# EUROPEAN PATENT SPECIFICATION

(11) **EP 3 814 332 B1**
(45) Date of publication and mention of the grant of the patent: **07.06.2023**
(21) Application number: 19736627.1
(22) Date of filing: 27.06.2019
(51) Int. Cl.: C07D 401/14, A01N 43/647, C07D 401/04, C07D 403/04

(54) **PESTICIDALLY ACTIVE AZOLE-AMIDE COMPOUNDS**
PESTIZIDE AZOL-AMIDWIRKSTOFFE
COMPOSÉS D'AMIDE D'AZOLE À ACTIVITÉ PESTICIDE

(30) Priority: 29.06.2018 EP 18180705; 01.02.2019 EP 19155123
(43) Date of publication of application: 05.05.2021
(73) Proprietor: SYNGENTA PARTICIPATIONS AG, 4058 Basel (CH)
(72) Inventor: GAGNEPAIN, Julien, Daniel, Henri, 4332 Stein (CH); EDMUNDS, Andrew, 4332 Stein (CH); EMERY, Daniel, 4332 Stein (CH); HALL, Roger, Graham, 4332 Stein (CH); HUETER, Ottmar, Franz, 4332 Stein (CH); KOLLETH KRIEGER, Amandine, 4332 Stein (CH); RENDLER, Sebastian, 4332 Stein (CH); SCHAETZER, Jürgen, Harry, 4332 Stein (CH)
(74) Representative: SYNGENTA IP
(86) International application number: PCT/EP2019/067266
(87) International publication number: WO 2020/002563

(56) References cited:
- WO-A1-2012/080376
- WO-A1-2017/192385

## Description

The present invention relates to pesticidally active, in particular insecticidally active azole-amide compounds, , to processes for their preparation, to compositions comprising those compounds, and to their use for controlling animal pests, including arthropods and in particular insects or representatives of the order *Acarina.*

WO2017192385 describes certain heteroaryl-1,2,4-triazole and heteroaryl-tetrazole compounds for use for controlling ectoparasites in animals (such as a mammal and a non-mammal animal). There have now been found novel pesticidally active azole-amide compounds.

The present invention accordingly relates, in a first aspect, to a compound of formula I, 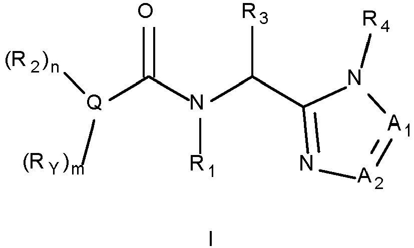 wherein:
A₁ is N, A₂ is CRs and R₅ is H, methyl, or (CH₂CH₂O)₂CH-;
R₁ is cyclopropyl-CH₂-, CH≡CCH₂-, H, or methyl;
Q is phenyl, pyridine, pyrimidine, pyrazine or pyridazine, wherein the phenyl, pyridine, pyrimidine, pyrazine or pyridazine is substituted by R₂ where n is 1 or 2, and, independent of the type of ring, is optionally substituted with R_{Y}, where m can be 0, 1 or 2;
R₂ is C₃-C₆cycloalkyl, phenyl or heteroaryl, each of which, independent of each other, is optionally substituted with one to three substituents independently selected from R_{x;} OR₆; piperidin-2-one-1-yl optionally substituted with one to two substituents independently selected from Rₓ; pyridin-2-one-1-yl optionally substituted with one to two substituents independently selected from Rₓ; azetidin-1-yl optionally substituted with one to two substituents independently selected from Rₓ; pyrrolidin-1-yl optionally substituted with one to two substituents independently selected from Rₓ; C₃-C₆cycloalkylC₁-C₄alkyl optionally substituted with one to two substituents independently selected from Rₓ; C₃-C₆cycloalkylC₁-C₃alkoxy optionally substituted with one to two substituents independently selected from Rₓ; C₁-C₅cyanoalkyl; C₁-C₅cyanoalkoxy; C₁-C₄alkylsulfonyl optionally substituted with one to two substituents independently selected from R_{x;} or C₁-C₄alkylsulfinyl optionally substituted with one to two substituents independently selected from Rₓ;
R_{Y} is selected from C₁-C₃alkyl, C₁-C₃haloalkyl, C₁-C₃haloalkylthio, C₁-C₃alkoxy, C₁-C₃haloalkoxy, halo, NOz, SF₅, CN, C(O)NH₂, C(O)OH and C(S)NH₂;
R₃ is methyl;
R₄ is 2-pyridine or 2-pyrimidine, each optionally substituted with C₁-C₃alkoxy or halogen;
R₆ is phenyl, benzyl, heteroaryl, or C₃-C₆ cycloalkyl, each of which, independent of each other, is optionally substituted with one to three substituents independently selected from Rₓ; and Rₓ is independently selected from halogen, C₁-C₃alkyl, C₁-C₃haloalkyl, C₁-C₃alkoxy, C₁-C₃haloalkoxy, NOz, SF₅, CN, CONH₂, C(S)NH₂, C₁-C₄haloalkylsulfanyl, C₁-C₄haloalkylsulfinyl, C₁-C₄haloalkylsulfonyl, C₁-C₄alkylsulfanyl, C₁-C₄alkylsulfinyl and C₁-C₄alkylsulfonyl;
or agrochemically acceptable salts, stereoisomers, enantiomers, tautomers and N-oxides of the compounds of formula I.

In the following, the invention as defined in the claims will be described in more detail. Although the description has been brought into conformity with the claims as far as possible, it is emphasized that the invention and the scope of protection are determined solely by the appended claims.

Compounds of formula I which have at least one basic centre can form, for example, acid addition salts, for example with strong inorganic acids such as mineral acids, for example perchloric acid, sulfuric acid, nitric acid, nitrous acid, a phosphorus acid or a hydrohalic acid, with strong organic carboxylic acids, such as C₁-C₄alkanecarboxylic acids which are unsubstituted or substituted, for example by halogen, for example acetic acid, such as saturated or unsaturated dicarboxylic acids, for example oxalic acid, malonic acid, succinic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids, such as C₁-C₄alkane- or arylsulfonic acids which are unsubstituted or substituted, for example by halogen, for example methane- or p-toluenesulfonic acid. Compounds of formula I which have at least one acidic group can form, for example, salts with bases, for example mineral salts such as alkali metal or alkaline earth metal salts, for example sodium, potassium or magnesium salts, or salts with ammonia or an organic amine, such as morpholine, piperidine, pyrrolidine, a mono-, di- or tri-lower-alkylamine, for example ethyl-, diethyl-, triethyl- or dimethylpropylamine, or a mono-, di- or trihydroxy-lower-alkylamine, for example mono-, di- or triethanolamine.

In each case, the compounds of formula (I) according to the invention are in free form, in oxidized form as a N-oxide or in salt form, e.g. an agronomically usable salt form.

N-oxides are oxidized forms of tertiary amines or oxidized forms of nitrogen containing heteroaromatic compounds. They are described for instance in the book "Heterocyclic N-oxides" by A. Albini and S. Pietra, CRC Press, Boca Raton 1991.

The compounds of formula I according to the invention also include hydrates which may be formed during the salt formation.

The term "C₁-Cₙalkyl" as used herein refers to a saturated straight-chain or branched hydrocarbon radical attached via any of the carbon atoms having 1 to n carbon atoms, for example, any one of the radicals methyl, ethyl, n-propyl, 1-methylbutyl, 2-methylbutyl, 3-methylbutyl, 2, 2-dimethylpropyl, 1-ethylpropyl, n-hexyl, n-pentyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, or 1-ethyl-2-methylpropyl.

The term "C₁-Cₙhaloalkyl" as used herein refers to a straight-chain or branched saturated alkyl radical attached via any of the carbon atoms having 1 to n carbon atoms (as mentioned above), where some or all of the hydrogen atoms in these radicals may be replaced by fluorine, chlorine, bromine and/or iodine, i.e., for example, any one of chloromethyl, dichloromethyl, trichloromethyl, fluoromethyl, difluoromethyl, trifluoromethyl, chlorofluoromethyl, dichlorofluoromethyl, chlorodifluoromethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3-pentafluoropropyl, heptafluoropropyl, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl. According a term "C₁-C₂fluoroalkyl" would refer to a C₁-C₂alkyl radical which carries 1, 2, 3, 4, or 5 fluorine atoms, for example, any one of difluoromethyl, trifluoromethyl, 1-fluoroethyl, 2-fluoroethyl, 2,2-difluoroethyl, 2,2,2-trifluoroethyl, 1,1,2,2-tetrafluoroethyl or pentafluoroethyl.

The term "C₁-Cₙalkoxy" as used herein refers to a straight-chain or branched saturated alkyl radical having 1 to n carbon atoms (as mentioned above) which is attached via an oxygen atom, i.e., for example, any one of the radicals methoxy, ethoxy, n-propoxy, 1-methylethoxy, n-butoxy, 1-methylpropoxy, 2-methylpropoxy or 1,1-dimethylethoxy. The term "haloC₁-Cₙalkoxy" as used herein refers to a C₁-Cₙalkoxy radical where one or more hydrogen atoms on the alkyl radical is replaced by the same or different halo atom(s) - examples include trifluoromethoxy, 2-fluoroethoxy, 3-fluoropropoxy, 3,3,3-trifluoropropoxy, 4-chlorobutoxy.

The term "C₁-Cₙcyanoalkyl" as used herein refers to a straight chain or branched saturated C₁-Cₙalkyl radical having 1 to n carbon atoms (as mentioned above), where one of the hydrogen atoms in these radicals is be replaced by a cyano group: for example, cyanomethyl, 2-cyanoethyl, 2-cyanopropyl, 3-cyanopropyl, 1-(cyanomethyl)-2-ethyl, 1-(methyl)-2-cyanoethyl, 4-cyanobutyl, and the like.

The term "C₁-Cₙcyanoalkoxy" as used herein refers to a straight chain or branched saturated C₁-Cₙalkoxy radical having 1 to n carbon atoms (as mentioned above), where one of the hydrogen atoms in these radicals is be replaced by a cyano group: for example, cyanomethoxy, 2-cyanoetoxy, 2-cyanopropoxy, 3-cyanoprooxyl, 1-(cyanomethyl)-2-ethyoxy, 1-(methyl)-2-cyanoetoxy, 4-cyanobutoxy, and the like.

The term "C₃-Cₙcycloalkyl" as used herein refers to 3 to n membered cycloalkyl groups such as cyclopropane, cyclobutane, cyclopropane, cyclopentane and cyclohexane.

The term "C₃-Cₙcycloalkyl-C₁-Cₙalkyl-" as used herein refers to 3 to n membered cycloalkyl group with a 1 to n carbon alkylene group, such as methylene or ethylene group, which alkylene group is connected to the rest of the molecule. In the instance, the C₃-Cₙcycloalkyl-C₁-Cₙalkyl- group is substituted, the substituent(s) can be on the cycloalkyl group and/or the C₁-Cₙalkyl- group.

The term "C₃-Cₙcycloalkyl-C₁-Cₙalkoxy" as used herein refers to 3 to n membered cycloalkyl group with a 1 to n carbon alkoxy group, which alkoxy group is connected to the rest of the molecule. In the instance, the C₃-Cₙcycloalkyl-C₁-Cnakoxy group is substituted, the substituent(s) is on the cycloalkyl group.

The term "aminocarbonylC₁-Cₙalkyl" as used herein refers to an alkyl radical where one of the hydrogen atoms in the radical is replaced by CONH2 group.

The term "hydroxycarbonylC₁-Cₙalkyl" as used herein refers to an alkyl radical where one of the hydrogen atoms in the radical is replaced by COOH group.

The term "C₁-Cₙnitroalkyl" as used herein refers to an alkyl radical where one of the hydrogen atoms in the radical is replaced by NO2 group.

The term "C₁-Cₙhaloalkylthio" as used herein refers to a C₁-C₃haloalkyl moiety linked through a sulfur atom.

The term "trimethylsilaneC₁-Cₙalkyl" as used herein refers to an alkyl radical where one of the hydrogen atoms in the radical is replaced by a -Si(CH₃)₃ group.

The term "C₂-Cₙalkenyl" as used herein refers to a straight or branched alkenyl chain having form two to n carbon atoms and one or two double bonds, for example, ethenyl, prop-1-enyl, but-2-enyl.

The term "C₂-Cₙhaloalkenyl" as used herein refers to a C₂-Cₙalkenyl moiety substituted with one or more halo atoms which may be the same or different.

The term "C₂-Cₙalkynyl" as used herein refers to a straight or branched alkynyl chain having from two to n carbon atoms and one triple bond, for example, ethynyl, prop-2-ynyl, but-3-ynyl,

The term "C₂-Cₙhaloalkynyl" as used herein refers to a C₂-Cₙalkynyl moiety substituted with one or more halo atoms which may be the same or different.

The term "C₁-Cₙalkylsulfinyl" as used herein refers to a straight chain or branched saturated alkyl radical having 1 to n carbon atoms which is attached via a sulfur atom and sulfur is in a partially oxidized form: -S(O)C₁-Cₙalkyl. i.e., for example, any one of methyS(O)-, ethylS(O)-, n-propylS(O)-, 1-methylethyS(O)-, butylS(O)-, 1-methylpropylS(O)-, 2-methylpropyIS(O)- and 1,1-dimethylethylS(O)-.

The term "C₁-Cₙalkylsulfonyl" as used herein refers to a straight chain or branched saturated alkyl radical having 1 to n carbon atoms which is attached via a sulfur atom and sulfur is in a fully oxidized form: -S(O)₂C₁-Cₙalkyl. i.e., for example, any one of methyS(O)₂-, ethylS(O)z-, n-propylS(O)z-, 1-methylethyS(O)₂-, butylS(O)z-, 1-methylpropylS(O)z-, 2-methylpropylS(O)z- and 1,1-dimethylethylS(O)z-.

The term "C₁-Cₙcyanoalkyl" as used herein refers to a straight chain or branched saturated C₁-Cₙalkyl radical having 1 to n carbon atoms (as mentioned above), where one of the hydrogen atoms in these radicals is be replaced by a cyano group: for example, cyanomethyl, 2-cyanoethyl, 2-cyanopropyl, 3-cyanopropyl, 1-(cyanomethyl)-2-ethyl, 1-(methyl)-2-cyanoethyl, 4-cyanobutyl, and the like.
Halogen is generally fluorine, chlorine, bromine or iodine. This also applies, correspondingly, to halogen in combination with other meanings, such as haloalkyl

The term "heteroaryl" as used herein refers to a 5- or 6-membered aromatic monocyclic ring having 1 to 3 heteroatoms independently selected from N, O and S. Examples are heteroaryls J1 to J30 shown in Scheme A below. Preferred heteroaryl preferred is pyridyl, pyrimidyl, and pyrazolyl.

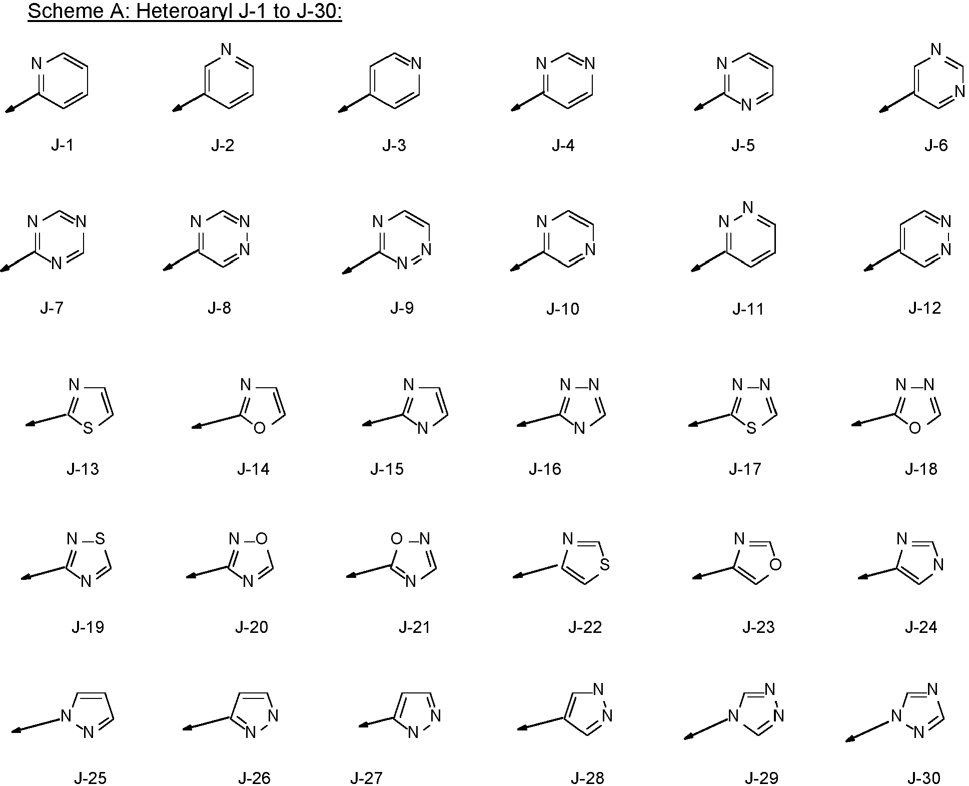

The pyridine, pyrimidine, pyrazine and pyridazine groups (unsubstituted or substituted) for Q and R₄ is connected via a carbon atom on the respective ring to the rest of the compound.

As used herein, the term "controlling" refers to reducing the number of pests, eliminating pests and/or preventing further pest damage such that damage to a plant or to a plant derived product is reduced.

As used herein, the term "pest" refers to insects, and molluscs that are found in agriculture, horticulture, forestry, the storage of products of vegetable origin (such as fruit, grain and timber); and those pests associated with the damage of man-made structures. The term pest encompasses all stages in the life cycle of the pest.

As used herein, the term "effective amount" refers to the amount of the compound, or a salt thereof, which, upon single or multiple applications provides the desired effect.

An effective amount is readily determined by the skilled person in the art, by the use of known techniques and by observing results obtained under analogous circumstances. In determining the effective amount a number of factors are considered including, but not limited to: the type of plant or derived product to be applied; the pest to be controlled & its lifecycle; the particular compound applied; the type of application; and other relevant circumstances.

As one of ordinary skill in the art will appreciate, compounds of Formula I contain a stereogenic centre which is indicated with an asterisk in the structure below: 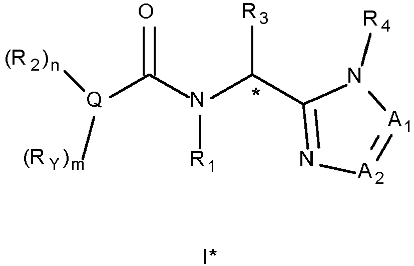 where R₁, Q, R_{Y}, R₂, R₃, R₄, A₁, A₂, m and n are as defined in the first aspect.

The present invention encompasses both racemates and individual enantiomers. Compounds having preferred stereochemistry are shown below.

Particularly preferred compounds of the present invention are compounds of formula I'a: 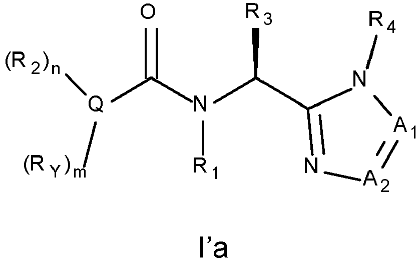 where R₁, Q, R_{Y}, R₂, R₃, R₄, A₁, A₂, m and n are as defined in the first aspect, and agrochemically acceptable salts, stereoisomers, enantiomers, tautomers and N-oxides of the compounds of formula (I'a).

The term "optionally substituted" as used herein means that the group referenced is either unsubstituted or is substituted by a designated substituent, for example, "pyridin-2-one-1-yl optionally substituted with Rₓ" means pyridin-2-one-1-yl and pyridin-2-one-1-yl substituted with Rₓ.

Embodiments according to the invention are provided as set out below.

In an embodiment of each aspect of the present invention, substituents R₂ and R_{Y} (if present) on Q are not attached to the carbon adjacent to the carbon bonded to the carbonyl (C=O) group-

In an embodiment of each aspect of the present invention, Q is
A. phenyl, pyridine or pyrimidine, wherein the phenyl, pyridine or pyrimidine is substituted by one or two R₂, and can be optionally further substituted, independent of the type of ring, with one or two R_{Y}; or
B. phenyl, or pyridine, wherein the phenyl, or pyridine is substituted by one or two R₂, and can be optionally further substituted, independent of the type of ring, with one or two R_{Y}; or
C. phenyl, or pyridine, wherein the phenyl, or pyridine is substituted by one or two R₂; or
D. phenyl wherein the phenyl is substituted by one or two R₂; or
E. pyridine wherein the pyridine is substituted by one or two R₂; or
F. phenyl, or pyridine, wherein the phenyl, or pyridine is substituted by one or two R₂, and can be optionally further substituted, independent of the type of ring, with one or two R_{Y}; or
G. phenyl, pyridine-2-yl, pyridine-4-yl, pyridine-3-yl or pyrimidin-5-yl, wherein the phenyl, pyridine-2-yl, pyridine-4-yl, pyridine-3-yl or pyrimidin-5-yl is substituted by one or two R₂, and can be optionally further substituted, independent of the type of ring, with one or two R_{Y}; or
H. phenyl, pyridine-2-yl, or pyridine-4-yl, wherein the phenyl, pyridine-2-yl, or pyridine-4-yl is substituted by one or two R₂, and can be optionally further substituted, independent of the type of ring, with one or two R_{Y}; or
I. phenyl, pyridine-2-yl, or pyridine-4-yl, wherein the phenyl, pyridine-2-yl, or pyridine-4-yl is substituted by one R₂, and can be optionally further substituted, independent of the type of ring, with one R_{Y}.

In an embodiment of each aspect of the present invention, R₂ is
A. C₃-C₄cycloalkyl, phenyl or heteroaryl selected from J-1 to J-30, each of C₃-C₄cycloalkyl, phenyl or heteroaryl, independent of each other, is optionally substituted with one to three substituents R_{x;} OR₆; piperidin-2-one-1-yl; pyridin-2-one-1-yl; azetidin-1-yl optionally substituted with Rₓ; pyrrolidin-1-yl; C₃-C₆cycloalkylC₁-C₄alkyl optionally substituted with Rₓ; C₃-C₆cycloalkylC₁-C₃alkoxy optionally substituted with Rₓ; C₁-C₅cyanoalkyl; C₁-C₅cyanoalkoxy; C₁-C₄alkylsulfonyl optionally substituted by Rₓ; or C₁-C₄alkylsulfinyl optionally substituted by Rₓ; or
B. C₃-C₄cycloalkyl, phenyl or heteroaryl selected from J-1 and J-25, each of C₃-C₄cycloalkyl, phenyl or heteroaryl, independent of each other, is optionally substituted with one to three substituents R_{x;} OR₆; piperidin-2-one-1-yl; pyridin-2-one-1-yl; azetidin-1-yl optionally substituted with Rₓ; pyrrolidin-1-yl; C₃-C₆cycloalkylC₁-C₄alkyl optionally substituted with Rₓ; C₃-C₆cycloalkylC₁-C₃alkoxy optionally substituted with Rₓ; C₁-C₅cyanoalkyl; C₁-C₅cyanoalkoxy; C₁-C₄alkylsulfonyl optionally substituted by Rₓ; or C₁-C₄alkylsulfinyl optionally substituted by Rₓ; or
C. C₃-C₄cycloalkyl, phenyl or pyrazolyl, each of which, independent of each other, is optionally substituted with one to three substituents R_{x;} OR₆; piperidin-2-one-1-yl; pyridin-2-one-1-yl; azetidin-1-yl optionally substituted with Rₓ; pyrrolidin-1-yl; C₃-C₆cycloalkylC₁-C₄alkyl optionally substituted with Rₓ; C₃-C₆cycloalkylC₁-C₃alkoxy optionally substituted with Rₓ; C₁-C₅cyanoalkyl; C₁-C₅cyanoalkoxy; C₁-C₄alkylsulfonyl optionally substituted by Rₓ; or C₁-C₄alkylsulfinyl optionally substituted by Rₓ; or
D. C₃-C₄cycloalkyl, phenyl or pyrazolyl, each of which, independent of each other, is optionally substituted with one to two substituents R_{x;} OR₆; azetidin-1-yl optionally substituted with Rₓ; C₃-C₆cycloalkylC₁-C₄alkyl optionally substituted with Rₓ; C₃-C₆cycloalkylC₁-C₃alkoxy optionally substituted with Rₓ; C₁-C₄alkylsulfonyl optionally substituted by Rₓ; or C₁-C₄alkylsulfinyl optionally substituted by Rₓ; or
E. C₃-C₄cycloalkyl or C₃-C₄cycloalkyl substituted with one to two substituents R_{x;} OR₆; C₃-C₆cycloalkylC₁-C₄alkyl, C₃-C₆cycloalkylC₁-C₄alkyl substituted with Rₓ; C₁-C₄alkylsulfonyl; ; C₁-C₄alkylsulfonyl substituted by R_{x;}; or C₁-C₄alkylsulfinyl; or C₁-C₄alkylsulfinyl substituted by Rₓ; or
F. C₃-C₄cycloalkyl or C₃-C₄cycloalkyl substituted with one to two halogen, C₁-C₃alkyl, C₁-C₃haloalkyl, C₃-C₄cycloalkylmethyl, C₃-C₄cycloalkylmethyl substituted with one to two halogen, C₁-C₃alkyl, C₁-C₃haloalkyl ; C₁-C₂alkylsulfonyl substituted with one to three halogen; or
G. cyclopropyl, cyclopropyl substituted with one to two halogen, methyl, trifluoromethyl, cyclopropylmethyl substituted with one to two halogen, trifluoromethyl, C₁₋ C₂alkylsulfonyl substituted with one to three halogen; or
H. cyclopropyl substituted with one to two fluorine, methyl, trifluoromethyl, cyano; cyclopropylmethyl substituted with one to two fluorine; trifluoromethylsulfonyl;
and in each embodiment A to H for R₂, n is 1 or 2, or n is 1.

In an embodiment of each aspect of the present invention, R₆ is
A. heteroaryl selected from J-1 to J-12, phenyl, benzyl, C₃-C₄ cycloalkyl, each of which, independent of each other, is optionally substituted with one substituent R_{x;}; or
B. C₃-C₄ cycloalkyl, or C₃-C₄ cycloalkyl substituted with one substituent R_{x;}

In an embodiment of each aspect of the present invention, R_{Y} is
A. C₁-C₃alkyl, C₁-C₃haloalkyl, C₁-C₃alkoxy, C₁-C₃haloalkoxy, halo, NOz, SF₅, and CN; or
B. C₁-C₃haloalkyl, C₁-C₃haloalkoxy and halo; or
C. C₁-C₃fluoroalkyl, C₁-C₃fluoroalkoxy, chlorine, and fluorine;
and in each embodiment A to C above, m is 0, 1 or 2; or m is 0 or 1; or m is 1.

In an embodiment of each aspect of the present invention, Rₓ is
A. independently selected from halogen, C₁-C₃alkyl, C₁-C₃haloalkyl, C₁-C₃alkoxy, C₁-C₃haloalkoxy, NOz, SF₅, CN, CONH₂, C(S)NH₂; or
B. independently selected from halogen, C₁-C₃haloalkyl, C₁-C₃haloalkoxy, and CN; or
C. independently selected from fluoro, C₁-C₃fluoroalkyl, C₁-C₃fluroroalkoxy, and CN.

In the present disclosure (items A to K listed below) and an embodiment of each aspect of the present invention (items L, M and N listed below), R₁ is
A. H; C₁-C₆alkyl optionally substituted with one substituent selected from: CN, CONH₂, COOH, NOz, and -Si(CH₃)₃; C₁-C₆haloalkyl; C₂-C₆alkenyl; C₂-C₆alkynyl; C₂-C₆haloalkynyl; C₃-C₄cycloalkyl- C₁-C₂alkyl - wherein the C₃-C₄cycloalkyl- is optionally substituted with 1 or 2 halo atoms; oxetan-3-yl-CH₂-; or benzyl optionally substituted with halo or C₁-C₃haloalkyl; or
B. H; C₁-C₆haloalkyl; C₁-C₆alkyl optionally substituted with CN or Si(CH₃)₃; C₃-C₆alkynyl; C₃-C₄cycloalkyl-C₁-C₂alkyl wherein the C₃-C₄cycloalkyl is optionally substituted with 1 or 2 halo atoms; oxetan-3-yl-CH₂-; or benzyl optionally substituted with halo; or
C. H, C₁-C₆alkyl, C₃-C₄cycloalkylC₁-C₂alkyl- wherein the C₃-C₄cycloalkyl is optionally substituted with 1 or 2 halo atoms, or oxetan-3-yl-CH₂-; or
D. H, C₁-C₆alkyl, or C₃-C₄cycloalkylC₁-C₂alkyl
E. H; C₁-C₆haloalkyl; C₁-C₆alkyl optionally substituted with CN or Si(CH₃)₃; C₃-C₆alkynyl; C₃-C₄cycloalkyl-C₁-C₂alkyl wherein the C₃-C₄cycloalkyl is optionally substituted with 1 or 2 halo atoms; or
F. H; C₁-C₆haloalkyl; C₁-C₆alkyl; C₃-C₆alkynyl; C₃-C₄cycloalkyl-C₁-C₂alkyl wherein the C₃-C₄cycloalkyl is optionally substituted with 1 or 2 halo atoms; or
G. cyclopropyl-CH₂-, n-propyl, CH≡CCH₂-, CF₃CH₂CH₂-, FCH₂CH₂-, FCH₂CH₂CH₂-, 2,2-difluorocyclopropyl-CH₂-, 2,2-dichlorocyclopropyl-CH₂-, H, CH₃, (CH₃)₃SiCH₂-, CH₃CH₂-, or CNCH₂-; or
H. cyclopropyl-CH₂-, n-propyl, CH≡CCH₂-, CF₃CH₂CH₂-, FCH₂CH₂-, FCH₂CH₂CH₂-, 2,2-difluorocyclopropyl-CH₂- or 2,2-dichlorocyclopropyl-CH₂-; or
I. cyclopropyl-CH₂-, n-propyl, CH≡CCH₂-, CF₃CH₂CH₂-, FCH₂CH₂-, FCH₂CH₂CH₂-, 2,2-difluorocyclopropyl-CH₂-, H, CH₃, (CH₃)₃SiCH₂-, or CH₃CH₂-; or
J. cyclopropyl-CH₂-, n-propyl, CH≡CCH₂-, CF₃CH₂CH₂-, FCH₂CH₂-, FCH₂CH₂CH₂-, 2,2-difluorocyclopropyl-CH₂-; or
K. cyclopropyl-CH₂-, n-propyl, CH≡CCH₂-, CF₃CH₂CH₂-, FCH₂CH₂-, or FCH₂CH₂CH₂-; or
L. cyclopropyl-CH₂-, CH≡CCH₂-, H or CH₃; or
M. CH=C-CH₂- or cyclopropyl-CH₂-; or
N. cyclopropyl-CH₂-.

In the present disclosure (item A listed below) and an embodiment of each aspect of the present invention (item B listed below), R₃ is
A. C₁-C₃alkyl or C₁-C₃haloalkyl; or
B. methyl.

In an embodiment of each aspect of the present invention, A₁ and A₂ is
A. A₁ is N and A₂ is CR₅ where R₅ is H, C₁-C₃alkyl, C₁-C₃haloaikyl, C₃-C₄cycloalkyl, C₁-C₃alkoxy, C₃-C₄alkoxyC(O)- or (C₁-C₃alkoxy)₂CH; or
B. A₁ is N and A₂ is CR₅ where R₅ is H, C₁-C₃alkyl, C₁-C₃alkoxyC(O)-, or (C₁-C₃alkoxy)₂CH-; or
C. A₁ is N and A₂ is CRs where R₅ is H, C₁-C₃alkyl, or (C₁-C₃alkoxy)₂CH-; or
D. A₁ is N and A₂ is CR₅ where R₅ is H or methyl; or
E. A₁ is N and A₂ is CR₅ where R₅ is H.

In the present disclosure (item A listed below) and an embodiment of each aspect of the invention (items B to E listed below), R₄ is
A. 2-pyridine, 2-pyrimidine, 2-pyrazine or 2-pyridazine, each optionally substituted with C₁-C₃alkoxy or halo;
B. 2-pyridine or 2-pyrimidine, each optionally substituted with C₁-C₃alkoxy or halo; or
C. selected from Y₁ to Y₃ 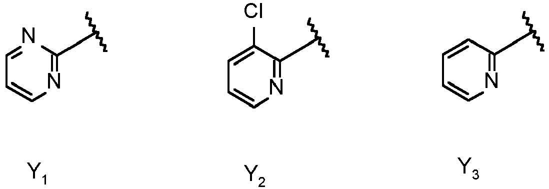
D. 2-pyridine (i.e Y₃), or 2-pyrimidine (i.e Y₁); or
E. 2-pyrimidine.

In the present disclosure (items A to E listed below) and an embodiment of each aspect of the invention (items F to H listed below), R₅ is
A. hydrogen, halogen, CN, C₁-C₃alkyl, C₁-C₃haloalkyl, C₃-C₄cycloalkyl, C₁-C₃alkoxy, or C₁-C₃haloalkoxy; or
B. hydrogen, halogen, C₁-C₃alkyl, C₁-C₃haloalkyl, C₃-C₄cycloalkyl, C₁-C₃alkoxy, or C₁-C₃haloalkoxy; or
C. hydrogen, C₁-C₃alkyl, C₁-C₃haloalkyl, C₁-C₃haloalkoxy, C₁-C₃alkoxy, or C₃-C₄cycloalkyl; or
D. hydrogen, halogen, C₁-C₃haloalkyl, or C₁-C₃alkoxy; or
E. hydrogen, C₁-C₃haloalkyl, or C₁-C₃alkoxy; or
F. hydrogen, methyl or (CH₂CH₂O)₂CH-; or
G. hydrogen or methyl;
H. hydrogen.

In an embodiment, the compound of formula I can be represented as 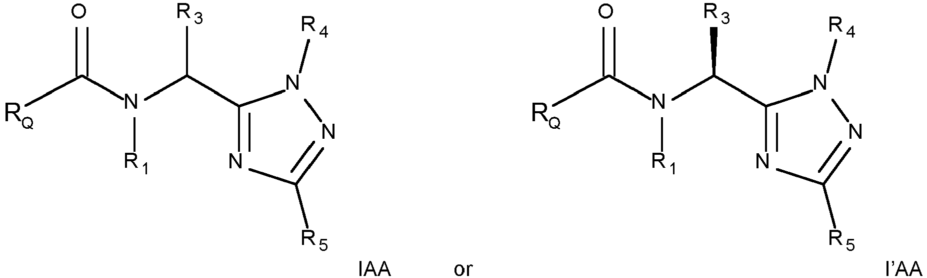 wherein R₁, R₃, R₄ and R₅ are as defined in the first aspect, R_{Q} corresponds to the group Q containing the one or two substituent R₂ and optionally one or two substituent R_{Y} as defined in the first aspect.

In an embodiment of each aspect of the invention, R_{Q} is selected from K-1 to K-8 (wherein the staggered line represents the point of connection/ attachment to the rest of the compound).

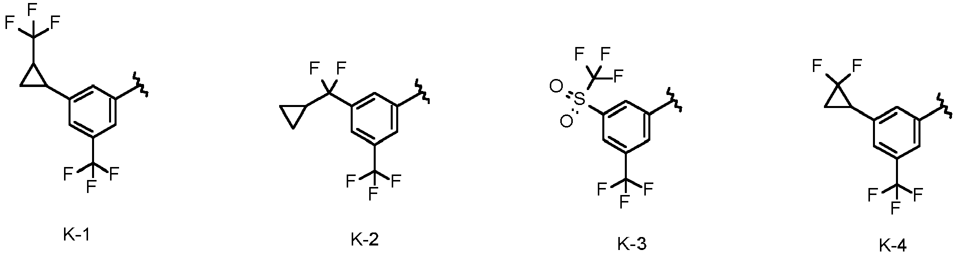

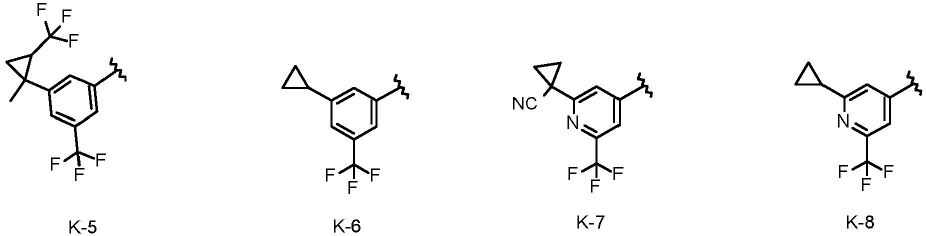

The present disclosure and invention, accordingly, makes available a compound of formula I or formula I'a having the substituents R₁, Q, R_{Y}, R₂, R₃, R₄, A₁ and A₂ as defined above in all combinations / each permutation. Accordingly, made available, for example, is a compound of formula I with R₁ being embodiment F (i.e. H; C₁-C₆haloalkyl; C₁-C₆alkyl; C₃-C₆alkynyl; C₃-C₄cycloalkyl-C₁-C₂alkyl wherein the C₃-C₄cycloalkyl is optionally substituted with 1 or 2 halo atoms), R₃ being embodiment B (i.e. methyl), R₄ being embodiment first aspect (i.e. pyridine, pyrimidine, pyrazine or pyridazine, wherein the pyridine, pyrimidine, pyrazine or pyridazine is optionally substituted with one substituent selected from: C₁-C₃alkyl, C₁-C₃haloalkyl, C₁-C₃alkoxy, C₃-C₄cycloalkyl, halogen or hydroxyl), A₁ and A₂ being embodiment D (i.e. A₁ is N and A₂ is CR₅ where R₅ is H or methyl); Q being an embodiment B ( i..e phenyl, or pyridine, wherein the phenyl, or pyridine is substituted by one or two R₂, and can be optionally further substituted, independent of the type of ring, with one or two R_{Y}), R₂ being an embodiment D (i.e. R₂ is C₃-C₄cycloalkyl, phenyl or pyrazolyl, each of which, independent of each other, is optionally substituted with one to two substituents R_{x;} OR₆; azetidin-1-yl optionally substituted with Rₓ; C₃-C₆cycloalkylC₁-C₄alkyl optionally substituted with Rₓ; C₃-C₆cycloalkylC₁-C₃alkoxy optionally substituted with Rₓ; C₁-C₄alkylsulfonyl optionally substituted by R_{x;} or C₁-C₄alkylsulfinyl optionally substituted by Rₓ; and n is 1 or 2), R₆ being an embodiment A (i.e. heteroaryl selected from J-1 to J-12, phenyl, benzyl, C₃-C₄ cycloalkyl, each of which, independent of each other, is optionally substituted with one substituent Rₓ), Rₓ being an embodiment B (i.e. independently selected from halogen, C₁-C₃haloalkyl, C₁-C₃haloalkoxy, and CN), and Rv being an embodiment of the first aspect (i.e C₁-C₃alkyl, C₁-C₃haloalkyl, C₁-C₃haloalkylthio, C₁-C₃alkoxy, C₁-C₃haloalkoxy, halo, NOz, SF₅, CN, C(O)NH₂, C(O)OH and C(S)NH₂; and m is 0, 1 or 2).

The present disclosure and invention also make available compound of formula I or formula I'a has R₁ as hydrogen, C₁-C₆alkyl, C₂-C₆alkynyl, C₃-C₄cycloalkyl-C₁-C₂alkyl-; R₃ as C₁-C₃alkyl; R₄ as phenyl, pyridine, or pyrimidine, wherein the phenyl, pyridine, or pyrimidine is optionally substituted, independent of the ring, with C₁-C₆alkyl, C₁-C₆haloalkyl, C₃-C₆cycloalkyl, and halo; Q as phenyl or pyridine, wherein the phenyl or pyridine is substituted, independent of the type of ring, by one or two R₂, and, can be further optionally substituted, independent of the type of ring, by one or two R_{Y}; R₂ as C₃-C₄cycloalkyl, phenyl or heteroaryl selected from J-1 to J-30, each of C₃-C₄cycloalkyl, phenyl or heteroaryl, independent of each other, is optionally substituted with one to three substituents R_{x;} OR₆; piperidin-2-one-1-yl; pyridin-2-one-1-yl; azetidin-1-yl optionally substituted with Rₓ; pyrrolidin-1-yl; C₃-C₆cycloalkylC₁-C₄alkyl optionally substituted with Rₓ; C₃-C₆cycloalkylC₁-C₃alkoxy optionally substituted with Rₓ; C₁-C₅cyanoalkyl; C₁-C₅cyanoalkoxy; C₁-C₄alkylsulfonyl optionally substituted by R_{x;} or C₁-C₄alkylsulfinyl optionally substituted by Rₓ; R₆ as heteroaryl selected from J-1 to J-12, phenyl, benzyl, C₃-C₄ cycloalkyl, each of which, independent of each other, is optionally substituted with one substituent Rₓ; Rₓ as independently selected from halogen, C₁-C₃haloalkyl, C₁-C₃haloalkoxy, and CN; R_{Y} as C₁-C₃haloalkyl, and halo; A₁ as N; A₂ as CRs (with R⁵ is H).

The present disclosure and invention also make available compound of formula I or formula I'a has R₁ as hydrogen, C₁-C₃alkyl, C₂-C₃alkynyl, C₃-C₄cycloalkyl-C₁-C₂alkyl-; R₃ as C₁-C₃alkyl; R₄ as phenyl, pyridine, or pyrimidine; Q as phenyl or pyridine, wherein the phenyl or pyridine is substituted, independent of the type of ring, by one or two R₂, and, can be further optionally substituted, independent of the type of ring, by one or two, independently selected from halogen and trifluoromethyl; R₂ as C₃-C₄cycloalkyl, phenyl or heteroaryl selected from J-1 to J-30, each of C₃-C₄cycloalkyl, phenyl or heteroaryl, independent of each other, is optionally substituted with one to three substituents, independently selected from halogen, trifluoromethyl, difluoromethoxy and CN_{;} OR₆; piperidin-2-one-1-yl; pyridin-2-one-1-yl; azetidin-1-yl optionally substituted with halogen and trifluoromethyl; C₁-C₅cyanoalkyl; C₃-C₆cycloalkylC₁-C₄alkyl optionally substituted with halogen, trifluoromethyl, difluoromethoxy and CN; C₃-C₆cycloalkylC₁-C₃alkoxy optionally substituted with halogen, trifluoromethyl, difluoromethoxy and CN; C₁-C₅cyanoalkyl; C₁-C₅cyanoalkoxy; C₁-C₄alkylsulfonyl optionally substituted by halogen, and trifluoromethyl; or C₁-C₄alkylsulfinyl optionally substituted by halogen, and trifluoromethyl; R₆ as heteroaryl selected from J-1 to J-12, phenyl, benzyl, C₃-C₄ cycloalkyl, each of which, independent of each other, is optionally substituted with one substituent halogen and trifluoromethyl; A₁ as N; and A₂ as CRs (with R₅ is H).

The present disclosure and invention also make available compound of formula I or formula I'a has R₁ as hydrogen, C₁-C₃alkyl, C₂-C₃alkynyl, C₃-C₄cycloalkyl-C₁-C₂alkyl-; R₃ as C₁-C₃alkyl; R₄ as phenyl, pyridine, or pyrimidine; Q as phenyl or pyridine, wherein the phenyl or pyridine is substituted, independent of the type of ring, by one or two R₂, and, independent of the type of ring, by one or two, independently selected from halogen and trifluoromethyl; R₂ as C₃-C₄cycloalkyl, phenyl or heteroaryl selected from J-1 and J-25, each of C₃-C₄cycloalkyl, phenyl or heteroaryl, independent of each other, is optionally substituted with one to three substituents R_{x;} OR₆; piperidin-2-one-1-yl; pyridin-2-one-1-yl; azetidin-1-yl optionally substituted with Rₓ; pyrrolidin-1-yl; C₃-C₆cycloalkylC₁-C₄alkyl optionally substituted with Rₓ; C₃-C₆cycloalkylC₁-C₃alkoxy optionally substituted with Rₓ; C₁-C₅cyanoalkyl; C₁-Cscyanoalkoxy; C₁-C₄alkylsulfonyl optionally substituted by R_{x;} or C₁-C₄alkylsulfinyl optionally substituted by Rₓ; R₆ as heteroaryl selected from J-1 to J-12, phenyl, benzyl, C₃-C₄ cycloalkyl, each of which, independent of each other, is optionally substituted with one substituent Rₓ; Rₓ as independently selected from halogen, C₁-C₃haloalkyl, C₁-C₃haloalkoxy, and CN; A₁ as N; and A₂ as CRs (with R₅ is H).

The present disclosure and invention also make available compound of formula I or formula I'a has R₁ as hydrogen, C₁-C₃alkyl, C₂-C₃alkynyl, C₃-C₄cycloalkyl-C₁-C₂alkyl-; R₃ as C₁-C₃alkyl; R₄ as phenyl, pyridine, or pyrimidine; Q as phenyl or pyridine, wherein the phenyl or pyridine is substituted by one R₂, and, independent of the type of ring, by one from halogen or trifluoromethyl; R₂ as C₃-C₄cycloalkyl, or phenyl, each of C₃-C₄cycloalkyl, or phenyl, independent of each other, is optionally substituted with one to three substituents R_{x;} OR₆; C₃-C₆cycloalkylC₁-C₄alkyl optionally substituted with Rₓ; C₃-C₆cycloalkylC₁-C₃alkoxy optionally substituted with Rₓ; C₁-C₅cyanoalkyl; C₁-C₅cyanoalkoxy; C₁-C₄alkylsulfonyl optionally substituted by R_{x;} or C₁-C₄alkylsulfinyl optionally substituted by Rₓ; R₆ as heteroaryl selected from J-1 to J-12, phenyl, benzyl, C₃-C₄ cycloalkyl, each of which, independent of each other, is optionally substituted with one substituent Rₓ; Rₓ as independently selected from halogen, C₁-C₃haloalkyl, C₁-C₃haloalkoxy, and CN;; A₁ as N; and A₂ as CRs (with R₅ is H).

The present disclosure and invention also make available compound of formula I or formula I'a has R₁ as hydrogen, C₁-C₃alkyl, C₂-C₃alkynyl, C₃-C₄cycloalkyl-C₁-C₂alkyl-; R₃ as C₁-C₃alkyl; R₄ as phenyl, pyridine, or pyrimidine; Q as phenyl or pyridine, wherein the phenyl or pyridine is substituted by one R₂, and by one trifluoromethyl group; R₂ as C₃-C₄cycloalkyl, optionally substituted with one to three substituents R_{x;}; C₃-C₆cycloalkylC₁-C₄alkyl optionally substituted with Rₓ; C₁-C₅cyanoalkyl; C₁-C₄alkylsulfonyl optionally substituted by R_{x;} or C₁-C₄alkylsulfinyl optionally substituted by Rₓ; Rₓ as independently selected from halogen, C₁-C₃haloalkyl, C₁-C₃haloalkoxy, and CN; A₁ as N; and A₂ as CRs (with R₅ is H).

The present disclosure and invention also make available compound of formula IAA or I'AA has R₁ as hydrogen, C₁-C₃alkyl, C₂-C₃alkynyl, C₃-C₄cycloalkyl-C₁-C₂alkyl-; R₃ as C₁-C₃alkyl; R₄ as phenyl, pyridine, or pyrimidine; A₁ as N; A₂ as CRs (with R₅ is H); and R_{Q} as selected from K-1 to K-8.

In a second aspect, the present invention makes available a composition comprising a compound as defined in the first aspect, one or more auxiliaries and diluent, and optionally one more other active ingredient.

In a third aspect, the present invention makes available a method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound as defined in the first aspect or a composition as defined in the second aspect. In an embodiment, the method excludes a method for treatment of the human or animal body by surgery or therapy and diagnostic methods practised on the human or animal body.

In a fourth aspect, the present invention makes available a method for the protection of plant propagation material from the attack by insects, acarines, nematodes or molluscs, which comprises treating the propagation material or the site, where the propagation material is planted, with an effective amount of a compound as defined in the first aspect or a composition as defined in the second aspect.

In a fifth aspect, the present invention makes available a plant propagation material, such as a seed, comprising, or treated with or adhered thereto, a compound as defined in the first aspect or a composition as defined in the second aspect.

The present invention in a further aspect provides a method of controlling parasites in or on an animal in need thereof comprising administering an effective amount of a compound of the first aspect. The present invention further provides a method of controlling ectoparasites on an animal in need thereof comprising administering an effective amount of a compound of the first aspect. The present invention further provides a method for preventing and/or treating diseases transmitted by ectoparasites comprising administering an effective amount of a compound of the first aspect, to an animal in need thereof.

In a further aspect, the present invention provides a compound as defined in the first aspect or a composition as defined in the second aspect
- for use in controlling parasites in or on an animal in need thereof; or
- for use in controlling ectoparasites on an animal in need thereof; or
- for preventing and/or treating diseases transmitted by ectoparasites.

Compounds of formula I can be prepared by those skilled in the art following methods known. More specifically compounds of formulae I, and I'a, and intermediates therefore can be prepared as described below in the Schemes and examples. Certain stereogenic centers have been left unspecified for the clarity and are not intended to limit the teaching of the Schemes in any way.

The processes for preparing compounds of formula I can be carried out by methods known to those skilled in the art. Compounds of formula I 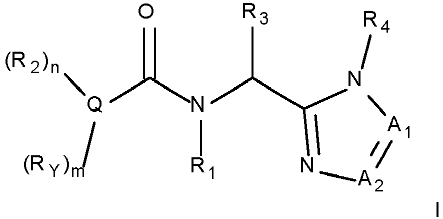 can be prepared by reaction of an amine of formula II 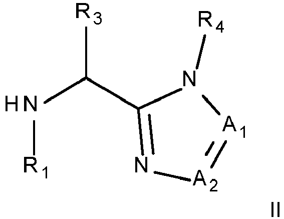 wherein R₁, R₃, R₄, A₁, and A₂ are as defined for compound of formula I, with a carboxylic acid derivative of formula III 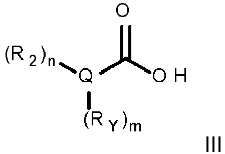 wherein Q, R₂, R_{Y}, m and n are as defined for compound of formula I. The chemistry is described in more detail in Scheme 1.

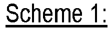

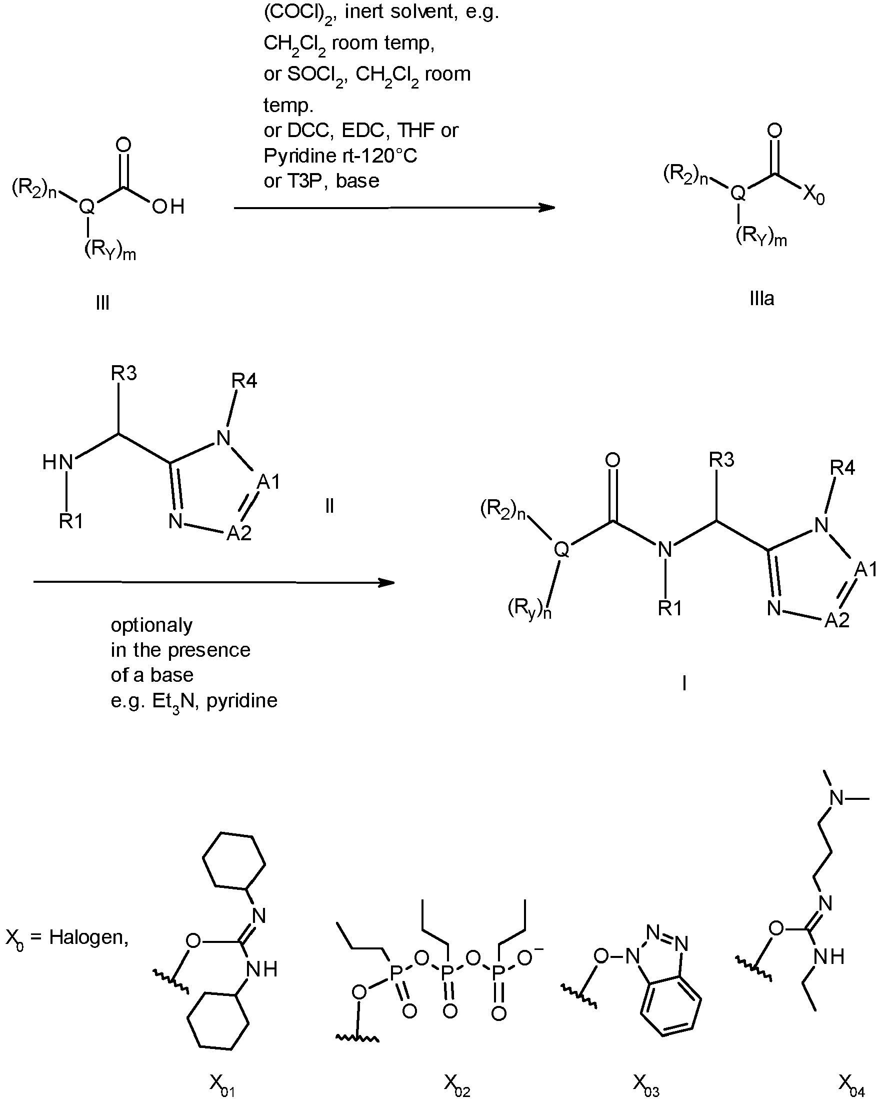

In Scheme 1, compounds of formula III wherein R₂, R_{Y}, m and n are defined in formula I, are activated to compounds of formula IIIa by methods known to those skilled in the art and described for example in Tetrahedron, 61 (46) , 10827-10852, 2005*.* For example, compounds where X₀ is halogen are formed by treatment of compounds of formula III with for example, oxalyl chloride orthionyl chloride in the presence of catalytic quantities of DMF in inert solvents such as methylene dichloride or THF at temperatures between 20°C to 100°C, preferably 25°C. Treatment of IIIa with compounds of formula II wherein R₁, R₃, R₄, A₁, and A₂ are defined as above , optionally in the presence of a base, e.g. triethylamine or pyridine leads to compounds of formula I. Alternatively, compounds of formula I can be prepared by treatment of compounds of formula III with dicyclohexyl carbodiimide (DCC), 1-Ethyl-3-(3-dimethylaminopropyl)carbodiimide (EDC) or 1-[Bis(dimethylamino)methylene]-1H-1,2,3-triazolo[4,5-b]pyridinium 3-oxide hexafluorophosphate (HATU) to give the activated species IIIa, wherein Xo is X₀₁, X₀₂ and X₀₃ respectively, in an inert solvent, e.g. pyridine, DMF, acetonitrile, CH₂Cl₂ or THF optionally in the presence of a base, e.g. triethylamine, at temperatures between 50-180 °C. Finally, an acid of the formula III can also be activated by reaction with a coupling reagent such as propanephosphonic acid anhydride (T3P^{®}) to provide compounds of formula IIIa wherein Xo is X₀₄ as described for example in Synthesis 2013, 45, 1569*.* Further reaction with an amine of the formula II provides compounds of formula I.

Processes for making compounds of formula Ila, wherein R₁, R₃ and R₄ are defined in formula I, are generally known or can be easily prepared by those skilled in the art. A typical example of such a synthesis is shown in Scheme 2.

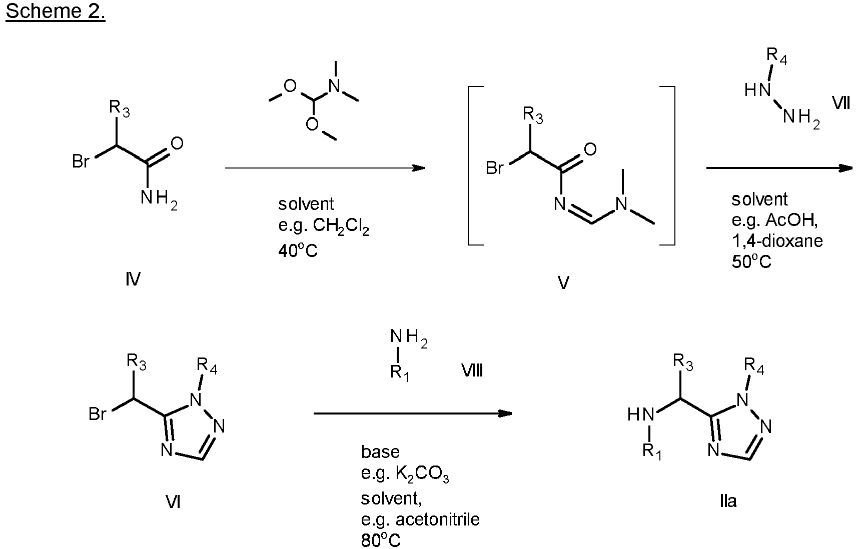

For example, compounds of formula Ila may be prepared by reaction between compounds of formula VI, wherein R₃ and R₄ are as defined in formula I, and compounds of formula VIII, wherein R₁ is defined in formula I, in suitable solvents that may include, for example, acetonitrile or dioxane, in the presence of a suitable base, such as sodium, potassium or cesium carbonate (or sodium or potassium hydrogene carbonate), usually upon heating at temperatures between room temperature and 200°C, preferably between 40 to the boiling point of the reaction mixture, optionally under microwave heating conditions.

Compounds of formula VI wherein R₃ and R₄ are as defined in formula I, may be prepared by reaction between compounds of formula V, wherein R₄ is as defined in formula I, and compounds of formula VII, wherein R₄ is defined in formula I, in suitable solvents that may include, for example, mixture of acetic acid and 1,4-dioxane, usually upon heating at temperatures between room temperature and 200°C, preferably between 40 to the boiling point of the reaction mixture, optionally under microwave heating conditions. Such processes have been described previously, for example, in Tetrahedron 2017, 73, 750.

Compounds of formula V wherein R₃ is defined as above, may be prepared by reaction between compounds of formula IV, wherein R₃ is as defined in formula I, and N,N-dimethylformamide dimethyl acetal (DMF-DMA), in suitable solvents that may include, for example, dichloromethane, usually upon heating at temperatures between room temperature and 200°C, preferably between 40 to the boiling point of the reaction mixture. Such processes have been described previously, for example, in Tetrahedron 2017, 73, 750.

Processes for making compounds of formula la, wherein Q, R₁, R₂, R₃, R₄, R_{Y}, m and n are as defined in formula I, are generally known or can be easily prepared by those skilled in the art. A typical example of such a synthesis is shown in Scheme 3.

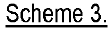

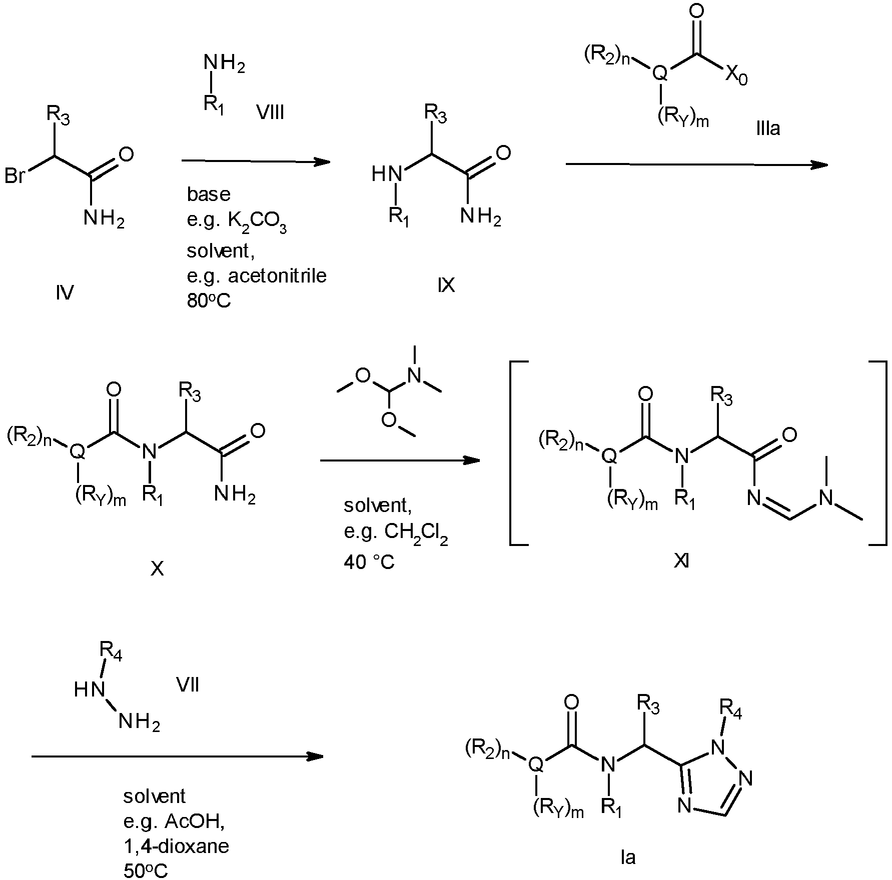

For example, compounds of formula la, wherein Q, R₁, R₂, R₃, R₄, R_{Y}, m and n are defined as for formula I, may be prepared by reaction between compounds of formula XI, wherein Q, R₁, R₂, R₃, R_{Y}, m and n are defined for formula I, and compounds of formula VII, wherein R₄ is defined in formula I, in suitable solvents that may include, for example, mixture of acetic acid and 1,4-dioxane, usually upon heating at temperatures between room temperature and 200°C, preferably between 40°C to the boiling point of the reaction mixture, optionally under microwave heating conditions. Such processes have been described previously, for example, in Tetrahedron 2017, 73, 750.

Compounds of formula XI, wherein Q, R₁, R₂, R₃, R_{Y}, m and n are defined as for formula I, may be prepared by reaction between compounds of formula X, wherein Q, R₁, R₂, R₃, R_{Y}, m and n are defined as for formula I, and N,N-dimethylformamide dimethyl acetal (DMF-DMA), in suitable solvents that may include, for example, dichloromethane, usually upon heating at temperatures between room temperature and 200°C, preferably between 40°C to the boiling point of the reaction mixture. Such processes have been described previously, for example, in Tetrahedron 2017, 73, 750, and US2016296501, preparation 7, page 29.

Compounds of formula X, wherein Q, R₁, R₂, R₃, R_{Y}, m and n are defined as for formula I, may be prepared by reaction between compounds of formula IX, wherein R₁ and R₃ are defined as for formula I, and compounds of formula IIIa, wherein R₂, R_{Y} and Xo are defined as in Scheme 1, in suitable inert solvents that may include, for example, pyridine, DMF, acetonitrile, CH₂Cl₂ or THF, optionally in the presence of a base, e.g. triethylamine or pyridine, usually upon heating at temperatures between room temperature and 200°C.

Compounds of formula IX, wherein R₁ and R₃ are defined as for formula I, may be prepared by reaction between compounds of formula IV, wherein R₃ is as defined in formula I, and compounds of formula VIII, wherein R₁ is defined in formula I, in suitable solvents that may include, for example, acetonitrile or dioxane, in the presence of a suitable base, such as sodium, potassium or cesium carbonate (or sodium or potassium hydrogene carbonate), usually upon heating at temperatures between room temperature and 200°C, preferably between 40°C to the boiling point of the reaction mixture, optionally under microwave heating conditions.

The desired enantiomer of formula I'a, wherein Q, R₁, R₂, R₃, R₄, R_{Y}, m and n are defined as for formula I, may be prepared by the same sequence depicted in Scheme 3 involving enantiopure compounds IV' or IX' which are commercially available (see scheme 3').

Alternatively, the desired enantiomer of formula I'a, wherein Q, R₁, R₂, R₃, R₄, R_{Y}, m and n are defined as for formula I, may be obtained by chiral chromatography of compound la.

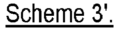

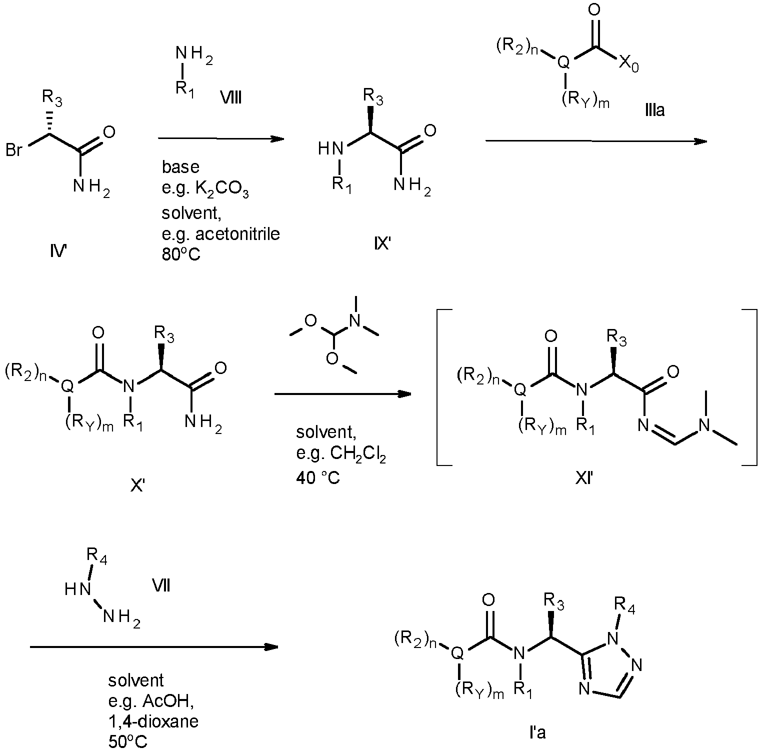

Compounds of formula Ib, wherein Q, R₁, R₂, R₃, R₄, R₅, R_{Y}, m and n are defined as for formula I, are generally known or can be easily prepared by those skilled in the art. A typical example of such a synthesis is shown in Scheme 4.

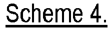

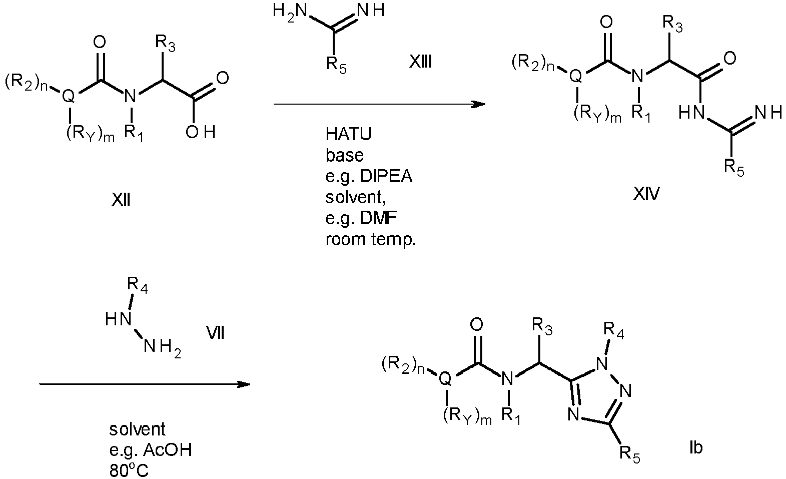

Compounds of formula Ib, wherein Q, R₁, R₂, R₃, R₄, R₅, R_{Y}, m and n are defined as for formula I, may be prepared by reaction between compounds of formula XIV, wherein Q, R₁, R₂, R₃, R₅, R_{Y}, m and n are defined as for formula I, and compounds of formula VII, wherein R₄ is defined in formula I, in suitable solvents that may include, for example, acetic acid, usually upon heating at temperatures between room temperature and 200°C, preferably between 40°C to the boiling point of the reaction mixture, optionally under microwave heating conditions. Such processes have been described previously, for example, in J. Org. Chem. 2011, 76, 1177.

Compounds of formula XIV, wherein, Q, R₁, R₂, R₃,, R₅, R_{Y}, m and n are defined as for formula I, may be prepared by reaction between compounds of formula XII, wherein Q, R₁, R₂, R₃, R_{Y}, m and n are defined as for formula I, and compounds of formula XIII, wherein, R₅ is defined as above, in the presence of a coupling agent, that may include, for example, HATU, in suitable solvents that may include, for example, DMF, usually upon heating at temperatures between room temperature and 200°C, preferably between 20°C to the boiling point of the reaction mixture, optionally under microwave heating conditions. Such processes have been described previously, for example, in J. Org. Chem. 2011, 76, 1177.

Alternatively, the desired enantiomer of formula I'b, wherein Q, R₁, R₂, R₃, R₄, R_{Y}, m and n are defined as for formula I, may be obtained by chiral chromatography of compound lb.

Compounds of formula Ic, wherein R₁, R₂, R₃, Rₓ, R_{Y}, A₁ and A₂ are defined as above are generally known or can be easily prepared by those skilled in the art. A typical example of such a synthesis is shown in Scheme 5.

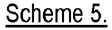

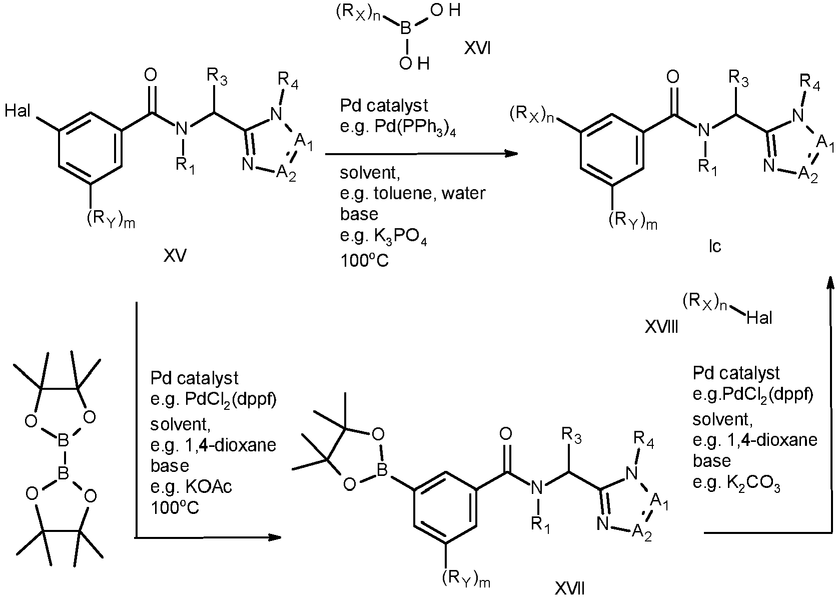

For example, compounds of formula Ic, wherein R₁, R₃, R₄, Rₓ, R_{Y}, A₁, and A₂ are defined as above, may be prepared by reaction between compounds of formula XV wherein, R₁, R₃, R₄, R_{Y}, A₁ and A₂ are defined as above, and compounds of formula XVI, wherein Rₓ is as defined above, and wherein Hal is a halogen such as, for example, bromine or iodine, in the presence of a palladium catalyst, for example, Pd(PPh₃)₄, in suitable solvents that may include, for example, toluene/water, 1,4-dioxane/water, in the presence of a suitable base, such as sodium, potassium or cesium carbonate or tripotassium phosphate usually upon heating at temperatures between room temperature and 200°C, preferably between 20°C to the boiling point of the reaction mixture, optionally under microwave heating conditions. Such processes have been described previously, for example, in Tetrahedron Letters 2002, 43, 6987-6990.

Compounds of formula Ic, wherein R₁, R₃, R₄, Rₓ, R_{Y}, A₁, and A₂ are defined as above, may also be prepared by reaction between compounds of formula XVII wherein, R₁, R₃, R₄, R_{Y}, A₁, and A₂ are defined as above, and compounds of formula XVIII, wherein Rₓ is as defined above, and wherein Hal is a halogen such as, for example, bromine or iodine, in the presence of a palladium catalyst, for example, PdCl₂(dppf), in suitable solvents that may include, for example, toluene/water, 1,4-dioxane/water, in the presence of a suitable base, such as sodium, potassium or cesium carbonate or tripotassium phosphate usually upon heating at temperatures between room temperature and 200°C, preferably between 20°C to the boiling point of the reaction mixture, optionally under microwave heating conditions. Such processes have been described previously, for example, in WO12139775 (see page 73).

Compounds of formula XVII, wherein, R₁, R₃, R₄, R_{Y}, A₁, and A₂ are defined as above, may be prepared by reaction of compounds of formula XV wherein, R₁, R₃, R₄, R_{Y}, A₁, and A₂ are defined as above and wherein Hal is a halogen such as, for example, bromine or iodine, with Bis(pinacolato)diboron (B₂pin₂), in the presence of a palladium catalyst, for example, PdCl₂(dppf), in suitable solvents that may include, for example, toluene/water, 1,4-dioxane/water, in the presence of a suitable base, such as sodium, potassium or cesium carbonate or potassium acetate, usually upon heating at temperatures between room temperature and 200°C, preferably between 20°C to the boiling point of the reaction mixture, optionally under microwave heating conditions. Such processes have been described previously, for example, in Bioorg. Med. Chem. Lett. 2015, 25, 1730, and WO12139775 (see preparative example 42, step 2, page 67).

Compounds of formula Id, wherein R₁, R₃, R₄, A₁, and A₂ are defined as above are generally known or can be easily prepared by those skilled in the art. A typical example of such a synthesis is shown in Scheme 6.

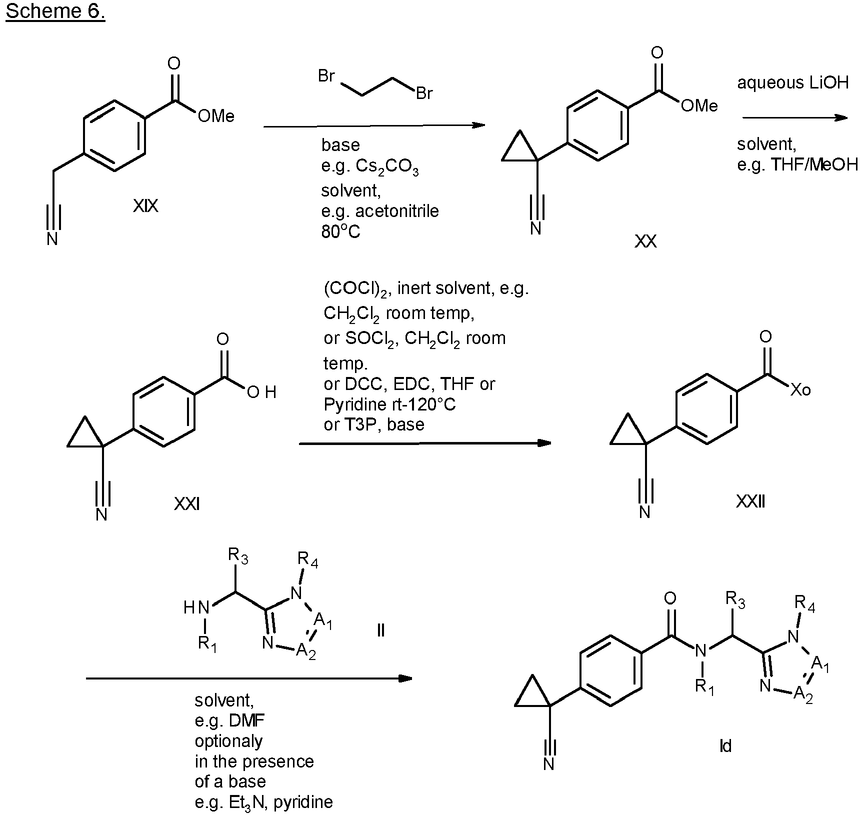

Compounds of formula Id, wherein R₁, R₃, R₄, A₁, and A₂ are defined as above, may be prepared, as described in Scheme 1, by reaction between compounds of formula XXII, wherein, X₀ is defined as in Scheme 1, and compounds of formula II, wherein R₁, R₃, R₄, A₁, and A₂ are defined in formula I.

Compounds of formula XXII, wherein, Xo is defined as in Scheme 1, may be prepared from compound of formula XXI, as described in Scheme 1 for the preparation of IIIa.

Compound of formula XXI, may be prepared from compound of formula XX, by treatment with aqueous LiOH, in suitable solvents that may include, for example, THF/MeOH mixture, usually upon heating at temperatures between room temperature and 200°C, preferably between 20°C to the boiling point of the reaction mixture, optionally under microwave heating conditions.

Compound of formula XX, may be prepared from compound of formula XIX, which is commercially available, by reaction, for example, with 1,2-dibromoethane, in suitable solvents that may include, for example, acetonitrile, in the presence of a suitable base, such as sodium, potassium or cesium carbonate, usually upon heating at temperatures between room temperature and 200°C, preferably between 20°C to the boiling point of the reaction mixture, optionally under microwave heating conditions.

Compounds of formula le, wherein R₁, R₃, R₄, A₁, and A₂ are defined as above are generally known or can be easily prepared by those skilled in the art. A typical example of such a synthesis is shown in Scheme 7.

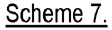

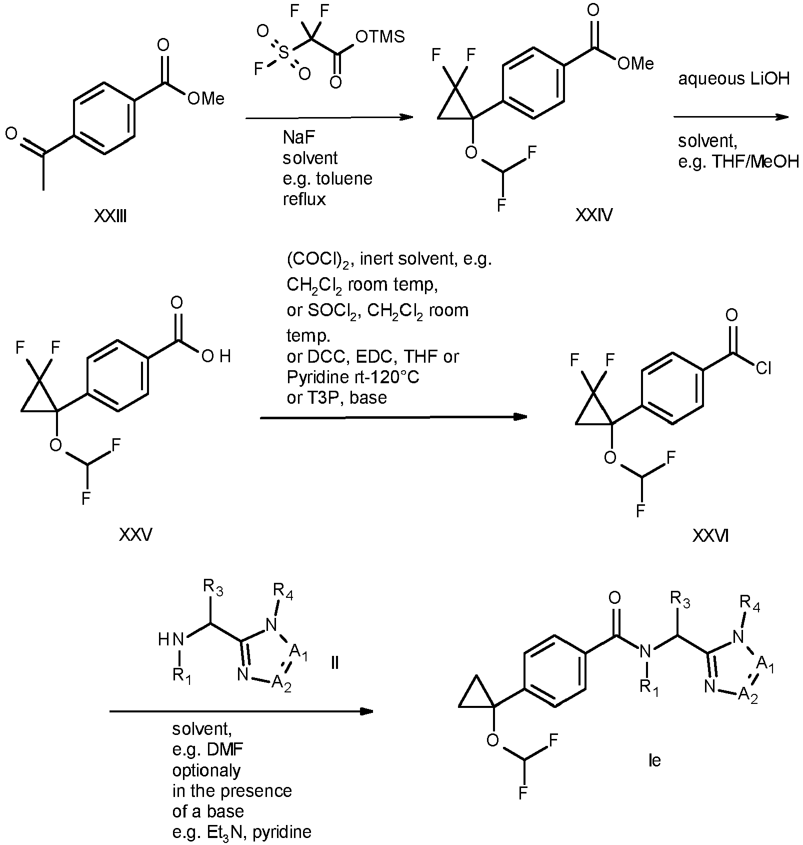

Compounds of formula le, wherein R₁, R₃, R₄, A₁, and A₂ are defined as above, may be prepared, as described in Scheme 1, by reaction between compounds of formula XXVI, wherein, Xo is defined as in Scheme 1, and compounds of formula II, wherein R₁, R₃, R₄, A₁, and A₂ are defined in formula I.

Compounds of formula XXVI, wherein, Xo is defined as in Scheme 1, may be prepared from compound of formula XXV, as described in Scheme 1 for the preparation of Illa.

Compound of formula XXV, may be prepared from compound of formula XXIV, by treatment with, for example aqueous LiOH, NaOH or KOH, in suitable solvents that may include, for example, THF/MeOH mixture, usually upon heating at temperatures between room temperature and 200°C, preferably between 20°C to the boiling point of the reaction mixture, optionally under microwave heating conditions.

Compound of formula XXIV, may be prepared from compound of formula XXIII, which is commercially available, by reaction, for example, with trimethylsilyl fluorosulfonyldifluoroacetate (TFDA), in suitable solvents that may include, for example, toluene, in the presence of a suitable base, such as sodium, potassium or cesium fluoride, usually upon heating at temperatures between room temperature and 200°C, preferably between 20°C to the boiling point of the reaction mixture, optionally under microwave heating conditions. Such processes have been described previously, for example, in J. Org. Chem. 2004, 69, 4210.

Compounds of formula If, wherein R₁, R₃, R₄, R_{Y}, R_{X}, A₁, and A₂ are defined as above, may be prepared as described in Scheme 1, e.g. by reaction of compounds of formula XXX, wherein Xo is defined as in Scheme 1, and compounds of formula II, wherein R₁, R₃, R₄, A₁, and A₂ are defined in formula I (Scheme 8).

Compounds of formula XXX, wherein, Xo is defined as in Scheme 1, may be prepared from compound of formula XXIX, as already described in Scheme 1 for the preparation of IIIa.

Compound of formula XXIX, may be prepared from compound of formula XXVIII analog as outlined in Scheme 7., by treatment with, for example aqueous LiOH, NaOH or KOH, in suitable solvents that may include, for example, THF/MeOH mixture, usually upon heating at temperatures between room temperature and 100°C, preferably between 20°C to the boiling point of the reaction mixture (Scheme 8).

Finally, compounds of formula XXVIII wherein R_{Y} is defined as above, may be prepared by treatment of compounds of formula XXVII, which are either commercially available or can be prepared by methods known to those skilled in the art (see e.g. Angew. Chem. Int. Ed. 2004, 43, 1132 and Pure Appl. Chem. 1985, 57, 1771) with (trifluoroethyl)-diphenyl-sulfonium triflate (Ph₂S⁺CH₂CF₃ ⁻OTf) in the presence of an Fe-catalyst and a base, preferable CsF at temperatures between 0 to 50 °, preferable 20 °C in DMA as solvent (analog to Org. Lett. 2016, 18, 2471) (Scheme 8).

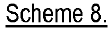

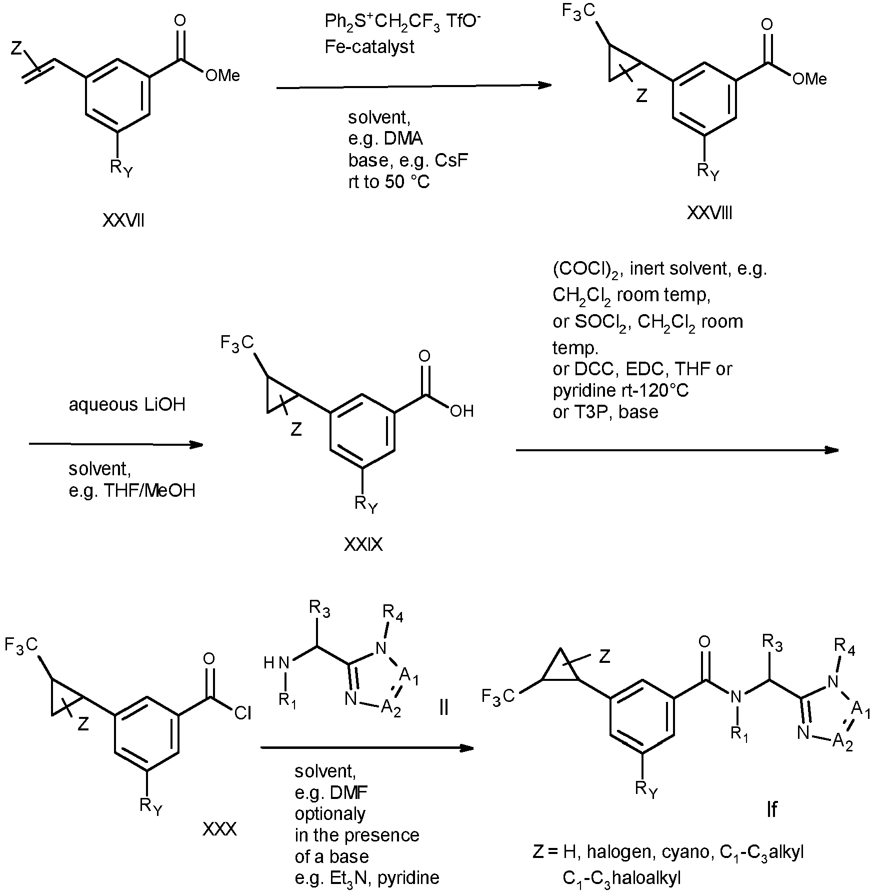

Compounds of formula Ig, wherein R₁, R₃, R₄, R_{Y}, A₁, and A₂ are defined as above and Z is H, halogen, cyano, C₁-C₃alkyl and C₁-C₃haloalkyl, and may be prepared in a very similar manner as shown in Scheme 9.

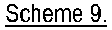

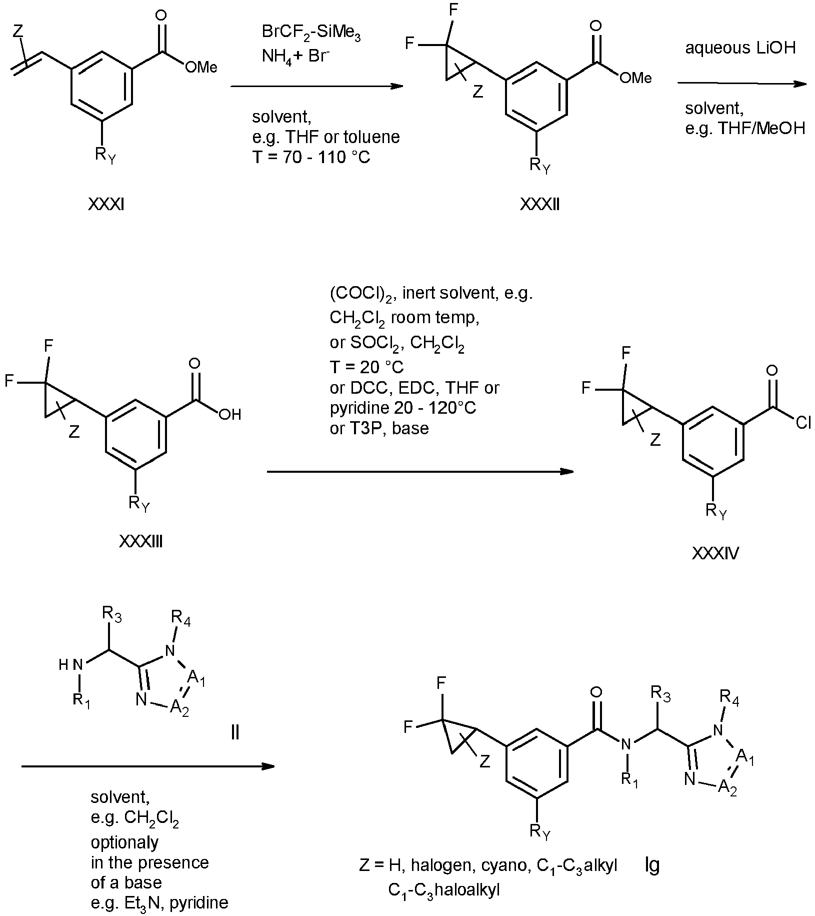

Thus, compounds of formula XXXII, wherein R_{Y} and Z is H, halogen, cyano, C₁-C₃alkyl and C₁-C₃haloalkyl, is defined as above, are prepared by reaction of compounds of formula XXXI (synthesized analog to ACS Med. Chem. Lett. 2013, 4, 514 or Tetrahedron Lett. 2001, 42, 4083) with (bromodifluoromethyl)-trimethylsilane in the presence of NH₄⁺Br⁻ in a suitable solvent, preferable in THF or toluene at temperatures between 70 to 110 °C. Subsequent saponification of the methyl ester, activation of the carboxylic acid and final amide coupling gives compounds of formula Ig as illustrated in Scheme 9.

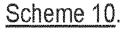

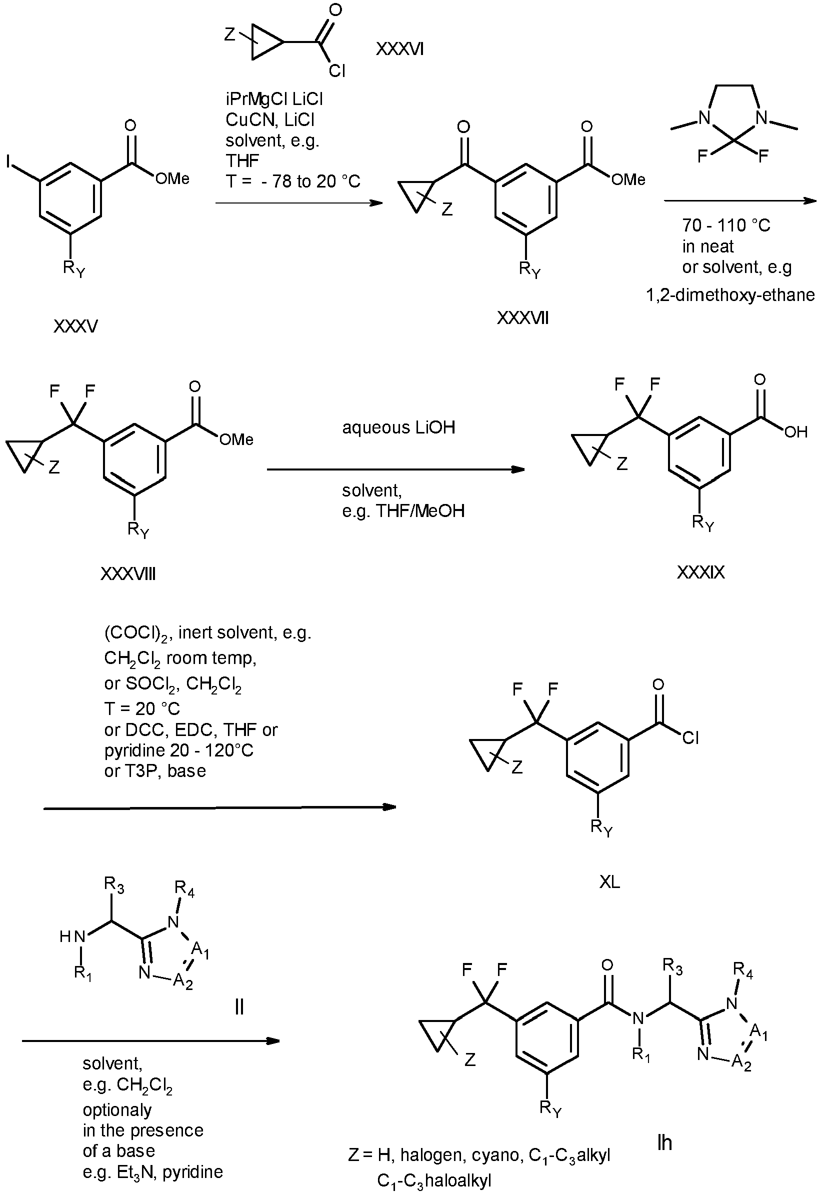

Carboxylic acids of formula XXXIX wherein Z is H, halogen, cyano, C₁-C₃alkyl and C₁-C₃haloalkyl and R_{y} is defined as above can be prepared according to reaction Scheme 10. Thus, compounds of formula XXXV wherein R_{Y} is defined as above are treated with iPrMgCl/LiCI-complex; subsequent reaction with CuCN and quenching with cyclopropane carbonyl chlorides of formula XXXVI wherein Rₓ is defined as in formula I provides compounds of formula XXXVII (analog to WO2006/067445*, page 148*). Following fluorination with 2,2-difluoro-1,3-dimethylimidazoline either in a solvent, e.g. in 1,2-dimethoxy-ethane or in neat (see Chem. Commun. 2002, (15), 1618) affords compound of formula XXXVIII. Hydrolysis with LiOH as already described gives carboxylic acids of formula XXXIX. Subsequent activation of the carboxylic acid of formula XXXIX and final amide coupling with amines of formula II delivers compounds of formula Ih as illustrated in Scheme 10.

Depending on the procedure or the reaction conditions, the reactants can be reacted in the presence of a base. Examples of suitable bases are alkali metal or alkaline earth metal hydroxides, alkali metal or alkaline earth metal hydrides, alkali metal or alkaline earth metal amides, alkali metal or alkaline earth metal alkoxides, alkali metal or alkaline earth metal acetates, alkali metal or alkaline earth metal carbonates, alkali metal or alkaline earth metal dialkylamides or alkali metal or alkaline earth metal alkylsilylamides, alkylamines, alkylenediamines, free or N-alkylated saturated or unsaturated cycloalkylamines, basic heterocycles, ammonium hydroxides and carbocyclic amines. Examples which may be mentioned are sodium hydroxide, sodium hydride, sodium amide, sodium methoxide, sodium acetate, sodium carbonate, potassium tert-butoxide, potassium hydroxide, potassium carbonate, potassium hydride, lithium diisopropylamide, potassium bis(trimethylsilyl)amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide and 1,8-diazabicyclo[5.4.0]undec-7-ene (DBU).

The reactants can be reacted with each other as such, i.e. without adding a solvent or diluent. In most cases, however, it is advantageous to add an inert solvent or diluent or a mixture of these. If the reaction is carried out in the presence of a base, bases which are employed in excess, such as triethylamine, pyridine, N-methylmorpholine or N,N-diethylaniline, may also act as solvents or diluents.

The reactions are advantageously carried out in a temperature range from approximately - 80°C to approximately +140°C, preferably from approximately -30°C to approximately +100°C, in many cases in the range between ambient temperature and approximately +80°C.

Depending on the choice of the reaction conditions and starting materials which are suitable in each case, it is possible, for example, in one reaction step only to replace one substituent by another substituent according to the invention, or a plurality of substituents can be replaced by other substituents according to the invention in the same reaction step.

Salts of compounds of formula I can be prepared in a manner known *per se.* Thus, for example, acid addition salts of compounds of formula I are obtained by treatment with a suitable acid or a suitable ion exchanger reagent and salts with bases are obtained by treatment with a suitable base or with a suitable ion exchanger reagent.

Salts of compounds of formula I can be converted in the customary manner into the free compounds I, acid addition salts, for example, by treatment with a suitable basic compound or with a suitable ion exchanger reagent and salts with bases, for example, by treatment with a suitable acid or with a suitable ion exchanger reagent.

Salts of compounds of formula I can be converted in a manner known *per se* into other salts of compounds of formula I, acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds of formula I, which have salt-forming properties can be obtained in free form or in the form of salts.

The compounds of formula I and, where appropriate, the tautomers thereof, in each case in free form or in salt form, can be present in the form of one of the isomers which are possible or as a mixture of these, for example in the form of pure isomers, such as antipodes and/or diastereomers, or as isomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule; the invention relates to the pure isomers and also to all isomer mixtures which are possible and is to be understood in each case in this sense hereinabove and hereinbelow, even when stereochemical details are not mentioned specifically in each case.

Diastereomer mixtures or racemate mixtures of compounds of formula I, in free form or in salt form, which can be obtained depending on which starting materials and procedures have been chosen can be separated in a known manner into the pure diasteromers or racemates on the basis of the physicochemical differences of the components, for example by fractional crystallization, distillation and/or chromatography.

Enantiomer mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl celulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained according to the invention not only by separating suitable isomer mixtures, but also by generally known methods of diastereoselective or enantioselective synthesis, for example by carrying out the process according to the invention with starting materials of a suitable stereochemistry.

N-oxides can be prepared by reacting a compound of the formula I with a suitable oxidizing agent, for example the H₂O₂/urea adduct in the presence of an acid anhydride, e.g. trifluoroacetic anhydride. Such oxidations are known from the literature, for example from J. Med. Chem., 32 (12), 2561-73, 1989 or WO 2000/15615.

It is advantageous to isolate or synthesize in each case the biologically more effective isomer, for example enantiomer or diastereomer, or isomer mixture, for example enantiomer mixture or diastereomer mixture, if the individual components have a different biological activity.

The compounds of formula I and, where appropriate, the tautomers thereof, in each case in free form or in salt form, can, if appropriate, also be obtained in the form of hydrates and/or include other solvents, for example those which may have been used for the crystallization of compounds which are present in solid form.

The compounds of formula I according to the following the Tables A-1 to A-64 can be prepared according to the methods described above. The examples which follow are intended to illustrate the invention and show preferred compounds of formula I.

The compounds according to the following Tables A-1 to A-64 below can be prepared according to the methods described above. The examples which follow are intended to illustrate the invention and show preferred compounds of formula I, in the form of a compound of formula IAA.

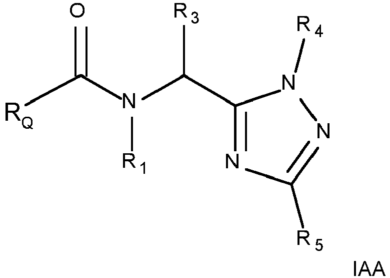

**Table X: Substituent definitions of R₁, R₃, R₄, A₁ and A₂ in compound of formula IAA**

| **Index** | **R₁** | **R₃** | **R₄** | **A₁** | **A₂** |
|---|---|---|---|---|---|
| 1 | H | Me | 2-pyrimidyl | N | CH |
| 2 | Me | Me | 2-pyrimidyl | N | CH |
| 3 | Et | Me | 2-pyrimidyl | N | CH |
| 4 | propargyl | Me | 2-pyrimidyl | N | CH |
| 5 | 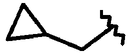 | Me | 2-pyrimidyl | N | CH |
| 6 | H | Me | 2-pyridyl | N | CH |
| 7 | Me | Me | 2-pyridyl | N | CH |
| 8 | Et | Me | 2-pyridyl | N | CH |
| 9 | propargyl | Me | 2-pyridyl | N | CH |
| 10 | 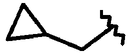 | Me | 2-pyridyl | N | CH |
| 11 | H | Me | 2- pyrazinyl | N | CH |
| 12 | Me | Me | 2- pyrazinyl | N | CH |
| 13 | Et | Me | 2- pyrazinyl | N | CH |
| 14 | propargyl | Me | 2- pyrazinyl | N | CH |
| 15 | 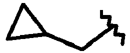 | Me | 2- pyrazinyl | N | CH |
| 16 | H | Me | 3- pyridazinyl | N | CH |
| 17 | Me | Me | 3- pyridazinyl | N | CH |
| 18 | Et | Me | 3- pyridazinyl | N | CH |
| 19 | propargyl | Me | 3- pyridazinyl | N | CH |
| 20 | 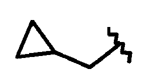 | Me | 3- pyridazinyl | N | CH |
| 21 | H | Me | 2-pyrimidyl | N | N |
| 22 | Me | Me | 2-pyrimidyl | N | N |
| 23 | Et | Me | 2-pyrimidyl | N | N |
| 24 | propargyl | Me | 2-pyrimidyl | N | N |
| 25 | 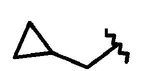 | Me | 2-pyrimidyl | N | N |
| 26 | H | Me | 2-pyridyl | N | N |
| 27 | Me | Me | 2-pyridyl | N | N |
| 28 | Et | Me | 2-pyridyl | N | N |
| 29 | propargyl | Me | 2-pyridyl | N | N |
| 30 | 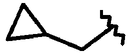 | Me | 2-pyridyl | N | N |
| 31 | H | Me | 2- pyrazinyl | N | N |
| 32 | Me | Me | 2- pyrazinyl | N | N |
| 33 | Et | Me | 2- pyrazinyl | N | N |
| 34 | propargyl | Me | 2- pyrazinyl | N | N |
| 35 | 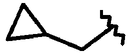 | Me | 2- pyrazinyl | N | N |
| 36 | H | Me | 3- pyridazinyl | N | N |
| 37 | Me | Me | 3- pyridazinyl | N | N |
| 38 | Et | Me | 3- pyridazinyl | N | N |
| 39 | propargyl | Me | 3- pyridazinyl | N | N |
| 40 | 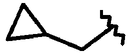 | Me | 3- pyridazinyl | N | N |
| 41 | H | Me | 5-bromopyrimidin-2-yl | N | CH |
| 42 | H | Me | 5-bromo-2-pyridyl | N | CH |
| 43 | H | Me | 5-bromopyrimidin-2-yl | N | CMe |
| 44 | H | Me | 5-bromo-2-pyridyl | N | CMe |
| 45 | H | Me | 2-pyrimidyl | N | CMe |
| 46 | H | Me | 2-pyridyl | N | CMe |

Table A-1 provides 46 compounds A-1.001 to A-1.046 of formula IAA wherein R₁, R₃, R₄, A₁ and A₂ are as defined in table X and R_{Q} is 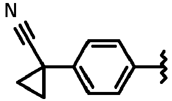 wherein the staggered line indicates the point of attachment of R_{Q} to the rest of the molecule, and in which the variables R₁, R₃, R₄, A₁ and A₂ have the specific meaning given in the corresponding line of Table X. For example, compound 1.005 has the following structure: 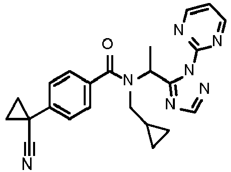
Table A-2 provides 46 compounds A-2.001 to A-2.046 of formula IAA wherein R₁, R₃, R₄, A₁ and A₂ are as defined in table X and R_{Q} is 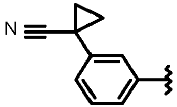
Table A-3 provides 46 compounds A-3.001 to A-3.046 of formula IAA wherein R₁, R₃, R₄, A₁ and A₂ are as defined in table X and R_{Q} is 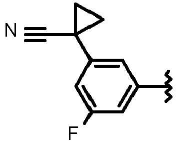
Table A-4 provides 46 compounds A-4.001 to A-4.046 of formula IAA wherein R₁, R₃, R₄, A₁ and A₂ are as defined in table X and R_{Q} is 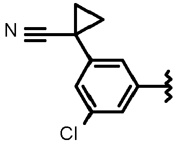
Table A-5 provides 46 compounds A-5.001 to A-5.046 of formula IAA wherein R₁, R₃, R₄, A₁ and A₂ are as defined in table X and R_{Q} is 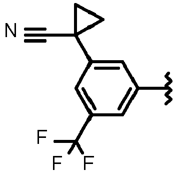
Table A-6 provides 46 compounds A-6.001 to A-6.046 of formula IAA wherein R₁, R₃, R₄, A₁ and A₂ are as defined in table X and R_{Q} is 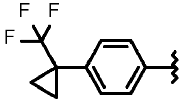
Table A-7 provides 46 compounds A-7.001 to A-7.046 of formula IAA wherein R₁, R₃, R₄, A₁ and A₂ are as defined in table X and R_{Q} is 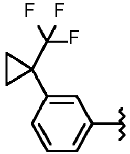
Table A-8 provides 46 compounds A-8.001 to A-8.046 of formula IAA wherein R₁, R₃, R₄, A₁ and A₂ are as defined in table X and R_{Q} is 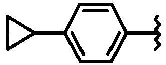
Table A-9 provides 46 compounds A-9.001 to A-9.046 of formula IAA wherein R₁, R₃, R₄, A₁ and A₂ are as defined in table X and R_{Q} is 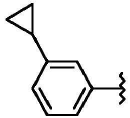
Table A-10 provides 46 compounds A-10.001 to A-10.046 of formula IAA wherein R₁, R₃, R₄, A₁ and A₂ are as defined in table X and R_{Q} is 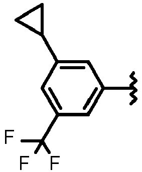
Table A-11 provides 46 compounds A-11.001 to A-11.046 of formula IAA wherein R₁, R₃, R₄, A₁ and A₂ are as defined in table X and R_{Q} is 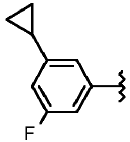
Table A-12 provides 46 compounds A-12.001 to A-12.046 of formula IAA wherein R₁, R₃, R₄, A₁ and A₂ are as defined in table X and R_{Q} is 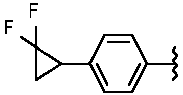
Table A-13 provides 46 compounds A-13.001 to A-13.046 of formula IAA wherein R₁, R₃, R₄, A₁ and A₂ are as defined in table X and R_{Q} is 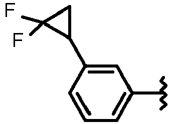
Table A-14 provides 46 compounds A-14.001 to A-14.046 of formula IAA wherein R₁, R₃, R₄, A₁ and A₂ are as defined in table X and R_{Q} is 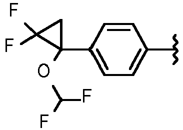
Table A-15 provides 46 compounds A-15.001 to A-15.046 of formula IAA wherein R₁, R₃, R₄, A₁ and A₂ are as defined in table X and R_{Q} is 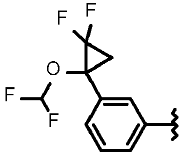
Table A-16 provides 46 compounds A-16.001 to A-16.046 of formula IAA wherein R₁, R₃, R₄, A₁ and A₂ are as defined in table X and R_{Q} is 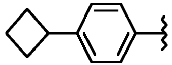
Table A-17 provides 46 compounds A-17.001 to A-17.046 of formula IAA wherein R₁, R₃, R₄, A₁ and A₂ are as defined in table X and R_{Q} is 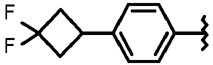
Table A-18 provides 46 compounds A-18.001 to A-18.046 of formula IAA wherein R₁, R₃, R₄, A₁ and A₂ are as defined in table X and R_{Q} is 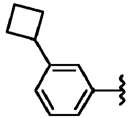
Table A-19 provides 46 compounds A-19.001 to A-19.046 of formula IAA wherein R₁, R₃, R₄, A₁ and A₂ are as defined in table X and R_{Q} is 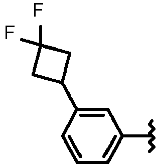
Table A-20 provides 46 compounds A-20.001 to A-20.046 of formula IAA wherein R₁, R₃, R₄, A₁ and A₂ are as defined in table X and R_{Q} is 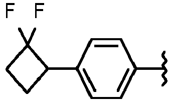
Table A-21 provides 46 compounds A-21.001 to A-21.046 of formula IAA wherein R₁, R₃, R₄, A₁ and A₂ are as defined in table X and R_{Q} is 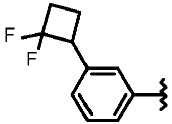
Table A-22 provides 46 compounds A-22.001 to A-22.046 of formula IAA wherein R₁, R₃, R₄, A₁ and A₂ are as defined in table X and R_{Q} is 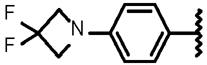
Table A-23 provides 46 compounds A-23.001 to A-23.046 of formula IAA wherein R₁, R₃, R₄, A₁ and A₂ are as defined in table X and R_{Q} is 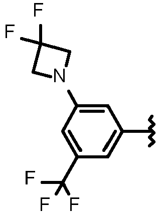
Table A-24 provides 46 compounds A-24.001 to A-24.046 of formula IAA wherein R₁, R₃, R₄, A₁ and A₂ are as defined in table X and R_{Q} is 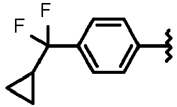
Table A-25 provides 46 compounds A-25.001 to A-25.046 of formula IAA wherein R₁, R₃, R₄, A₁ and A₂ are as defined in table X and R_{Q} is 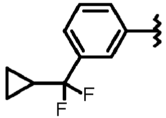
Table A-26 provides 46 compounds A-26.001 to A-26.046 of formula IAA wherein R₁, R₃, R₄, A₁ and A₂ are as defined in table X and R_{Q} is 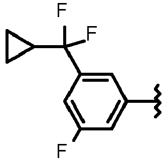
Table A-27 provides 46 compounds A-27.001 to A-27.046 of formula IAA wherein R₁, R₃, R₄, A₁ and A₂ are as defined in table X and R_{Q} is 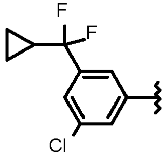
Table A-28 provides 46 compounds A-28.001 to A-28.046 of formula IAA wherein R₁, R₃, R₄, A₁ and A₂ are as defined in table X and R_{Q} is 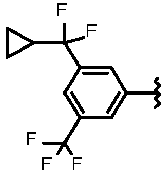
Table A-29 provides 46 compounds A-29.001 to A-29.046 of formula IAA wherein R₁, R₃, R₄, A₁ and A₂ are as defined in table X and R_{Q} is 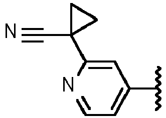
Table A-30 provides 46 compounds A-30.001 to A-30.046 of formula IAA wherein R₁, R₃, R₄, A₁ and A₂ are as defined in table X and R_{Q} is 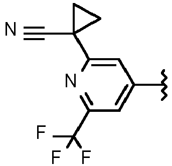
Table A-31 provides 46 compounds A-31.001 to A-31.046 of formula IAA wherein R₁, R₃, R₄, A₁ and A₂ are as defined in table X and R_{Q} is 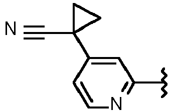
Table A-32 provides 46 compounds A-32.001 to A-32.046 of formula IAA wherein R₁, R₃, R₄, A₁ and A₂ are as defined in table X and R_{Q} is 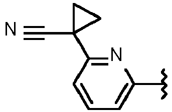
Table A-33 provides 46 compounds A-33.001 to A-33.046 of formula IAA wherein R₁, R₃, R₄, A₁ and A₂ are as defined in table X and R_{Q} is 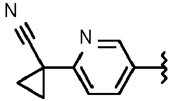
Table A-34 provides 46 compounds A-34.001 to A-34.046 of formula IAA wherein R₁, R₃, R₄, A₁ and A₂ are as defined in table X and R_{Q} is 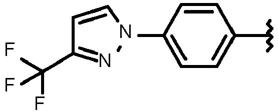
Table A-35 provides 46 compounds A-35.001 to A-35.046 of formula IAA wherein R₁, R₃, R₄, A₁ and A₂ are as defined in table X and R_{Q} is 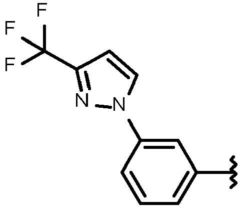
Table A-36 provides 46 compounds A-36.001 to A-36.046 of formula IAA wherein R₁, R₃, R₄, A₁ and A₂ are as defined in table X and R_{Q} is 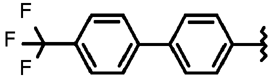
Table A-37 provides 46 compounds A-37.001 to A-37.046 of formula IAA wherein R₁, R₃, R₄, A₁ and A₂ are as defined in table X and R_{Q} is 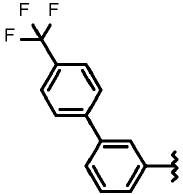
Table A-38 provides 46 compounds A-38.001 to A-38.046 of formula IAA wherein R₁, R₃, R₄, A₁ and A₂ are as defined in table X and R_{Q} is 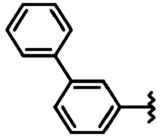
Table A-39 provides 46 compounds A-39.001 to A-39.046 of formula IAA wherein R₁, R₃, R₄, A₁ and A₂ are as defined in table X and R_{Q} is 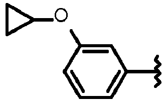
Table A-40 provides 46 compounds A-40.001 to A-40.046 of formula IAA wherein R₁, R₃, R₄, A₁ and A₂ are as defined in table X and R_{Q} is 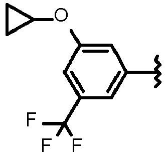
Table A-41 provides 46 compounds A-41.001 to A-41.046 of formula IAA wherein R₁, R₃, R₄, A₁ and A₂ are as defined in table X and R_{Q} is 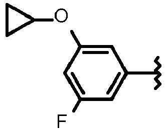
Table A-42 provides 46 compounds A-42.001 to A-42.046 of formula IAA wherein R₁, R₃, R₄, A₁ and A₂ are as defined in table X and R_{Q} is 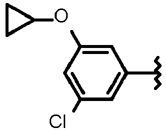
Table A-43 provides 46 compounds A-43.001 to A-43.046 of formula IAA wherein R₁, R₃, R₄, A₁ and A₂ are as defined in table X and R_{Q} is 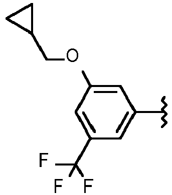
Table A-44 provides 46 compounds A-44.001 to A-44.046 of formula IAA wherein R₁, R₃, R₄, A₁ and A₂ are as defined in table X and R_{Q} is 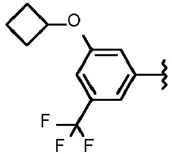
Table A-45 provides 46 compounds A-45.001 to A-45.046 of formula IAA wherein R₁, R₃, R₄, A₁ and A₂ are as defined in table X and R_{Q} is 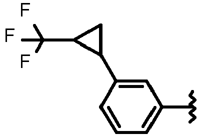
Table A-46 provides 46 compounds A-46.001 to A-46.046 of formula IAA wherein R₁, R₃, R₄, A₁ and A₂ are as defined in table X and R_{Q} is 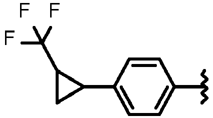
Table A-47 provides 46 compounds A-47.001 to A-47.046 of formula IAA wherein R₁, R₃, R₄, A₁ and A₂ are as defined in table X and R_{Q} is 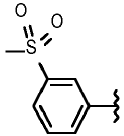
Table A-48 provides 46 compounds A-48.001 to A-48.046 of formula IAA wherein R₁, R₃, R₄, A₁ and A₂ are as defined in table X and R_{Q} is 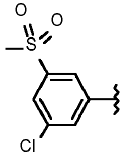
Table A-49 provides 46 compounds A-49.001 to A-49.046 of formula IAA wherein R₁, R₃, R₄, A₁ and A₂ are as defined in table X and R_{Q} is 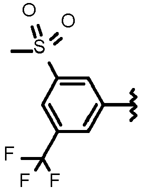
Table A-50 provides 46 compounds A-50.001 to A-50.046 of formula IAA wherein R₁, R₃, R₄, A₁ and A₂ are as defined in table X and R_{Q} is 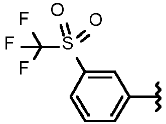
Table A-51 provides 46 compounds A-51.001 to A-51.046 of formula IAA wherein R₁, R₃, R₄, A₁ and A₂ are as defined in table X and R_{Q} is 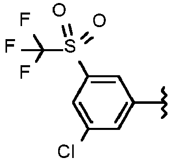
Table A-52 provides 46 compounds A-52.001 to A-52.046 of formula IAA wherein R₁, R₃, R₄, A₁ and A₂ are as defined in table X and R_{Q} is 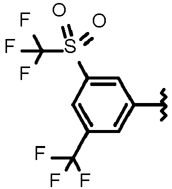
Table A-53 provides 46 compounds A-53.001 to A-53.046 of formula IAA wherein R₁, R₃, R₄, A₁ and A₂ are as defined in table X and R_{Q} is 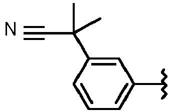
Table A-54 provides 46 compounds A-54.001 to A-54.046 of formula IAA wherein R₁, R₃, R₄, A₁ and A₂ are as defined in table X and R_{Q} is 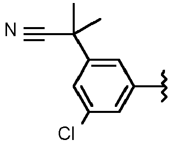
Table A-55 provides 46 compounds A-55.001 to A-55.046 of formula IAA wherein R₁, R₃, R₄, A₁ and A₂ are as defined in table X and R_{Q} is 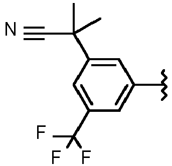
Table A-56 provides 46 compounds A-56.001 to A-56.046 of formula IAA wherein R₁, R₃, R₄, A₁ and A₂ are as defined in table X and R_{Q} is 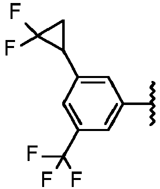
Table A-57 provides 46 compounds A-57.001 to A-57.046 of formula IAA wherein R₁, R₃, R₄, A₁ and A₂ are as defined in table X and R_{Q} is 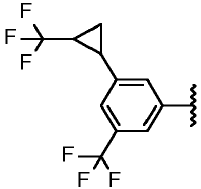
Table A-58 provides 46 compounds A-58.001 to A-58.046 of formula IAA wherein R₁, R₃, R₄, A₁ and A₂ are as defined in table X and R_{Q} is 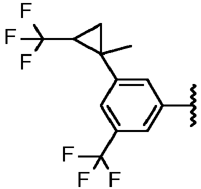
Table A-59 provides 46 compounds A-59.001 to A-59.046 of formula IAA wherein R₁, R₃, R₄, A₁ and A₂ are as defined in table X and R_{Q} is 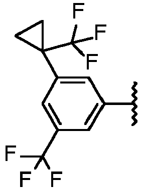
Table A-60 provides 46 compounds A-60.001 to A-60.046 of formula IAA wherein R₁, R₃, R₄, A₁ and A₂ are as defined in table X and R_{Q} is 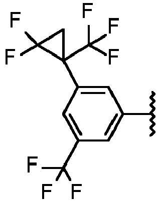
Table A-61 provides 46 compounds A-61.001 to A-61.046 of formula IAA wherein R₁, R₃, R₄, A₁ and A₂ are as defined in table X and R_{Q} is 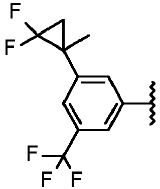
Table A-62 provides 46 compounds A-62.001 to A-62.046 of formula IAA wherein R₁, R₃, R₄, A₁ and A₂ are as defined in table X and R_{Q} is 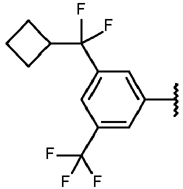
Table A-63 provides 46 compounds A-63.001 to A-63.046 of formula IAA wherein R₁, R₃, R₄, A₁ and A₂ are as defined in table X and R_{Q} is 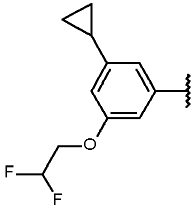
Table A-64 provides 46 compounds A-64.001 to A-64.046 of formula IAA wherein R₁, R₃, R₄, A₁ and A₂ are as defined in table X and R_{Q} is

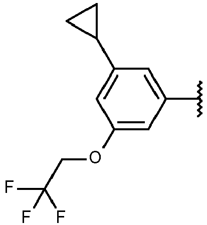

Certain compounds of formula III and IIIB are novel and can be prepared by methods described above. 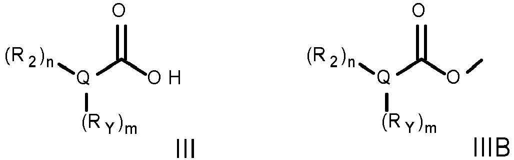 where Q, R₂, R_{Y}, m and n are as defined for formula I. Accordingly, 64 compounds of formula III and 64 compounds of formula IIIB are made available wherein the group Q containing the substituent R₂ and optionally R_{Y} corresponds to .R_{Q}, as defined in Tables A-1 to A-64.

The compounds of formula I according to the invention are preventively and/or curatively valuable active ingredients in the field of pest control, even at low rates of application, which have a very favorable biocidal spectrum and are well tolerated by warm-blooded species, fish and plants. The active ingredients according to the invention act against all or individual developmental stages of normally sensitive, but also resistant, animal pests, such as insects or representatives of the order Acarina. The insecticidal or acaricidal activity of the active ingredients according to the invention can manifest itself directly, i. e. in destruction of the pests, which takes place either immediately or only after some time has elapsed, for example during ecdysis, or indirectly, for example in a reduced oviposition and/or hatching rate.

Examples of the above mentioned animal pests are:
from the order *Acarina,* for example,
Acalitus spp, Aculus spp, Acaricalus spp, Aceria spp, Acarus siro, Amblyomma spp., Argas spp., Boophilus spp., Brevipalpus spp., Bryobia spp, Calipitrimerus spp., Chorioptes spp., Dermanyssus gallinae, Dermatophagoides spp, Eotetranychus spp, Eriophyes spp., Hemitarsonemus spp, Hyalomma spp., Ixodes spp., Olygonychus spp, Ornithodoros spp., Polyphagotarsone latus, Panonychus spp., Phyllocoptruta oleivora, Phytonemus spp, Polyphagotarsonemus spp, Psoroptes spp., Rhipicephalus spp., Rhizoglyphus spp., Sarcoptes spp., Steneotarsonemus spp, Tarsonemus spp. and Tetranychus spp.;
from the order *Anoplura,* for example, Haematopinus spp., Linognathus spp., Pediculus spp., Pemphigus spp. and Phylloxera spp.;
from the order *Coleoptera,* for example, Agriotes spp., Amphimallon majale, Anomala orientalis, Anthonomus spp., Aphodius spp, Astylus atromaculatus, Ataenius spp, Atomaria linearis, Chaetocnema tibialis, Cerotoma spp, Conoderus spp, Cosmopolites spp., Cotinis nitida, Curculio spp., Cyclocephala spp, Dermestes spp., Diabrotica spp., Diloboderus abderus, Epilachna spp., Eremnus spp., Heteronychus arator, Hypothenemus hampei, Lagria vilosa, Leptinotarsa decemLineata, Lissorhoptrus spp., Liogenys spp, Maecolaspis spp, Maladera castanea, Megascelis spp, Melighetes aeneus, Melolontha spp., Myochrous armatus, Orycaephilus spp., Otiorhynchus spp., Phyllophaga spp, Phlyctinus spp., Popillia spp., Psylliodes spp., Rhyssomatus aubtilis, Rhizopertha spp., Scarabeidae, Sitophilus spp., Sitotroga spp., Somaticus spp, Sphenophorus spp, Sternechus subsignatus, Tenebrio spp., Tribolium spp. and Trogoderma spp.; from the order *Diptera,* for example,
Aedes spp., Anopheles spp, Antherigona soccata,Bactrocea oleae, Bibio hortulanus, Bradysia spp, Calliphora erythrocephala, Ceratitis spp., Chrysomyia spp., Culex spp., Cuterebra spp., Dacus spp., Delia spp, Drosophila melanogaster, Fannia spp., Gastrophilus spp., Geomyza tripunctata, Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis spp, Rivelia quadrifasciata, Scatella spp, Sciara spp., Stomoxys spp., Tabanus spp., Tannia spp. and Tipula spp.;
from the order *Hemiptera,* for example, Acanthocoris scabrator, Acrosternum spp, Adelphocoris lineolatus, Amblypelta nitida, Bathycoelia thalassina, Blissus spp, Cimex spp., Clavigralla tomentosicollis, Creontiades spp, Distantiella theobroma, Dichelops furcatus, Dysdercus spp., Edessa spp, Euchistus spp., Eurydema pulchrum, Eurygaster spp., Halyomorpha halys, Horcias nobilellus, Leptocorisa spp., Lygus spp, Margarodes spp, Murgantia histrionic, Neomegalotomus spp, Nesidiocoris tenuis, Nezara spp., Nysius simulans, Oebalus insularis, Piesma spp., Piezodorus spp, Rhodnius spp., Sahlbergella singularis, Scaptocoris castanea, Scotinophara spp. , Thyanta spp , Triatoma spp., Vatiga illudens;
Acyrthosium pisum, Adalges spp, Agalliana ensigera, Agonoscena targionii, Aleurodicus spp, Aleurocanthus spp, Aleurolobus barodensis, Aleurothrixus floccosus, Aleyrodes brassicae, Amarasca biguttula, Amritodus atkinsoni, Aonidiella spp., Aphididae, Aphis spp., Aspidiotus spp., Aulacorthum solani, Bactericera cockerelli, Bemisia spp, Brachycaudus spp, Brevicoryne brassicae, Cacopsylla spp, Cavariella aegopodii Scop., Ceroplaster spp., Chrysomphalus aonidium, Chrysomphalus dictyospermi, Cicadella spp, Cofana spectra, Cryptomyzus spp, Cicadulina spp, Coccus hesperidum, Dalbulus maidis, Dialeurodes spp, Diaphorina citri, Diuraphis noxia, Dysaphis spp, Empoasca spp., Eriosoma larigerum, Erythroneura spp., Gascardia spp., Glycaspis brimblecombei, Hyadaphis pseudobrassicae, Hyalopterus spp, Hyperomyzus pallidus, Idioscopus clypealis, Jacobiasca lybica, Laodelphax spp., Lecanium corni, Lepidosaphes spp., Lopaphis erysimi, Lyogenys maidis, Macrosiphum spp., Mahanarva spp, Metcalfa pruinosa, Metopolophium dirhodum, Myndus crudus, Myzus spp., Neotoxoptera sp, Nephotettix spp., Nilaparvata spp., Nippolachnus piri Mats, Odonaspis ruthae, Oregma lanigera Zehnter, Parabemisia myricae, Paratrioza cockerelli, Parlatoria spp., Pemphigus spp., Peregrinus maidis, Perkinsiella spp, Phorodon humuli, Phylloxera spp, Planococcus spp., Pseudaulacaspis spp., Pseudococcus spp., Pseudatomoscelis seriatus, Psylla spp., Pulvinaria aethiopica, Quadraspidiotus spp., Quesada gigas, Recilia dorsalis, Rhopalosiphum spp., Saissetia spp., Scaphoideus spp., Schizaphis spp., Sitobion spp., Sogatella furcifera, Spissistilus festinus, Tarophagus Proserpina, Toxoptera spp, Trialeurodes spp, Tridiscus sporoboli, Trionymus spp, Trioza erytreae , Unaspis citri, Zygina flammigera, Zyginidia scutellaris, ;
from the order *Hymenoptera,* for example, Acromyrmex, Arge spp, Atta spp., Cephus spp., Diprion spp., Diprionidae, Gilpinia polytoma, Hoplocampa spp., Lasius spp., Monomorium pharaonis, Neodiprion spp., Pogonomyrmex spp, Slenopsis invicta, Solenopsis spp. and Vespa spp.;
from the order *Isoptera,* for example, Coptotermes spp, Corniternes cumulans, Incisitermes spp, Macrotermes spp, Mastotermes spp, Microtermes spp, Reticulitermes spp.; Solenopsis geminate
from the order *Lepidoptera,* for example, Acleris spp., Adoxophyes spp., Aegeria spp., Agrotis spp., Alabama argillaceae, Amylois spp., Anticarsia gemmatalis, Archips spp., Argyresthia spp, Argyrotaenia spp., Autographa spp., Bucculatrix thurberiella, Busseola fusca, Cadra cautella, Carposina nipponensis, Chilo spp., Choristoneura spp., Chrysoteuchia topiaria, Clysia ambiguella, Cnaphalocrocis spp., Cnephasia spp., Cochylis spp., Coleophora spp., Colias lesbia, Cosmophila flava, Crambus spp, Crocidolomia binotalis, Cryptophlebia leucotreta, Cydalima perspectalis, Cydia spp., Diaphania perspectalis, Diatraea spp., Diparopsis castanea, Earias spp., Eldana saccharina, Ephestia spp., Epinotia spp, Estigmene acrea, Etiella zinckinella, Eucosma spp., Eupoecilia ambiguella, Euproctis spp., Euxoa spp., Feltia jaculiferia, Grapholita spp., Hedya nubiferana, Heliothis spp., Hellula undalis, Herpetogramma spp, Hyphantria cunea, Keiferia lycopersicella, Lasmopalpus lignosellus, Leucoptera scitella, Lithocollethis spp., Lobesia botrana, Loxostege bifidalis, Lymantria spp., Lyonetia spp., Malacosoma spp., Mamestra brassicae, Manduca sexta, Mythimna spp, Noctua spp, Operophtera spp., Orniodes indica, Ostrinia nubilalis, Pammene spp., Pandemis spp., Panolis flammea, Papaipema nebris, Pectinophora gossypiela, Perileucoptera coffeella, Pseudaletia unipuncta, Phthorimaea operculella, Pieris rapae, Pieris spp., Plutella xylostella, Prays spp., Pseudoplusia spp, Rachiplusia nu, Richia albicosta, Scirpophaga spp., Sesamia spp., Sparganothis spp., Spodoptera spp., Sylepta derogate, Synanthedon spp., Thaumetopoea spp., Tortrix spp., Trichoplusia ni, Tuta absoluta, and Yponomeuta spp.;
from the order *Mallophaga,* for example, Damalinea spp. and Trichodectes spp.;
from the order *Orthoptera,* for example, Blatta spp., Blattella spp., Gryllotalpa spp., Leucophaea maderae, Locusta spp., Neocurtilla hexadactyla, Periplaneta spp. , Scapteriscus spp, and Schistocerca spp.;
from the order *Psocoptera,* for example, Liposcelis spp.;
from the order *Siphonaptera,* for example, Ceratophyllus spp., Ctenocephalides spp. and Xenopsylla cheopis;
from the order *Thysanoptera,* for example, Calliothrips phaseoli, Frankliniella spp., Heliothrips spp, Hercinothrips spp., Parthenothrips spp, Scirtothrips aurantii, Sericothrips variabilis, Taeniothrips spp., Thrips spp;
from the order *Thysanura,* for example, Lepisma saccharina.

The active ingredients according to the invention can be used for controlling, i. e. containing or destroying, pests of the abovementioned type which occur in particular on plants, especially on useful plants and ornamentals in agriculture, in horticulture and in forests, or on organs, such as fruits, flowers, foliage, stalks, tubers or roots, of such plants, and in some cases even plant organs which are formed at a later point in time remain protected against these pests.

Suitable target crops are, in particular, cereals, such as wheat, barley, rye, oats, rice, maize or sorghum; beet, such as sugar or fodder beet; fruit, for example pomaceous fruit, stone fruit or soft fruit, such as apples, pears, plums, peaches, almonds, cherries or berries, for example strawberries, raspberries or blackberries; leguminous crops, such as beans, lentils, peas or soya; oil crops, such as oilseed rape, mustard, poppies, olives, sunflowers, coconut, castor, cocoa or ground nuts; cucurbits, such as pumpkins, cucumbers or melons; fibre plants, such as cotton, flax, hemp or jute; citrus fruit, such as oranges, lemons, grapefruit or tangerines; vegetables, such as spinach, lettuce, asparagus, cabbages, carrots, onions, tomatoes, potatoes or bell peppers; Lauraceae, such as avocado, Cinnamonium or camphor; and also tobacco, nuts, coffee, eggplants, sugarcane, tea, pepper, grapevines, hops, the plantain family and latex plants.

The compositions and/or methods of the present invention may be also used on any ornamental and/or vegetable crops, including flowers, shrubs, broad-leaved trees and evergreens.

For example the invention may be used on any of the following ornamental species: *Ageratum* spp., *Alonsoa* spp., *Anemone* spp., *Anisodontea capsenisis, Anthemis* spp., *Antirrhinum* spp., *Aster* spp., *Begonia* spp. (e.g. *B. elatior, B. semperflorens, B. tubéreux*), *Bougainvillea* spp., *Brachycome* spp., *Brassica* spp. (ornamental), *Calceolaria* spp., *Capsicum annuum, Catharanthus roseus, Canna* spp., *Centaurea* spp., *Chrysanthemum* spp., *Cineraria* spp. (*C*. *maritime*), *Coreopsis* spp., *Crassula coccinea, Cuphea ignea, Dahlia* spp., *Delphinium* spp., *Dicentra spectabilis, Dorotheantus* spp., *Eustoma grandiflorum, Forsythia* spp., *Fuchsia* spp., *Geranium gnaphalium, Gerbera* spp., *Gomphrena globosa, Heliotropium* spp., *Helianthus* spp., *Hibiscus* spp., *Hortensia* spp., *Hydrangea* spp., *Hypoestes phyllostachya, Impatiens* spp. (*I*. *Walleriana*)*, Iresines* spp., *Kalanchoe* spp., *Lantana camara, Lavatera trimestris, Leonotis leonurus, Lilium* spp., *Mesembryanthemum* spp., *Mimulus* spp., *Monarda* spp., *Nemesia* spp., *Tagetes* spp., *Dianthus* spp. (carnation), *Canna* spp., *Oxalis* spp., *Bellis* spp., *Pelargonium* spp. (*P. peltatum, P. Zonale), Viola* spp. (pansy), *Petunia* spp., *Phlox* spp., *Plecthranthus* spp., *Poinsettia* spp., *Parthenocissus* spp. (*P. quinquefolia, P. tricuspidata), Primula* spp., *Ranunculus* spp., *Rhododendron* spp., *Rosa* spp. (rose), *Rudbeckia* spp., *Saintpaulia* spp., *Salvia* spp., *Scaevola aemola, Schizanthus wisetonensis, Sedum* spp., *Solanum* spp., *Surfinia* spp., *Tagetes* spp., *Nicotinia* spp., *Verbena* spp., *Zinnia* spp. and other bedding plants.

For example the invention may be used on any of the following vegetable species: *Allium* spp. (*A. sativum, A.. cepa, A. oschaninii, A. Porrum, A. ascalonicum, A. fistulosum), Anthriscus cerefolium, Apium graveolus, Asparagus officinalis, Beta vulgarus, Brassica* spp. (*B. Oleracea, B. Pekinensis, B. rapa), Capsicum annuum, Cicer arietinum, Cichorium endivia, Cichorum* spp. (*C*. *intybus, C. endivia), Citrillus lanatus, Cucumis* spp. (*C*. *sativus, C. melo), Cucurbita* spp. (*C*. *pepo, C. maxima), Cyanara* spp. (*C*. *scolymus, C. cardunculus), Daucus carota, Foeniculum vulgare, Hypericum* spp., *Lactuca sativa, Lycopersicon* spp. (*L. esculentum, L. lycopersicum), Mentha* spp., *Ocimum basilicum, Petroselinum crispum, Phaseolus* spp. (*P*. *vulgaris, P. coccineus*), *Pisum sativum, Raphanus sativus, Rheum rhaponticum, Rosemarinus* spp., *Salvia* spp., *Scorzonera hispanica, Solanum melongena, Spinacea oleracea, Valerianella* spp. (*V. locusta, V. eriocarpa*) and *Vicia faba.*

Preferred ornamental species include African violet, *Begonia, Dahlia, Gerbera, Hydrangea, Verbena, Rosa, Kalanchoe, Poinsettia, Aster, Centaurea, Coreopsis, Delphinium, Monarda, Phlox, Rudbeckia, Sedum, Petunia, Viola, Impatiens, Geranium, Chrysanthemum, Ranunculus, Fuchsia, Salvia, Hortensia,* rosemary, sage, St. Johnswort, mint, sweet pepper, tomato and cucumber.

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca (preferably in vegetables, vineyards), Leptinotarsa (preferably in potatos) and Chilo supressalis (preferably in rice).

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca (preferably in vegetables, vineyards), Leptinotarsa (preferably in potatos) and Chilo supressalis (preferably in rice).

In a further aspect, the invention may also relate to a method of controlling damage to plant and parts thereof by plant parasitic nematodes (Endoparasitic-, Semiendoparasitic- and Ectoparasitic nematodes), especially plant parasitic nematodes such as root knot nematodes, Meloidogyne hapla, Meloidogyne incognita, Meloidogyne javanica, Meloidogyne arenaria and other Meloidogyne species; cyst-forming nematodes, Globodera rostochiensis and other Globodera species; Heterodera avenae, Heterodera glycines, Heterodera schachtii, Heterodera trifolii, and other Heterodera species; Seed gall nematodes, Anguina species; Stem and foliar nematodes, Aphelenchoides species; Sting nematodes, Belonolaimus longicaudatus and other Belonolaimus species; Pine nematodes, Bursaphelenchus xylophilus and other Bursaphelenchus species; Ring nematodes, Criconema species, Criconemella species, Criconemoides species, Mesocriconema species; Stem and bulb nematodes, Ditylenchus destructor, Ditylenchus dipsaci and other Ditylenchus species; Awl nematodes, Dolichodorus species; Spiral nematodes, Heliocotylenchus multicinctus and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, Longidorus elongatus and other Longidorus species; Pin nematodes, Pratylenchus species; Lesion nematodes, Pratylenchus neglectus, Pratylenchus penetrans, Pratylenchus curvitatus, Pratylenchus goodeyi and other Pratylenchus species; Burrowing nematodes, Radopholus similis and other Radopholus species; Reniform nematodes, Rotylenchus robustus, Rotylenchus reniformis and other Rotylenchus species; Scutellonema species; Stubby root nematodes, Trichodorus primitivus and other Trichodorus species, Paratrichodorus species; Stunt nematodes, Tylenchorhynchus claytoni, Tylenchorhynchus dubius and other Tylenchorhynchus species; Citrus nematodes, Tylenchulus species; Dagger nematodes, Xiphinema species; and other plant parasitic nematode species, such as Subanguina spp., Hypsoperine spp., Macroposthonia spp., Melinius spp., Punctodera spp., and Quinisulcius spp..

The compounds of the invention may also have activity against the molluscs. Examples of which include, for example, Ampullariidae; Arion (A. ater, A. circumscriptus, A. hortensis, A. rufus); Bradybaenidae (Bradybaena fruticum); Cepaea (C. hortensis, C. Nemoralis); ochlodina; Deroceras (D. agrestis, D. empiricorum, D. laeve, D. reticulatum); Discus (D. rotundatus); Euomphalia; Galba (G. trunculata); Helicelia (H. itala, H. obvia); Helicidae Helicigona arbustorum); Helicodiscus; Helix (H. aperta); Limax (L. cinereoniger, L. flavus, L. marginatus, L. maximus, L. tenellus); Lymnaea; Milax (M. gagates, M. marginatus, M. sowerbyi); Opeas; Pomacea (P. canaticulata); Vallonia and Zanitoides.

The term "crops" is to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins, for example insecticidal proteins from Bacillus cereus or Bacillus popilliae; or insecticidal proteins from Bacillus thuringiensis, such as δ-endotoxins, e.g. Cry1Ab, Cry1Ac, Cry1F, Cry1 Fa2, Cry2Ab, Cry3A, Cry3Bb1 or Cry9C, or vegetative insecticidal proteins (Vip), e.g. Vip1, Vip2, Vip3 or Vip3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

In the context of the present invention there are to be understood by δ-endotoxins, for example Cry1Ab, Cry1Ac, Cry1F, Cry1Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO 02/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO 03/018810).

Examples of such toxins or transgenic plants capable of synthesising such toxins are disclosed, for example, in EP-A-0 374 753, WO 93/07278, WO 95/34656, EP-A-0 427 529, EP-A-451 878 and WO 03/052073.

The processes for the preparation of such transgenic plants are generally known to the person skilled in the art and are described, for example, in the publications mentioned above. Cryl-type deoxyribonucleic acids and their preparation are known, for example, from WO 95/34656, EP-A-0 367 474, EP-A-0 401 979 and WO 90/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and moths (Lepidoptera).

Transgenic plants containing one or more genes that code for an insecticidal resistance and express one or more toxins are known and some of them are commercially available. Examples of such plants are: YieldGard^{®} (maize variety that expresses a Cry1Ab toxin); YieldGard Rootworm^{®} (maize variety that expresses a Cry3Bb1 toxin); YieldGard Plus^{®} (maize variety that expresses a Cry1Ab and a Cry3Bb1 toxin); Starlink^{®} (maize variety that expresses a Cry9C toxin); Herculex I^{®} (maize variety that expresses a Cry1Fa2 toxin and the enzyme phosphinothricine N-acetyltransferase (PAT) to achieve tolerance to the herbicide glufosinate ammonium); NuCOTN 33B^{®} (cotton variety that expresses a Cry1Ac toxin); Bollgard I^{®} (cotton variety that expresses a Cry1Ac toxin); Bollgard II^{®} (cotton variety that expresses a Cry1Ac and a Cry2Ab toxin); VipCot^{®} (cotton variety that expresses a Vip3A and a CrylAb toxin); NewLeaf^{®} (potato variety that expresses a Cry3A toxin); NatureGard^{®}, Agrisure^{®} GT Advantage (GA21 glyphosate-tolerant trait), Agrisure^{®} CB Advantage (Bt11 corn borer (CB) trait) and Protecta^{®}.

Further examples of such transgenic crops are:
1. **Bt11 Maize** from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a truncated Cry1Ab toxin. Bt11 maize also transgenically expresses the enzyme PAT to achieve tolerance to the herbicide glufosinate ammonium.
2. **Bt176 Maize** from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer (*Ostrinia nubilalis* and *Sesamia nonagrioides*) by transgenic expression of a CrylAb toxin. Bt176 maize also transgenically expresses the enzyme PAT to achieve tolerance to the herbicide glufosinate ammonium.
3. **MI**R₆**04 Maize** from Syngenta Seeds SAS, Chemin de l'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 03/018810.
4. **MON 863 Maize** from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. **IPC 531 Cotton** from Monsanto Europe S.A. 270-272 Avenue de Tervuren, B-1150 Brussels, Belgium, registration number C/ES/96/02.
**6. 1507 Maize** from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. **NK603** × **MON 810 Maize** from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 × MON 810
Maize transgenically expresses the protein CP4 EPSPS, obtained from *Agrobacterium sp.* strain CP4, which imparts tolerance to the herbicide Roundup^{®} (contains glyphosate), and also a CrylAb toxin obtained from *Bacillus thuringiensis subsp. kurstaki* which brings about tolerance to certain Lepidoptera, include the European corn borer.
Transgenic crops of insect-resistant plants are also described in BATS (Zentrum für Biosicherheit und Nachhaltigkeit, Zentrum BATS, Clarastrasse 13, 4058 Basel, Switzerland) Report 2003, (http://bats.ch) .

The term "crops" is to be understood as including also crop plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818 and EP-A-0 353 191. The methods of producing such transgenic plants are generally known to the person skilled in the art and are described, for example, in the publications mentioned above.

Crops may also be modified for enhanced resistance to fungal (for example Fusarium, Anthracnose, or Phytophthora), bacterial (for example Pseudomonas) or viral (for example potato leafroll virus, tomato spotted wilt virus, cucumber mosaic virus) pathogens.

Crops also include those that have enhanced resistance to nematodes, such as the soybean cyst nematode.

Crops that are tolerance to abiotic stress include those that have enhanced tolerance to drought, high salt, high temperature, chill, frost, or light radiation, for example through expression of NF-YB or other proteins known in the art.

Antipathogenic substances which can be expressed by such transgenic plants include, for example, ion channel blockers, such as blockers for sodium and calcium channels, for example the viral KP1, KP4 or KP6 toxins; stilbene synthases; bibenzyl synthases; chitinases; glucanases; the so-called "pathogenesis-related proteins" (PRPs; see e.g. EP-A-0 392 225); antipathogenic substances produced by microorganisms, for example peptide antibiotics or heterocyclic antibiotics (see e.g. WO 95/33818) or protein or polypeptide factors involved in plant pathogen defence (so-called "plant disease resistance genes", as described in WO 03/000906).

Further areas of use of the compositions according to the invention are the protection of stored goods and store rooms and the protection of raw materials, such as wood, textiles, floor coverings or buildings, and also in the hygiene sector, especially the protection of humans, domestic animals and productive livestock against pests of the mentioned type.

The present invention provides a compound of the first aspect for use in therapy. The present invention provides a compound of the first aspect, for use in controlling parasites in or on an animal. The present invention further provides a compound of the first aspect, for use in controlling ectoparasites on an animal. The present invention further provides a compound of the first aspect, for use in preventing and/or treating diseases transmitted by ectoparasites.

The present invention provides the use of a compound of the first aspect, for the manufacture of a medicament for controlling parasites in or on an animal. The present invention further provides the use of a compound of the first aspect, for the manufacture of a medicament for controlling ectoparasites on an animal. The present invention further provides the use of a compound of the first aspect, for the manufacture of a medicament for preventing and/or treating diseases transmitted by ectoparasites.

The present invention provides the use of a compound of the first aspect, in controlling parasites in or on an animal. The present invention further provides the use of a compound of the first aspect, in controlling ectoparasites on an animal.

The term "controlling" when used in context of parasites in or on an animal refers to reducing the number of pests or parasites, eliminating pests or parasites and/or preventing further pest or parasite infestation.

The term "treating" when used in context of parasites in or on an animal refers to restraining, slowing, stopping or reversing the progression or severity of an existing symptom or disease.

The term "preventing" when used in context of parasites in or on an animal refers to the avoidance of a symptom or disease developing in the animal.

The term "animal" when used in context of parasites in or on an animal may refer to a mammal and a non-mammal, such as a bird or fish. In the case of a mammal, it may be a human or non-human mammal. Non-human mammals include, but are not limited to, livestock animals and companion animals. Livestock animals include, but are not limited to, cattle, camellids, pigs, sheep, goats and horses. Companion animals include, but are not limited to, dogs, cats and rabbits.

A "parasite" is a pest which lives in or on the host animal and benefits by deriving nutrients at the host animal's expense. An "endoparasite" is a parasite which lives in the host animal. An "ectoparasite" is a parasite which lives on the host animal. Ectoparasites include, but are not limited to, acari, insects and crustaceans (e.g. sea lice). The Acari (or Acarina) sub-class comprises ticks and mites. Ticks include, but are not limited to, members of the following genera: *Rhipicaphalus,* for example, *Rhipicaphalus (Boophilus) microplus* and *Rhipicephalus sanguineus; Amblyomrna; Dermacentor*; *Haemaphysalis; Hyalomma; Ixodes; Rhipicentor*; *Margaropus; Argas; Otobius;* and *Ornithodoros.* Mites include, but are not limited to, members of the following genera: *Chorioptes,* for example *Chorioptes bovis; Psoroptes,* for example *Psoroptes ovis; Cheyletiella; Dermanyssus*; for example *Dermanyssus gallinae; Ortnithonyssus; Demodex,* for example *Demodex canis; Sarcoptes,* for example *Sarcoptes scabiei;* and *Psorergates.* Insects include, but are not limited to, members of the orders: Siphonaptera, Diptera, Phthiraptera, Lepidoptera, Coleoptera and Homoptera. Members of the Siphonaptera order include, but are not limited to, *Ctenocephalides felis* and *Ctenocephatides canis.* Members of the Diptera order include, but are not limited to, *Musca spp.;* bot fly, for example *Gasterophilus intestinalis* and *Oestrus ovis;* biting flies; horse flies, for example *Haematopota spp.* and *Tabunus spp.; haematobia,* for example *haematobia irritans; Stomoxys; Lucilia;* midges; and mosquitoes. Members of the Phthiraptera class include, but are not limited to, blood sucking lice and chewing lice, for example *Bovicola Ovis* and *Bovicola Bovis.*

The term "effective amount" when used in context of parasites in or on an animal refers to the amount or dose of the compound of the invention, or a salt thereof, which, upon single or multiple dose administration to the animal, provides the desired effect in or on the animal. The effective amount can be readily determined by the attending diagnostician, as one skilled in the art, by the use of known techniques and by observing results obtained under analogous circumstances. In determining the effective amount a number of factors are considered by the attending diagnostician, including, but not limited to: the species of mammal; its size, age, and general health; the parasite to be controlled and the degree of infestation; the specific disease or disorder involved; the degree of or involvement or the severity of the disease or disorder; the response of the individual; the particular compound administered; the mode of administration; the bioavailability characteristics of the preparation administered; the dose regimen selected; the use of concomitant medication; and other relevant circumstances.

The compounds of the invention may be administered to the animal by any route which has the desired effect including, but not limited to topically, orally, parenterally' and subcutaneously. Topical administration is preferred. Formulations suitable for topical administration include, for example, solutions, emulsions and suspensions and may take the form of a pour-on, spot-on, sprayon, spray race or dip. In the alternative, the compounds of the invention may be administered by means of an ear tag or collar.

Salt forms of the compounds of the invention include both pharmaceutically acceptable salts and veterinary acceptable salts, which can be different to agrochemically acceptable salts. Pharmaceutically and veterinary acceptable salts and common methodology for preparing them are well known in the art. See, for example, Gould, P.L., "Salt selection for basic drugs", International Journal of Pharmaceutics, 33: 201 -217 (1986); Bastin, R.J., et al. "Salt Selection and Optimization Procedures for Pharmaceutical New Chemical Entities", Organic Process Research and Development, 4: 427-435 (2000); and Berge, S.M., et al., "Pharmaceutical Salts", Journal of Pharmaceutical Sciences, 66: 1-19, (1977). One skilled in the art of synthesis will appreciate that the compounds of the invention are readily converted to and may be isolated as a salt, such as a hydrochloride salt, using techniques and conditions well known to one of ordinary skill in the art. In addition, one skilled in the art of synthesis will appreciate that the compounds of the invention are readily converted to and may be isolated as the corresponding free base from the corresponding salt.

The present invention also provides a method for controlling pests (such as mosquitoes and other disease vectors; see also http://www.who.int/malaria/vector_control/irs/en/). In one embodiment, the method for controlling pests comprises applying the compositions of the invention to the target pests, to their locus or to a surface or substrate by brushing, rolling, spraying, spreading or dipping. By way of example, an IRS (indoor residual spraying) application of a surface such as a wall, ceiling or floor surface is contemplated by the method of the invention. In another embodiment, it is contemplated to apply such compositions to a substrate such as non-woven or a fabric material in the form of (or which can be used in the manufacture of) netting, clothing, bedding, curtains and tents.

In one embodiment, the method for controlling such pests comprises applying a pesticidally effective amount of the compositions of the invention to the target pests, to their locus, or to a surface or substrate so as to provide effective residual pesticidal activity on the surface or substrate. Such application may be made by brushing, rolling, spraying, spreading or dipping the pesticidal composition of the invention. By way of example, an IRS application of a surface such as a wall, ceiling or floor surface is contemplated by the method of the invention so as to provide effective residual pesticidal activity on the surface. In another embodiment, it is contemplated to apply such compositions for residual control of pests on a substrate such as a fabric material in the form of (or which can be used in the manufacture of) netting, clothing, bedding, curtains and tents.

Substrates including non-woven, fabrics or netting to be treated may be made of natural fibres such as cotton, raffia, jute, flax, sisal, hessian, or wool, or synthetic fibres such as polyamide, polyester, polypropylene, polyacrylonitrile or the like. The polyesters are particularly suitable. The methods of textile treatment are known, e.g. WO 2008/151984, WO 2003/034823, US 5631072, WO 2005/64072, WO2006/128870, EP 1724392, WO 2005113886 or WO 2007/090739.

Further areas of use of the compositions according to the invention are the field of tree injection/trunk treatment for all ornamental trees as well all sort of fruit and nut trees.

In the field of tree injection/trunk treatment, the compounds according to the present invention are especially suitable against wood-boring insects from the order *Lepidoptera* as mentioned above and from the order *Coleoptera,* especially against woodborers listed in the following tables A and B:

**Table A. Examples of exotic woodborers of economic importance.**

| Family | Species | Host or Crop Infested |
|---|---|---|
| Buprestidae | *Agrilus planipennis* | Ash |
| Cerambycidae | *Anoplura glabripennis* | Hardwoods |
| Scolytidae | *Xylosandrus crassiusculus* | Hardwoods |
| | *X. mutilatus* | Hardwoods |
| | *Tomicus piniperda* | Conifers |

**Table B. Examples of native woodborers of economic importance.**

| Family | Species | Host or Crop Infested |
|---|---|---|
| Buprestidae | *Agrilus anxius* | Birch |
| | *Agrilus politus* | Willow, Maple |
| | *Agrilus sayi* | Bayberry, Sweetfern |
| | *Agrilus vittaticolllis* | Apple, Pear, Cranberry, Serviceberry, Hawthorn |
| | *Chrysobothris femorata* | Apple, Apricot, Beech, Boxelder, Cherry, Chestnut, Currant, Elm, Hawthorn, Hackberry, Hickory, Horsechestnut, Linden, Maple, Mountain-ash, Oak, Pecan, Pear, Peach, Persimmon, Plum, Poplar, Quince, Redbud, Serviceberry, Sycamore, Walnut, Willow |
| | *Texania campestris* | Basswood, Beech, Maple, Oak, Sycamore, Willow, Yellow-poplar |
| Cerambycidae | *Goes pulverulentus* | Beech, Elm, Nuttall, Willow, Black oak, Cherrybark oak, Water oak, Sycamore |
| | *Goes tigrinus* | Oak |
| | *Neoclytus acuminatus* | Ash, Hickory, Oak, Walnut, Birch, Beech, Maple, Eastern hophornbeam, Dogwood, Persimmon, Redbud, Holly, Hackberry, Black locust, Honeylocust, Yellow-poplar, Chestnut, Osage-orange, Sassafras, Lilac, Mountain-mahogany, Pear, Cherry, Plum, Peach, Apple, Elm, Basswood, Sweetgum |
| | *Neoptychodes trilineatus* | Fig, Alder, Mulberry, Willow, Netleaf hackberry |
| | *Oberea ocellata* | Sumac, Apple, Peach, Plum, Pear, Currant, Blackberry |
| | *Oberea tripunctata* | Dogwood, Viburnum, Elm, Sourwood, Blueberry, Rhododendron, Azalea, Laurel, Poplar, Willow, Mulberry |
| | *Oncideres cingulata* | Hickory, Pecan, Persimmon, Elm, Sourwood, Basswood, Honeylocust, Dogwood, Eucalyptus, Oak, Hackberry, Maple, Fruit trees |
| | *Saperda calcarata* | Poplar |
| | *Strophiona nitens* | Chestnut, Oak, Hickory, Walnut, Beech, Maple |
| Scolytidae | *Corthylus columbianus* | Maple, Oak, Yellow-poplar, Beech, Boxelder, Sycamore, Birch, Basswood, Chestnut, Elm |
| | *Dendroctonus frontalis* | Pine |
| | *Dryocoetes betulae* | Birch, Sweetgum, Wild cherry, Beech, Pear |
| | *Monarthrum fasciatum* | Oak, Maple, Birch, Chestnut, Sweetgum, Blackgum, Poplar, Hickory, Mimosa, Apple, Peach, Pine |
| | *Phloeotribus liminaris* | Peach, Cherry, Plum, Black cherry, Elm, Mulberry, Mountain-ash |
| | *Pseudopityophthorus pruinosus* | Oak, American beech, Black cherry, Chickasaw plum, Chestnut, Maple, Hickory, Hornbeam, Hophornbeam |
| Sesiidae | *Paranthrene simulans* | Oak, American chestnut |
| | *Sannina uroceriformis* | Persimmon |
| | *Synanthedon exitiosa* | Peach, Plum, Nectarine, Cherry, Apricot, Almond, Black cherry |
| | *Synanthedon pictipes* | Peach, Plum, Cherry, Beach, Black Cherry |
| | *Synanthedon rubrofascia* | Tupelo |
| | *Synanthedon scitula* | Dogwood, Pecan, Hickory, Oak, Chestnut, Beech, Birch, Black cherry, Elm, Mountain-ash, Viburnum, Willow, Apple, Loquat, Ninebark, Bayberry |
| | *Vitacea polistiformis* | Grape |

The present invention may be also used to control any insect pests that may be present in turfgrass, including for example beetles, caterpillars, fire ants, ground pearls, millipedes, sow bugs, mites, mole crickets, scales, mealybugs, ticks, spittlebugs, southern chinch bugs and white grubs. The present invention may be used to control insect pests at various stages of their life cycle, including eggs, larvae, nymphs and adults.

In particular, the present invention may be used to control insect pests that feed on the roots of turfgrass including white grubs (such as *Cyclocephala spp.* (e.g. masked chafer, *C*. *lurida), Rhizotrogus spp.* (e.g. European chafer, *R. majalis), Cotinus spp.* (e.g. Green June beetle, *C*. *nitida), Popillia spp.* (e.g. Japanese beetle, *P. japonica), Phyllophaga spp.* (e.g. May/June beetle), *Ataenius spp.* (e.g. Black turfgrass ataenius, *A*. *spretulus*), *Maladera spp.* (e.g. Asiatic garden beetle, *M. castanea)* and *Tomarus spp*.), ground pearls (*Margarodes* spp.), mole crickets (tawny, southern, and short-winged; *Scapteriscus* spp., *Gryllotalpa africana*) and leatherjackets (European crane fly, *Tipula spp*.).

The present invention may also be used to control insect pests of turfgrass that are thatch dwelling, including armyworms (such as fall armyworm *Spodoptera frugiperda,* and common armyworm *Pseudaletia unipuncta*), cutworms, billbugs (*Sphenophorus spp.,* such as *S*. *venatus verstitus* and *S*. *parvulus*), and sod webworms (such as *Crambus spp.* and the tropical sod webworm, *Herpetogramma phaeopteralis).*

The present invention may also be used to control insect pests of turfgrass that live above the ground and feed on the turfgrass leaves, including chinch bugs (such as southern chinch bugs, *Blissus insularis*), Bermudagrass mite (*Eriophyes cynodoniensis*), rhodesgrass mealybug (*Antonina graminis*), two-lined spittlebug (*Propsapia bicincta*), leafhoppers, cutworms (*Noctuidae* family), and greenbugs.

The present invention may also be used to control other pests of turfgrass such as red imported fire ants (*Solenopsis invicta*) that create ant mounds in turf.

In the hygiene sector, the compositions according to the invention are active against ectoparasites such as hard ticks, soft ticks, mange mites, harvest mites, flies (biting and licking), parasitic fly larvae, lice, hair lice, bird lice and fleas.

Examples of such parasites are:
Of the order Anoplurida: Haematopinus spp., Linognathus spp., Pediculus spp. and Phtirus spp., Solenopotes spp..
Of the order Mallophagida: Trimenopon spp., Menopon spp., Trinoton spp., Bovicola spp., Werneckiella spp., Lepikentron spp., Damalina spp., Trichodectes spp. and Felicola spp..
Of the order Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp. and Melophagus spp..
Of the order Siphonapterida, for example Pulex spp., Ctenocephalides spp., Xenopsylla spp., Ceratophyllus spp..
Of the order Heteropterida, for example Cimex spp., Triatoma spp., Rhodnius spp., Panstrongylus spp..
Of the order Blattarida, for example Blatta orientalis, Periplaneta americana, Blattelagermanica and Supella spp..
Of the subclass Acaria (Acarida) and the orders Meta- and Meso-stigmata, for example Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp. and Varroa spp..
Of the orders Actinedida (Prostigmata) and Acaridida (Astigmata), for example Acarapis spp., Cheyletiella spp., Ornithocheyletia spp., Myobia spp., Psorergatesspp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp..

The compositions according to the invention are also suitable for protecting against insect infestation in the case of materials such as wood, textiles, plastics, adhesives, glues, paints, paper and card, leather, floor coverings and buildings.

The compositions according to the invention can be used, for example, against the following pests: beetles such as Hylotrupes bajulus, Chlorophorus pilosis, Anobium punctatum, Xestobium rufovillosum, Ptilinuspecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthesrugicollis, Xyleborus spec.,Tryptodendron spec., Apate monachus, Bostrychus capucins, Heterobostrychus brunneus, Sinoxylon spec. and Dinoderus minutus, and also hymenopterans such as Sirexjuvencus, Urocerus gigas, Urocerus gigas taignus and Urocerus augur, and termites such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis and Coptotermes formosanus, and bristletails such as Lepisma saccharina.The compounds of formulae (I), and (I'a), or salts thereof, are especially suitable for controlling one or more pests selected from the family: Noctuidae, Plutellidae, Chrysomelidae, Thripidae, Pentatomidae, Tortricidae, Delphacidae, Aphididae, Noctuidae, Crambidae, Meloidogynidae, and Heteroderidae. In a preferred embodiment of each aspect, a compound TX (where TX means "one compound selected from the compounds defined in the Tables A-1 to A-64 and Table P") controls one or more of pests selected from the family: Noctuidae, Plutellidae, Chrysomelidae, Thripidae, Pentatomidae, Tortricidae, Delphacidae, Aphididae, Noctuidae, Crambidae, Meloidogynidae, and Heteroderidae.

The compounds of formulae (I), and (I'a), or salts thereof, are especially suitable for controlling one or more of pests selected from the genus: *Spodoptera spp, Plutella spp, Frankliniella spp, Thrips spp, Euschistus spp, Cydia spp, Nilaparvata spp, Myzus spp, Aphis spp, Diabrotica spp, Rhopalosiphum spp, Pseudoplusia spp* and *Chilo spp..* In a preferred embodiment of each aspect, a compound TX (where TX means "one compound selected from the compounds defined in the Tables A-1 to A-64 and Table P") controls one or more of pests selected from the genus: *Spodoptera spp, Plutella spp, Frankliniella spp, Thrips spp, Euschistus spp, Cydia spp, Nilaparvata spp, Myzus spp, Aphis spp, Diabrotica spp, Rhopalosiphum spp, Pseudoplusia spp* and *Chilo spp.*

The compounds of formulae (I), and (I'a), or salts thereof, are especially suitable for controlling one or more of *Spodoptera littoralis, Plutella xylostella, Frankliniella occidentalis, Thrips tabaci, Euschistus heros, Cydia pomonella, Nilaparvata lugens, Myzus persicae, Chrysodeixis includens, Aphis craccivora, Diabrotica balteata, Rhopalosiphum padi,* and *Chilo suppressalis.*

In a preferred embodiment of each aspect, a compound TX (where TX means "one compound selected from the compounds defined in the Tables A-1 to A-64 and Table P") controls one or more of *Spodoptera littoralis, Plutella xylostella, Frankliniella occidentalis, Thrips tabaci, Euschistus heros, Cydia pomonella, Nilaparvata lugens, Myzus persicae, Chrysodeixis includens, Aphis craccivora, Diabrotica balteata, Rhopalosiphum Padia,* and *Chilo Suppressalis,* such as *Spodoptera littoralis* + TX, *Plutella xylostella* + TX; *Frankliniella occidentalis* + TX, *Thrips tabaci* + TX, *Euschistus heros* + TX, *Cydia pomonella* + TX, *Nilaparvata lugens* + TX, *Myzus persicae* + TX, *Chrysodeixis includens* + TX, *Aphis craccivora* + TX, *Diabrotica balteata* + TX, *Rhopalosiphum Padi* + TX, and *Chilo suppressalis +* TX.

In an embodiment, of each aspect, one compound from in the Tables A-1 to A-64 and Table P is suitable for controlling *Spodoptera littoralis, Plutella xylostella, Frankliniella occidentalis, Thrips tabaci, Euschistus heros, Cydia pomonella, Nilaparvata lugens, Myzus persicae, Chrysodeixis includens, Aphis craccivora, Diabrotica balteata, Rhopalosiphum Padia,* and *Chilo Suppressalis* in cotton, vegetable, maize, cereal, rice and soya crops.

In an embodiment, one compound from the Tables A-1 to A-64 and Table P is suitable for controlling Mamestra (preferably in vegetables), Cydia pomonella (preferably in apples), Empoasca (preferably in vegetables, vineyards), Leptinotarsa (preferably in potatos) and Chilo supressalis (preferably in rice).

Compounds according to the invention may possess any number of benefits including, inter alia, advantageous levels of biological activity for protecting plants against insects or superior properties for use as agrochemical active ingredients (for example, greater biological activity, an advantageous spectrum of activity, an increased safety profile (against non-target organisms above and below ground (such as fish, birds and bees), improved physico-chemical properties, or increased biodegradability). In particular, it has been surprisingly found that certain compounds of formula I may show an advantageous safety profile with respect to non-target arthropods, in particular pollinators such as honey bees, solitary bees, and bumble bees. Most particularly, Apis mellifera.

The compounds according to the invention can be used as pesticidal agents in unmodified form, but they are generally formulated into compositions in various ways using formulation adjuvants, such as carriers, solvents and surface-active substances. The formulations can be in various physical forms, e.g. in the form of dusting powders, gels, wettable powders, water-dispersible granules, water-dispersible tablets, effervescent pellets, emulsifiable concentrates, microemulsifiable concentrates, oilin-water emulsions, oil-flowables, aqueous dispersions, oily dispersions, suspo-emulsions, capsule suspensions, emulsifiable granules, soluble liquids, water-soluble concentrates (with water or a watermiscible organic solvent as carrier), impregnated polymer films or in other forms known e.g. from the Manual on Development and Use of FAO and WHO Specifications for Pesticides, United Nations, First Edition, Second Revision (2010). Such formulations can either be used directly or diluted prior to use. The dilutions can be made, for example, with water, liquid fertilisers, micronutrients, biological organisms, oil or solvents.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof.

The active ingredients can also be contained in very fine microcapsules. Microcapsules contain the active ingredients in a porous carrier. This enables the active ingredients to be released into the environment in controlled amounts (e.g. slow-release). Microcapsules usually have a diameter of from 0.1 to 500 microns. They contain active ingredients in an amount of about from 25 to 95 % by weight of the capsule weight. The active ingredients can be in the form of a monolithic solid, in the form of fine particles in solid or liquid dispersion or in the form of a suitable solution. The encapsulating membranes can comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of the compositions according to the invention are known *per se.* As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, *N,N*-dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, m-xylene, *n*-hexane, *n*-octylamine, octadecanoic acid, octylamine acetate, oleic acid, oleylamine, o-xylene, phenol, polyethylene glycol, propionic acid, propyl lactate, propylene carbonate, propylene glycol, propylene glycol methyl ether, p-xylene, toluene, triethyl phosphate, triethylene glycol, xylenesulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, ethylene glycol, propylene glycol, glycerol, N-methyl-2-pyrrolidone and the like.

Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances.

A large number of surface-active substances can advantageously be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2-ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and dialkylphosphate esters; and also further substances described e.g. in McCutcheon's Detergents and Emulsifiers Annual, MC Publishing Corp., Ridgewood New Jersey (1981).

Further adjuvants that can be used in pesticidal formulations include crystallisation inhibitors, viscosity modifiers, suspending agents, dyes, anti-oxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and liquid and solid fertilisers.

The compositions according to the invention can include an additive comprising an oil of vegetable or animal origin, a mineral oil, alkyl esters of such oils or mixtures of such oils and oil derivatives. The amount of oil additive in the composition according to the invention is generally from 0.01 to 10 %, based on the mixture to be applied. For example, the oil additive can be added to a spray tank in the desired concentration after a spray mixture has been prepared. Preferred oil additives comprise mineral oils or an oil of vegetable origin, for example rapeseed oil, olive oil or sunflower oil, emulsified vegetable oil, alkyl esters of oils of vegetable origin, for example the methyl derivatives, or an oil of animal origin, such as fish oil or beef tallow. Preferred oil additives comprise alkyl esters of C₈-C₂₂ fatty acids, especially the methyl derivatives of C₁₂-C₁₈ fatty acids, for example the methyl esters of lauric acid, palmitic acid and oleic acid (methyl laurate, methyl palmitate and methyl oleate, respectively). Many oil derivatives are known from the Compendium of Herbicide Adjuvants, 10th Edition, Southern Illinois University, 2010.

The inventive compositions generally comprise from 0.1 to 99 % by weight, especially from 0.1 to 95 % by weight, of compounds of the present invention and from 1 to 99.9 % by weight of a formulation adjuvant which preferably includes from 0 to 25 % by weight of a surface-active substance. Whereas commercial products may preferably be formulated as concentrates, the end user will normally employ dilute formulations.

The rates of application vary within wide limits and depend on the nature of the soil, the method of application, the crop plant, the pest to be controlled, the prevailing climatic conditions, and other factors governed by the method of application, the time of application and the target crop. As a general guideline compounds may be applied at a rate of from 1 to 2000 l/ha, especially from 10 to 1000 l/ha.

Preferred formulations can have the following compositions (weight %):

### Emulsifiable concentrates:

| | |
|---|---|
| active ingredient: | 1 to 95 %, preferably 60 to 90 % |
| surface-active agent: | 1 to 30 %, preferably 5 to 20 % |
| liquid carrier: | 1 to 80 %, preferably 1 to 35 % |

### Dusts:

| | |
|---|---|
| active ingredient: | 0.1 to 10 %, preferably 0.1 to 5 % |
| solid carrier: | 99.9 to 90 %, preferably 99.9 to 99 % |

### Suspension concentrates:

| | |
|---|---|
| active ingredient: | 5 to 75 %, preferably 10 to 50 % |
| water: | 94 to 24 %, preferably 88 to 30 % |
| surface-active agent: | 1 to 40 %, preferably 2 to 30 % |

### Wettable powders:

| | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

### Granules:

| | |
|---|---|
| active ingredient: | 0.1 to 30 %, preferably 0.1 to 15 % |
| solid carrier: | 99.5 to 70 %, preferably 97 to 85 % |

The following Examples further illustrate, but do not limit, the invention.

| Wettable powders | a) | b) | c) |
|---|---|---|---|
| active ingredients | 25 % | 50 % | 75 % |
| sodium lignosulfonate | 5% | 5 % | - |
| sodium lauryl sulfate | 3% | - | 5% |
| sodium diisobutylnaphthalenesulfonate | - | 6% | 10% |
| phenol polyethylene glycol ether (7-8 mol of ethylene oxide) | - | 2% | - |
| highly dispersed silicic acid | 5% | 10% | 10% |
| Kaolin | 62 % | 27 % | - |

The combination is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredients | 25 % | 50 % | 75 % |
| light mineral oil | 5 % | 5 % | 5 % |
| highly dispersed silicic acid | 5 % | 5 % | - |
| Kaolin | 65 % | 40 % | - |
| Talcum | - | | 20 % |

The combination is thoroughly mixed with the adjuvants and the mixture is thoroughly ground in a suitable mill, affording powders that can be used directly for seed treatment.

| Emulsifiable concentrate | |
|---|---|
| active ingredients | 10 % |
| octylphenol polyethylene glycol ether (4-5 mol of ethylene oxide) | 3 % |
| calcium dodecylbenzenesulfonate | 3 % |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredients | 5 % | 6 % | 4 % |
| Talcum | 95 % | - | - |
| Kaolin | - | 94 % | - |
| mineral filler | - | - | 96 % |

Ready-for-use dusts are obtained by mixing the combination with the carrier and grinding the mixture in a suitable mill. Such powders can also be used for dry dressings for seed.

| Extruder granules | |
|---|---|
| Active ingredients | 15 % |
| sodium lignosulfonate | 2 % |
| carboxymethylcellulose | 1 % |
| Kaolin | 82 % |

The combination is mixed and ground with the adjuvants, and the mixture is moistened with water. The mixture is extruded and then dried in a stream of air.

| Coated granules | |
|---|---|
| Active ingredients | 8% |
| polyethylene glycol (mol. wt. 200) | 3% |
| Kaolin | 89 % |

The finely ground combination is uniformly applied, in a mixer, to the kaolin moistened with polyethylene glycol. Non-dusty coated granules are obtained in this manner.

### Suspension concentrate

| | |
|---|---|
| active ingredients | 40 % |
| propylene glycol | 10 % |
| nonylphenol polyethylene glycol ether (15 mol of ethylene oxide) | 6 % |
| Sodium lignosulfonate | 10 % |
| carboxymethylcellulose | 1 % |
| silicone oil (in the form of a 75 % emulsion in water) | 1 % |
| Water | 32 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredients | 40 % |
| propylene glycol | 5 % |
| copolymer butanol PO/EO | 2 % |
| Tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of the combination are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed. The obtained capsule suspension is stabilized by adding 0.25 parts of a thickener and 3 parts of a dispersing agent. The capsule suspension formulation contains 28% of the active ingredients. The medium capsule diameter is 8-15 microns. The resulting formulation is applied to seeds as an aqueous suspension in an apparatus suitable for that purpose.

Formulation types include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP), a soluble granule (SG) or any technically feasible formulation in combination with agriculturally acceptable adjuvants.

### Preparatory Examples:

"Mp" means melting point in °C. Free radicals represent methyl groups. ¹ H NMR measurements were recorded on a Brucker 400MHz spectrometer, chemical shifts are given in ppm relevant to a TMS standard. Spectra measured in deuterated solvents as indicated. Either one of the LC-MS methods below was used to characterize the compounds. The characteristic LC-MS values obtained for each compound were the retention time ("Rt", recorded in minutes) and the measured molecular ion (M+H)⁺.

### LC-MS and GC-MS Methods:

### LC-MS Method 1:

Spectra were recorded on a Mass Spectrometer from Waters (SQD, SQDII Single quadrupole mass spectrometer) equipped with an electrospray source (Polarity: positive and negative ions, Capillary: 3.00 kV, Cone range: 30 V, Extractor: 2.00 V, Source Temperature: 150°C, Desolvation Temperature: 350°C, Cone Gas Flow: 50 l/h, Desolvation Gas Flow: 650 l/h, Mass range: 100 to 900 Da) and an Acquity UPLC from Waters: Binary pump, heated column compartment, diode-array detector and ELSD detector. Column: Waters UPLC HSS T3, 1.8 µm, 30 × 2.1 mm, Temp: 60 °C, DAD Wavelength range (nm): 210 to 500, Solvent Gradient: A = water + 5% MeOH + 0.05 % HCOOH, B= Acetonitrile + 0.05 % HCOOH, gradient: 10-100% B in 1.2 min; Flow (ml/min) 0.85

### LC-MS Method 2:

Spectra were recorded on a ACQUITY Mass Spectrometer from Waters Corporations (SQD or SQDII Single quadrupole mass spectrometer) equipped with an electrospray source (Polarity: positive or negative ions, Capillary: 3.0 kV, Cone: 30V, Extractor: 3.00 V, Source Temperature: 150°C, Desolvation Temperature: 400°C, Cone Gas Flow: 60 L/hr, Desolvation Gas Flow: 700 L/hr, Mass range: 140 to 800 Da) and an ACQUITY UPLC from Waters Corporations with solvent degasser, binary pump, heated column compartment and diode-array detector. Column: Waters UPLC HSS T3, 1.8 µm, 30 × 2.1 mm, Temp: 60 °C, DAD Wavelength range (nm): 210 to 400, Solvent Gradient: A = Water/Methanol 9:1 + 0.1% formic acid, B= Acetonitrile + 0.1% formic acid, gradient: 0-100% B in 2.5 min; Flow (ml/min) 0.75.

### GC-MS Method 3:

GC-MS was conducted on a Thermo, MS: ISQ and GC: Trace GC 1310 with a column from Zebron phenomenex: Phase ZB-5ms 15 m, diam: 0.25 mm, 0.25 µm, He flow 1.5 ml/min, temp injector: 250°C, temp detector: 220°C, method: hold 0.7min at 60 °C, 80°C/min until 320°C, hold 2 min at 320°C, total time 6min. CI reagent gas: Methane, flow 1ml/min, ionization mode Cl, polarity positive, scan time 0.2 sec, Scan mass range 50-650amu

### LC-MS Method 4:

Spectra were recorded on a Mass Spectrometer from Agilent (Single quad mass spectrometer) equipped with an Multimode- Electron Spray and APCI (Polarity: positive and negative ions), Capillary: 4.00KV, Corona Current 4.0µA, Charging Voltage, 2.00kV, Nitrogen Gas Flow:9.0L/min, Nebulizer Pressure: 40psig, Mass range: 100 to 1000 m/z), dry gas temperature 250°C,Vaporizer temperature 200°C and Spectra were recorded on LCMS from Agilent: quaternary pump, heated column compartment, Variable wave length detector. Column: Eclipse XDB C18, 5.0 µm, 150x4.6 mm, column Temp: Ambient, Wavelength (nm): 220nm, Solvents: A =0.05% TFA in water, B = 0.05% TFA in Acetonitrile. Gradient: time/%B: 0/5, 0.5/5, 3.5/90, 5/90, 5.1/5, 7/5; Flow rate: 1.0ml/min.

### Example 1: Preparation of 4-(1-cyanocyclopropyl)-N-(cyclopropylmethyl)-N-[1-(2-pyrimidin-2-yl-1,2,4-triazol-3-yl)ethyl]benzamide (Compound P2)

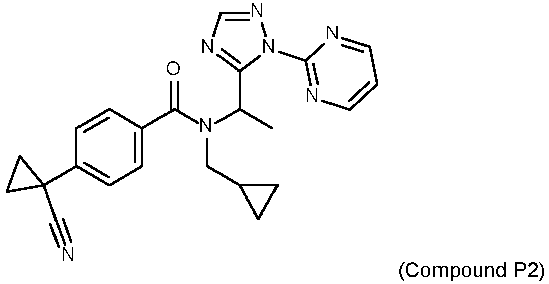

### Step A: Preparation of 2-[5-(1-bromoethyl)-1,2,4-triazol-1-yl]pyrimidine (I1).

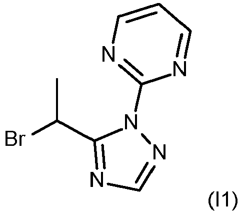

A 250 mL flask was charged with 2-bromopropanamide (2.8 g, 18 mmol), dichloromethane (54 mL), and 1,1-dimethoxy-N,N-dimethyl-methanamine (3.2 g, 27 mmol). The suspension was refluxed for one hour, and the resulting colorless solution was evaporated. The residue was transferred in a 100 mL flask and dissolved in 1,4-dioxane (18 mL) and acetic acid (16 mL). Pyrimidin-2-ylhydrazine (2 g, 18 mmol) was then added and the white suspension stirred at 90°C for two hours. The resulting homogeneous mixture was evaporated and quenched with a saturated aqueous solution of sodium hydrogen carbonate (30 mL). The aqueous phase was extracted with ethyl acetate (2 × 10 mL) and the combined organic phases were washed with water (2 × 5 mL), brine (5 mL), dried with Na2SO4, filtered and evaporated to give a crude orange oil. Purification by chromatography over silica gel (cyclohexane/ethyl acetate gradient, 100:5 to 0:100) afforded 2-[5-(1-bromoethyl)-1,2,4-triazol-1-yl]pyrimidine as a white solid.
¹H-NMR (400 MHz, CDCI₃, ppm): δ = 8.90 (d, J=4.8 Hz, 2H), 8.07 (s, 1H), 7.40 (t, J=4.8 Hz, 1H), 6.41 (q, J=6.9 Hz, 1H), 2.24 (d, J=6.9 Hz, 3H);
LC-MS (method 1): Rt 0.64, m/z = 255/256 (M+H⁺).

### Step B: Preparation of N-(cyclopropylmethyl)-1-(2-pyrimidin-2-yl-1,2,4-triazol-3-yl)ethanamine (I2)

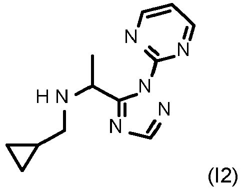

A 50 mL flask was charged with 2-[5-(1-bromoethyl)-1,2,4-triazol-1-yl]pyrimidine (0.5 g, 2.0 mmol), acetonitrile (6 mL), potassium carbonate (0.55 g, 4 mmol) and cyclopropylmethanamine (170 mg, 2.36 mmol). The resulting suspension was stirred at 80°C for two hours, evaporated and quenched with water (5 mL). The aqueous phase was extracted with ethyl acetate (2 × 3 mL), the combined organic phases were washed with water (2× 3 mL), brine (3 mL), dried with Na2SO4, filtered and evaporated to give *N*-(cyclopropylmethyl)-1-(2-pyrimidin-2-yl-1,2,4-triazol-3-yl)ethanamine as a beige oil.
¹H-NMR (400 MHz, CDCI₃): δ = 8.88 (d, J=4.8 Hz, 2H), 8.05-8.05 (m, 1H), 8.03 (s, 1H), 7.37 (t, J=4.8 Hz, 1H), 4.96 (q, J=7.0 Hz, 1H), 2.55-2.33 (m, 2H), 2.22 (dd, J=7.5, 11.6 Hz, 1H), 1.54 (d, J=7.0 Hz, 3H), 0.96-0.81 (m, 1H), 0.43-0.34 (m, 2H), 0.07-0.09 (m, 2H);
LC-MS (method 1): Rt 0.19, m/z = 245 (M+H⁺).

### Step C: Preparation of methyl 4-(1-cyanocyclopropyl)benzoate

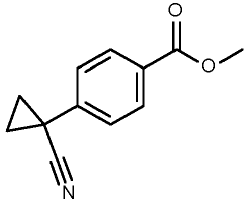

A 100 mL flask was charged with methyl 4-(cyanomethyl)benzoate (1 g, 5.7 mmol), acetonitrile (46 mL), cesium carbonate (5.6 g, 17 mmol) and 1,2-dibromoethane (1.3 g, 5.8 mmol). The resulting suspension was stirred at 80°C for three hours, cooled at room temperature and quenched with water (30 mL). The aqueous mixture was extracted with ethyl acetate (2 × 20 mL), the combined organic phases were washed with water (5 mL), brine (5 mL), dried with Na₂SO₄, filtered and evaporated. Purification by chromatography over silica gel (cyclohexane/ethyl acetate gradient, 1:0→1:1) affords methyl 4-(1-cyanocyclopropyl)benzoate.
¹H-NMR (400 MHz, CDCI₃): *δ*= 8.05-8.00 (m, 2H), 7.37-7.33 (m, 2H), 3.93 (s, 3H), 1.85-1.80 (m, 2H), 1.51-1.47 (m, 2H); );
LC-MS (method 1): Rt 0.87, *m*/*z* = 202 (M+1).

### Step D: Preparation of 4-(1-cyanocyclopropyl)benzoic acid

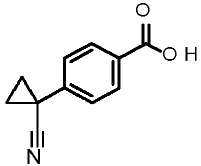

A 10 mL flask was charged with methyl 4-(1-cyanocyclopropyl)benzoate (140 mg, 0,7 mmol), tetrahydrofuran (2 mL), water (1 mL) and lithium hydroxyde monohydrate (29.5 mg, 0.7 mmol). The reaction mixture was vigorously stirred for two hours and was acidified with a 1M aqueous solution of hydrochloric acid. The aqueous phase was extracted with ethyl acetate (2 × 2 mL), the combined organic phases were washed with water (2 mL), dried with Na₂SO₄, filtered and evaporated to give 4-(1-cyanocyclopropyl)benzoic acid, which was used in the next step without any further purification.

¹H-NMR (400 MHz, CDCI₃, ppm): *δ*= 7.99-7.93 (m, 2H), 7.29-7.24 (m, 2H), 1.77-1.71 (m, 2H), 1.46-1.39 (m, 2H); LC-MS (method 1): Rt 0.69, m/z = 188 (M+H⁺).

### Step E: Preparation of 4-(1-cyanocyclopropyl)-N-(cyclopropylmethyl)-N-[1-(2-pyrimidin-2-yl-1,2,4-triazol-3-yl)ethyl1benzamide (Compound P2)

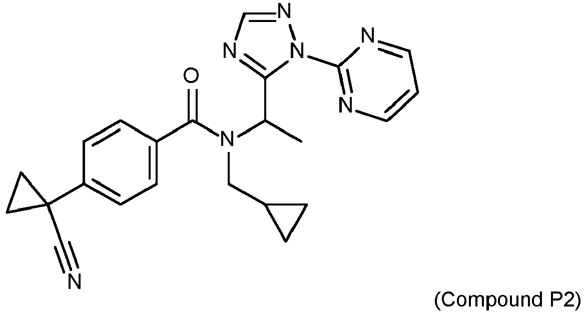

A 10 mL flask was charged with N-(cyclopropylmethyl)-1-(2-pyrimidin-2-yl-1,2,4-triazol-3-yl)ethanamine (90 mg, 0.37 mmol), N,N-dimethylformamide (1 mL), N-ethyl-N-isopropyl-propan-2-amine (130 µL, 0.74 mmol), 4-(1-cyanocyclopropyl)benzoic acid (76 mg, 0.41 mmol) and 1-[Bis(dimethylamino)methylene]-1H-1,2,3-triazolo[4,5-b]pyridinium 3-oxide hexafluorophosphate (210 mg, 0.55 mmol). The resulting yellow/beige solution was stirred at room temperature for one hour and was evaporated. The aqueous phase was extracted with ethyl acetate (2 × 3 mL), the combined organic phases were washed with water (2× 3 mL), brine (3 mL), dried with Na₂SO₄, filtered and evaporated. Purification by chromatography over silica gel (dichloromethane/methanol gradient, 1:0 to 9:1) afford 4-(1-cyanocyclopropyl)-N-(cyclopropylmethyl)-N-[1-(2-pyrimidin-2-yl-1,2,4-triazol-3-yl)ethyl]benzamide.
LC-MS (method 1): Rt 0.83, m/z = 414 (M+H⁺).

### Example 2: Preparation of N-(cyclopropylmethyl)-N-[1-(2-pyrimidin-2-yl-1,2,4-triazol-3-yl)ethyl]-3-[2-(trifluoromethyl)cyclopropyl]benzamide (Compound P42)

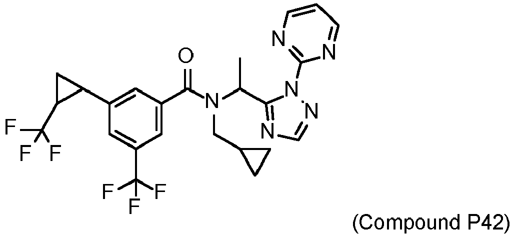

### Step A: Preparation of methyl 3-(trifluoromethyl)-5-vinyl-benzoate

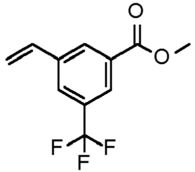

A three necked round bottom flask was charged with methyl 3-bromo-5-(trifluoromethy)-benzoate (3. 00g, 10 mmol), tributyl(vinyl)tin (4.20 g, 3.8 mL, 12 mmol), tetrakis(triphenylphosphine)palladium (0.12 g, 0.10 mmol) and toluene (62 mL) at 20 °C under argon. The light orange solution was heated at 110 °C and stirred for 2 h. The reaction mixture was cooled down to room temperature, diluted with ethyl acetate, and filtrated through a pad of Celite^{®}. After evaporation under vacuum the crude product was obtained as a viscous liquid. The resulting crude material was purified by column chromatography over silica gel, to give pure methyl 3-(trifluoromethyl)-5-vinyl-benzoate (2.10 g) as colorless liquid.
¹H-NMR (400 MHz, CDCI₃, ppm): *δ*= 3.94 - 4.01 (m, 3 H), 5.46 (d, J=10.64 Hz, 1 H), 5.93 (d, J=17.61 Hz, 1 H), 6.73 - 6.84 (m, 1 H), 7.80 - 7.84 (m, 1 H) 8.17 - 8.21 (m, 1 H) 8.23 - 8.28 (m, 1 H).

### Step B: Preparation of methyl 3-(trifluoromethyl)-5-[2-(trifluoromethyl)cyclopropyl1benzoate

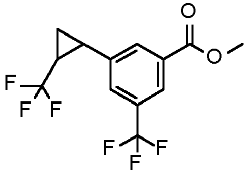

A vial was charged with methyl 3-(trifluoromethyl)-5-vinyl-benzoate (0.41 g, 1.78 mmol), CsF (0.38 g, 2.14 mmol), (0.08 g, 011 mmol) Iron(III) meso-tetraphenylporphine chloride, (trifluoroethyl)-diphenyl-sulfonium triflate (1.00 g, 2.39 mmol) and DMA (8.7 mL) under argon. The reaction was stirred for 4 days at 20 °C under argon and was then diluted with CH₂Cl₂ and water. The organic phase was separated, washed with water, dried over Na₂SO₄, filtered and evaporated. The residue was subjected to flash chromatography using silica gel and with a gradient 0 - 10% ethyl acetate in cyclohexane as eluent to yield methyl 3-(trifluoromethyl)-5-[2-(trifluoromethyl)cyclopropyl]benzoate (0.28 g) as colorless liquid.

¹H-NMR (400 MHz, CDCI₃, ppm): *δ*= 1.25 - 1.34 (m, 1 H), 1.48 - 1.55 (m, 1 H), 1.88 - 2.00 (m, 1 H), 2.46 - 2.53 (m, 1 H), 3.98 (s, 3 H), 7.60 (s, 1 H), 7.98 (s, 1 H), 8.19 (s, 1 H).

### Step C: Preparation of methyl 3-(trifluoromethyl)-5-[2-(trifluoromethyl)cyclopropyl1benzoic acid (I6)

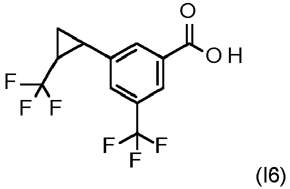

A vial was loaded with 3-(trifluoromethyl)-5-[2-(trifluoromethyl)cyclopropyl]benzoate (0.28 g, 0.66 mmol), LiOH (7.0 mg, 1.73 mmol), 3.0 mL THF and 1.3 mL water. The reaction mixture was stirred at 20 °C for 2 h. Then the reaction mixture was cooled to 0 °C , acidified with HCl (1M), extracted with 15 mL ethyl acetate (2x), and washed with brine. The organic phase was separated, washed with water, dried over Na₂SO₄, filtered and evaporated to give 3-(trifluoromethyl)-5-[2-(trifluoromethyl)cyclopropyl]benzoic acid as white solid. This product was used for the next step without further purification.

LC-MS (method 1): Rt 1.00, *m*/*z* = 297 (M-H⁺).

### Step D: Preparation of N-(cyclopropylmethyl)-N-[1-(2-pyrimidin-2-yl-1,2,4-triazol-3-yl)ethyl1-3-(trifluoromethyl)-5-[2-(trifluoromethyl)cyclopropyl1benzamide (Compound P42)

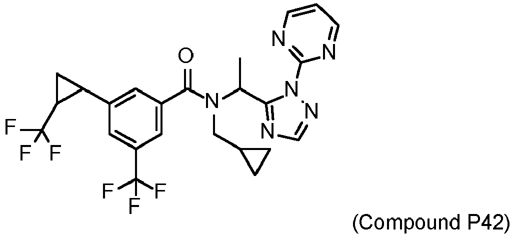

A 10 mL flask was charged with N-(cyclopropylmethyl)-1-(2-pyrimidin-2-yl-1,2,4-triazol-3-yl)ethanamine (0.10 g, 0.41 mmol), N,N-dimethylformamide (2.50 mL), N-ethyl-N-isopropyl-propan-2-amine (0.11g, 0.82 mmol), 3-(trifluoromethyl)-5-[2-(trifluoromethyl)cyclopropyl]benzoic acid (0.13 g, 0.45 mmol) and 1-[bis(dimethylamino)methylene]-1H-1,2,3-triazolo[4,5-b]pyridinium 3-oxide hexafluorophosphate (0.23 g, 0.61 mmol). The reaction mixture was stirred at room temperature for two hours and then poured into a cold aqueous solution of NH₄Cl. The aqueous phase was then extracted with ethyl acetate, and washed with water and brine. The combined organic phases were dried over Na2SO4, filtered and evaporated. Purification of the crude product by chromatography over silica gel (gradient of 0 - 10% ethyl acetate in cyclohexane then methanol in dichloromethane) yielded N-(cyclopropylmethyl)-N-[1-(2-pyrimidin-2-yl-1,2,4-triazol-3-yl)ethyl]-3-(trifluoromethyl)-5-[2-(trifluoromethyl)cyclopropyl]benzamide as colorless resin.

¹H-NMR (600 MHz, DMSO-d6, ppm) *δ*= -0.19 to -0.06 (m, 1 H), -0.04 - 0.06 (m, 1 H), 0.27 - 0.41 (m, 2 H), 0.78 (br s, 1 H), 1.29 - 1.35 (m, 1 H), 1.37 - 1.47 (m, 1H), 1.77 (d, J=6.9 Hz, 3 H), 2.23 - 2.32 (m, 1 H), 2.53 - 2.58 (m, 1 H), 3.12 (br dd, J=15.1 , 6.2 Hz, 1 H), 3.26 (br dd, J=14.8, 5.7 Hz, 1 H), 6.22 (q, J=6.7 Hz, 1H), 7.19 (s, 1 H), 7.23 (s, 1 H), 7.54 (s, 1 H), 7.57 (td, J=4.7, 3.2 Hz, 1 H), 8.12 (s, 1 H), 8.84 (d, J=4.7 Hz, 2 H).

LC-MS (method 1): Rt 1.07, m/z = 525 (M+H⁺).

### Example 3: Preparation of 3-[cyclopropyl(difluoro)methyl]-N-(cyclopropylmethyl)-N-[1-(2-pyrimidin-2-yl-1,2,4-triazol-3-yl)ethyl]-5-(trifluoromethyl)benzamide (Compound P49)

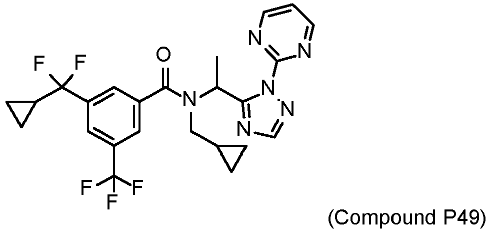

### Step A: Preparation of methyl 3-[cyclopropyl(difluoro)methyl1-5-(trifluoromethyl)benzoate

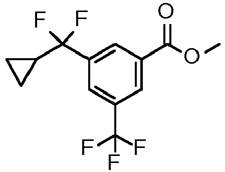

A 50 mL round-bottom flask equipped with a magnetic stirrer was charged with methyl 3-(cyclopropanecarbonyl)-5-(trifluoromethyl)benzoate (0.80 g, 2.94 mmol, prepared analog to WO2006/067445*, page 148*) and 2,2-difluoro-1,3-dimethylimidazoline at 20 °C under argon. The reaction mixture was stirred at 110 °C for 14 h. The reaction mixture was cooled to 0 °C and carefully quenched with a saturated aqueous solution of Na₂CO₃. The aqueous phase was then extracted with ethyl acetate, the combined organic phases were dried over Na₂SO₄, filtered, and concentrated under vacuum. Finally, the crude product was purified by flash chromatography on silica gel (gradient of 0 - 30% ethyl acetate in cyclohexane) to provide methyl 3-[cyclopropyl(difluoro)methyl]-5-(trifluoromethyl)benzoate (0.64 g) as colorless oil.

¹H-NMR (400 MHz, CDCI₃, ppm) *δ*= 8.40 (1 H, s), 8.38 (1 H, s), 7.98 (1 H, t, J=0.74 Hz), 4.00 (3 H, s), 1.50 - 1.58 (1 H, m), 0.81 - 0.87 (2 H, m), 0.72 - 0.80 (2 H, m).

### Step B: Preparation of 3-[cyclopropyl(difluoro)methyl1-5-(trifluoromethyl)benzoic acid (I26)

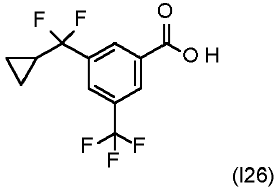

A vial was loaded with methyl 3-[cyclopropyl(difluoro)methyl]-5-(trifluoromethyl)benzoate (0.64 g, 2.18 mmol), LiOH (9.2 mg, 2.18 mmol), 6.50 mL THF and 3.30 mL water. The reaction mixture was stirred at 20 °C for 1.5 h. The reaction mixture was cooled to 0 °C , acidified (pH = 2) with HCl (1M), extracted with ethyl acetate (3x), and washed with brine. The organic phase was separated, washed with water, dried over Na₂SO₄, filtered and evaporated to give 3-[cyclopropyl(difluoro)methyl]-5-(trifluoromethyl)benzoic acid (0.57 g) as white solid. The crude product was pure enough and used as such for the next step.

¹H-NMR (400 MHz, CDCI₃, ppm): *δ*= 10.70 - 12.60 (1 H, br s), 8.48 (1 H, s), 8.45 (1 H, s), 8.05 (1 H, m), 1.46 - 1.58 (1 H, m), 0.80 - 0.88 (2 H, m), 0.73 - 0.79 (2 H, m).

### Step C: Preparation of 3-[cyclopropyl(difluoro)methyl1-N-(cyclopropylmethyl)-N-[1-(2-pyrimidin-2-yl-1,2,4-triazol-3-yl)ethyl1-5-(trifluoromethyl)benzamide (Compound P49)

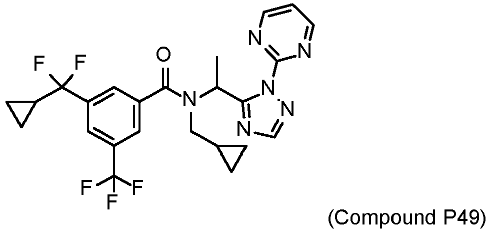

A vial was charged with N-(cyclopropylmethyl)-1-(2-pyrimidin-2-yl-1,2,4-triazol-3-yl)ethanamine (0.16 g, 0.63 mmol), acetonitrile (1.90 mL), N-ethyl-N-isopropyl-propan-2-amine (0.17 g, 0.22 mL, 1.27 mmol), 3-[cyclopropyl(difluoro)methyl]-5-(trifluoromethyl)benzoic acid (0.19 g, 0.67 mmol) and 1-[bis(dimethylamino)methylene]-1H-1,2,3-triazolo[4,5-b]pyridinium 3-oxide hexafluorophosphate (0.37 g, 0.0.95 mmol). The resulting yellow solution was stirred at room temperature for 1.5 h and then concentrated under vacuum. Purification of the crude product by flash chromatography over silica gel (gradient of 0 - 80% ethyl acetate in cyclohexane) gave 3-[cyclopropyl(difluoro)methyl]-N-(cyclopropylmethyl)-N-[1-(2-pyrimidin-2-yl-1,2,4-triazol-3-yl)ethyl]-5-(trifluoromethyl)benzamide (0.28 g) as light yellow resin.

LC-MS (method 1): Rₜ 1.06 , *m*/*z* = 507 (M+H⁺).

### Example 4: Preparation of 3-cyclopropyl-N-[1-(2-pyrimidin-2-yl-1,2,4-triazol-3-yl)ethyl]-5-(trifluoromethyl)benzamide (Compound P24)

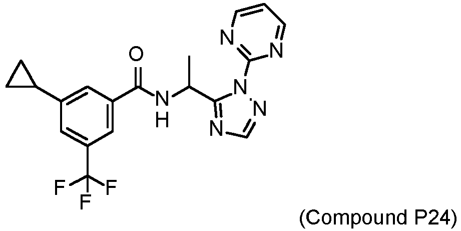

### Step A: Preparation of methyl 3-cyclopropyl-5-(trifluoromethyl)benzoate

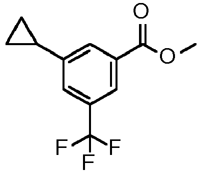

In a vial charged with methyl 3-bromo-5-(trifluoromethyl)benzoate (1.00 g, 3.46 mmol), were added 1,4-dioxane (8.70 mL), 3N solution of Na₂CO₃ (1.10 g, 10.40 mmol, 3.50 mL), cyclopropylboronic acid (0.62 g, 6.92 mmol) and Pd(Cl₂(dppf) (0.14 g, 0.173 mmol)(analog to WO2013/171712*, page 109*). The reaction mixture was degazed with argon for 5 min and then stirred at 100 °C for 3 h. The reaction mixture was cooled to room temperature and diluted with water and extracted with ethyl acetate (3x). The combined organic layers were dried, (Na₂SO₄), filtered and evaporated in vacuo. The crude product was purified by flash column chromatography over silica gel, (gradient of 0 - 15% ethyl acetate in cyclohexane) to afford methyl 3-cyclopropyl-5-(trifluoromethyl)benzoate (0.77 g) as light yellow oil.

¹H-NMR (400 MHz, CDCI₃, ppm): *δ*= 8.07 (1 H, s), 7.90 (1 H, s), 7.51 (1 H, s), 3.95 (3 H, s), 2.02 (1 H, tt, J=8.44, 5.14 Hz), 1.04 - 1.14 (2 H, m), 0.74 - 0.87 (2 H, m).

### Step B: Preparation of 3-cyclopropyl-5-(trifluoromethyl)benzoic acid (I5)

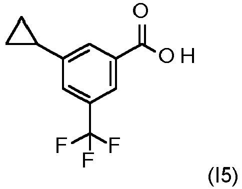

A vial was loaded with methyl 3-cyclopropyl-5-(trifluoromethyl)benzoate (0.77 g g, 3.14 mmol), LiOH monohydrate (0.133 g, 3.14 mmol), 9.4 mL THF, and 4.7 mL water. The reaction mixture was stirred at 20 °C for 3 h. Then the reaction was acidified (pH = 2) with HCl (1M) and extracted with ethyl acetate (3x). The combined organic phases were dried over Na₂SO₄, filtered and evaporated to give 3-cyclopropyl-5-(trifluoromethyl)benzoic acid (0.72 g) as white solid. The crude product was pure enough and used as such for the next step.

¹H-NMR (400 MHz, CDCI₃, ppm): *δ*= 10.4 - 10.9 (1H, br s), 8.14 (1 H, s), 7.97 (1 H, s), 7.57 (1 H, s), 2.08 - 2.01 (1 H, m), 1.08 - 1.14 (2 H, m), 0.80 - 0.86 (2 H, m).

LC-MS (method 1): Rₜ 0.96 , *m*/*z* = 229 (M-H⁺).

### Step C: Preparation of 3-cyclopropyl-N-[1-(2-pyrimidin-2-yl-1,2,4-triazol-3-yl)ethyl1-5-(trifluoromethyl)benzamide (Compound P24)

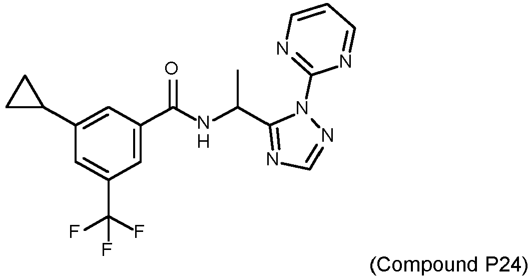

A vial was charged with 1-(2-pyrimidin-2-yl-1,2,4-triazol-3-yl)ethanamine (0.20 g, 1.05 mmol) (prepared according to WO2017/192385*, page 30*), acetonitrile (3.2 mL), N-ethyl-N-isopropyl-propan-2-amine (0.28 g, 0.37 mL, 2.10 mmol), (3-cyclopropyl-5-(trifluoromethyl)benzoic acid (0.242 g, 1.05 mmol) and 1-[bis(dimethylamino)methylene]-1H-1,2,3-triazolo[4,5-b]pyridinium 3-oxide hexafluorophosphate (0.62 g, 1.58 mmol) (analog to Tetrahedron Lett. 1999, 40, 3109*).* The resulting yellow solution was stirred at room temperature for one hour and then concentrated under vacuum. Purification of the crude product by flash chromatography over silica gel (gradient of 0 - 80% acetonitrile in H₂O) yielded 3-cyclopropyl-N-[1-(2-pyrimidin-2-yl-1,2,4-triazol-3-yl)ethyl]-5-(trifluoromethyl)benzamide (0.17 g) as light orange powder.

¹H-NMR (400 MHz, CDCI₃, ppm): *δ*= 8.93 (2 H, d, J=4.77 Hz), 8.07 (1 H, s), 7.81 (1 H, m), 7.71 (1 H, m), 7.44 (2 H, m), 7.41 (1 H, t, J=4.77 Hz), 6.42 - 6.50 (1 H, m), 1.98 - 2.06 (1 H, m), 1.71 (3 H, d, J=6.97 Hz), 1.06 - 1.12 (2 H, m), 0.78 - 0.83 (2 H, m).

LC-MS (method 1): Rt 0.94 , *m*/*z* = 403 (M+H⁺).

### Example 5: Preparation of 3-(cyclopropylmethoxy)-N-(cyclopropylmethyl)-N-[1-(2-pyrimidin-2-yl-1,2,4-triazol-3-yl)ethyl]-5-(trifluoromethyl)benzamide ( Compound P47)

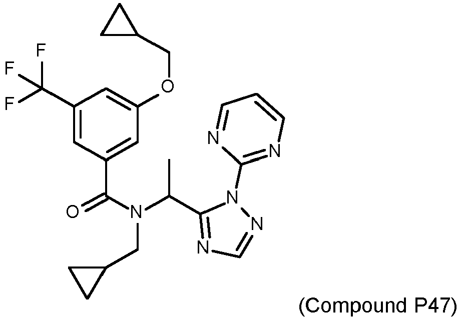

### Step A: Preparation of methyl 3-(cyclopropylmethoxy)-5-(trifluoromethyl)benzoate

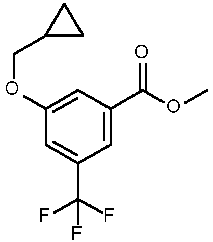

In a vial, under argon, methyl 3-hydroxy-5-(trifluoromethyl)benzoate ( CAS: 796119-63-6, 0.258 g, 1.17 mmol) was dissolved in DMF (6 mL). Cesium carbonate ( 1.15 g, 3.52 mmol) and bromomethylcyclopropane ( 1.14 mL, 11.7 mmol) were added and the solution was stirred for 4 hours at 100°C. The reaction mixture was quenched with water and the aqueous layer was extracted 2 times with ethyl acetate. The organic phases were washed 4 times with water then brine, dried over Na₂SO₄, filtered and evaporated to afford methyl 3-(cyclopropylmethoxy)-5-(trifluoromethyl)benzoate. LC-MS (method 1): Rt 1.16 min , m/z = 275 (M+H+).

### Step B: Preparation of 3-(cyclopropylmethoxy)-5-(trifluoromethyl)benzoic acid (I24)

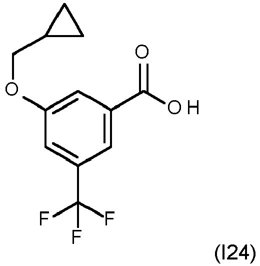

Methyl 3-(cyclopropylmethoxy)-5-(trifluoromethyl)benzoate (0.315 g, 1.15 mmol) was dissolved in THF (3.5 mL) and water (1.7 mL). Lithium hydroxide monohydrate (0.073g, 1.72 mmol) was added and the resulting mixture was stirred overnight at room temperature. The reaction mixture was quenched with water and the aqueous layer was extracted with ethyl acetate. Then the aqueous layer was acidified with HCl 2M solution until pH 2 and extracted again with ethyl acetate 2 times . The combined organic layers were washed with water, dried over Na₂SO₄ , filtered and evaporated to afford 3-(cyclopropylmethoxy)-5-(trifluoromethyl)benzoic acid.

LC-MS (method 1): Rt 1.00 min , m/z = 259 (M-H+).

### Step C: Preparation of 3-(cyclopropylmethoxy)-N-(cyclopropylmethyl)-N-[1-(2-pyrimidin-2-yl-1,2,4-triazol-3-yl)ethyl1-5-(trifluoromethyl)benzamide (Compound P47)

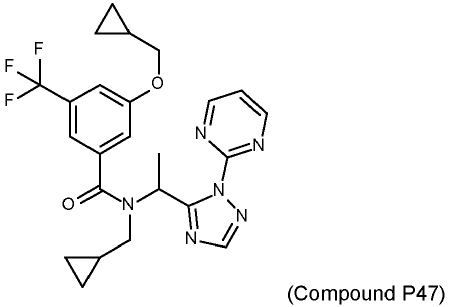

Compound P47 was prepared in using 3-(cyclopropylmethoxy)-5-(trifluoromethyl)benzoic acid and the conditions described for Compound P49 (Step C)

LC-MS (method 1): Rt 1.07 , m/z = 486 (M+H+).

### Example 6: Preparation of 3-(cyclobutoxy)-N-(cyclopropylmethyl)-N-[1-(2-pyrimidin-2-yl-1,2,4-triazol-3-yl)ethyl]-5-(trifluoromethyl)benzamide (Compound P48)

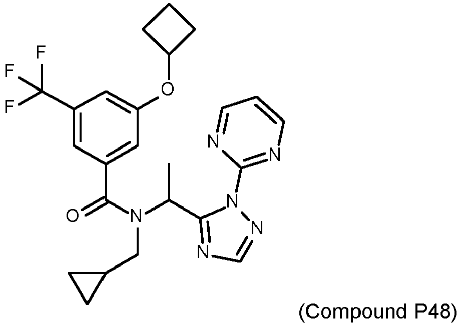

### Step A: Preparation of cyclobutyl 3-(cyclobutoxy)-5-(trifluoromethyl)benzoate

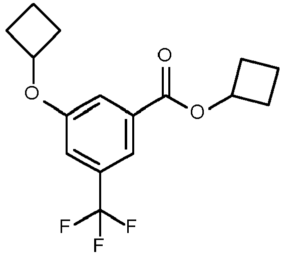

In a vial, under argon, methyl 3-hydroxy-5-(trifluoromethyl)benzoate ( CAS: 796119-63-6, 0.150 g, 0.728 mmol) was dissolved in DMF (4 mL). Cesium carbonate (0.711 g, 2.18 mmol) and bromocyclobutane (0.685 mL, 7.28 mmol) were added and the solution was stirred for 4 hours at 100°C. The reaction mixture was quenched with water and the aqueous layer was extracted 2 times with ethyl acetate. The organic phases were washed 4 times with water then with brine, dried over Na₂SO₄, filtered and evaporated to afford cyclobutyl 3-(cyclobutoxy)-5-(trifluoromethyl)benzoate. ¹H-NMR (400 MHz, CDCI₃, ppm) *δ*= 1.68 - 1.82 (m, 2 H), 1.84 - 1.98 (m, 2 H), 2.13 - 2.31 (m, 4 H), 2.43 - 2.57 (m, 4 H), 4.74 (t, J=7.15 Hz, 1 H), 5.24 (dd, J=8.07, 6.97 Hz, 1 H), 7.24 (t, J=1.65 Hz, 1 H), 7.64 (d, J=2.20 Hz, 1 H), 7.86 (s, 1 H).

LC-MS (method 1): Rt 1.33 min , m/z = 315 (M+H+).

### Step B: Preparation of 3-(cyclobutoxy)-5-(trifluoromethyl)benzoic acid (I25)

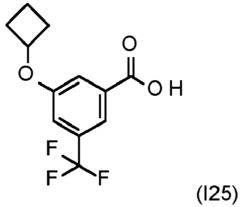

Cyclobutyl 3-(cyclobutoxy)-5-(trifluoromethyl)benzoate (0.204 g, 0.649 mmol) was dissolved in THF (2 mL) and water (1 mL). Lithium hydroxide monohydrate (0.041g, 0.974 mmol) was added and the resulting mixture was stirred overnight at 60°C. The reaction mixture was quenched with water and the aqueous layer was extracted with ethyl acetate. Then the aqueous layer was acidified with HCl 2 M solution until pH 2 and extracted again with ethyl acetate 2 times. The organic layers were washed with water, dried over Na₂SO₄ , filtered and evaporated to afford 3-(cyclobutoxy)-5-(trifluoromethyl)benzoic acid.

LC-MS (method 1): Rt 1.03 min , m/z = 261 (M+H+).

### Step C: Preparation of 3-(cyclobutoxy)-N-(cyclopropylmethyl)-N-[1-(2-pyrimidin-2-yl-1,2,4-triazol-3-yl)ethyl1-5-(trifluoromethyl)benzamide (Compound P48)

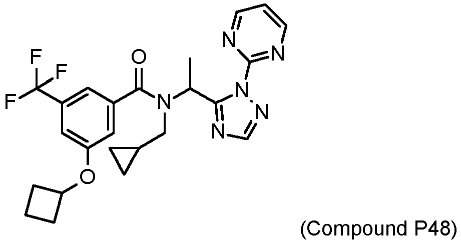

Compound P48 was prepared in using 3-(cyclobutoxy)-5-(trifluoromethyl)benzoic acid and the conditions described for Compound P49 (Step C)

LC-MS (method 1): Rt 1.08 , m/z = 487 (M+H+).

### Example 7: Preparation of 3-(cyclopropoxy)-N-(cyclopropylmethyl)-N-[1-(2-pyrimidin-2-yl-1,2,4-triazol-3-yl)ethyl]-5-(trifluoromethyl)benzamide (Compound P51)

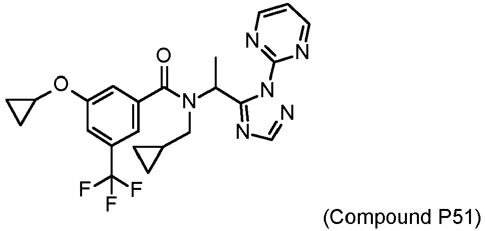

Compound P51 was prepared in using the conditions described for compound P48 to afford 3-(cyclopropoxy)-N-(cyclopropylmethyl)-N-[1-(2-pyrimidin-2-yl-1,2,4-triazol-3-yl)ethyl]-5-(trifluoromethyl)benzamide.

LC-MS (method 1): Rt 1.02 min , m/z = 473 (M+H+).

### Example 8: Preparation of 3-(1-cyano-1-methyl-ethoxy)-N-[1-(2-pyrimidin-2-yl-1 ,2,4-triazol-3-yl)ethyl]-5-(trifluoromethyl)benzamide (Compound P56)

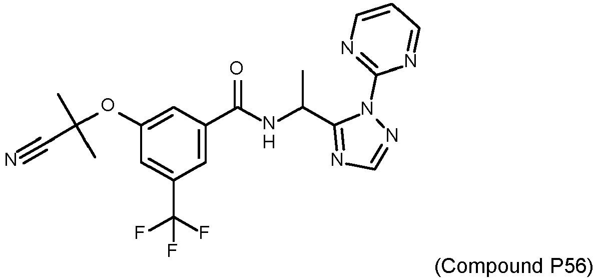

### Step A: Preparation of methyl 3-(2-amino-1,1-dimethyl-2-oxo-ethoxy)-5-(trifluoromethyl)benzoate

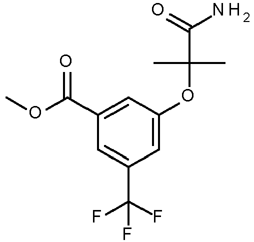

Methyl-3-hydroxy-5-(trifluoromethyl)benzoate (0.50 g, 2.2 mmol) was dissolved in acetonitrile (11 mL). Then cesium carbonate (1.1 g, 3.3 mmol) was added. The resulting suspension was stirred 5 min and 2-bromo-2-methyl-propanamide (0.59 g, 3.6 mmol) was added. Reaction mixture was stirred overnight at 70°C. The reaction mixture was evaporated and water and ethyl acetate were added. The organic phase was separated and washed with brine, dried over Na₂SO₄, filtered and evaporated to afford methyl 3-(2-amino-1,1-dimethyl-2-oxo-ethoxy)-5-(trifluoromethyl)benzoate.

LC-MS (method 1): Rt 0.89 min , m/z = 306 (M+H+).

### Step B: Preparation of methyl 3-(1-cyano-1-methyl-ethoxy)-5-(trifluoromethyl)benzoate

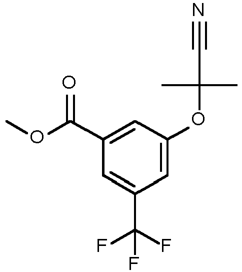

Methyl-3-(2-amino-1,1-dimethyl-2-oxo-ethoxy)-5-(trifluoromethyl)benzoate (0.72 g, 2.4 mmol) was suspended in dichloromethane (24 mL) and triethylamine (.3 mL, 9.4 mmol) was added. The mixture was cooled down at 0°C and trifluoroacetic anhydride (0.99 mL, 7.1 mmol) was added dropwise. The resulting yellow solution was stirred at room temperature overnight. The reaction mixture was carefully quenched with methanol and then with NaHCOs (gas formed) and then the aqueous layer was extracted 2 times with dichloromethane. The combined organic layers were dried over Na₂SO₄, filtered and evaporated. The crude residue was purified by chromatography over silica gel to afford methyl 3-(1 -cyano-1 -methyl-ethoxy)-5-(trifluoromethyl) benzoate.

¹H-NMR (400 MHz, CDCI₃, ppm) *δ*= 1.80 (s, 6 H), 3.99 (s, 3 H), 7.63 (t, J=1.65 Hz, 1 H), 8.05 (d, J=1.47 Hz, 1 H), 8.10 - 8.16 (m, 1 H).

LC-MS (method 1): Rt 1.09 min , m/z = 286 (M+H+).

### Step C: Preparation of 3-(1-cyano-1-methyl-ethoxy)-5-(trifluoromethyl)benzoic acid (I30)

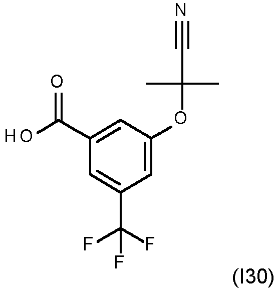

Methyl-3-(1-cyano-1-methyl-ethoxy)-5-(trifluoromethyl)benzoate (0.36 g, 1.3 mmol) was dissolved in THF (5.4 mL) and water (1.8 mL), then lithium hydroxide monohydrate (0.11 g, 2.5 mmol) was added and the mixture was stirred at room temperature for 3 hours. THF was evaporated, then water (2 mL) was added and the mixture was acidified with hydrochloric acid (2.0 mol/L) to pH 5. Ethyl acetate and water were added and the organic phase was separated, dried over Na₂SO₄, filtered and concentrated under vacuum to afford 3-(1-cyano-1-methyl-ethoxy)-5-(trifluoromethyl)benzoic.

LC-MS (method 1): Rt 0.92 min , m/z = 272 (M-H+).

### Step D: Preparation of 3-(1-cyano-1-methyl-ethoxy)-N-f1-(2-pyrimidin-2-yl-1,2,4-triazol-3-yl)ethyll-5-(trifluoromethyl)benzamide (Compound P56)

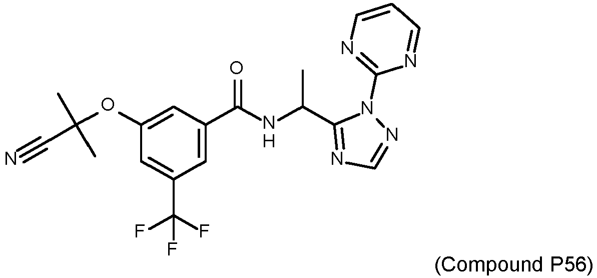

Compound P56 was prepared in using 3-(1-cyano-1-methyl-ethoxy)-5-(trifluoromethyl)benzoic acid and the conditions described for Compound P49 (Step C)

¹H-NMR (400 MHz, DMSO, ppm) *δ*=1.65 (d, J=6.97Hz, 3 H) ,1.74 (s, 6 H), 6.01 (t, J=6.97 Hz, 1 H), 7.55 - 7.70 (m, 2 H), 7.84 (s, 1 H), 8.00 (s, 1 H), 8.18 (s, 1 H), 8.99 (d, J=5.14 Hz, 2 H), 9.39 (d, J=7.34 Hz, 1 H).

LC-MS (method 1): Rt 0.89 min , m/z = 446 (M+H+).

### Example 9: Preparation of 3,5-bis(cyclopropoxy)-N-(cyclopropylmethyl)-N-[1-(2-pyrimidin-2-yl-1,2,4-triazol-3-yl)ethyl]benzamide (Compound P60)

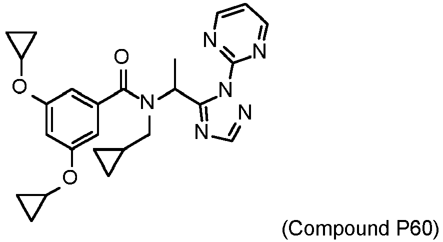

### Step A: Preparation of methyl 3,5-bis(cyclopropoxy)benzoate

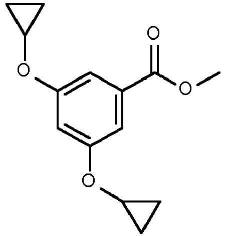

In a vial, under argon, methyl 3,5-dihydroxybenzoate (CAS: 2150-44-9, 0.100 g, 0.595 mmol) was dissolved in DMF (3 mL). Cesium carbonate ( 0.585 g, 1.78 mmol) and bromocyclopropane ( 0.476 mL, 5.95 mmol) were added and the vial was closed and stirred for 1 hour at 200°C under microwaves system. The reaction mixture was quenched with water and the aqueous layer was extracted 2 times with ethyl acetate. The organic phases were washed 4 times with water then with brine, dried over Na₂SO₄, filtered and evaporated to afford methyl 3,5-bis(cyclopropoxy)benzoate

LC-MS (method 1): Rt 1.09 min , m/z = 249 (M+H+).

### Step B: Preparation of 3,5-bis(cyclopropoxy)benzoic acid (I31)

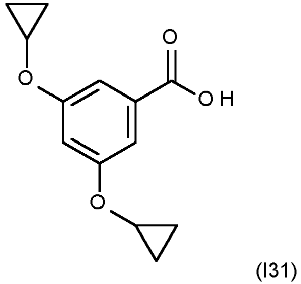

Methyl-3,5-bis(cyclopropoxy)benzoate (0.111 g, 0.447 mmol) was dissolved in THF (1.5 mL) and water (1 mL). Lithium hydroxide monohydrate (0.018g, 0.447 mmol) was added and the resulting mixture was stirred overnight at room temperature. The reaction mixture was quenched with water and the aqueous layer was extracted with ethyl acetate. Then the aqueous layer was acidified with HCl (2 M) until pH 2 and extracted again with ethyl acetate (2x) . The organic layers were washed with water, dried over Na₂SO₄ , filtered and evaporated to afford 3,5-bis(cyclopropoxy)benzoic acid.

LC-MS (method 1): Rt 0.91min , m/z = 233 (M-H+).

### Step C: Preparation of 3,5-bis(cyclopropoxy)-N-(cyclopropylmethyl)-N-[1-(2-pyrimidin-2-yl-1,2,4-triazol-3-yl)ethyl1benzamide (Compound P60)

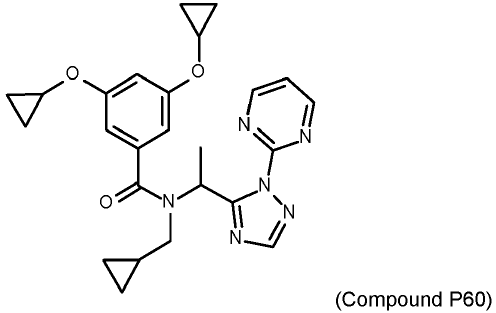

Compound P60 was prepared in using 3,5-bis(cyclopropoxy)benzoic acid and the conditions described for Compound P49 (Step C)

LC-MS (method 1): Rt 0.99 min , m/z = 461 (M+H+).

### Example 10: Preparation of 3-cyclopropyl-5-(2,2-difluoroethoxy)-N-[1-(2-pyrimidin-2-yl-1 ,2,4-triazol-3-yl)ethyl]benzamide (Compound P61)

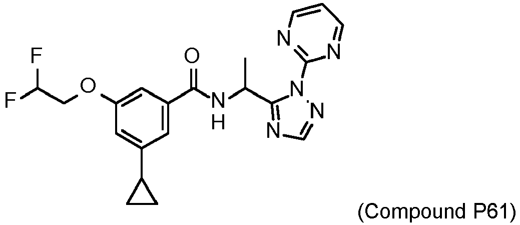

### Step A: Preparation of methyl 3-bromo-5-(2,2-difluoroethoxy)benzoate

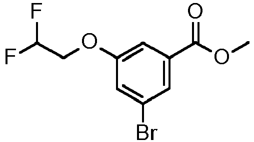

Methyl-3-bromo-5-hydroxy-benzoate (CAS: 192810-12-1, 0.200 g, 0.866 mmol) was charged in a vial and dissolved in acetonitrile (2 mL) and cesium carbonate (0.570 mg, 1.73 mmol) added. The resulting yellow mixture was purged with Argon, 2,2-difluoroethyl trifluoromethanesulfonate (0.195 mg, 0.909 mmol) was added and the solution was stirred 1 hour at room temperature. The mixture was poured into water, extracted (2x) with ethyl acetate. Combined organic layers were washed with brine, dried over Na₂SO₄, filtered and evaporated to afford methyl 3-bromo-5-(2,2-difluoroethoxy)benzoate. ¹H-NMR (400 MHz, CDCI₃, ppm) δ 3.94 (s, 3 H) 4.24 (td, J=12.84, 4.03 Hz, 2 H) 5.94 - 6.30 (m, 1 H) 7.30 (dd, J=2.57, 1.83 Hz, 1 H) 7.52 (dd, J=2.38, 1.28 Hz, 1 H) 7.85 (t, J=1.47 Hz, 1 H)

LC-MS (method 1): Rt 1.07 min , m/z = 293 (M-H+).

### Step B: Preparation of methyl 3-cyclopropyl-5-(2,2-difluoroethoxy)benzoate

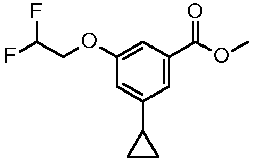

Methyl-3-bromo-5-(2,2-difluoroethoxy)benzoate (0.255 g, 0.864 mmol) was charged in a vial and dissolved in 1 ,4-Dioxane (2 mL) to get a yellow clear solution. The solution was purged with Argon followed by the addition of cyclopropylboronic acid (0.155 g, 1.73 mmol,) sodium carbonate (0.275 g 2.59 mmol) , water (0.8 mL) and PdCl₂(dppf) (0.035 g, 0.0432 mmol)

The orange solution was heated to 100°C and stirred for 2 hours. Water was added to the mixture and extraction was carried out with ethyl acetate (3x). The combined organic layers were dried over Na₂SO₄, filtered through a sintered disc filter funnel and concentrated under reduced pressure at 40°C. The crude residue was purified by chromatography over silica gel to afford methyl 3-cyclopropyl-5-(2,2-difluoroethoxy)benzoate.
¹H-NMR (400 MHz, CDCI₃, ppm) δ 0.70 - 0.79 (m, 2 H) 0.97 - 1.07 (m, 2 H) 1.88 - 1.99 (m, 1 H) 3.92 (s, 3 H) 4.23 (td, J=13.11, 4.22 Hz, 2 H) 5.92 - 6.29 (m, 1 H) 6.84 - 6.90 (m, 1 H) 7.35 (dd, J=2.38, 1.28 Hz, 1 H) 7.44 (t, J=1.47 Hz, 1 H)
LC-MS (method 1): Rt 1.07 min , m/z = 257 (M+H+).

### Step C: Preparation of 3-cyclopropyl-5-(2,2-difluoroethoxy)benzoic acid (I32)

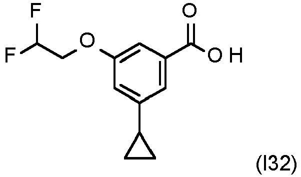

Methyl 3-cyclopropyl-5-(2,2-difluoroethoxy)benzoate (0.128 g, 0.500 mmol) was charged in a flask and dissolved in tetrahydrofuran (1.5 mL) and water (1 mL) to give a colorless solution.

To this solution was added hydroxylithium monohydrate (0.024 mg, 0.999 mmol) and the resulting mixture was stirred at room temperature overnight. The reaction solution was acidified with 1M HCl, and then extracted with ethyl acetate (2x). The combined organic phases were dried over Na₂SO₄, filtered and then concentrated to afford 3-cyclopropyl-5-(2,2-difluoroethoxy)benzoic acid.
¹H-NMR (400 MHz, DMSO-d6, ppm) δ 0.70 - 0.77 (m, 2 H) 0.94 - 1.00 (m, 2 H) 1.95 - 2.06 (m, 1 H) 4.36 (td, J=14.76, 3.48 Hz, 2 H) 6.21 - 6.54 (m, 1 H) 6.91 - 6.97 (m, 1 H) 7.26 (dd, J=2.38, 1.28 Hz, 1 H) 7.31 (t, J=1.28 Hz, 1 H) 11.73 - 13.25 (m, 1 H)
LC-MS (method 1): Rt 0.91 min , m/z = 241 (M-H+).

### Step D: Preparation of 3-cyclopropyl-5-(2,2-difluoroethoxy)-N-[1-(2-pyrimidin-2-yl-1,2,4-triazol-3-yl)ethyl]benzamide (Compound P61)

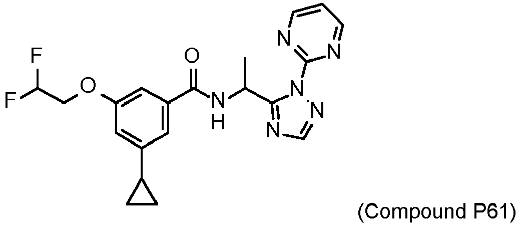

1-(2-pyrimidin-2-yl-1,2,4-triazol-3-yl)ethanamine;hydrochloride (0.110 g, 0.485 mmol) was dissolved in N, N-dimethylformamide (1.5 mL). N-ethyl-N-isopropyl-propan-2-amine (0.249 mL, 1.46 mmol) was added and the resultin mixture was stirred for 5 min at room temperature. Then 3-cyclopropyl-5-(2,2-difluoroethoxy)benzoic acid (0.129 g, 0.534 mmol) was added followed by HATU (0.277 mg, 0.728 mmol) to get a brown solution which was stirred overnight at RT. The reaction mixture was poured into NH₄Cl and extracted with ethyl acetate. The combined organic phases were washed with water, dried over Na₂SO₄, filtered and concentrated to get crude. The crude was purified by chromatography two times to afford 3-cyclopropyl-5-(2,2-difluoroethoxy)-N-[1-(2-pyrimidin-2-yl-1,2,4-triazol-3-yl)ethyl]benzamide.
¹H-NMR (400 MHz, CDCl₃, ppm) δ 0.66 - 0.78 (m, 2 H) 0.87 - 1.12 (m, 2 H) 1.69 (d, J=6.97 Hz, 3 H) 1.91 (tt, J=8.34, 5.04 Hz, 1 H) 4.09 - 4.24 (m, 2 H) 5.90 - 6.24 (m, 1 H) 6.41 - 6.49 (m, 1 H) 6.73 - 6.77 (m, 1 H) 7.13 - 7.19 (m, 2 H) 7.40 (t, J=4.95 Hz, 1 H) 7.43 - 7.51 (m, 1 H) 8.05 (s, 1 H) 8.91 (d, J=4.77 Hz, 2 H)
LC-MS (method 1): Rt 0.88 min , m/z = 415 (M+H+).
m. p:128-132°C

### Example 11: Preparation of N-[1-[2-(5-bromo-2-pyridyl)-1,2,4-triazol-3-yl]ethyl]-3-cyclopropyl-5-(trifluoromethyl)benzamide (Compound P74)

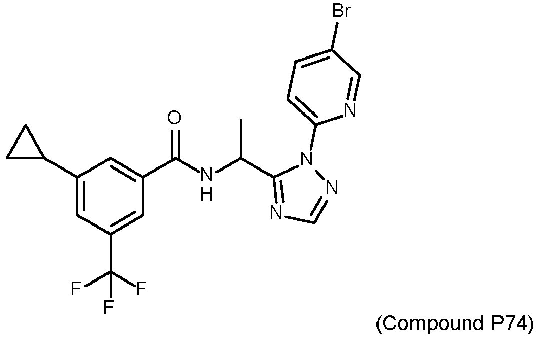

### Step A: Preparation of N-(2-amino-1-methyl-2-oxo-ethyl)-3-cyclopropyl-5-(trifluoromethyl)benzamide

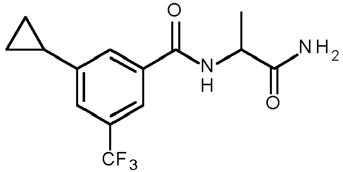

To a stirred solution of 3-cyclopropyl-5-(trifluoromethyl)benzoic acid (300 mg, 1.17 mmol) in ethyl acetate (4.0 mL) was added 2-aminopropanamide (287 mg, 2.93 mmol) , followed by T3P (1.49 g, 2.35 mmol) and DIPEA (0.615 mL, 3.52 mmol) and stirred at room temperature for 16 h. The reaction mixture was diluted with water (100 mL) and extracted with dichloromethane (2 x 150 mL). The combined organic layers were washed with brine (30 mL), dried over Na₂SO₄ and concentrated under reduced pressure. The crude material was purified by column chromatography over silica gel to afford N-(2-amino-1-methyl-2-oxo-ethyl)-3-cyclopropyl-5-(trifluoromethyl)benzamide as a white solid. ¹H-NMR (400 MHz, DMSO-d6, ppm): δ 8.71(d, J = 7.6 Hz, 1H), 7.98 (s, 1H), 7.81 (s, 1H), 7.62 (s, 1H), 7.40 (s, 1H), 7.01 (s, 1H) 4.42 (m, 1 H), 2.11 (m, 1H), 1.33 (d, J = 7.2 Hz, 3H), 1.06 -1.04 (m, 2H), 1.03 - 0.83 (m, 2H).

### Step B: Preparation of 3-cyclopropyl-N-[2-[(E)-dimethylaminomethyleneamino]-1-methyl-2-oxo-ethyl]-5-(trifluoromethyl)benzamide

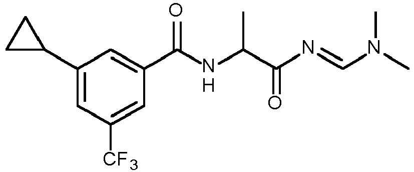

To a stirred solution of N-(2-amino-1-methyl-2-oxo-ethyl)-3-cyclopropyl-5-(trifluoromethyl)benzamide (50 mg, 0.1 mmol) in dichloromethane (2.0 mL) was added 1,1-dimethoxy-N,N-dimethyl-methanamine (21.4 mg, 0.180 mmol) and stirred at 50 °C for 2 h. The reaction mixture was concentrated under reduced pressure to afford 3-cyclopropyl-N-[2-[(E)-dimethylaminomethyleneamino]-1-methyl-2-oxoethyl]-5-(trifluoromethyl)benzamide (50 mg, crude weight) as a brown liquid. The crude compound was used in the next step without further purification.

### Step C: Preparation of N-[1-[2-(5-bromo-2-pyridyl)-1,2,4-triazol-3-yl1ethyl1-3-cyclopropyl-5-(trifluoromethyl)benzamide (Compound P74)

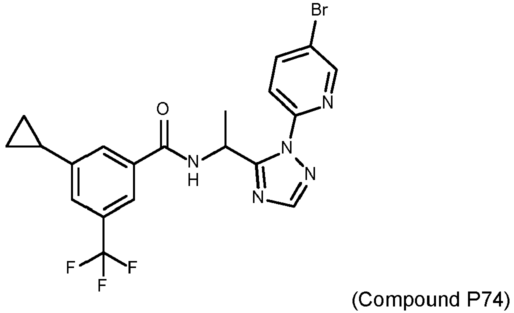

To a stirred solution of 3-cyclopropyl-N-[2-[(E)-dimethylaminomethyleneamino]-1-methyl-2-oxo-ethyl]-5-(trifluoromethyl)benzamide (300 mg, 0.760 mmol) in 1,4-Dioxane (5 mL) was added (5-bromo-2-pyridyl)hydrazine (214 mg, 1.14 mmol) and AcOH (3 mL) and the resulting mixture was stirred at 90 °C for 2 h. The reaction mixture was cooled to room temperature, diluted with water (30 mL) and extracted with dichloromethane (2 x 150 mL).The combined organic layers were washed with brine (30 mL), dried over Na₂SO₄ and concentrated under reduced pressure. The crude material was purified by column chromatography over silica gel to N-[1-[2-(5-bromo-2-pyridyl)-1,2,4-triazol-3-yl]ethyl]-3-cyclopropyl-5-(trifluoromethyl)benzamide.

¹H-NMR (400 MHz, DMSO-d6, ppm): δ 9.20 (d, J = 6.8 Hz, 1H), 8.68 (d, J = 2.4 Hz, 1H), 8.30 (dd, J = 6.4 Hz, 1H), 8.16 (s, 1H), 7.81 (m, 2H), 7.62 (d, J = 19.2 Hz, 2H), 5.99 - 5.92 (m, 1H), 2.12-2.06 (m, 1H), 1.62 (d, J = 7.2 Hz, 3H), 1.05 - 1.01 (m, 2H), 1.01 - 0.78 (m, 2H).

LC-MS (method 4): Rt 5.24 min , m/z = 482 (M+H+).

### Example 12: Preparation of N-[1-[2-(5-bromopyrimidin-2-yl)-5-methyl-1,2,4-triazol-3-yl]ethyl]-3-(1-cyano-1-methyl-ethyl)-5-(trifluoromethyl)benzamide (Compound P77)

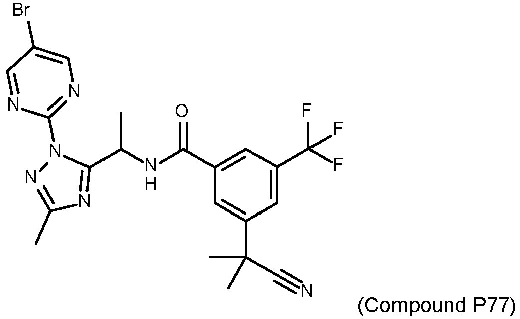

### Step A: Preparation of N-(2-amino-1-methyl-2-oxo-ethyl)-3-(1-cyano-1-methyl-ethyl)-5-(trifluoromethyl)benzamide

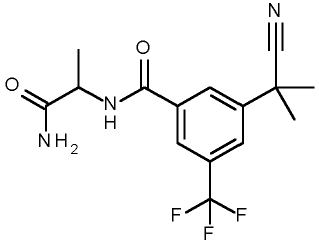

To a stirred solution of 2-aminopropanamide (500 mg, 5.11 mmol) in DMF (25 mL) at 0 °C was added 3-(1-cyano-1-methyl-ethyl)-5-(trifluoromethyl)benzoic acid (1.32 g, 5.13 mmol), DIPEA (2.7 mL, 15.3 mmol) followed by EDC.HCl (1.95 g, 10.2 mmol) , 1-Hydroxybenzotriazole (1.38 g, 10.2 mmol) and reaction mixture stirred at room temperature for 2 h. The reaction mixture was diluted with H₂O (50 mL), washed with saturated aqueous sodium bicarbonate solution (100 mL) and extracted with EtOAc (2 x 70 mL). The combined organic layers were washed with brine (100 mL), dried over Na₂SO₄ and concentrated under reduced pressure. The crude mixture was purified by column chromatography over silica gel to afford N-(2-amino-1-methyl-2-oxo-ethyl)-3-(1-cyano-1-methyl-ethyl)-5-(trifluoromethyl)benzamide.

¹H-NMR (400 MHz, DMSO-d6, ppm): δ 8.90 (d, J = 7.6 Hz, 1H), 8.29 (d, J = 16.8Hz, 2H), 7.99 (s, 1H), 7.47 (s, 1H), 7.03 (s, 1H), 4.47 - 4.43 (t, 1H), 1.78 (s, 6H), 1.35 (d, J = 7.2 Hz, 3H).

### Step B: Preparation of 3-(1-cyano-1-methyl-ethyl)-N-[2-[(E)-1-(dimethylamino)ethylideneamino]-1-methyl-2-oxo-ethyl1-5-(trifluoromethyl) benzamide

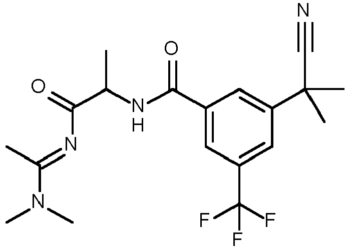

To a stirred solution of N-(2-amino-1-methyl-2-oxo-ethyl)-3-(1-cyano-1-methyl-ethyl)-5-(trifluoromethyl)benzamide (500 mg, 1.45 mmol) in dichloromethane (20 mL) was added N,N-dimethylacetamide dimethyl acetal (0.319 mL, 2.18 mmol) and the reaction mixture was stirred at 50°C for 1 h. The reaction mixture was concentrated under reduced pressure to afford 3-(1-cyano-1-methyl-ethyl)-N-[2-[(E)-1-(dimethylamino)ethylideneamino]-1-methyl-2-oxo-ethyl]-5-(trifluoromethyl)benzamide as colourless liquid. The crude compound was used in the next step without further purification.

### Step C: Preparation of N-[1-[2-(5-bromopyrimidin-2-yl)-5-methyl-1,2,4-triazol-3-yl1ethyl1-3-(1-cyano-1-methyl-ethyl)-5-(trifluoromethyl)benzamide (Compound P77)

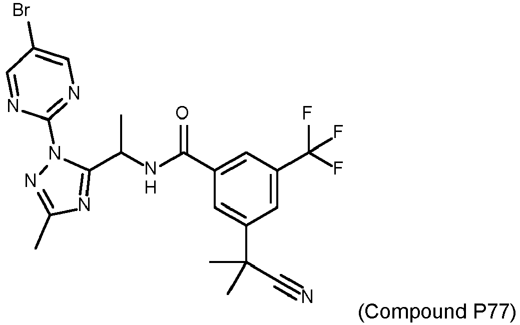

To a stirred solution of 3-(1-cyano-1-methyl-ethyl)-N-[2-[(E)-1-(dimethylamino)ethylideneamino]-1-methyl-2-oxo-ethyl]-5-(trifluoromethyl)benzamide (250 mg, 0.568 mmol) in 1,4-dioxane (5 mL) and AcOH (5 mL) was added (5-bromopyrimidin-2-yl)hydrazine (161 mg, 0.851 mmol) and the resulting mixture was stirred at 80 °C for 2 h. The reaction mixture was allowed to reach room temperature, diluted with H₂O (50 mL), washed with saturated aqueous sodium bicarbonate solution (100 mL) and extracted with ethyl acetate (2 x 70 mL). The combined organic layers were washed with brine (100 mL), dried over Na₂SO₄ and concentrated under reduced pressure. The crude residue was purified by column chromatography over silica gel to afford N-[1-[2-(5-bromopyrimidin-2-yl)-5-methyl-1,2,4-triazol-3-yl]ethyl]-3-(1-cyano-1-methyl-ethyl)-5-(trifluoromethyl)benzamide as a white solid. ¹H-NMR (400 MHz, DMSO-d6, ppm): δ 9.40 (d, J = 7.2 Hz, 1H), 9.13 (s, 2H), 8.17 (d, J = 22.0 Hz, 2H), 7.98 (s, 1H), 6.0 - 5.96 (t, 1H), 2.33 (s, 3H), 1.75 (s, 6H), 1.63 (d, J = 6.8 Hz, 3H).

LC-MS (method 4): Rt 4.78 min , m/z = 522 (M+H+).

### Example 13: Preparation of N-[1-(2-pyrimidin-2-yl-1 ,2,4-triazol-3-yl)ethyl]-3-(trifluoromethyl)-5-[2-(trifluoromethyl)phenyl]benzamide (Compound P69)

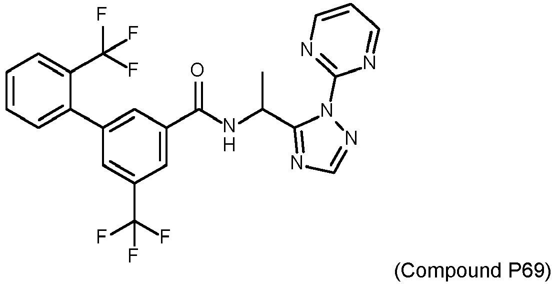

### Step A: Preparation of methyl 3-(trifluoromethyl)-5-[2-(trifluoromethyl)phenyl]benzoate

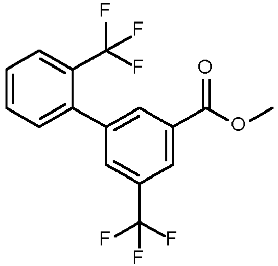

In a round bottom flask, methyl-3-iodo-5-(trifluoromethyl)benzoate (0.400 g, 1.15 mmol) was dissolved in tetrahydrofuran (9.21 mL), 2-(trifluoromethyl)phenylboronic acid (0.338 g, 1.73 mmol) and potassium carbonate (1.4 mL, 2.0 mol/L) were added. The resulting mixture was degassed under Argon for 5 min, tetrakis(triphenylphosphine)palladium(0) (0.0672 g, 0.058 mmol) was added before the vial was sealed.The reaction was heated at 80°C for 18 h. The mixture was diluted with ethyl acetate and filtered though a filter paper, diluted with water and extracted with ethyl acetate (2x 20 ml). The combined organic layers were dried over Na₂SO₄, filtered and evaporated. The crude was purified by chromatography over silica gel to afford methyl 3-(trifluoromethyl)-5-[2-(trifluoromethyl)phenyl]benzoate.
¹H-NMR (400 MHz, CDCl₃, ppm) δ 8.4 (s, 1 H) 8.2 (s, 1 H) 7.8 - 7.9 (m, 2 H) 7.6 - 7.7 (m, 1 H) 7.6 (d, *J*=7.70 Hz, 1 H) 7.3 - 7.4 (m, 1 H) 4.0 (s, 3 H)
GCMS (method 3): Rt 3.30 min , m/z = 349 (M+H+).

### Step B: Preparation of 3-(trifluoromethyl)-5-[2-(trifluoromethyl)phenyl1benzoic acid (139)

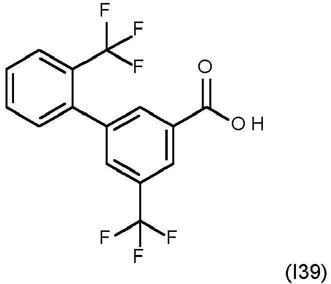

3-(trifluoromethyl)-5-[2-(trifluoromethyl)phenyl]benzoic acid was prepared in using 3-(trifluoromethyl)-5-[2-(trifluoromethyl)phenyl]benzoate and the conditions described for Compound P60 (Step B) ¹H-NMR (400 MHz, DMSO-d6, ppm) δ 7.53 - 7.57 (m, 1 H) 7.68 - 7.74 (m, 1 H) 7.77 - 7.84 (m, 1 H) 7.89 - 7.93 (m, 1 H) 7.95 - 7.99 (m, 1 H) 8.11 - 8.14 (m, 1 H) 8.24 - 8.27 (m, 1 H) 13.71 (br s, 1 H). LC-MS (method 1): Rt 1.09 min , m/z = 333 (M-H+).

### Step C: Preparation of N-[1-(2-pyrimidin-2-yl-1,2,4-triazol-3-yl)ethyl1-3-(trifluoromethyl)-5-[2-(trifluoromethyl)phenyl]benzamide (Compound P69)

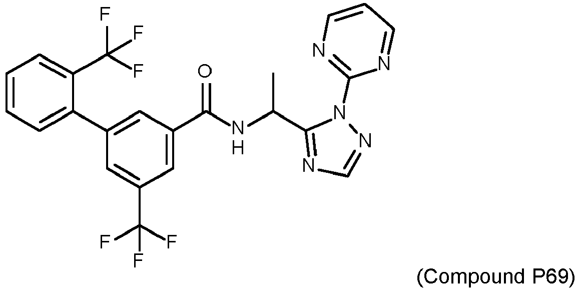

Compound P69 was prepared in using 3-(trifluoromethyl)-5-[2-(trifluoromethyl)phenyl]benzoic acid and the conditions described for Compound P24 (Step C)

¹H-NMR (400 MHz, CDCl₃, ppm) δ 1.73 (d, J=6.60 Hz, 3 H) 6.43 - 6.53 (m, 1 H) 7.35 - 7.39 (m, 1 H) 7.40 - 7.44 (m, 1 H) 7.47 - 7.54 (m, 1 H) 7.52 (br d, J=8.07 Hz, 1 H) 7.55 - 7.61 (m, 1 H) 7.62 - 7.68 (m, 1 H) 7.77 (s, 1 H) 7.82 (d, J=7.34 Hz, 1 H) 7.97 (s, 1 H) 8.05 - 8.08 (m, 1 H) 8.15 (s, 1 H) 8.94 (d, J=4.77 Hz, 2 H).

LC-MS (method 1): Rt 1.07 min , m/z = 507 (M+H+).

### Example 14: Preparation of 2-(3-chloropyrazol-1-yl)-N-[1-(2-pyrimidin-2-yl-1,2,4-triazol-3-yl)ethyl]-6-(trifluoromethyl)pyridine-4-carboxamide (Compound P70)

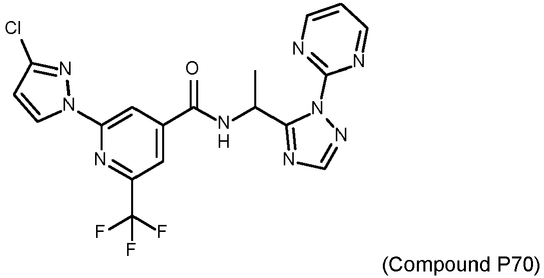

### Step A: Preparation of methyl 2-(3-chloropyrazol-1-yl)-6-(trifluoromethyl)pyridine-4-carboxylate

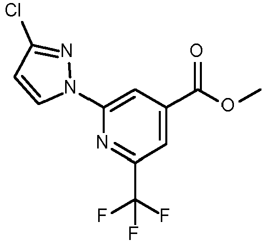

Methyl-2-chloro-6-(trifluoromethyl)pyridine-4-carboxylate (143 mg, 0.59 mmol) was dissolved in 1,4-dioxane (6 mL) and the resulting solution was flushed with argon for 10 min. Then 3-chloro-1H-pyrazole (77 mg, 0.72 mmol), potassium phosphate (191 mg, 0.90 mmol) and [tBuXPhosPd(allyl)]OTf (44 mg, 0.06 mmol) were added and the reaction was stirred for 18 h at 80°C. The mixture was diluted with water and the aqueous layer was extracted with ethyl acetate (2x). The organic layers were combined, dried over Na₂SO₄, filtered and evaporated.The crude residue was purified by chromatography over silica gel (-cyclohexane + 0-20 % ethyl acetate) to afford methyl 2-(3-chloropyrazol-1-yl)-6-(trifluoromethyl)pyridine-4-carboxylate.
¹H-NMR (400 MHz, CDCl₃, ppm) δ 8.7 (s, 1 H) 8.6 (d, J=2.57 Hz, 1 H) 8.1 (d, J=1.10 Hz, 1 H) 6.5 (d, J=2.57 Hz, 1 H) 4.1 (s, 3 H)
LC-MS (method 1): Rt 1.14 min , m/z = 306 (M+H+).

### Step B: Preparation of 2-(3-chloropyrazol-1-yl)-6-(trifluoromethyl)pyridine-4-carboxylic acid (I40)

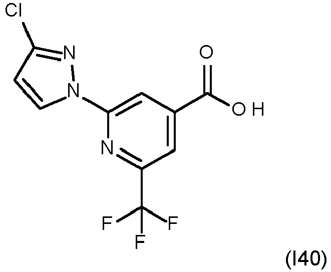

2-(3-chloropyrazol-1-yl)-6-(trifluoromethyl)pyridine-4-carboxylic acid was prepared in using methyl 2-(3-chloropyrazol-1-yl)-6-(trifluoromethyl)pyridine-4-carboxylate and the conditions described for Compound P60 (Step B)
¹H-NMR (400 MHz, DMSO-d6, ppm) δ 13.9 - 15.0 (m, 1 H) 8.7 (d, J=2.57 Hz, 1 H) 8.4 (s, 1 H) 8.1 (d, J=1.10 Hz, 1 H) 6.8 (d, J=2.57 Hz, 1 H)
LC-MS (method 1): Rt 0.96 min , m/z = 292 (M+H+).

### Step C: Preparation of 2-(3-chloropyrazol-1-yl)-N-[1-(2-pyrimidin-2-yl-1,2,4-triazol-3-yl)ethyl1-6-(trifluoromethyl)pyridine-4-carboxamide (Compound P70)

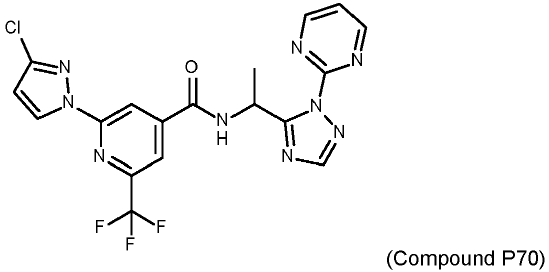

Compound P70 was prepared in using 2-(3-chloropyrazol-1-yl)-6-(trifluoromethyl)pyridine-4-carboxylic acid and the conditions described for Compound P24 (Step C)
¹H-NMR (400 MHz, CDCl₃, ppm) δ 1.75 (d, J=6.6 Hz, 3 H) 6.43 - 6.53 (m, 2 H) 7.46 (t, J=4.8 Hz, 1 H) 7.86 - 7.93 (m, 1 H) 8.02 (d, J=1.1 Hz, 1 H) 8.09 (s, 1 H) 8.45 (d, J=0.7 Hz, 1 H) 8.60 (d, J=2.9 Hz, 1 H) 8.90 - 9.07 (m, 2 H)
LC-MS (method 1): Rt 0.97 min , m/z = 464 (M+H+).

### Example 15: Preparation of 2-cyclopropyl-N-[1-(2-pyrimidin-2-yl-1,2,4-triazol-3-yl)ethyl]-6-(trifluoromethyl)pyridine-4-carboxamide (Compound P54)

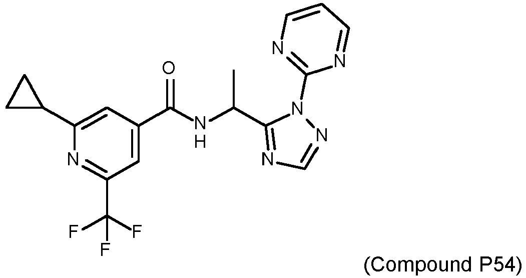

### Step A: Preparation of methyl 2-cyclopropyl-6-(trifluoromethyl)pyridine-4-carboxylate and 2-cyclopropyl-6-(trifluoromethyl)pyridine-4-carboxylic acid (I29)

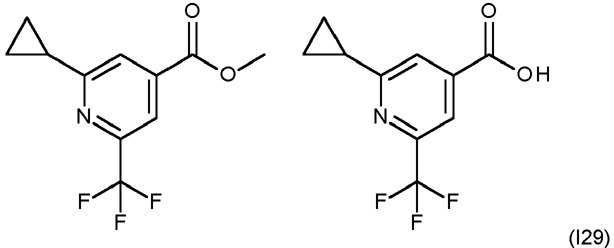

Cyclopropylboronic acid (1.43 g, 16.7 mmol, 2.00 equiv.) and sodium hydrogenocarbonate (2.10 g, 25.1 mmol,) were added to a solution of methyl 2-chloro-6-(trifluoromethyl)pyridine-4-carboxylate (2.00 g, 8.35 mmol) in 1,4-dioxane (20.9 mL) and water (8.35 mL), and the resulting suspension was flushed with argon for 10 min. [1,1'-bis(diphenylphosphino)ferrocene] dichloropalladium (II) (0.322 g, 0.417 mmol) was added and the resulting suspension was stirred at 100 °C for 1 hour under argon. After cooling down to room temperature, the reaction mixture was quenched with water and extracted (2x) with ethyl acetate. The combined organic phases were dried over Na₂SO₄, filtered and evaporated to give of first crude material, which gave after purification by flash chromatography over silica gel (ethyl acetate in cyclohexane) the desired methyl 2-cyclopropyl-6-(trifluoromethyl)pyridine-4-carboxylate. ¹H-NMR (400 MHz, CDCl₃, ppm) δ : 1.04 - 1.23 (m, 4 H), 2.14 - 2.28 (m, 1 H), 4.00 (s, 3 H), 7.88 (s, 1 H), 7.95 (d, J=1.47 Hz, 1 H).

LC-MS (method 1): Rt 1.12 min, m/z 246 [M+H⁺].

After acidification to pH 1, the aqueous layer was extracted again (2x) with ethyl acetate, the combined organic phases were dried over Na₂SO₄, filtered and evaporated to give a second crude material, which upon purification by flash chromatography over silica gel (methanol in dichloromethane) afforded 2-cyclopropyl-6-(trifluoromethyl)pyridine-4-carboxylic acid.

¹H-NMR (400 MHz, DMSO-d6, ppm) δ : 0.94 - 1.03 (m, 2 H), 1.06 - 1.15 (m, 2 H), 2.37 - 2.46 (m, 1 H), 7.88 (d, J=1.10 Hz, 1 H), 8.05 (d, J=0.73 Hz, 1 H), 13.89 - 14.33 (m, 1 H).

LC-MS (method 1): Rt 0.94 min, m/z 232 [M+H⁺].

### Step B: Preparation of 2-cyclopropyl-N-[1-(2-pyrimidin-2-yl-1,2,4-triazol-3-yl)ethyl]-6-(trifluoromethyl)pyridine-4-carboxamide (Compound P54)

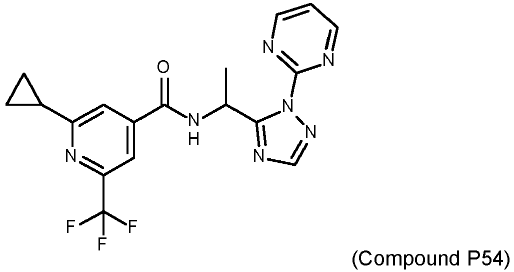

Compound P54 was prepared in using 2-cyclopropyl-6-(trifluoromethyl)pyridine-4-carboxylic acid and the conditions described for Compound P24 (Step C)

¹H-NMR (400 MHz, CDCl₃, ppm) δ : 8.9 (d, J=5.13 Hz, 2 H) 8.1 (s, 1 H) 7.7 - 7.8 (m, 3 H) 7.4 (t, J=4.95 Hz, 1 H) 6.4 - 6.6 (m, 1 H) 2.1 - 2.2 (m, 1 H) 1.7 (d, J=6.60 Hz, 3 H) 1.2 - 1.3 (m, 3 H) 1.1 - 1.2 (m, 4 H).

LC-MS (method 1): Rt 0.92 min , m/z = 404 (M+H+).

### Example 16: Preparation of N-(cyclopropylmethyl)-3-(4-fluorophenyl)-N-[1-(2-pyrimidin-2-yl-1,2,4-triazol-3-yl)ethyl]-5-(trifluoromethyl)benzamide (Compound P36)

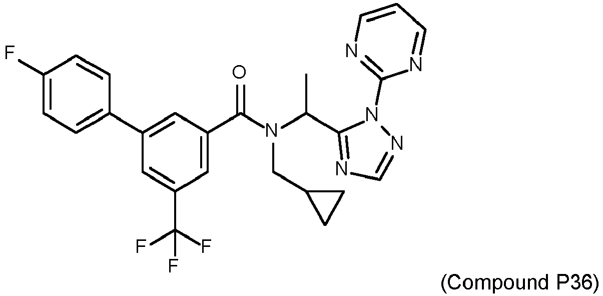

### Step A: Preparation of 3-bromo-N-(cyclopropylmethyl)-N-[1-(2-pyrimidin-2-yl-1,2,4-triazol-3-yl)ethyl1-5-(trifluoromethyl)benzamide

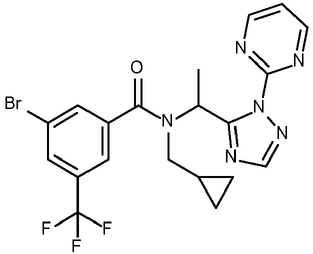

Intermediate 3-bromo-N-(cyclopropylmethyl)-N-[1-(2-pyrimidin-2-yl-1,2,4-triazol-3-yl)ethyl]-5-(trifluoromethyl)benzamide was prepared in using 3-bromo-5-(trifluoromethyl)benzoic acid and the conditions described for Compound P49 (Step C)

LC-MS (method 1): Rt 1.01 min , m/z = 495/497 (M+H+).

### Step B: Preparation of N-(cyclopropylmethyl)-3-(4-fluorophenyl)-N-[1-(2-pyrimidin-2-yl-1,2,4-triazol-3-yl)ethyl1-5-(trifluoromethyl)benzamide (Compound P36)

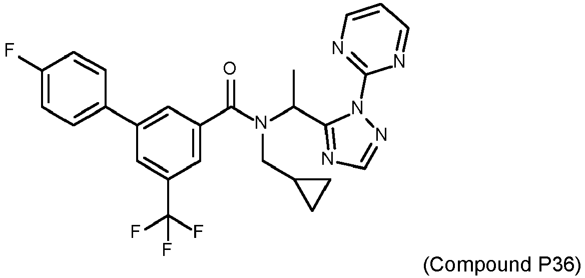

3-bromo-N-(cyclopropylmethyl)-N-[1-(2-pyrimidin-2-yl-1,2,4-triazol-3-yl)ethyl]-5-(trifluoromethyl)benzamide (100 mg, 0.202 mmol), (4-fluorophenyl)boronic acid (34 mg, 0.242 mmol) and Na₂CO₃ (0.15 ml, 0.75 mL/mmol) were added under argon in dioxane,

tetrakis(phosphanyl)palladium (2.5 mg, 0.01 mmol) was then added and the mixture was degased for 5 min with argon before the vial was closed. The reaction mixture was stirred for 4h at 100°C. The suspension was filtered over Celite^{®}, diluted with ethyl acetate and the organic phase was evaporated to give a yellow oil which was purified by reversed phase chromatography to afford of N-(cyclopropylmethyl)-3-(4-fluorophenyl)-N-[1-(2-pyrimidin-2-yl-1,2,4-triazol-3-yl)ethyl]-5-(trifluoromethyl)benzamide.

LC-MS (method 1): Rt 1.07 min , m/z = 511 (M+H+).

### Example 17: Preparation of N-(cyclopropylmethyl)-3-(3,3-difluoroazetidin-1-yl)-N-[1-(2-pyrimidin-2-yl-1,2,4-triazol-3-yl)ethyl]-5-(trifluoromethyl)benzamide (Compound P20)

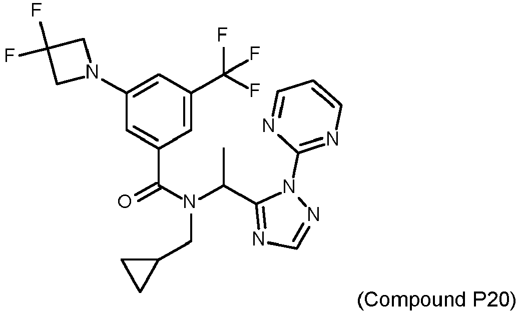

A vial was charged with 3-bromo-N-(cyclopropylmethyl)-N-[1-(2-pyrimidin-2-yl-1,2,4-triazol-3-yl)ethyl]-5-(trifluoromethyl)benzamide (0.100 g, 0.202 mmol), 3,3-difluoroazetidine hydrochloride (0,0314 g, 0,242 mmol), sodium tert-butoxide (0.0588 g, 0.606 mmol), toluene (0,606 ml) and tBuXPhos Pd G3 (0.0160 g, 0.0202 mmol). The reaction mixture was flushed with argon for 5 min and then heated to 40 °C for 3 hours. Then the suspension was filtered over a Celite^{®} pad. The filtrate was diluted with ethyl acetate and concentrated. The crude material was purified by chromatography over silica gel to give N-(cyclopropylmethyl)-3-(3,3-difluoroazetidin-1-yl)-N-[1-(2-pyrimidin-2-yl-1,2,4-triazol-3-yl)ethyl]-5-(trifluoromethyl)benzamide as a yellow oil.

LC-MS (method 1): Rt 1.01 , m/z = 507 (M+H⁺).

### Example 18: Preparation of N-(cyclopropylmethyl)-N-[1-(2-pyrimidin-2-yl-1,2,4-triazol-3-yl)ethyl]-3-(trifluoromethyl)-5-(trifluoromethylsulfinyl)benzamide (Compound P26)

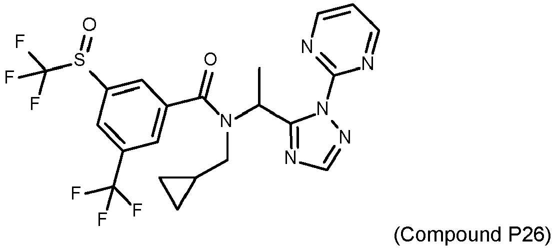

### Step A: Preparation of methyl 3-(trifluoromethyl)-5-(trifluoromethylsulfanyl)benzoate

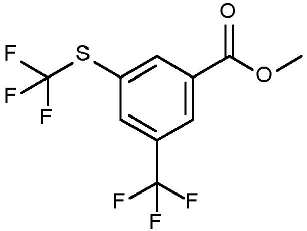

(2,2'-bipyridine)(trifluoromethanethiolato)copper (CAS 1413732-47-4) (3.9 g, 12 mmol) was added to a solution of methyl 3-iodo-5-(trifluoromethyl)benzoate (2.0 g, 6.1 mmol) in acetonitrile (18 mL) under argon. The reaction mixture was heated up to 90 °C and stirred overnight. After cooling down to room temperature, the reaction mixture was filtered over a pad of Celite^{®} and concentrated. The crude material was purified by two flash chromatographies over silica gel (ethyl acetate in cyclohexane) to afford the desired product as a yellow gum.

¹H-NMR (400 MHz, CDCl₃, ppm) δ : 4.02 (s, 3 H), 8.11 (s, 1 H), 8.44 (s. 1H), 8.53 (s, 1 H).

LC-MS (method 1): Rt 1.21 , m/z = 279 (M+H⁺).

### Step B: Preparation of methyl 3-(trifluoromethyl)-5-(trifluoromethylsulfinyl)benzoate

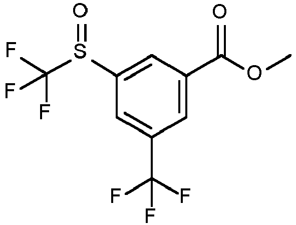

3-chlorobenzenecarboperoxoic acid (3.6 g, 17 mmol) was added to a solution of methyl 3-(trifluoromethyl)-5-(trifluoromethylsulfanyl)benzoate in dichloromethane (25 mL). After stirring for 3 hour at room temperature, more 3-chlorobenzenecarboperoxoic acid (3.6 g, 17 mmol) was added and the reaction mixture was stirred overnight. 3-chlorobenzenecarboperoxoic acid (3.6 g, 17 mmol) was added and the reaction mixture was stirred overnight again. The precipitate formed was filtered. The filtrate was washed with 10% aqueous solution of sodium thiosulfate and with NaHCOs sat solution. The organic phase was dried over sodium sulfate, filtered and concentrated under reduced pressure. The crude was purified by chromatography over silica gel to afford methyl 3-(trifluoromethyl)-5-(trifluoromethylsulfinyl)benzoate.

LC-MS (method 1): Rt 1.05 , m/z = 321 (M+H⁺).

### Step C: Preparation of 3-(trifluoromethyl)-5-(trifluoromethylsulfinyl)benzoic acid (17)

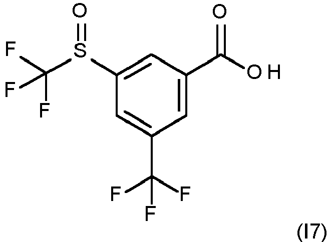

Methyl 3-(trifluoromethyl)-5-(trifluoromethylsulfinyl)benzoate (0.900 g, 2.81 mmol, 1 equiv.) was charged in a flask and dissolved in tetrahydrofuran (8.43 mL) and water (5.62 mL). To this mixture was added lithium hydroxide (0.135 g, 5.62 mmol, 2 equiv.) and the reaction was stirred 2 hours at room temperature. The reaction mixture was acidified with 1M hydrochloric acid, and the aqueous phase was extracted with ethyl acetate (2x) . The combined organic phases were dried over sodium sulfate, filtered and then concentrated to afford 3-(trifluoromethyl)-5-(trifluoromethylsulfinyl)benzoic acid.
¹H-NMR (400 MHz, DMSO-d6, ppm) δ : 8.47 (s, 1 H) 8.51 (s, 1 H) 8.64 (s, 1 H) 14.06 (brs, 1 H)
¹⁹F NMR (377 MHz, DMSO-d6, ppm) δ : -73.46 (s, 3 F) -61.49 (s, 3 F)
LC-MS (method 1): Rt 0.89 , m/z = 307 (M+H⁺).

### Step D: Preparation of N-(cyclopropylmethyl)-N-[1-(2-pyrimidin-2-yl-1,2,4-triazol-3-yl)ethyl1-3-(trifluoromethyl)-5-(trifluoromethylsulfinyl)benzamide (Compound P26)

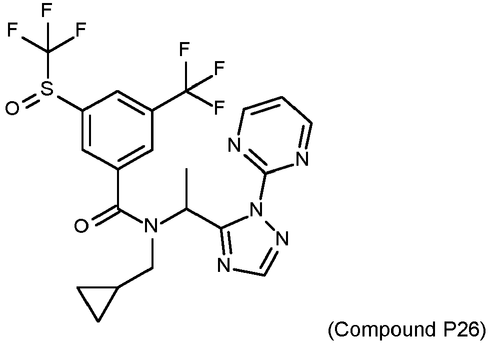

The desired product was prepared using 3-(trifluoromethyl)-5-(trifluoromethylsulfinyl)benzoic acid and the conditions described for Compound P2 (Step E).
¹H-NMR (600 MHz, DMSO-d6, ppm) δ : -0.21 - -0.10 (m, 1 H) -0.10 - 0.00 (m, 1 H) 0.30 - 0.38 (m, 2 H) 0.70 - 0.79 (m, 1 H) 1.80 (dd, J=6.90, 1.27 Hz, 3 H) 3.07 - 3.18 (m, 1 H) 3.18 - 3.30 (m, 1 H) 6.26 (quin, J=7.04 Hz, 1 H) 7.60 (td, J=4.86, 1.91 Hz, 1 H) 7.81 (s, 1 H) 7.93 (brd, J=4.54 Hz, 1 H) 8.15 (d, J=2.18 Hz, 1 H) 8.22 (br d, J=5.45 Hz, 1 H) 8.88 (d, J=5.08 Hz, 2 H)
LC-MS (method 1): Rt 0.98, m/z = 533 (M+H⁺).

### Example 19: Preparation of N-(cyclopropylmethyl)-3-(difluoromethylsulfonyl)-N-[1-(2-pyrimidin-2-yl-1,2,4-triazol-3-yl)ethyl]-5-(trifluoromethyl)benzamide (Compound P30)

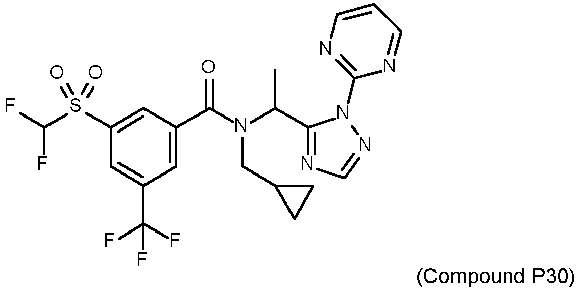

### Step A: Preparation of methyl 3-(difluoromethylsulfanyl)-5-(trifluoromethyl)benzoate

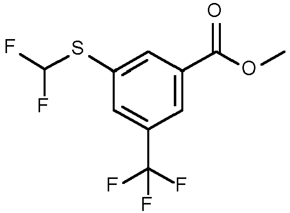

To a solution of methyl 3-sulfanyl-5-(trifluoromethyl)benzoate (0.540 g, 2.29 mmol) in N,N-dimethylformamide (11.4 mL) was added under argon potassium carbonate (0.479 g, 3.43 mmol) and sodium chlorodifluoroacetate (0.704 g, 4.57 mmol) The reaction mixture was heated to 95°C for 3 hours. The mixture was then diluted with water and the aqueous phase was extracted with ethyl acetate. The combined organic phases were washed with water and brine, dried over sodium sulfate, filtered and then concentrated to afford methyl 3-(difluoromethylsulfanyl)-5-(trifluoromethyl)benzoate as an orange solid.
¹H-NMR (400 MHz, CDCl₃, ppm) δ : 3.99 (s, 3 H) 6.90 (t, 1 H) 8.02 (s, 1 H) 8.36 (s, 1 H) 8.43 (s, 1 H)
¹⁹F NMR (377 MHz, CDCl₃, ppm) δ : -91.49 (s, 2 F) -62.91 (s, 3 F)

### Step B: Preparation of methyl 3-(difluoromethylsulfonyl)-5-(trifluoromethyl)benzoate

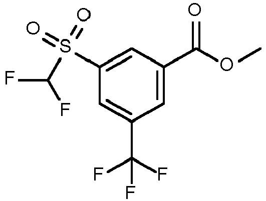

To a solution of methyl 3-(difluoromethylsulfanyl)-5-(trifluoromethyl)benzoate (0.540 g, 1.89 mmol) in carbon tetrachloride (0.943 ml), acetonitrile (0.943 ml) and water (2.36 ml), was added sodium periodate (1.87 g, 8.68 mmol) and trichlororuthenium hydrate (0.0106 g, 0.0472 mmol). The reaction mixture was stirred at room temperature for 30 min. The mixture was diluted with water and extracted with dichloromethane. The combined organic layers were dried over sodium sulfate, filtered and concentrated under reduced pressure. The crude was purified by chromatography over silica gel to afford methyl 3-(difluoromethylsulfonyl)-5-(trifluoromethyl)benzoate as a white solid.
¹H-NMR (400 MHz, CDCl₃, ppm) δ : 4.06 (s, 3 H) 6.31 (t, 1 H) 8.43 (s, 1 H) 8.72 (s, 1 H) 8.83 (s, 1 H)
¹⁹F NMR (376 MHz, CDCl₃, ppm) δ : -120.50 (s, 2 F) -62.93 (s, 3 F)
LC-MS (method 1): Rt 1.54 , m/z = 219 (M+H⁺).

### Step C: Preparation of 3-(difluoromethylsulfonyl)-5-(trifluoromethyl)benzoic acid (111)

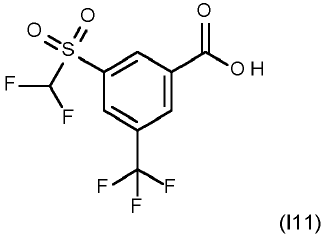

Methyl 3-(difluoromethylsulfonyl)-5-(trifluoromethyl)benzoate (0.280 g, 0.880 mmol, 1.0 equiv.) was charged in a flask and dissolved in tetrahydrofuran (2.64 mL) and water (1.76 mL). To this mixture was added portion wise at 0°C lithium hydroxide (0.0421 g, 1.76 mmol, 2 equiv.) and the reaction was stirred 10 minutes at 0°C and then 0.5 hours at room temperature. The reaction mixture was acidified with 1M hydrochloric acid, and the aqueous phase was extracted with ethyl acetate (3x) . The combined organic phases were washed with water, dried over sodium sulfate, filtered and then concentrated to afford 3-(difluoromethylsulfonyl)-5-(trifluoromethyl)benzoic acid as a white solid.
¹H-NMR (400 MHz, DMSO-d6, ppm) δ : 7.50 (t, 1 H) 8.48 (s, 1 H) 8.59 - 8.73 (m, 2 H) 14.28 (br s, 1 H)
¹⁹F NMR (377 MHz, DMSO-d6, ppm) δ : -124.39 (s, 2 F) -61.54 (s, 3 F)
LC-MS (method 1): Rt 1.20 , m/z = 303 (M-H⁺).

### Step D: Preparation of N-(cyclopropylmethyl)-3-(difluoromethylsulfonyl)-N-[1-(2-pyrimidin-2-yl-1,2,4-triazol-3-yl)ethyl1-5-(trifluoromethyl)benzamide (Compound P30)

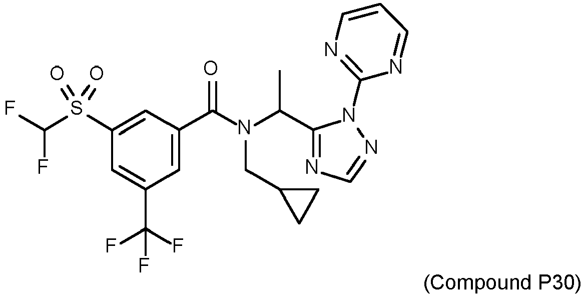

The desired product was prepared using 3-(difluoromethylsulfonyl)-5-(trifluoromethyl)benzoic acid and the conditions described for Compound P2 (Step E).

LC-MS (method 1): Rt 1.48 , m/z = 531 (M+H⁺).

### Example 20: Preparation of N-(cyclopropylmethyl)-2-methylsulfonyl-N-[1-(2-pyrimidin-2-yl-1,2,4-triazol-3-yl)ethyl]-6-(trifluoromethyl)pyridine-4-carboxamide (Compound P53)

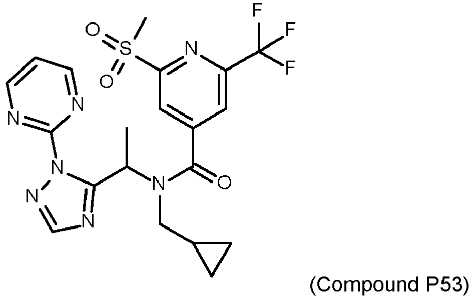

### Step A: Preparation of methyl 2-chloro-6-(trifluoromethyl)pyridine-4-carboxylate

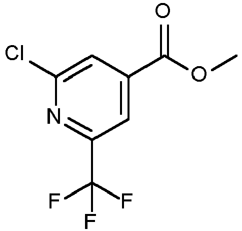

To a solution of 2-chloro-6-(trifluoromethyl)pyridine-4-carboxylic acid (20 g, 88.7 mmol) in methanol (266 mL) was added dropwise at room temperature sulfuric acid (4.92 mL, 88.7 mmol). After the addition the reaction mixture was heated up to 65°C and stirred overnight. The reaction mixture was allowed to cool down to room temperature and poured into sat. NaHCOs aq. sol. (250 ml), stirred for 15 minutes and extracted with dichloromethane (3x). The combined organic layers were dried over sodium sulfate, filtered and concentrated. Then the residue was dissolved in dichloromethane, filtered over Celite^{®}, and concentrated under reduce pressure to afford methyl 2-chloro-6-(trifluoromethyl)pyridine-4-carboxylate.

¹H-NMR (400 MHz, CDCl₃, ppm) δ : 4.04 (s, 3 H) 8.11 (s, 1 H) 8.17 (d, J=1.10 Hz, 1 H)

### Step B: Preparation of methyl 2-methylsulfanyl-6-(trifluoromethyl)pyridine-4-carboxylate

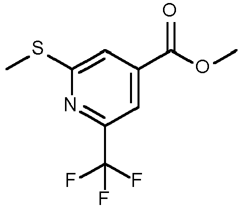

To s solution of methyl 2-chloro-6-(trifluoromethyl)pyridine-4-carboxylate (0.130 g,0.543 mmol) in N,N-dimethylformamide (2.71 mL) under argon was added sodium thiomethoxide (0.0423 g, 0.543 mmol). After 1 hour at room temperature, the reaction mixture was diluted with ethyl acetate and water. The aqueous phase was extracted with ethyl acetate and the combined organic layers were dried over sodium sulfate, filtered and concentrated to afford methyl 2-methylsulfanyl-6-(trifluoromethyl)pyridine-4-carboxylate.
¹H-NMR (400 MHz, CDCl₃, ppm) δ : 2.65 (s, 3 H) 4.00 (s, 3 H) 7.86 (d, J=1.10 Hz, 1 H) 7.91 - 7.95 (m, 1 H)
LC-MS (method 1): Rt 1.09, m/z = 252 (M+H⁺).

### Step C: Preparation of 2-methylsulfanyl-6-(trifluoromethyl)pyridine-4-carboxylic acid

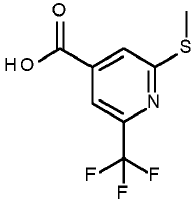

The desired product was prepared using 2-methylsulfanyl-6-(trifluoromethyl)pyridine-4-carboxylate and the conditions described for Compound P26 (Step C).
¹H-NMR (400 MHz, DMSO-d6, ppm) δ : 2.60 (s, 3 H) 7.81 (d, J=1.10 Hz, 1 H) 7.96 (s, 1 H) 14.18 (br s, 1 H)
LC-MS (method 1): Rt 0.89, m/z = 238 (M+H⁺).

### Step D: Preparation of N-(cyclopropylmethyl)-2-methylsulfanyl-N-[1-(2-pyrimidin-2-yl-1,2,4-triazol-3-v!)ethv!1-6-(trif!uoromethv!)pyridine-4-carboxamide

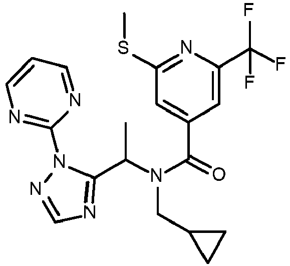

The desired product was prepared using 2-methylsulfanyl-6-(trifluoromethyl)pyridine-4-carboxylic acid. and the conditions described for Compound P2 (Step E).
¹H-NMR (600 MHz, DMSO-d6, ppm) δ : -0.07 - 0.09 (m, 2 H) 0.39 (ddt, J=12.92, 8.65, 4.47, 4.47 Hz, 2 H) 0.78 - 0.86 (m, 1 H) 1.77 (d, J=6.90 Hz, 3 H) 2.57 (s, 3 H) 3.13 - 3.35 (m, 2 H) 6.15 - 6.25 (m, 1 H) 7.19 (s, 1 H) 7.26 (s, 1 H) 7.61 (s, 1 H) 8.15 (s, 1 H) 8.88 (d, J=4.90 Hz, 2 H)
LC-MS (method 1): Rt 1.00, m/z = 464 (M+H⁺).

### Step E: Preparation of N-(cyclopropylmethyl)-2-methylsulfonyl-N-[1-(2-pyrimidin-2-yl-1,2,4-triazol-3-v!)ethv!1-6-(trif!uoromethv!)pyridine-4-carboxamide (compound P53)

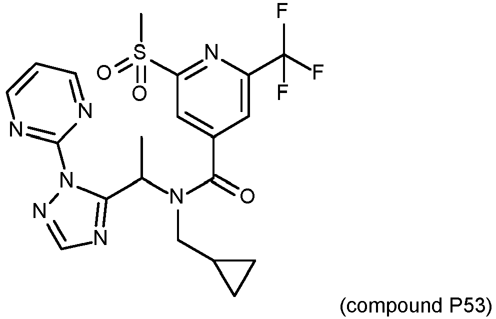

To a solution of N-(cyclopropylmethyl)-2-methylsulfanyl-N-[1-(2-pyrimidin-2-yl-1,2,4-triazol-3-yl)ethyl]-6-(trifluoromethyl)pyridine-4-carboxamide (0.050 g, 0.11 mmol) in dichloromethane (2.2 ml) at 0°C, under argon, was added 3-chlorobenzenecarboperoxoic acid (0.051 g, 0.23 mmol). The reaction mixture was stirred for 2 hours and then quenched with sodium thiosulfate sat. sol. and 1M sodium hydroxide. The mixture was stirred for 30 min and then the aqueous layer was extracted 3 times with dichloromethane. The combined organic layers were washed (2x) with 1 M sodium hydroxide, dried over sodium sulfate, filtered and concentrated to afford N-(cyclopropylmethyl)-2-methylsulfonyl-N-[1-(2-pyrimidin-2-yl-1,2,4-triazol-3-yl)ethyl]-6-(trifluoromethyl)pyridine-4-carboxamide.
¹H-NMR (600 MHz, DMSO-d6, ppm) δ : -0.11 - 0.07 (m, 2 H) 0.39 (ddt, J=12.97, 8.65, 4.45, 4.45 Hz, 2 H) 0.76 - 0.86 (m, 1 H) 1.80 (d, J=6.90 Hz, 3 H) 3.18 (br s, 2 H) 3.34 (s, 3 H) 6.15 - 6.27 (m, 1 H) 7.62 (s, 1 H) 7.96 (s, 1 H) 8.01 - 8.05 (m, 1 H) 8.14 - 8.17 (m, 1 H) 8.87 - 8.94 (m, 2 H)
LC-MS (method 1): Rt 0.85, m/z = 496 (M+H⁺).

### Example 21: Preparation of N-(cyclopropylmethyl)-N-[1-(2-pyrimidin-2-yl-1,2,4-triazol-3-yl)ethyl]-2-(2,2,2-trifluoroethylsulfinyl)-6-(trifluoromethyl)pyridine-4-carboxamide (Compound P57)

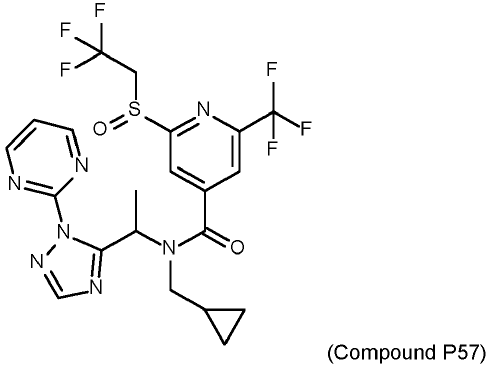

### Step A: Preparation of 2-chloro-N-(cyclopropylmethyl)-N-[1-(2-pyrimidin-2-yl-1,2,4-triazol-3-yl)ethyl]-6-(trifluoromethyl)pyridine-4-carboxamide

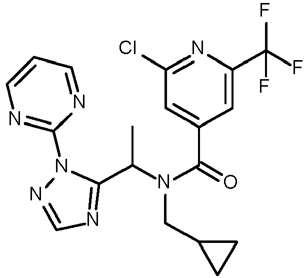

The desired product was prepared using 2-chloro-6-(trifluoromethyl)pyridine-4-carboxylic acid and the conditions described for Compound P2 (Step E).
¹H-NMR (600 MHz, DMSO-d6, ppm) δ : -0.17 - 0.13 (m, 2 H) 0.39 (ddd, J=17.98, 9.08, 4.54 Hz, 2 H) 0.74 - 0.92 (m, 1 H) 1.78 (d, *J*=7.08 Hz, 3 H) 2.76 - 3.39 (m, 2 H) 6.01 - 6.41 (m, 1 H) 7.58 (s, 1 H) 7.60 (s, 1 H) 7.61 - 7.64 (m, 1 H) 8.15 (s, 1 H) 8.90 (d, *J*=4.72 Hz, 2 H)
LC-MS (method 1): Rt 0.94, m/z = 452 (M+H⁺).

### Step B: Preparation of N-(cyclopropylmethyl)-N-[1-(2-pyrimidin-2-yl-1,2,4-triazol-3-yl)ethyl1-2-(2,2,2-trifluoroethylsulfanyl)-6-(trifluoromethyl)pyridine-4-carboxamide

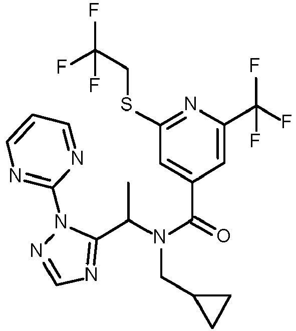

A vial was charged with 2-chloro-N-(cyclopropylmethyl)-N-[1-(2-pyrimidin-2-yl-1,2,4-triazol-3-yl)ethyl]-6-(trifluoromethyl)pyridine-4-carboxamide (0.1 g, 0.2213 mmol), 2,2,2-trifluoroethanethiol (0.03246 g, 0.02487 mL, 0.2656 mmol), dipotassium carbonate (0.06271 g, 0.4537 mmol) and N,N-dimethylformamide (3 mL). The reaction mixture was stirred under microwave radiation at room temperature for 1 hour. The reaction mixture was quenched with water, and the aqueous layer was extracted with ethyl acetate (3x). The combined organic layers were washed with water (5x) then dried over sodium sulfate, filtered and concentrated to afford N-(cyclopropylmethyl)-N-[1-(2-pyrimidin-2-yl-1,2,4-triazol-3-yl)ethyl]-2-(2,2,2-trifluoroethylsulfanyl)-6-(trifluoromethyl)pyridine-4-carboxamide. ¹H-NMR (600 MHz, DMSO-d6,ppm) δ : -0.18 - 0.16 (m, 2 H) 0.37 (ddd, *J*=13.31, 8.95, 4.45 Hz, 2 H) 0.78 (br s, 1 H) 1.76 (d, *J*=6.90 Hz, 3 H) 3.09 - 3.37 (m, 2 H) 4.21 (q, *J*=10.17 Hz, 2 H) 6.20 (brd, *J*=2.91 Hz, 1 H) 7.31 (s, 1 H) 7.50 (s, 1 H) 7.59 (t, *J*=4.90 Hz, 1 H) 8.14 (s, 1 H) 8.86 (brd, *J*=4.72 Hz, 2 H)

LC-MS (method 1): Rt 1.06, m/z = 532 (M+H⁺).

### Step C: Preparation of N-(cyclopropylmethyl)-N-[1-(2-pyrimidin-2-yl-1,2,4-triazol-3-yl)ethyl1-2-(2,2,2-trifluoroethylsulfinyl)-6-(trifluoromethyl)pyridine-4-carboxamide (Compound P57)

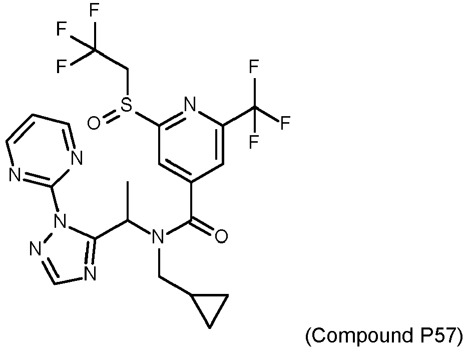

The desired product was prepared using N-(cyclopropylmethyl)-N-[1-(2-pyrimidin-2-yl-1 ,2,4-triazol-3-yl)ethyl]-2-(2,2,2-trifluoroethylsulfanyl)-6-(trifluoromethyl)pyridine-4-carboxamide and the conditions described for Compound P26 (Step B) to afford N-(cyclopropylmethyl)-N-[1-(2-pyrimidin-2-yl-1,2,4-triazol-3-yl)ethyl]-2-(2,2,2-trifluoroethylsulfinyl)-6-(trifluoromethyl)pyridine-4-carboxamide ¹H-NMR (600 MHz, DMSO-d6, ppm) δ : -0.20 - 0.12 (m, 2 H) 0.21 - 0.52 (m, 2 H) 0.77 (br s, 1 H) 1.78 (d, *J*=6.90 Hz, 3 H) 3.01 - 3.40 (m, 2 H) 4.00 -4.15 (m, 1 H) 4.18 - 4.30 (m, 1 H) 6.18 (br d, *J*=1.09 Hz, 1 H) 7.60 (t, *J*=4.81 Hz, 1 H) 7.76 - 7.85 (m, 1 H) 7.95 (d, *J*=13.62 Hz, 1 H) 8.14 (s, 1 H) 8.89 (t, *J*=4.18 Hz, 2 H)

LC-MS (method 1): Rt 0.95, m/z = 548 (M+H⁺).

### Example 22: Preparation of 4-chloro-6-(1-cyano-1-methyl-ethyl)-N-(cyclopropylmethyl)-N-[1-(2-pyrimidin-2-yl-1,2,4-triazol-3-yl)ethyl]pyridine-2-carboxamide (Compound P28)

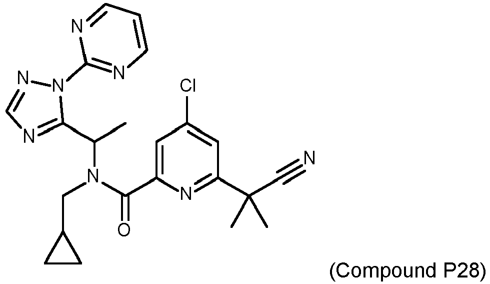

### Step A : Preparation of 4-chloro-6-(cyanomethyl)pyridine-2-carboxylic acid and 6-chloro-4-(cyanomethyl)pyridine-2-carboxylic acid

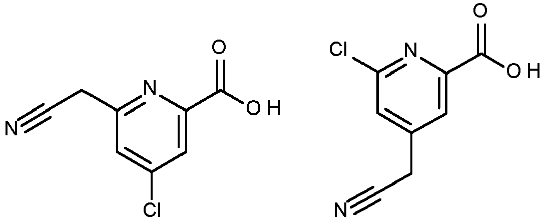

Butyllithium (2.5 M/L in THF) (3.9 mL, 9.77 mmol) was diluted in tetrahydrofuran (10 mL) and cooled to -78 °C under argon. Acetonitrile (0.548 mL, 10.4 mmol) was added dropwise and the solution was stirred at -78°C for 30 min. A solution of 4,6-dichloropyridine-2-carboxylic acid (0.250 g, 1.30 mmol) in tetrahydrofuran (10 mL) was added and the reaction mixture was stirred 2 hours at -78 °C and then 1 hour at room temperature. The reaction mixture was quenched with methanol followed by a saturated aqueous NaHCOs. The mixture was extracted with dichloromethane (2x). The combined organic layers were dried over sodium sulfate, filtered and concentrated to afford a mixture of 4-chloro-6-(cyanomethyl)pyridine-2-carboxylic acid and 6-chloro-4-(cyanomethyl)pyridine-2-carboxylic acid as a yellow oil that was used as it in the next step.

LC-MS (method 1): Rt 0.51, *m*/*z* = 197 (M+H⁺).

### Step B : Preparation of 4-chloro-6-(1-cyano-1-methyl-ethyl)pyridine-2-carboxylic (I9) acid and 6-chloro-4-(1-cyano-1-methyl-ethyl)pyridine-2-carboxylic acid (I8)

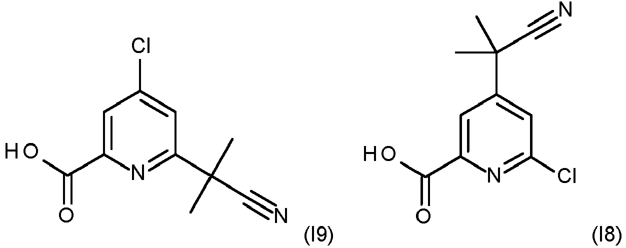

To a mixture of 4-chloro-6-(cyanomethyl)pyridine-2-carboxylic acid and 6-chloro-4-(cyanomethyl)pyridine-2-carboxylic acid (250 mg, 1.27 mmol) in acetonitrile (2.54 mL) were added tetrabutylammonium bromide (0.820 g, 2.54 mmol) and iodomethane (0.379 g, 2.67 mmol). The solution was stirred 30 min at room temperature and then cooled at 0 °C. Sodium hydroxide (50% in water) (0.671 mL, 12.7 mmol) was added dropwise and the reaction mixture was stirred at room temperature overnight. The reaction mixture was quenched with water and extracted with ethyl acetate. The combined organic layers were dried over sodium sulfate, filtered and concentrated. The aqueous was acidified with 5M hydrochloric acid and extracted with dichloromethane (3x), the organic phases were dried over sodium sulfate, filtered and concentrated. The crude residue was purified by reverse phase chromatography to afford 4-chloro-6-(1-cyano-1-methyl-ethyl)pyridine-2-carboxylic acid (intermediate 19) and 6-chloro-4-(1-cyano-1-methyl-ethyl)pyridine-2-carboxylic acid (intermediate 18) which could be separated.
Intermediate I9: ¹H-NMR (400 MHz, CDCl₃, ppm) δ : 1.82 (s, 6 H) 7.84 (d, *J*=1.83 Hz, 1 H) 8.20 (d, *J*=1.83 Hz, 1 H)
LC-MS (method 1): Rt 0.75, m/z = 225 (M+H⁺).
Intermediate I8: ¹H-NMR (400 MHz, CDCl₃, ppm) δ : 1.78 (s, 6 H) 7.69 (s, 1 H) 8.18 (d, *J*=1.47 Hz, 1 H)
LC-MS (method 1): Rt 0.66, m/z = 225 (M+H⁺).

### Step C : Preparation of 4-chloro-6-(1-cyano-1-methyl-ethyl)-N-(cyclopropylmethyl)-N-[1-(2-pyrimidin-2-yl-1,2,4-triazol-3-yl)ethyllpyridine-2-carboxamide (Compound P28)

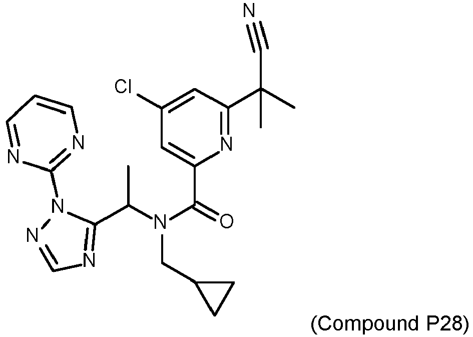

The desired product was prepared using 4-chloro-6-(1-cyano-1-methyl-ethyl)pyridine-2-carboxylic acid and the conditions described for Compound P2 (Step E).

LC-MS (method 1): Rt 0.91, m/z = 451 (M+H⁺).

### Example 23: Preparation of 6-chloro-4-(1-cyano-1-methyl-ethyl)-N-(cyclopropylmethyl)-N-[1-(2-pyrimidin-2-yl-1,2,4-triazol-3-yl)ethyl]pyridine-2-carboxamide (Compound P27)

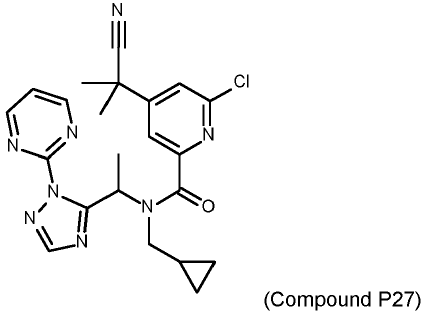

The desired product was prepared in using 6-chloro-4-(1-cyano-1-methyl-ethyl)pyridine-2-carboxylic acid and the conditions described in for Compound P2 (Step E).

LC-MS (method 1): Rt 0.90, m/z = 451 (M+H⁺).

### Example 24: Preparation of 4-chloro-6-(1-cyanocyclopropyl)-N-(cyclopropylmethyl)-N-[1-(2-pyrimidin-2-yl-1 ,2,4-triazol-3-yl)ethyl]pyridine-2-carboxamide (Compound P29)

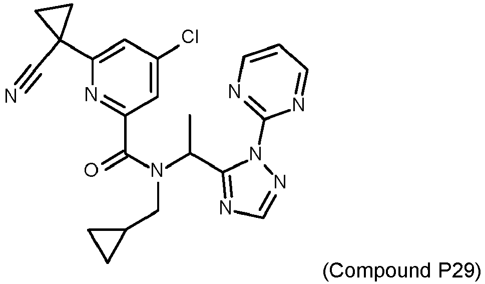

### Step A : Preparation of 4-chloro-6-(1-cyanocyclopropyl)pyridine-2-carboxylic acid (I10)

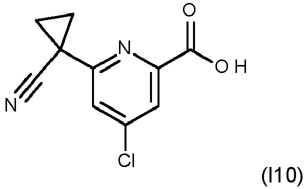

To a solution of 4-chloro-6-(cyanomethyl)pyridine-2-carboxylic acid (0.182 g, 0.926 mmol) (prepared in step A for Compound P28) in acetonitrile (1.85 mL) were added tetrabutylammonium bromide (0.298 g, 0.926 mmol) and 1,2-dibromoethane (0.183 g, 0.972 mmol). The solution was stirred 30 min at room temperature and then cooled at 0 °C. Sodium hydroxide (50% in water) (0.244 mL, 4.63 mmol) was added dropwise and the reaction mixture was stirred at room temperature overnight. The reaction mixture was quenched with water and extracted with ethyl acetate. The combined organic layers were dried over sodium sulfate, filtered and concentrated. The aqueous was acidified with 5 M hydrochloric acid and extracted with dichloromethane (3x), the organic phases were dried over sodium sulfate, filtered and concentrated. The combined crudes were purified by reverse phase chromatography to afford 4-chloro-6-(1-cyanocyclopropyl)pyridine-2-carboxylic acid.

LC-MS (method 1): Rt 0.61, m/z = 223 (M+H⁺).

### Step B : Preparation of 4-chloro-6-(1-cyanocyclopropyl)-N-(cyclopropylmethyl)-N-[1-(2-pyrimidin-2-yl-1,2,4-triazol-3-yl)ethyllpyridine-2-carboxamide (Compound P29)

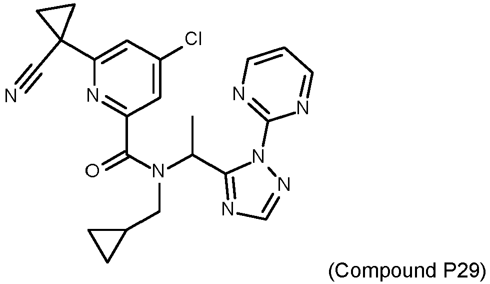

The desired product was prepared in using 4-chloro-6-(1-cyanocyclopropyl)pyridine-2-carboxylic acid and the conditions described for Compound P2 (Step E).

LC-MS (method 1): Rt 0.88, m/z = 449 (M+H⁺).

### Example 25: Preparation of 6-(1-cyano-1-methyl-ethyl)-N-(cyclopropylmethyl)-N-[1-(2-pyrimidin-2-yl-1,2,4-triazol-3-yl)ethyl]-4-(trifluoromethyl)pyridine-2-carboxamide (Compound P31)

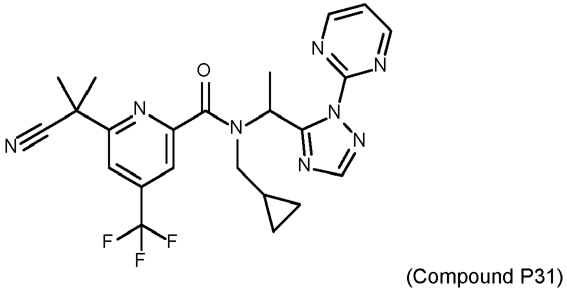

### Step A : Preparation of ethyl 2-[6-chloro-4-(trifluoromethyl)-2-pyridyl]-2-cyano-acetate

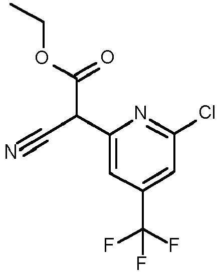

A suspension of sodium hydride (1.80 g, 45.1 mmol) in N-methyl-2-pyrrolidone (12.0 mL) was cooled at 8 °C, and ethyl cyanoacetate (4.90 mL, 45.1 mmol) was added dropwise over 40 minutes. The mixture was stirred at room temperature for 15 minutes until no gas evolution, then a solution of 2-chloro-6-fluoro-4-(trifluoromethyl)pyridine (3.00 g, 15.0 mmol) in N-methyl-2-pyrrolidone (3.0 mL) was added dropwise. The reaction mixture was stirred at room temperature for 17 hours. The reaction medium was quenched at 0°C by slow addition of water. Then it was acidified with 2 N hydrochloric acid solution until pH reach 3-4 and extraction was carried out with ethyl acetate (3x). The combined organic layers were washed with water (2x), brine, dried over sodium sulfate, filtered and concentrated under reduced pressure. The crude residue was purified by chromatography over silica gel. The resulting product was triturated into cold diethyl ether and filtered. The solid press cake was washed with cold diethyl ether and dried to afford ethyl 2-[6-chloro-4-(trifluoromethyl)-2-pyridyl]-2-cyanoacetate as a yellow solid.

¹H-NMR (400 MHz, CDCl₃, ppm) δ : 14.50 (1 H, br s), 7.40 (1 H, m), 6.65 (1 H, t, J=1.65 Hz), 4.27 - 4.37 (3 H, q, *J*=6.97 Hz), 1.32 - 1.40 (3 H, t, J=6.97 Hz).

LC-MS (method 1): Rt 0.98, m/z = 291 (M-H⁺).

### Step B : Preparation of 2-[6-chloro-4-(trifluoromethyl)-2-pyridyl]acetonitrile

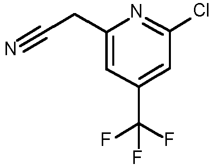

To a suspension of 2-[6-chloro-4-(trifluoromethyl)-2-pyridyl]-2-cyano-acetate (1.00 g, 3.42 mmol) in ethanol (6.49 mL) was added hydrochloric acid 6 N in water (10.3 mL, 61.5 mmol). The reaction mixture was stirred at reflux for 18 hours and then temperature was allowed to reach room temperature. The reaction medium was poured onto ice/water and extraction was carried out with ethyl acetate (3x). The combined organic layers were washed with brine, dried over anhydrous sodium sulfate, filtered and concentrated under reduced pressure. The crude residue was purified by chromatography over silica gel to afford 2-[6-chloro-4-(trifluoromethyl)-2-pyridyl]acetonitrile (0.5623 g, 2.549 mmol) as a light yellow oil.

¹H-NMR (400 MHz, CDCl₃, ppm) δ : 7.63 (1 H, s), 7.58 (1 H, s), 4.02 (2 H, s).

LC-MS (method 1): Rt 0.88, m/z = 221 (M+H⁺).

### Step C : Preparation of 2-[6-bromo-4-(trifluoromethyl)-2-pyridyl]acetonitrile

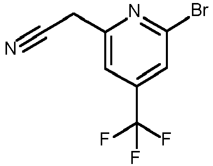

To a solution of 2-[6-chloro-4-(trifluoromethyl)-2-pyridyl]acetonitrile ( 0.562 g, 2.55 mmol, 1.00 equiv.) in propionitrile (2.55 mL) was added dropwise trimethylbromosilane (0.804 g, 0.693 mL, 5.10 mmol, 2.00 equiv.). The reaction mixture was stirred at 95°C for 16h, then it was allowed to reach room temperature. The reaction mixture was concentrated under reduced pressure and the crude was purified by chromatography over silica gel to afford 2-[6-bromo-4-(trifluoromethyl)-2-pyridyl]acetonitrile as an orange oil.

¹H-NMR (400 MHz, CDCl₃, ppm) δ : 7.73 (1 H, s), 7.66 (1 H, s), 4.03 (2 H, s).

### Step D : Preparation of 2-[6-bromo-4-(trifluoromethyl)-2-pyridyl]-2-methyl-propanenitrile

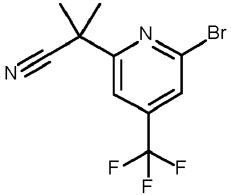

A solution of 2-[6-bromo-4-(trifluoromethyl)-2-pyridyl]acetonitrile (0.250 g, 0.943 mmol) in dimethyl sulfoxide (1.89 mL) was cooled to 8°C. Then sodium hydride (0.113 g, 2.83 mmol) was added portionwise over 2 minutes. The mixture was stirred at room temperature for 20 minutes until no gas evolution, then iodomethane (0.177 mL, 2.83 mmol) was added dropwise. The reaction mixture was stirred at room temperature for 1h 15 minutes. Then it was carefully quenched at 8°C by slow addition of water and extracted with ethyl acetate (3x). The combined organic layers were washed with water (2x) , brine, dried over anhydrous sodium sulfate, filtered and concentrated under reduced pressure. The crude was purified by chromatography over silica gel to afford 2-[6-bromo-4-(trifluoromethyl)-2-pyridyl]-2-methyl-propanenitrile as a yellow oil.

¹H-NMR (400 MHz, CDCl₃, ppm) δ : 7.79 (1 H, d, J=0.73 Hz), 7.69 (1 H, s), 1.80 (6 H, s).

### Step E : Preparation of methyl 6-(1-cyano-1-methyl-ethyl)-4-(trifluoromethyl)pyridine-2-carboxylate

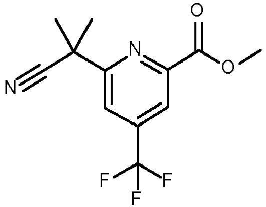

An autoclave was charged at room temperature, under argon, with palladium(II) acetate (0.00234 g, 0.0102 mmol), Pd(dppf)Cl₂ (0.0111 g, 0.0136 mmol), dppf (0.00585 g, 0.0102 mmol), 2-[6-bromo-4-(trifluoromethyl)-2-pyridyl]-2-methyl-propanenitrile (0.100 g, 0.341 mmol), previously degassed methanol (3.5 mL) and triethylamine (0.0976 mL, 0.699 mmol). The autoclave was closed, flushed with argon, then with carbon monoxide. The reaction mixture was stirred at 100°C under 5 bars pressure of carbon monoxide for 4.5 hours. The reaction mixture was allowed to reach room temperature and filtered. The solid cake was washed with ethyl acetate and the filtrate poured into water and extracted with ethyl acetate (3x) . The combined organic layers were washed with brine, dried over anhydrous sodium sulfate, filtered and concentrated under reduced pressure. The crude residue was purified by chromatography over silica gel to afford 6-(1-cyano-1-methyl-ethyl)-4-(trifluoromethyl)pyridine-2-carboxylate as a light yellow oil.

¹H-NMR (400 MHz, CDCl₃, ppm) δ : 8.27 (1 H, s), 8.02 (1 H, d, J=0.73 Hz), 4.04 (3 H, s), 1.85 (6 H, s).

LC-MS (method 1): Rt 0.96 min, m/z = 273 (M+H⁺).

### Step F : Preparation of 6-(1-cyano-1-methyl-ethyl)-4-(trifluoromethyl)pyridine-2-carboxylic acid (112)

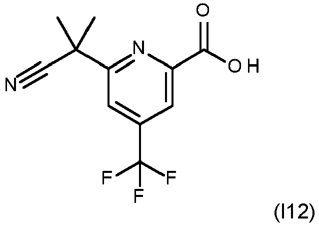

To a solution of 6-(1-cyano-1-methyl-ethyl)-4-(trifluoromethyl)pyridine-2-carboxylate (0.083 g, 0.30 mmol) in tetrahydrofuran (0.91 mL) and water (0.46 mL) was added lithium hydroxide monohydrate (0.013 g, 0.30 mmol) and the resulting light yellow solution was stirred at room temperature for 2 hours. The reaction mixture was acidified with 1M hydrochloric acid, and the aqueous phase was extracted with ethyl acetate (3x) . The combined organic phases were dried over sodium sulfate, filtered and then concentrated to afford 6-(1-cyano-1-methyl-ethyl)-4-(trifluoromethyl)pyridine-2-carboxylic acid as a light yellow gum.
¹H-NMR (400 MHz, CDCl₃, ppm) δ :8.46 (1 H, s), 8.06 (1 H, s), 1.88 (6 H, s)
LC-MS (method 1): Rt 0.80 min, m/z = 257 (M-H⁺).

### Step G : Preparation of 6-(1-cyano-1-methyl-ethyl)-N-(cyclopropylmethyl)-N-[1-(2-pyrimidin-2-yl-1,2,4-triazol-3-yl)ethyl]-4-(trifluoromethyl)pyridine-2-carboxamide (Compound P31)

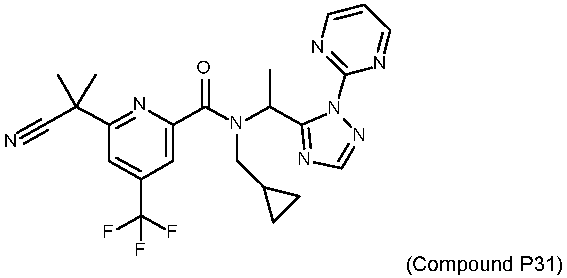

The desired product was prepared using 6-(1-cyano-1-methyl-ethyl)-4-(trifluoromethyl)pyridine-2-carboxylic acid and the condition described for Compound P2 (Step E).

LC-MS (method 1): Rt 0.95 min, m/z = 485 (M+H⁺).

### Example 26: Preparation of 6-(1-cyanocyclopropyl)-N-(cyclopropylmethyl)-N-[1-(2-pyrimidin-2-yl-1,2,4-triazol-3-yl)ethyl]-4-(trifluoromethyl)pyridine-2-carboxamide (Compound P33)

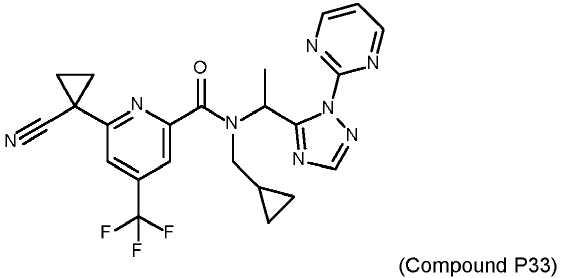

### Step A : Preparation of 1-[6-bromo-4-(trifluoromethyl)-2-pyridyl]cyclopropanecarbonitrile

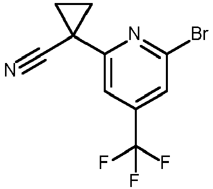

To a solution of 2-[6-bromo-4-(trifluoromethyl)-2-pyridyl]acetonitrile (prepared in step C for Compound P31), (0.364 g, 1.37 mmol) diluted in dimethyl sulfoxide (4.81 mL) at 8°C was added sodium hydride (0.165 g, 4.12 mmol). The mixture was stirred at room temperature for 20 minutes until no gas evolution, then 1,2-dibromoethane (0.179 mL, 2.06 mmol) was added dropwise. The reaction mixture was stirred at room temperature for 1.5 hours. Then, the reaction mixture was carefully quenched at 8°C with water and extracted with ethyl acetate. The combined organic layers were washed with water (2x) , brine, dried over anhydrous sodium sulfate, filtered and concentrated under reduced pressure. The crude was purified by chromatography over silica gel to afford 1-[6-bromo-4-(trifluoromethyl)-2-pyridyl]cyclopropanecarbonitrile as a white solid .

¹H-NMR (400 MHz, CDCl₃, ppm) δ : 7.88 (1 H, s), 7.58 (1 H, s), 1.88 - 1.94 (2 H, m), 1.82 - 1.87 (2 H, m).

LC-MS (method 1): Rt 1.06 min, m/z = 291/293 (M+H⁺).

### Step B: Preparation of methyl 6-(1-cyanocyclopropyl)-4-(trifluoromethyl)pyridine-2-carboxylate

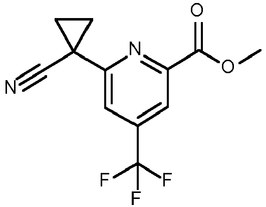

The desired product was prepared using 1-[6-bromo-4-(trifluoromethyl)-2-pyridyl]cyclopropanecarbonitrile and the conditions described for Compound P31 (Step E).

¹H-NMR (400 MHz, CDCl₃, ppm) δ : 8.17 (1 H, s), 8.11 (1 H, d, J=0.73 Hz), 4.00 (3 H, s), 1.95 - 2.04 (2 H, m), 1.80 - 1.93 (2 H, m).

LC-MS (method 1): Rt 0.94 min, m/z = 271 (M+H⁺).

### Step C : Preparation of 6-(1-cyanocyclopropyl)-4-(trifluoromethyl)pyridine-2-carboxylic acid (114)

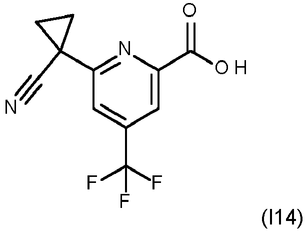

The desired product was prepared using 6-(1-cyanocyclopropyl)-4-(trifluoromethyl)pyridine-2-carboxylate and the conditions described for Compound P31 (Step F).

¹H-NMR (400 MHz, CDCl₃, ppm) δ : 9.74 - 10.27 (1 H, br s), 8.37 (1 H, m), 8.17 (1 H, m), 1.97 - 2.04 (2 H, m), 1.88 - 1.96 (2 H, m).

LC-MS (method 1): Rt 0.79 min, *m*/*z* = 257 (M+H⁺).

### Step D : Preparation of 6-(1-cyanocyclopropyl)-N-(cyclopropylmethyl)-N-[1-(2-pyrimidin-2-yl-1,2,4-triazol-3-yl)ethyll-4-(trifluoromethyl)pyridine-2-carboxamide (Compound P33)

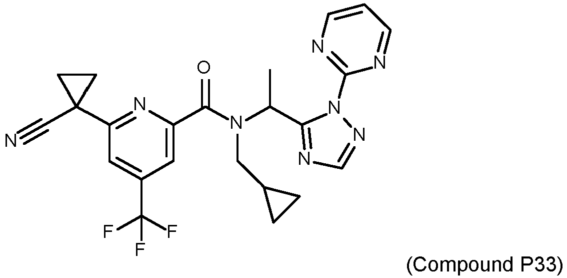

The desired product was prepared using 6-(1-cyanocyclopropyl)-4-(trifluoromethyl)pyridine-2-carboxylic acid and the conditions described for Compound P2 (Step E).

LC-MS (method 1): Rt 0.94 min, m/z = 483 (M+H⁺).

### Example 27: Preparation of N-(cyclopropylmethyl)-3-(1-methylcyclopropyl)-N-[1-(2-pyrimidin-2-yl-1,2,4-triazol-3-yl)ethyl]-5-(trifluoromethyl)benzamide (Compound P38)

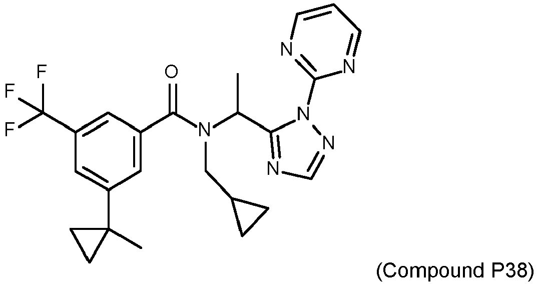

### Step A : Preparation of methyl 3-isopropenyl-5-(trifluoromethyl)benzoate

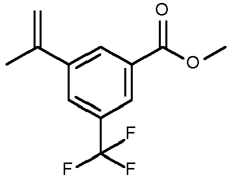

A solution of methyl 3-bromo-5-(trifluoromethyl)benzoate (5.00 g, 17.3 mmol) in dimethoxyethane (69.2 mL) was degased under argon for 5 minutes, then Pd(PPh₃)₄ (0.808 g, 0.692 mmol) was added, followed by isopropenylboronic acid pinacol ester (3.06 g, 3.42 mL, 17.3 mmol). Then a degassed 2 N solution of sodium carbonate (7.35 g, 69.2 mmol) in water (34.6 mL) was added dropwise. The reaction mixture was stirred at 95°C for 3.5 hours and then temperature was allowed to reach room temperature. Water was added to the reaction mixture and it was extracted (3x) with ethyl acetate. The combined organic layers were washed with brine, dried over sodium sulfate, filtered and concentrated under reduced pressure. The crude was purified by chromatography over silica gel to afford methyl 3-isopropenyl-5-(trifluoromethyl)benzoate.

¹H-NMR (400 MHz, CDCl₃, ppm) δ : 8.31 (1 H, s), 8.20 (1 H, s), 7.88 (1 H, s), 5.51 (1 H, t, *J*=1.11 Hz), 5.26 (1 H, t, *J*=1.46 Hz), 3.98 (3 H, s), 2.21 (3 H, dd, *J*=1.47, 0.73 Hz).

### Step B : Preparation of methyl 3-(1-methylcyclopropyl)-5-(trifluoromethyl)benzoate

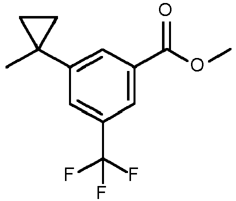

Diethylzinc 1.0 M solution in hexanes (6.1 mL, 6.14 mmol) was diluted in dichloromethane (3.4 mL) and the solution was cooled down to 0°C. Then, a solution of trifluoroacetic acid (0.473 mL, 6.14 mmol) in dichloromethane (3.4 mL) was added dropwise. The mixture was stirred at 0°C for 15 minutes and diiodomethane (0.506 mL, 6.14 mmol) was added dropwise. The reaction mixture was stirred at 0°C for 15 minutes, then a solution of methyl 3-isopropenyl-5-(trifluoromethyl)benzoate (0.500 g, 2.05 mmol) in dichloromethane (3.4 mL) was added dropwise. The reaction mixture was stirred at 0°C for 15 minutes, then at room temperature for 4.5 hours. The reaction mixture was cooled down to 0°C and carefully quenched with methanol, then sat. NH₄Cl aq. solution. Extraction was carried out with ethyl acetate (3x) . The combined organic layers were washed with brine, dried over anhydrous sodium sulfate, filtered and concentrated under reduced pressure. The crude was purified by chromatography over silica gel to afford methyl 3-(1-methylcyclopropyl)-5-(trifluoromethyl)benzoate as a light yellow oil.

¹H-NMR (400 MHz, DMSO-d6, ppm) δ : 8.09 (2 H, m), 7.68 (1 H, m), 3.93 (3 H, s), 1.46 (3 H, s), 0.88 - 0.97 (2 H, m), 0.83 - 0.88 (2 H, m).

### Step C : Preparation of 3-(1-methylcyclopropyl)-5-(trifluoromethyl)benzoic acid (I17)

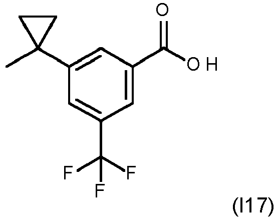

The desired product was prepared using methyl 3-(1-methylcyclopropyl)-5-(trifluoromethyl)benzoate and the conditions described for Compound P31 (Step F).

¹H-NMR (400 MHz, CDCl₃, ppm) δ : 8.17 (1 H, m), 8.16 (1 H, m), 7.74 (1 H, m), 1.48 (3 H, s), 0.91 - 0.98 (2 H, m), 0.79 - 0.90 (2 H, m).

LC-MS (method 1): Rt 1.02 min, m/z = 243 (M-H⁺).

### Step D : Preparation of N-(cyclopropylmethyl)-3-(1-methylcyclopropyl)-N-[1-(2-pyrimidin-2-yl-1,2,4-triazol-3-yl)ethyl1-5-(trifluoromethyl)benzamide (Compound P38)

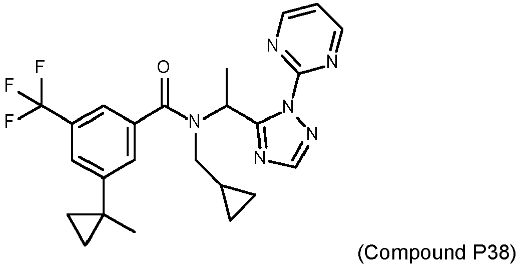

The desired product was prepared using 3-(1-methylcyclopropyl)-5-(trifluoromethyl)benzoic acid and the conditions described for Compound P2 (Step E).

¹H-NMR (600 MHz, DMSO-d6, ppm) δ : -0.05 - 0.03 (m, 1 H) 0.08 (br dd, J=8.9, 4.4 Hz, 1 H) 0.45 (ddt, J=12.7, 8.4, 4.3, 4.3 Hz, 2 H) 0.84 - 0.91 (m, 1 H) 0.92 - 0.98 (m, 4 H) 1.48 (s, 3 H) 1.88 (d, J=7.1 Hz, 3 H) 3.19 (brdd, J=15.0, 6.1 Hz, 1 H) 3.34 (br dd, J=14.9, 6.0 Hz, 1 H) 6.34 (q, J=6.9 Hz, 1 H) 7.25 (s, 1 H) 7.28 (s, 1 H) 7.59 (s, 1 H) 7.69 (t, J=4.8 Hz, 1 H) 8.23 (s, 1 H) 8.95 (d, J=4.9 Hz, 2 H).

LC-MS (method 1): Rt 1.07 min, m/z = 471 (M+H⁺).

### Example 28: Preparation of N-(cyclopropylmethyl)-3-(2,2-difluoro-1-methyl-cyclopropyl)-N-[1-(2-pyrimidin-2-yl-1,2,4-triazol-3-yl)ethyl]-5-(trifluoromethyl)benzamide (Compound P44)

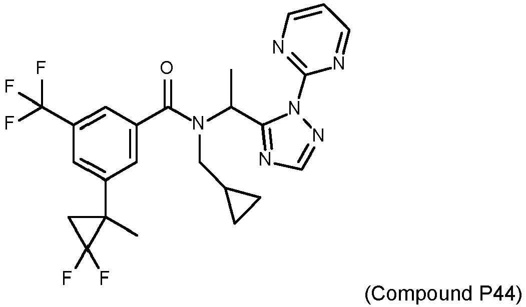

### Step A : Preparation of methyl 3-(2,2-difluoro-1-methyl-cyclopropyl)-5-(trifluoromethyl)benzoate

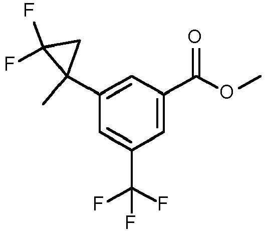

To a solution of methyl 3-isopropenyl-5-(trifluoromethyl)benzoate (prepared in step A for Compound P38) (0.500 g, 2.05 mmol) in toluene (14.3 mL) was added tetrabutylammonium bromide (0.0200 g, 0.0614 mmol) and trimethyl(bromodifluoromethylsilane (0.478 mL, 3.07 mmol). The reaction mixture was heated to 110°C for 2 hours and then temperature was allowed to reach room temperature. The reaction mixture was poured onto ice/water and extraction was carried out with cyclohexane (3x) . The combined organic layers were washed with brine, dried over anhydrous sodium sulfate, filtered and concentrated under reduced pressure. The crude was purified by chromatography over silica gel to afford methyl 3-(2,2-difluoro-1-methyl-cyclopropyl)-5-(trifluoromethyl)benzoate as a light yellow oil. ¹H-NMR (400 MHz, CDCl₃, ppm) δ : 8.22 (1 H, t), 8.19 (1 H, t), 7.76 (1 H, t), 3.98 (3 H, s), 1.72-1.79 (1 H, m), 1.56 (3 H, s), 1.58-1.51 (1 H, m).

### Step B : Preparation of 3-(2,2-difluoro-1-methyl-cyclopropyl)-5-(trifluoromethyl)benzoic acid (I21)

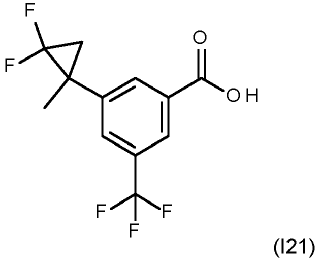

The desired product was prepared using methyl 3-(2,2-difluoro-1-methyl-cyclopropyl)-5-(trifluoromethyl)benzoate and the conditions described for Compound P31 (Step F).

¹H-NMR (400 MHz, CDCl₃, ppm) δ : 8.31 (1 H, s), 8.26 (1 H, s), 7.83 (1 H, s), 1.75 - 1.81 (1 H, m), 1.60 (3 H, m), 0.54 - 1.60 (1 H, m).

LC-MS (method 1): Rt 0.98 min, m/z = 279 (M-H⁺).

### Step C : Preparation of N-(cyclopropylmethyl)-3-(2,2-difluoro-1-methyl-cyclopropyl)-N-[1-(2-pyrimidin-2-yl-1,2,4-triazol-3-yl)ethyl1-5-(trifluoromethyl)benzamide (Compound P44)

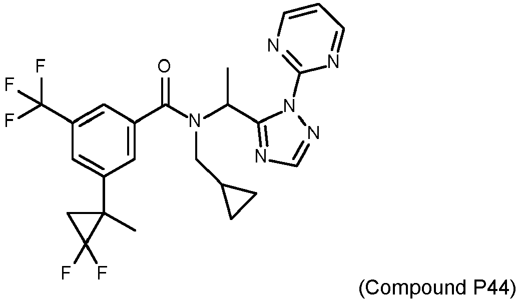

The desired product was prepared using 3-(2,2-difluoro-1-methyl-cyclopropyl)-5-(trifluoromethyl)benzoic acid and the conditions described for Compound P2 (Step E).

LC-MS (method 1): Rt 1.04 min, m/z = 507 (M+H⁺).

### Example 29: Preparation of N-(cyclopropylmethyl)-3-[2,2-difluoro-1-(trifluoromethyl)cyclopropyl]-N-[1-(2-pyrimidin-2-yl-1,2,4-triazol-3-yl)ethyl]-5-(trifluoromethyl)benzamide (Compound P41)

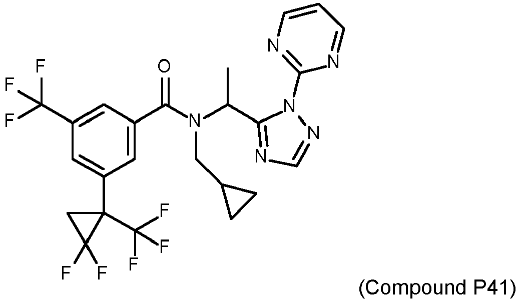

### Step A : Preparation of methyl 3-(trifluoromethyl)-5-[1-(trifluoromethyl)vinyl]benzoate

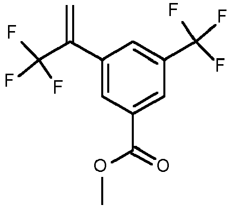

Methyl 3-bromo-5-(trifluoromethyl)benzoate (0.500 g, 1.68 mmol) was dissolved in dimethoxyethane (6.71 mL) and degased under argon for 5 minutes. Then Pd(PPh₃)₄ (0.0784 g, 0.0671 mmol) was added followed by 4,4,6-trimethyl-2-[1-(trifluoromethyl)vinyl]-1,3,2-dioxaborinane (0.348 mL, 1.68 mmol). Then a 2N solution of sodium carbonate (0.713 g, 6.71 mmol) in water (3.36 mL) was added dropwise. The reaction mixture was heated to 85°C for 3.5 hours and then the temperature was allowed to reach room temperature.The reaction mixture was quenched with water and extracted ethyl acetate (3x) . The combined organic layers were washed with brine, dried over sodium sulfate, filtered and concentrated. The crude was purified by chromatography over silica gel to afford methyl 3-(trifluoromethyl)-5-[1-(trifluoromethyl)vinyl]benzoate as a colorless liquid.
¹H-NMR (400 MHz, CDCl₃, ppm) δ : 3.99 (s, 3 H) 5.92 (q, J=1.22 Hz, 1 H) 6.14 (d, J=0.73 Hz, 1 H) 7.88 (s, 1 H) 8.30 - 8.35 (m, 2 H)

### Step B : Preparation of methyl 3-[2,2-difluoro-1-(trifluoromethyl)cyclopropyl1-5-(trifluoromethyl)benzoate

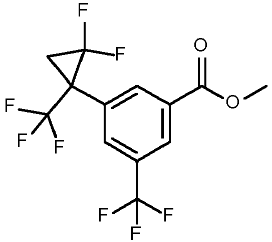

The desired product was prepared using methyl methyl 3-(trifluoromethyl) -5-[1-(trifluoromethyl)vinyl]benzoate and the conditions described for Compound P44 (Step A).
¹H-NMR (400 MHz, CDCl₃, ppm) δ : 8.2 - 8.6 (m, 2 H) 7.9 (s, 1 H) 4.0 (s, 3 H) 2.5 (ddd, J=12.56, 8.89, 6.05 Hz, 1 H) 2.0 (dddt, J=12.56, 8.16, 3.12, 1.47, 1.47 Hz, 1 H)

### Step C : Preparation of 3-[2,2-difluoro-1-(trifluoromethyl)cyclopropyl1-5-(trifluoromethyl)benzoic acid (I19)

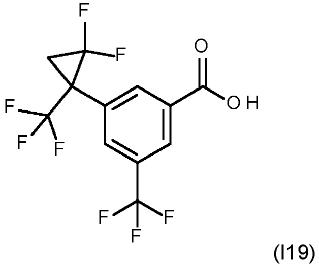

The desired product was prepared using methyl 3-[2,2-difluoro-1-(trifluoromethyl)cyclopropyl]-5-(trifluoromethyl)benzoate and the conditions described for Compound P31 (Step F).

LC-MS (method 1): Rt 1.01 min, m/z = 333 (M-H⁺).

### Step D : Preparation of N-(cyclopropylmethyl)-3-[2,2-difluoro-1-(trifluoromethyl)cyclopropyl]-N-[1-(2-pyrimidin-2-yl-1,2,4-triazol-3-yl)ethyl1-5-(trifluoromethyl)benzamide (Compound 41)

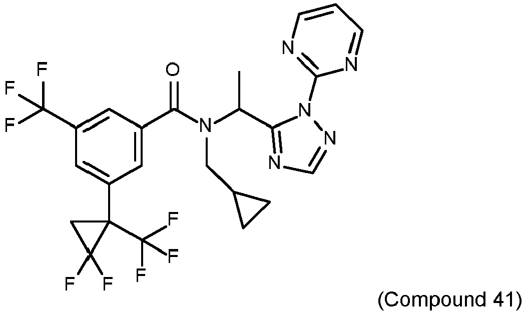

The desired product was prepared using 3-[2,2-difluoro-1-(trifluoromethyl)cyclopropyl]-5-(trifluoromethyl)benzoic acid and the conditions described for Compound P2 (Step E).

LC-MS (method 1): Rt 1.06 min, m/z = 561 (M+H⁺).

### Example 30: Preparation of N-(cyclopropylmethyl)-N-[1-(2-pyrimidin-2-yl-1,2,4-triazol-3-yl)ethyl]-3-(trifluoromethyl)-5-[1-(trifluoromethyl)cyclopropyl]benzamide (Compound P45)

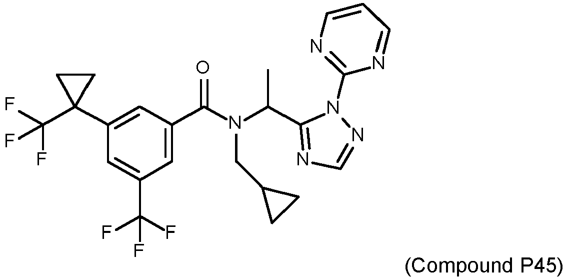

### Step A : Preparation of methyl 3-(trifluoromethyl)-5-[1-(trifluoromethyl)cyclopropyl]benzoate

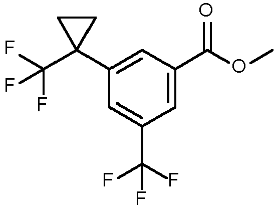

A solution of methyl 3-(trifluoromethyl)-5-[1-(trifluoromethyl)vinyl]benzoate (prepared in step A for Compound P41) (0.097 g, 0.33 mmol) in tetrahydrofuran (1.3 mL), under argon, was cooled to -78°C. Then diphenyl(methyl)sulfonium tetrafluoroborate (0.190 g, 0.65 mmol) and 1M lithium bis(trimethylsilyl)amide in THF (1.3 mL, 1.3 mmol) were added. The reaction mixture was allowed to warm to room temperature and it was stirred for 2 hours at room temperature. The reaction mixture was quenched with NH₄Cl aq. sol. and extracted (2x) with ethyl acetate. The combined organic phases were washed (2x) with water followed by brine, dried over sodium sulfate, filtered and concentrated. The crude was purified by chromatography over silica gel to afford methyl 3-(trifluoromethyl)-5-[1-(trifluoromethyl)cyclopropyl]benzoate as a colorless oil.

LC-MS (method 1): Rt 1.00 min, m/z = 311 (M-H⁺).

### Step B : Preparation of 3-(trifluoromethyl)-5-[1-(trifluoromethyl)cyclopropyl]benzoic acid (I22)

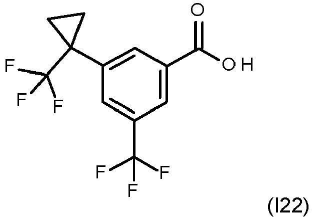

The desired product was prepared using 3-(trifluoromethyl)-5-[1-(trifluoromethyl)cyclopropyl]benzoate and the conditions described for Compound P31 (Step F).
¹H-NMR (400 MHz, CDCl₃, ppm) δ : 1.10 - 1.18 (m, 2 H) 1.21 - 1.35 (m, 1 H) 1.47 - 1.54 (m, 2 H) 7.98 (s, 1 H) 8.34 - 8.42 (m, 2 H)
LC-MS (method 1): Rt 1.03 min, m/z = 297 (M-H⁺).

### Step C : Preparation of N-(cyclopropylmethyl)-N-[1-(2-pyrimidin-2-yl-1,2,4-triazol-3-yl)ethyl]-3-(trifluoromethyl)-5-[1-(trifluoromethyl)cyclopropyl]benzamide (Compound P45)

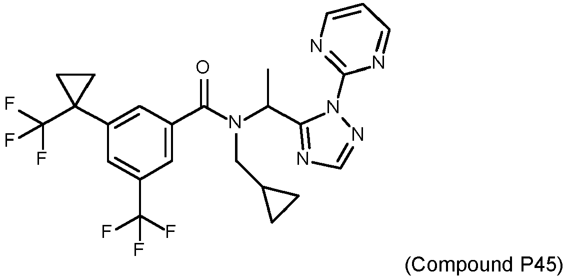

The desired product was prepared using 3-(trifluoromethyl)-5-[1-(trifluoromethyl)cyclopropyl]benzoic acid and the conditions described for Compound P2 (Step E).

LC-MS (method 1): Rt 1.09 min, m/z = 525 (M+H⁺).

### Example 31: Preparation of N-(cyclopropylmethyl)-N-[1-(2-pyrimidin-2-yl-1,2,4-triazol-3-yl)ethyl]-3-(trifluoromethyl)-5-(trifluoromethylsulfonyl)benzamide (Compound P89)

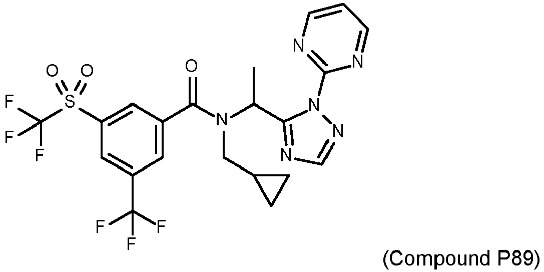

### Step A: Preparation of methyl 3-(trifluoromethyl)-5-(trifluoromethylsulfonyl)benzoate

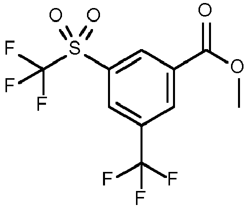

3-Chloroperbenzoic acid (2.3 g, 11 mmol) was added portionwise to a 0°C cooled solution of methyl 3-(trifluoromethyl)-5-(trifluoromethylsulfanyl)benzoate (prepared in step A for Compound P26) (1.8 g, 5.3 mmol) in dichloromethane (16 mL). After stirring for 1 hour at room temperature, more 3-chloroperbenzoic acid (2.3 g, 11 mmol) was added and the reaction mixture was stirred overnight. The precipitate formed was filtered. The filtrate was washed with 10% aqueous solution of sodium thiosulfate and with NaHCOs sat solution. The organic phase was dried over sodium sulfate, filtered and concentrated under reduced pressure. The crude was purified by chromatography over silica gel to afford methyl 3-(trifluoromethyl)-5-(trifluoromethylsulfonyl) benzoate.
¹H-NMR (400 MHz, CDCl₃, ppm) δ : 4.07 (s, 3 H) 8.43 - 8.51 (m, 1 H) 8.70 - 8.80 (m, 1 H) 8.84 - 8.91 (m, 1 H).
¹⁹F NMR (377 MHz, CDCl₃, ppm) δ : -77.49 (s, 3 F) -62.96 (s, 3 F)

### Step B: Preparation of 3-(trifluoromethyl)-5-(trifluoromethylsulfonyl)benzoic acid (I41)

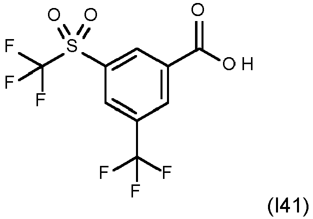

Methyl 3-(trifluoromethyl)-5-(trifluoromethylsulfonyl)benzoate (1.8 g, 5.4 mmol) was charged in a flask and dissolved in tetrahydrofuran (16 mL) and water (11 mL). To this mixture was added lithium hydroxide monohydrate (0.26 g, 11 mmol) and the reaction was stirred for 1 hour at room temperature. The reaction mixture was acidified with 1M hydrochloric acid, and the aqueous phase was extracted with ethyl acetate twice. The combined organic phases were dried over sodium sulfate, filtered and then concentrated to afford 3-(trifluoromethyl)-5-(trifluoromethylsulfonyl)benzoic acid which was used without further purification.

¹H-NMR (400 MHz, DMSO-d6, ppm) δ : 8.68 (s, 2 H) 8.71 - 8.76 (m, 1 H) 13.33 - 15.22 (m, 1 H). LC-MS (method 1): Rt 0.98 min, m/z = 321 (M-H⁺).

### Step C: Preparation of N-(cyclopropylmethyl)-N-[1-(2-pyrimidin-2-yl-1,2,4-triazol-3-yl)ethyl1-3-(trifluoromethyl)-5-(trifluoromethylsulfonyl)benzamide (Compound 89)

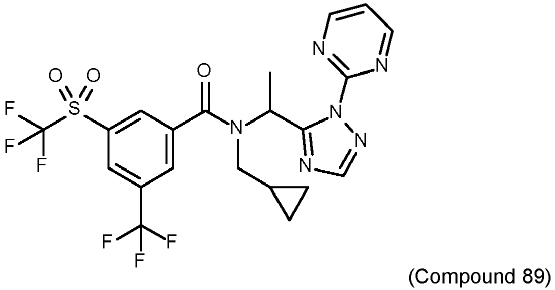

The desired product was prepared using 3-(trifluoromethyl)-5-(trifluoromethylsulfonyl)benzoic acid and the condition described for Compound P2 (Step E).
¹H-NMR (600 MHz, DMSO-d6, ppm) δ : -0.19 - -0.10 (m, 1 H) -0.06 (brdd, *J*=8.81 , 4.27 Hz, 1 H) 0.29 - 0.39 (m, 2 H) 0.71 - 0.78 (m, 1 H) 1.78 (d, *J*=6.90 Hz, 3 H) 3.13 (br dd, *J*=15.26, 6.18 Hz, 1 H) 3.23 (br dd, *J*=15.26, 5.99 Hz, 1 H) 6.19 - 6.27 (m, 1 H) 7.59 (t, *J*=4.90 Hz, 1 H) 8.08 - 8.20 (m, 3 H) 8.33 (s, 1 H) 8.87 (d, *J*=4.90 Hz, 2 H)
LC-MS (method 1): Rt 1.03 min, m/z = 549 (M+H⁺).

### Example 32: Preparation of 2-(1-cyanocyclopropyl)-N-(cyclopropylmethyl)-N-[1-(2-pyrimidin-2-yl-1,2,4-triazol-3-yl)ethyl]-6-(trifluoromethyl)pyridine-4-carboxamide (Compound P32)

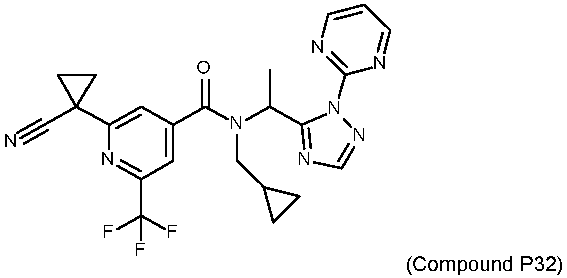

### Step A: Preparation of methyl 2-(1-cyano-2-ethoxy-2-oxo-ethyl)-6-(trifluoromethyl)pyridine-4-carboxylate

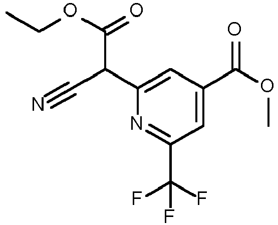

Methyl 2-chloro-6-(trifluoromethyl)pyridine-4-carboxylate (1.05 g, 4.40 mmol) was dissolved in dimethylsulfoxide (13.2 mL). Then ethyl 2-cyanoacetate (0.702 mL, 6.60 mmol), potassium carbonate (1.535 g, 11.00 mmol) and tetrabutylammonium bromide (0.145 g, 0.440 mmol) were added successively at room temperature. The resulting suspension was stirred 1 hour at 90 °C and then stirred overnight at room temperature. The reaction mass was diluted with 50 mL of water and 100 mL of ethyl acetate, cooled to 0-10 °C and slowly quenched with 1N hydrochloric acid until pH 3. The aqueous phase was extracted with ethyl acetate. The combined organic layers were dried over sodium sulfate and concentrated under reduced pressure at 50 °C. The crude material was purified by chromatography over silica gel with ethyl acetate in cyclohexane to afford methyl 2-(1-cyano-2-ethoxy-2-oxo-ethyl)-6-(trifluoromethyl)pyridine-4-carboxylate.

¹H-NMR (400 MHz, CDCl₃, ppm) δ : 1.36 - 1.43 (m, 3 H) 4.01 (s, 3 H) 4.34 (q, *J* = 7.58 Hz, 2 H) 7.34 (s, 1 H) 8.06 (s, 1 H) 14.46 - 14.67 (m, 1 H).

LC-MS (method 1): retention time 1.01 min, m/z 317 [M+H]⁺.

### Step B: Preparation of methyl 2-(cyanomethyl)-6-(trifluoromethyl)pyridine-4-carboxylate

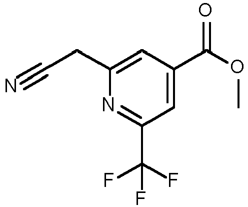

To a solution of methyl 2-(1-cyano-2-ethoxy-2-oxo-ethyl)-6-(trifluoromethyl)pyridine-4-carboxylate (0.800 g, 2.53 mmol) in dimethyl sulfoxide (20 mL) was added sodium chloride (0.299 g, 5.06 mmol) in water (10 mL). The resulting mixture was stirred for 4 hours at 95 °C. After cooling down to room temperature, the reaction mixture was diluted with water (50 mL) and extracted with ethyl acetate (3x50 mL). The combined organic layers were dried over sodium sulfate, filtered and contracted under reduced pressure to afford methyl 2-(cyanomethyl)-6-(trifluoromethyl)pyridine-4-carboxylate which was used without further purification.

¹H-NMR (400 MHz, CDCl₃, ppm) δ : 4.05 (s, 3 H) 4.13 (s, 2 H) 8.24 (s, 1 H) 8.26 (s, 1 H).

LC-MS (method 1): retention time 0.89 min, m/z 243 [M-H]⁻.

### Step C: Preparation of 2-(1-cyanocyclopropyl)-6-(trifluoromethyl)pyridine-4-carboxylic acid (113)

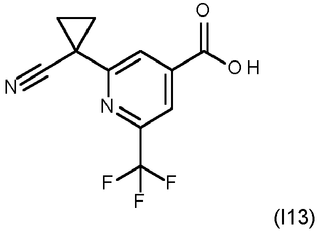

Methyl 2-(cyanomethyl)-6-(trifluoromethyl)pyridine-4-carboxylate (0.05 g, 0.20 mmol) was dissolved in dimethylformamide (2 mL). Sodium hydride (24 mg, 0.61 mmol) was added at room temperature and the colorless solution became a dark purple suspension. After 10 min, 1,2-dibromoethane (0.02 mL, 0.24 mmol) was added and the resulting suspension was stirred for 15 min at room temperature. The reaction mixture was quenched with a saturated ammonium chloride solution at 0-5 °C and diluted with ethyl acetate. The aqueous layer was acidified to pH 2-3 with 1N hydrochloric acid and extracted with ethyl acetate (2x). The combined organic layers were dried over sodium sulfate, filtered and evaporated under reduced pressure. The crude was purified by reverse phase chromatography to afford 2-(1-cyanocyclopropyl)-6-(trifluoromethyl)pyridine-4-carboxylic acid.

¹H-NMR (400 MHz, DMSO-d6, ppm) δ : 1.76 - 1.83 (m, 2 H) 1.96 - 2.03 (m, 2 H) 8.07 (d, *J* = 1.10 Hz, 1 H) 8.17 (s, 1 H) 13.35 - 15.45 (m, 1 H).

### Step D: Preparation of 2-(1-cyanocyclopropyl)-N-(cyclopropylmethyl)-N-[1-(2-pyrimidin-2-yl-1,2,4-triazol-3-yl)ethyl]-6-(trifluoromethyl)pyridine-4-carboxamide (Compound P32)

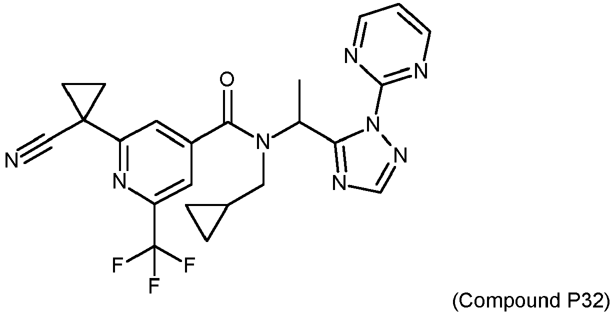

The desired product was prepared using 2-(1-cyanocyclopropyl)-6-(trifluoromethyl)pyridine-4-carboxylic acid and the condition described for Compound P2 (Step E).

LC-MS (method 1): Rt 0.96 min, m/z = 483 (M+H⁺).

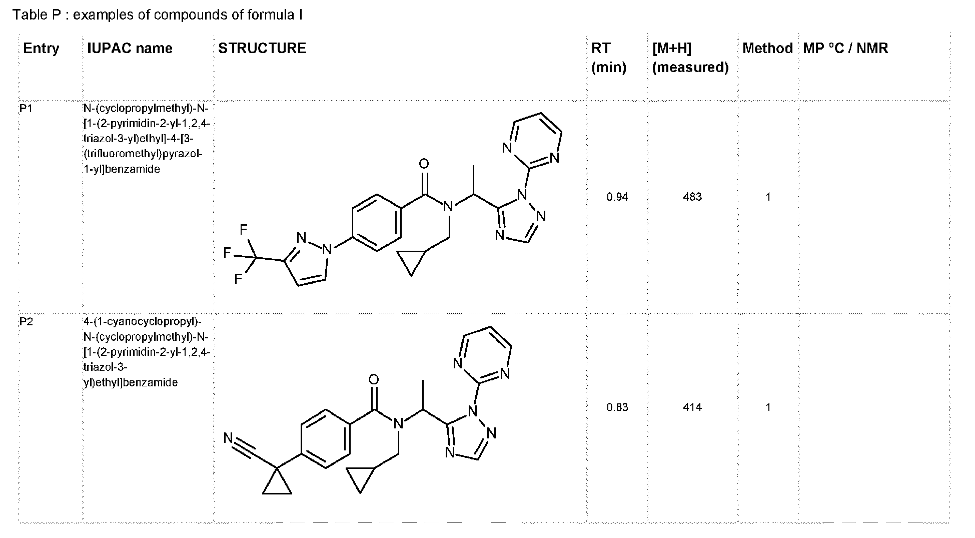

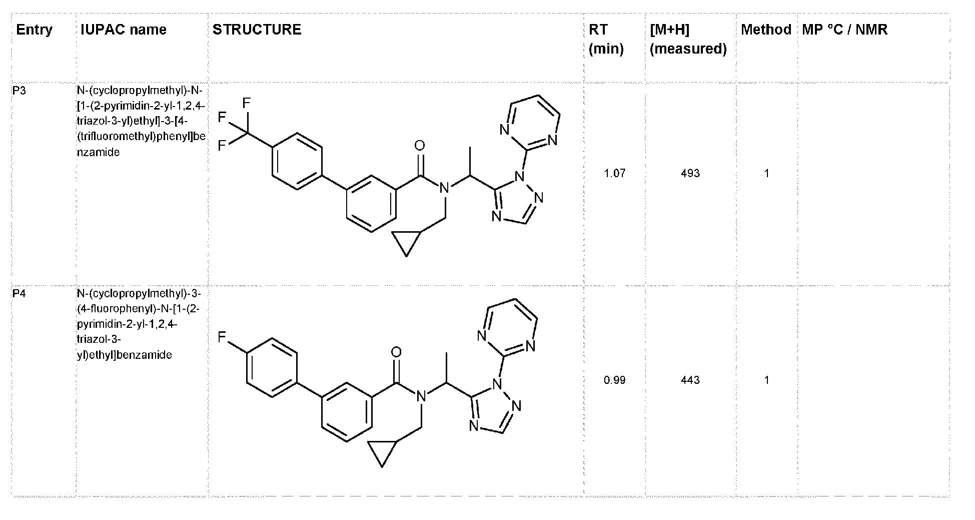

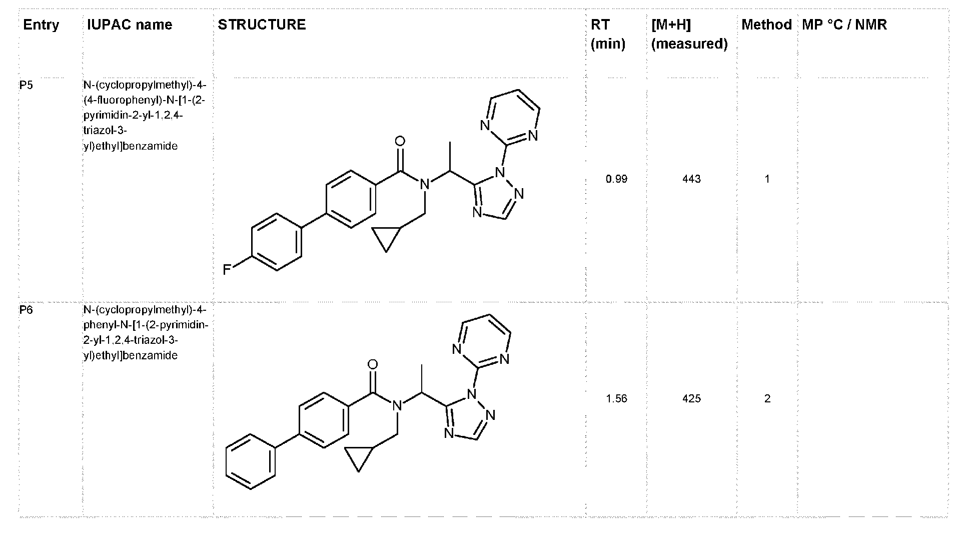

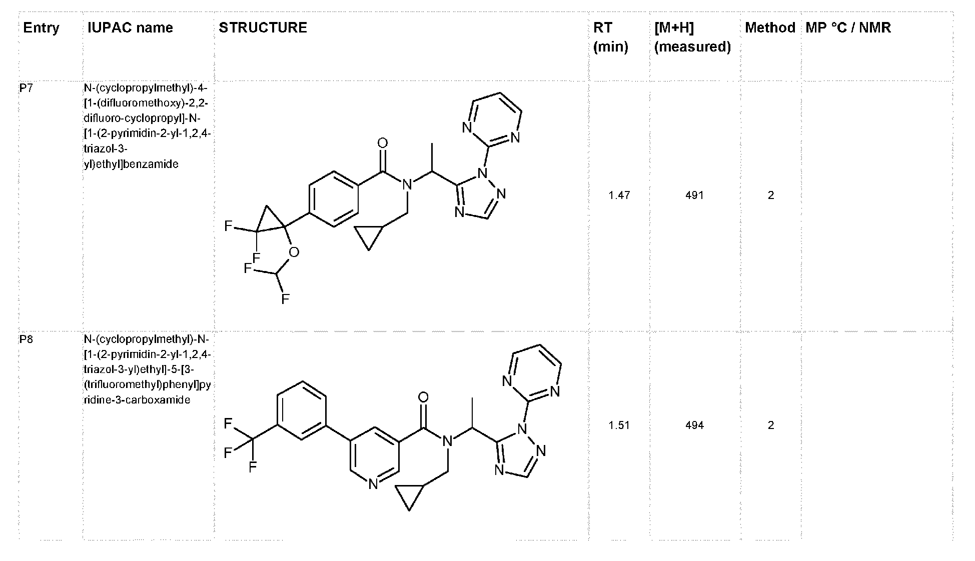

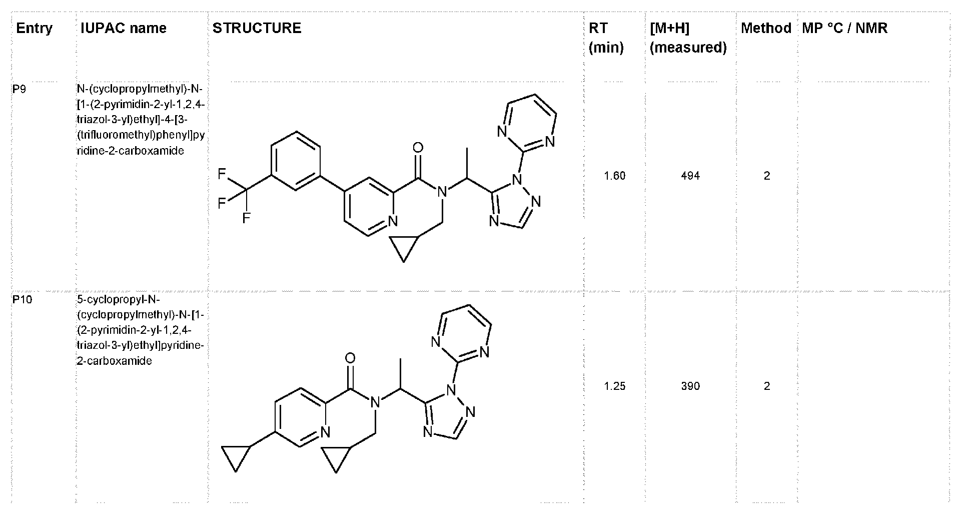

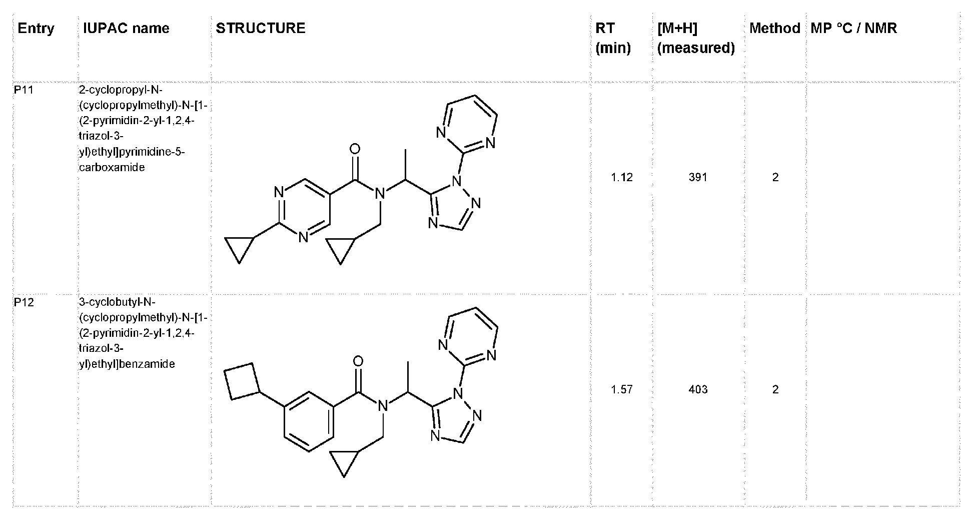

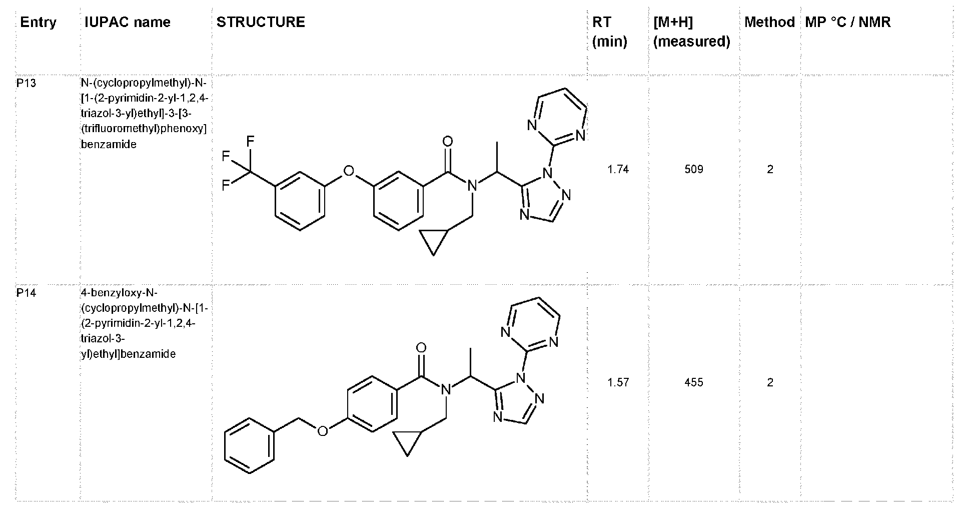

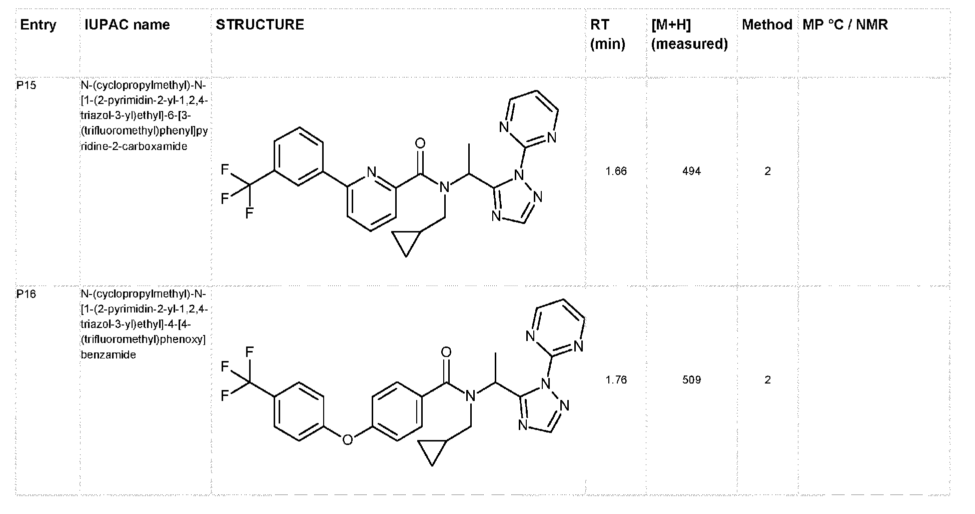

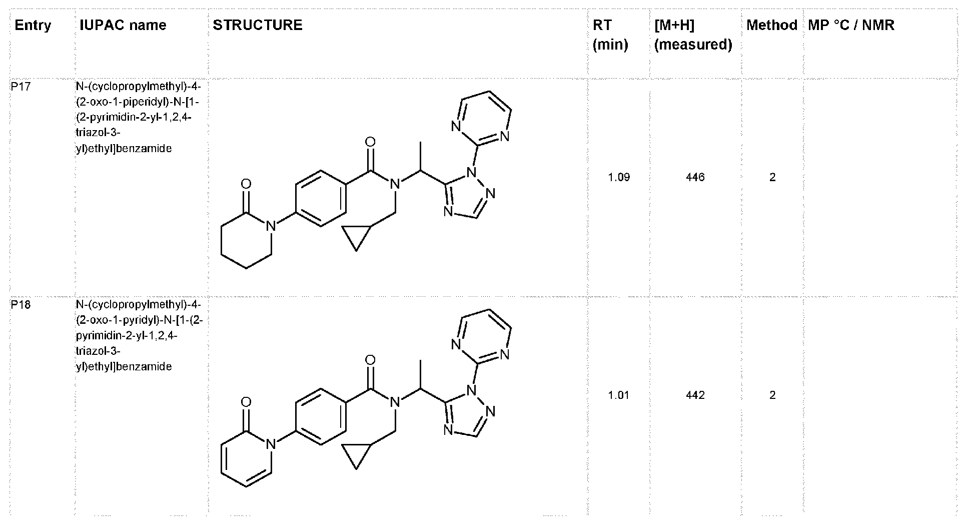

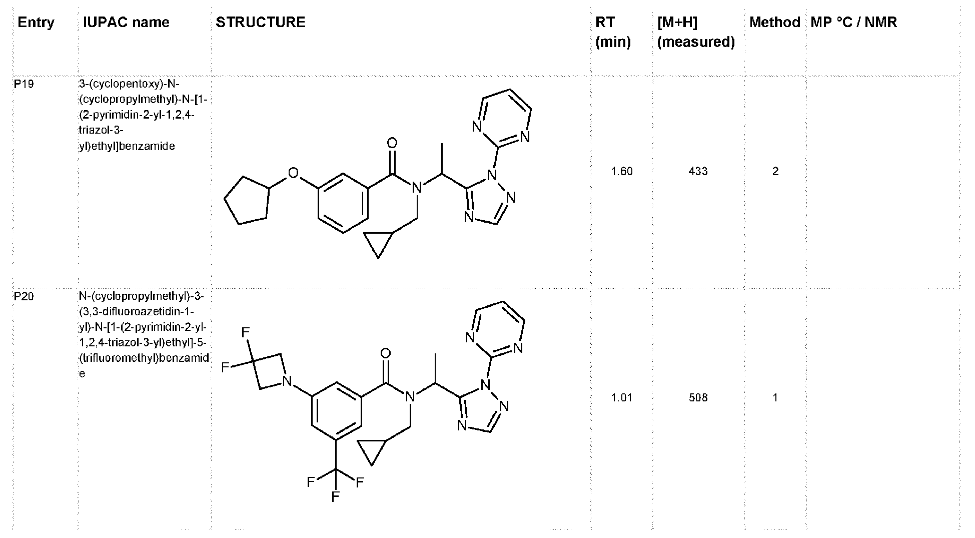

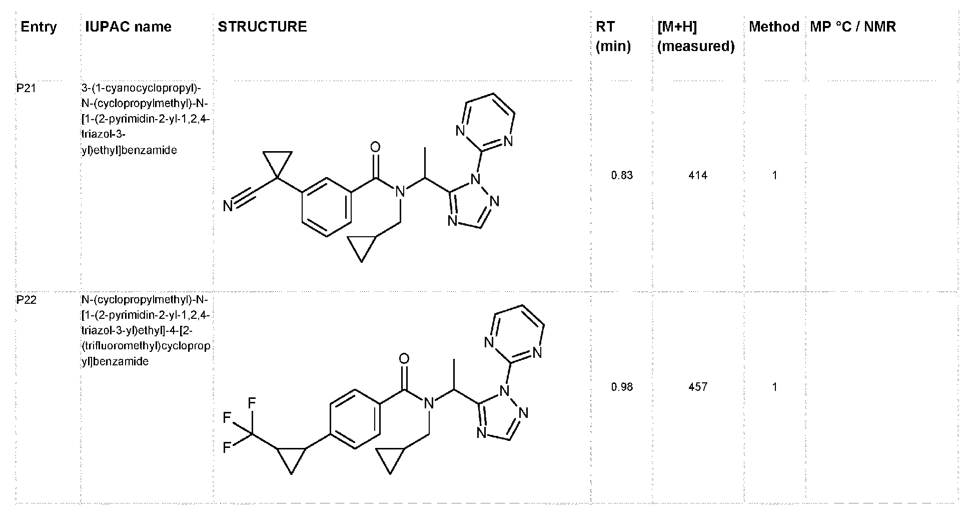

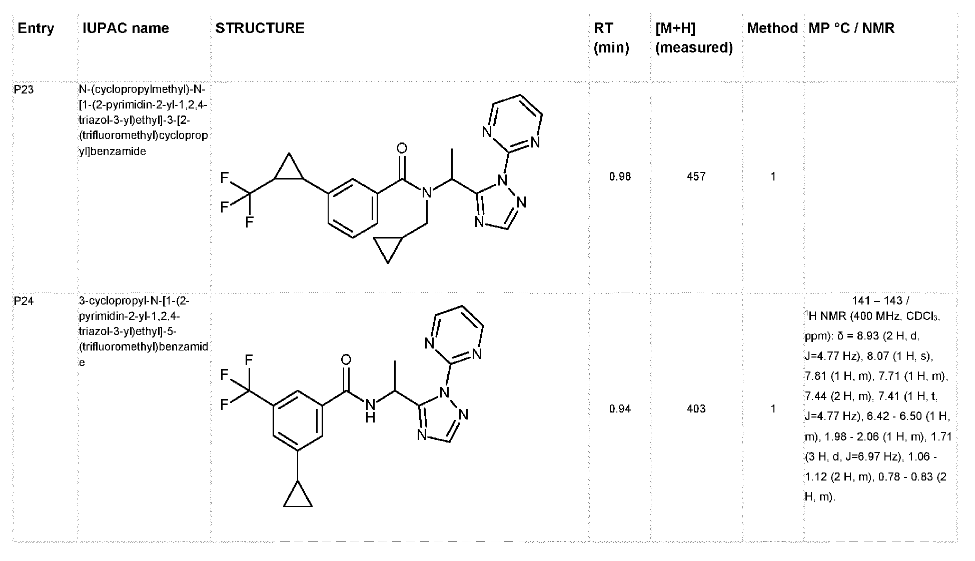

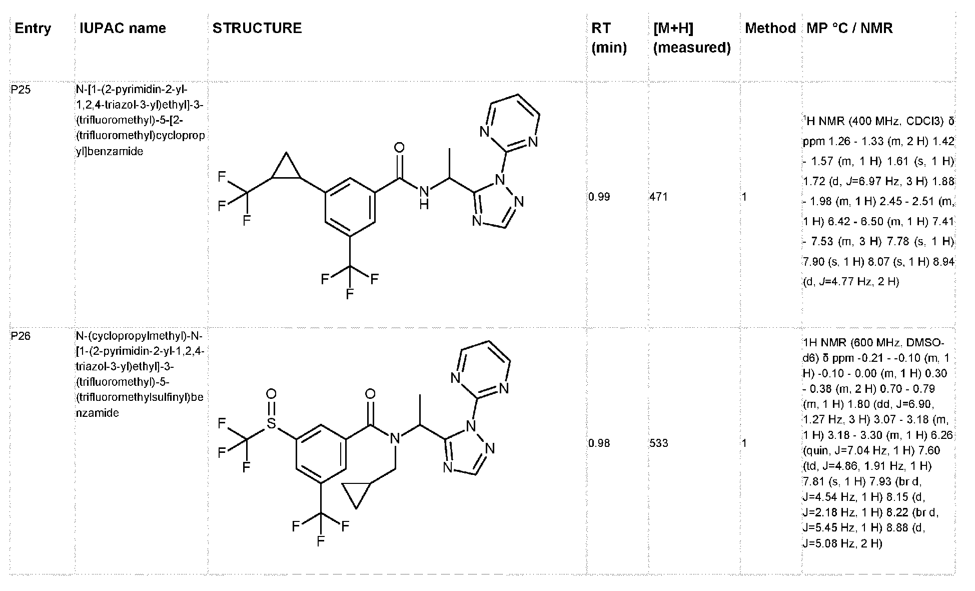

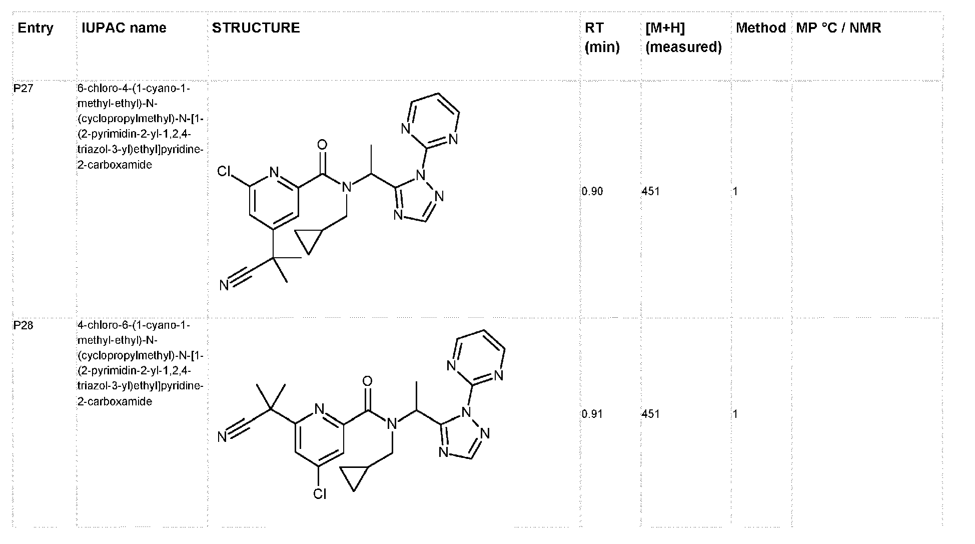

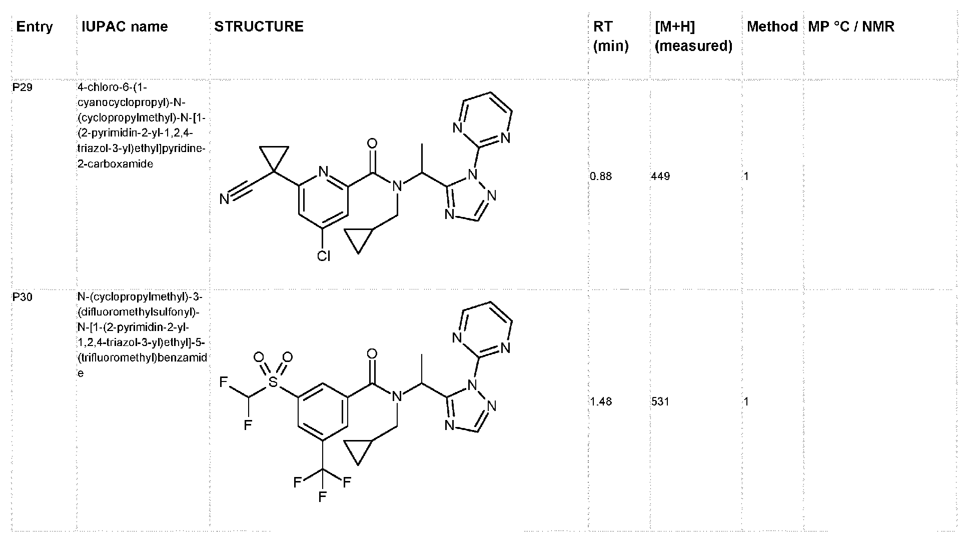

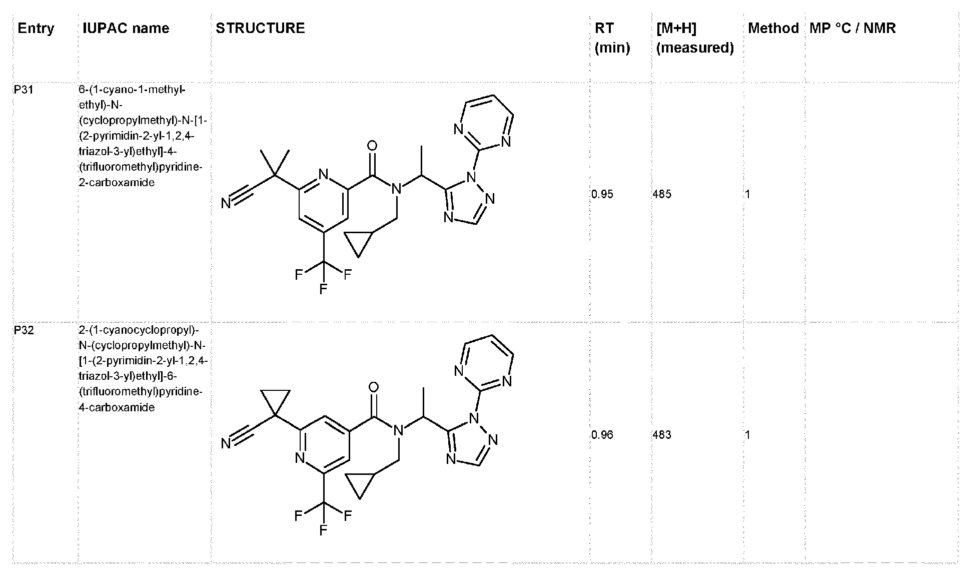

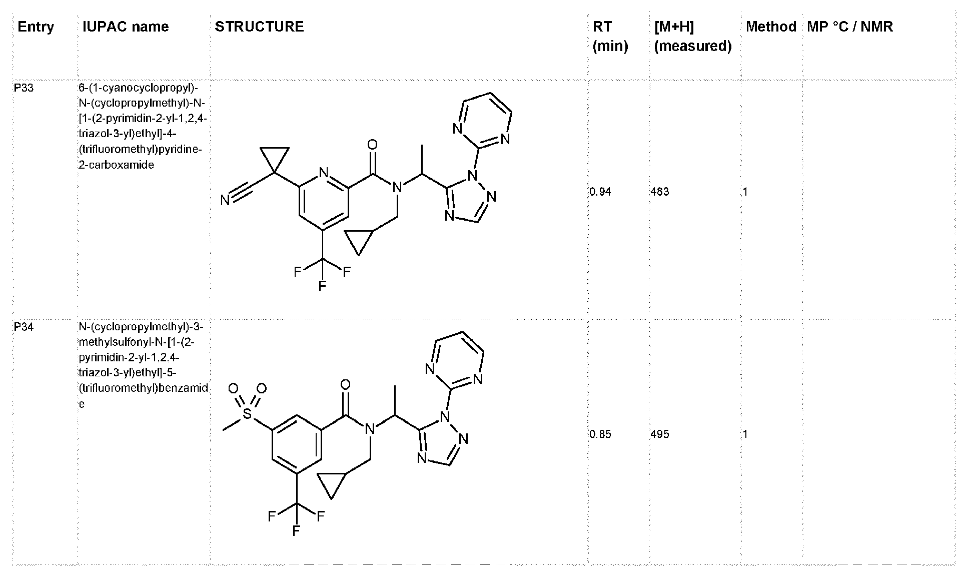

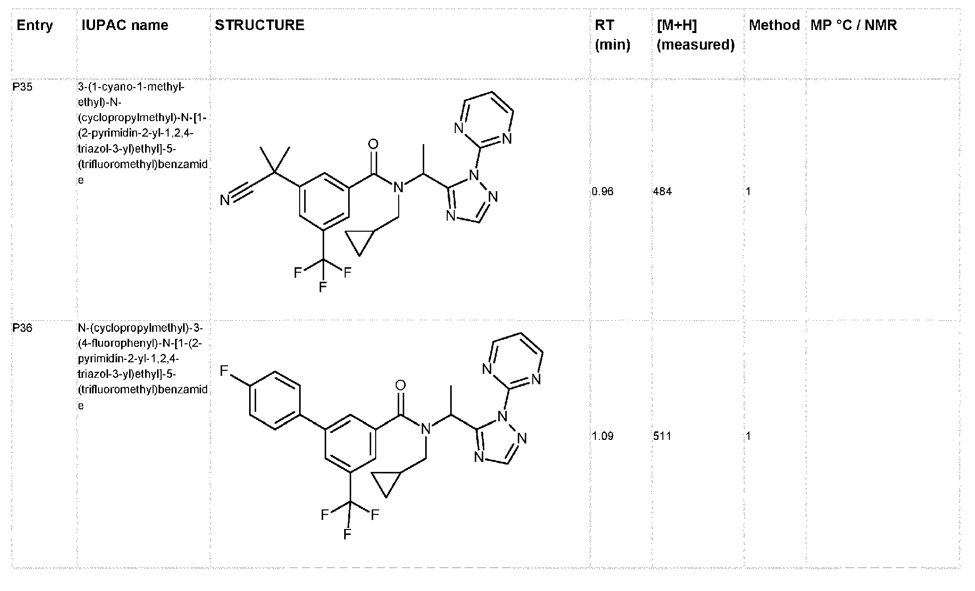

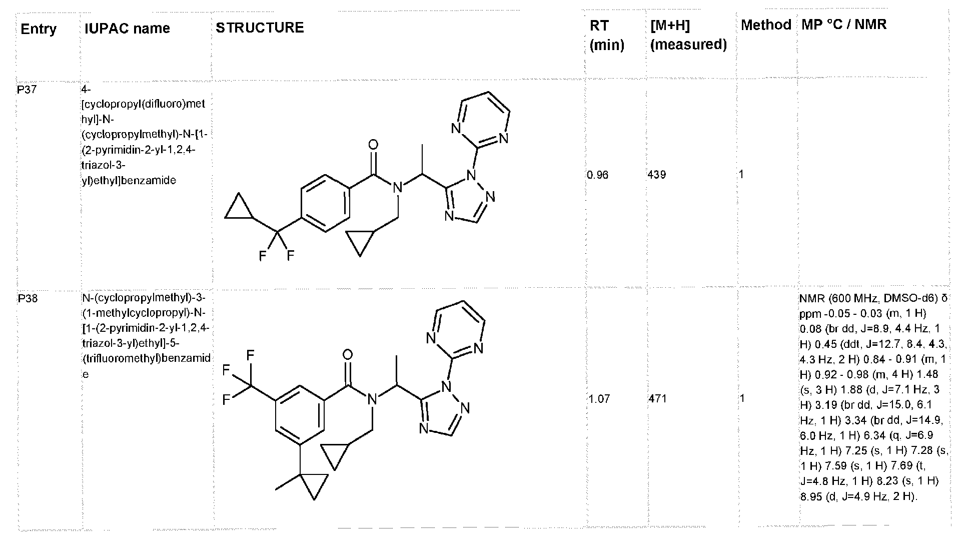

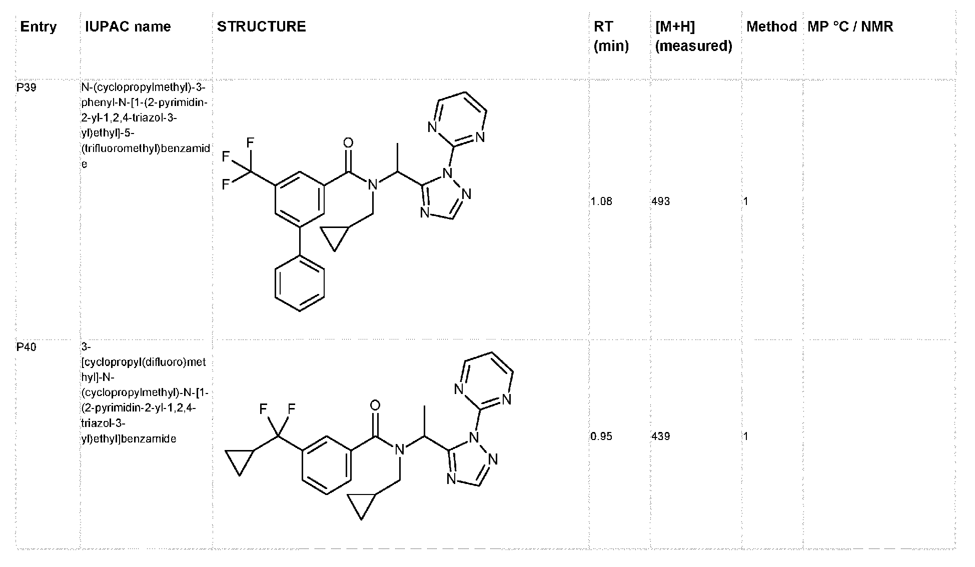

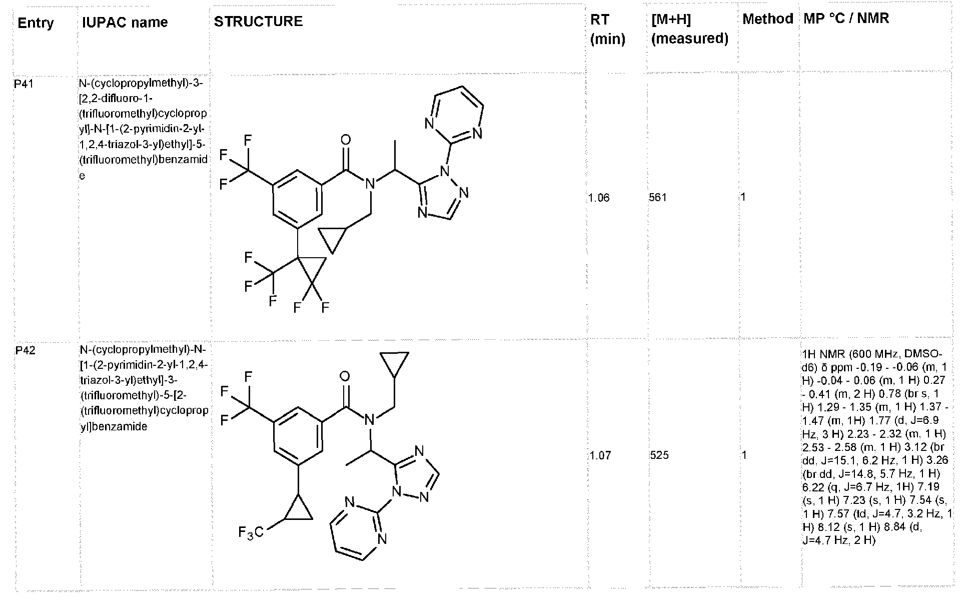

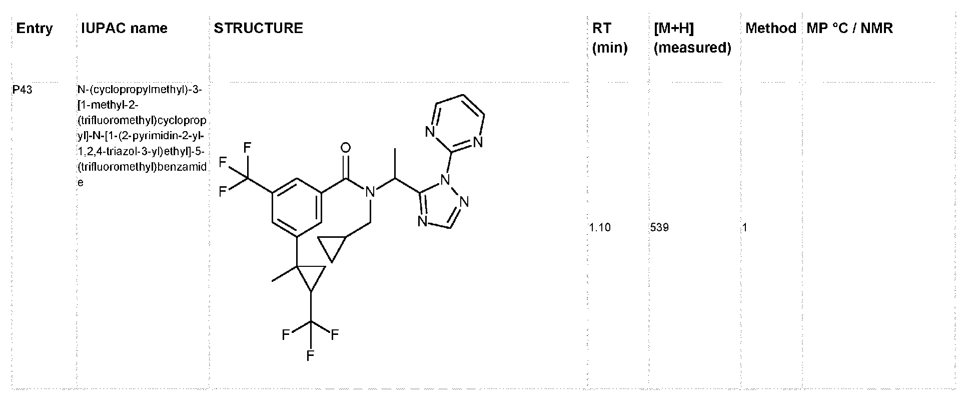

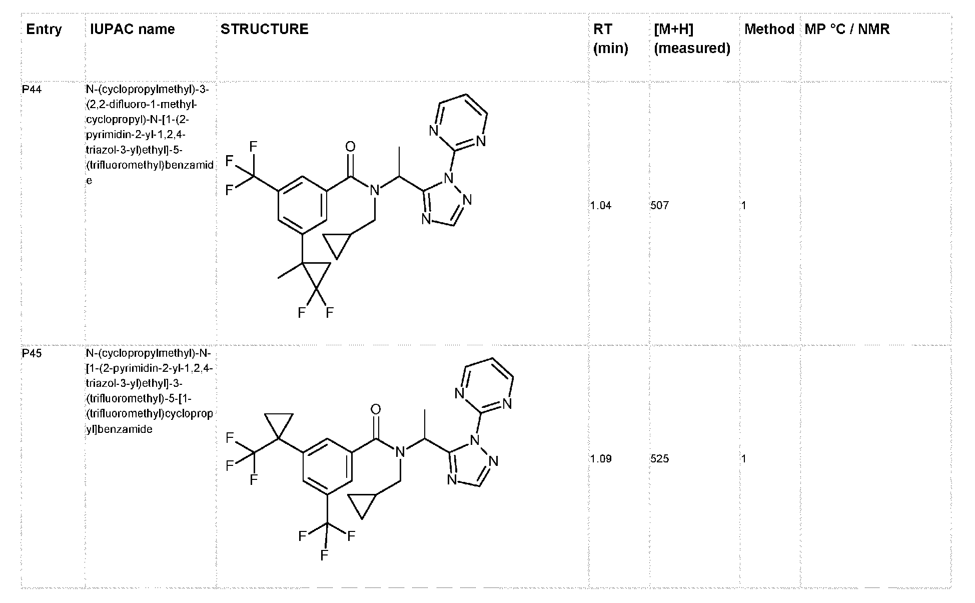

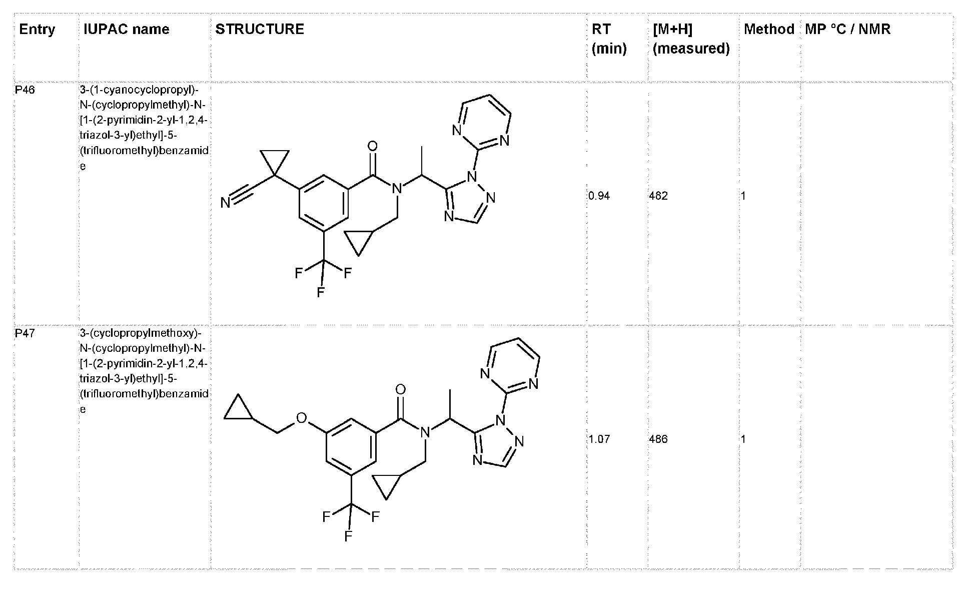

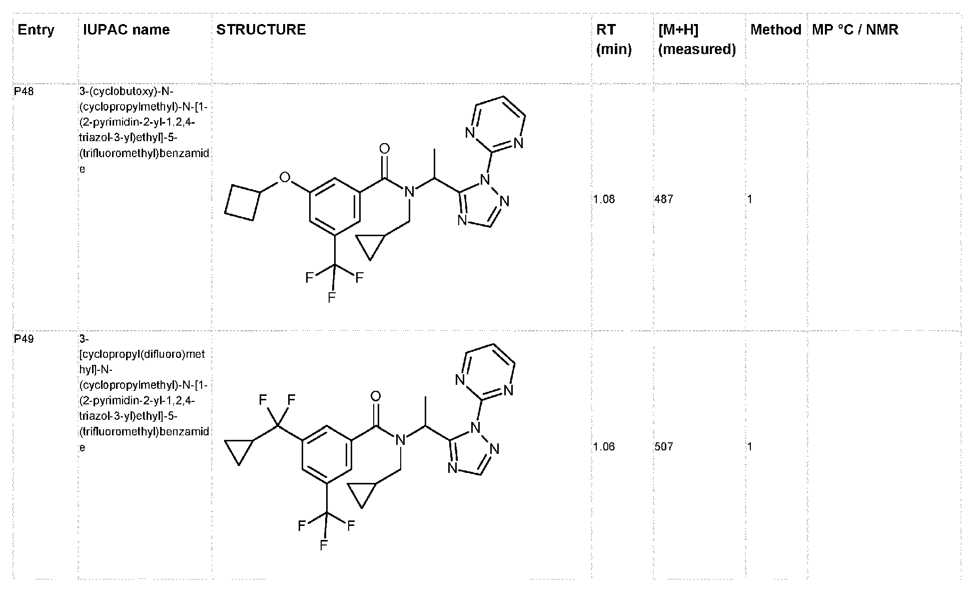

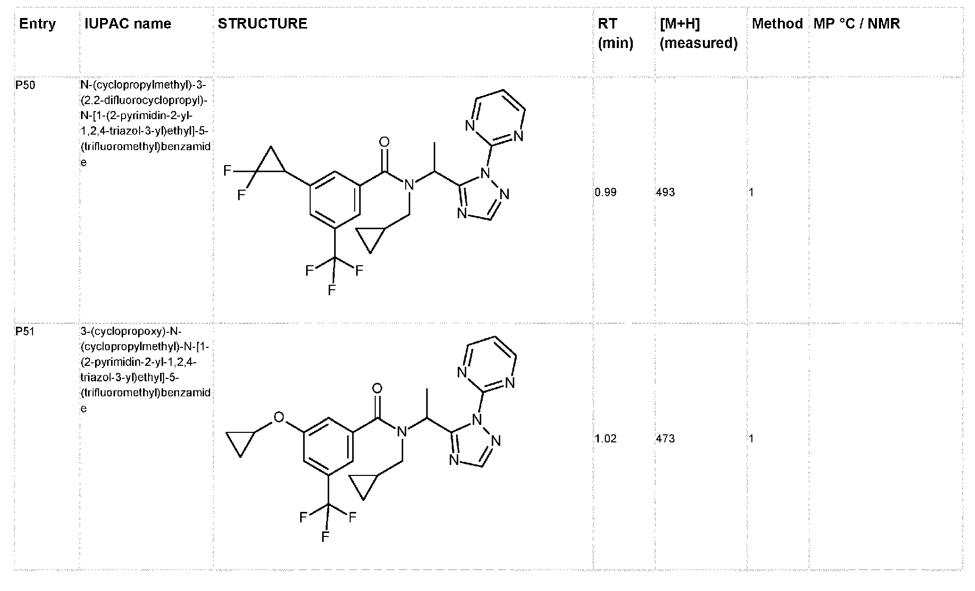

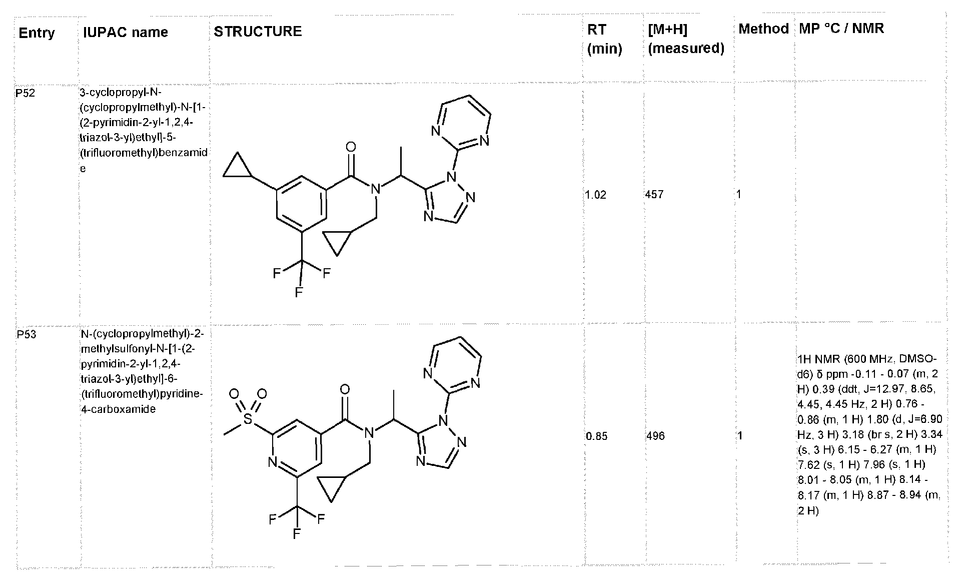

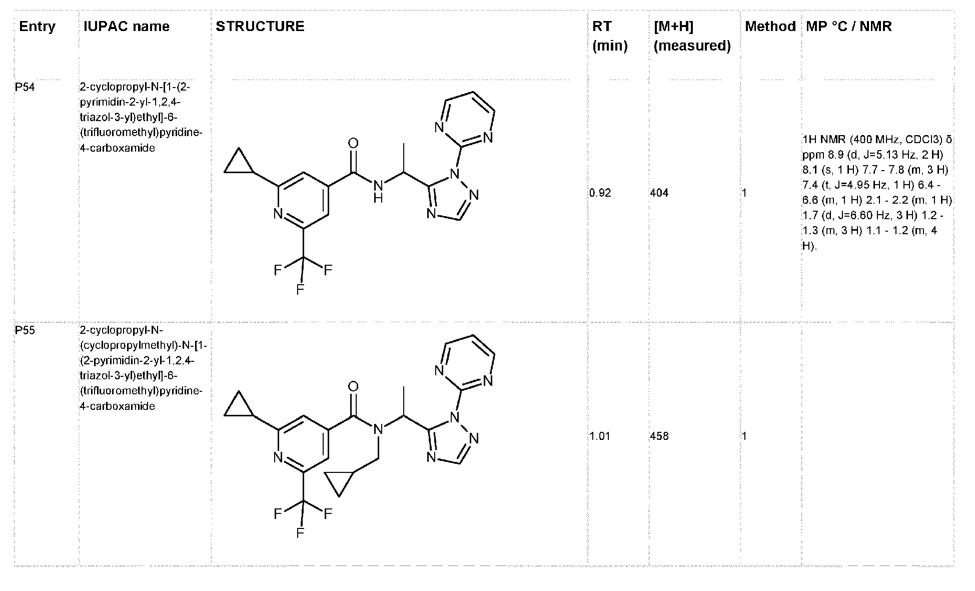

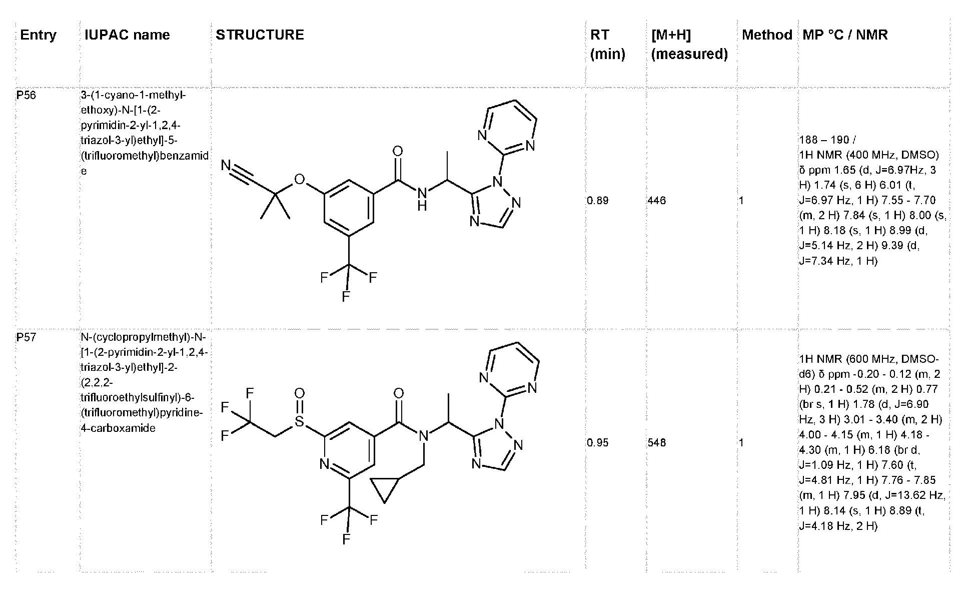

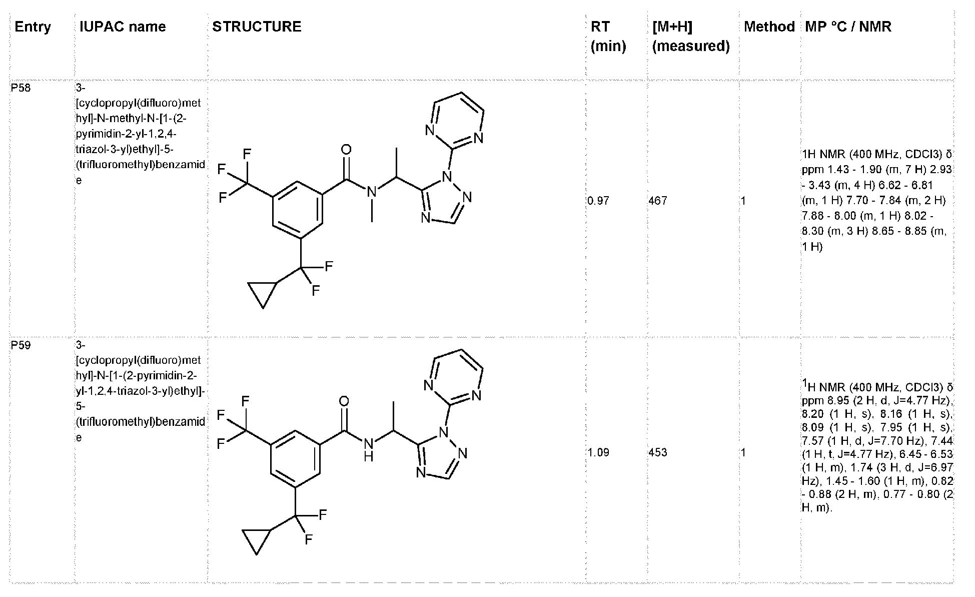

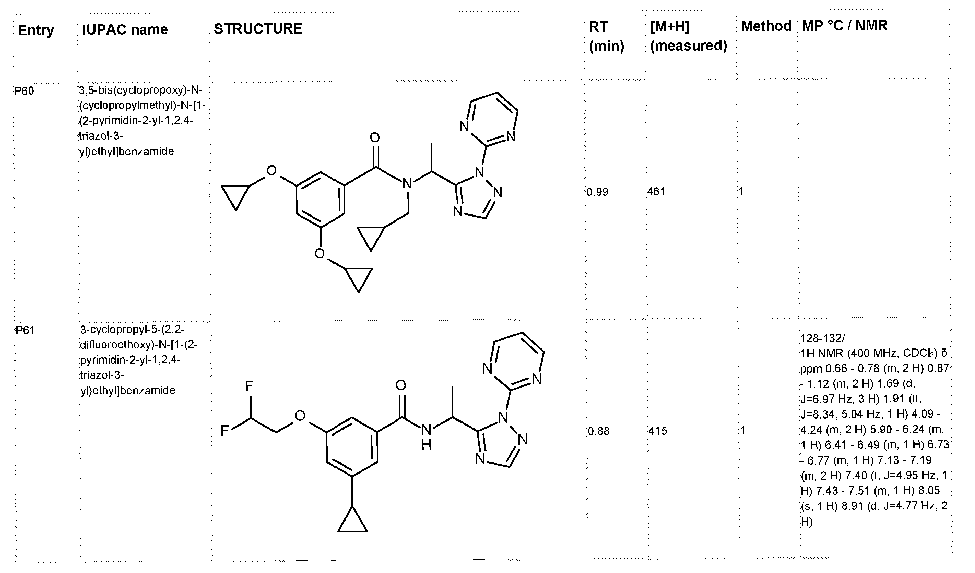

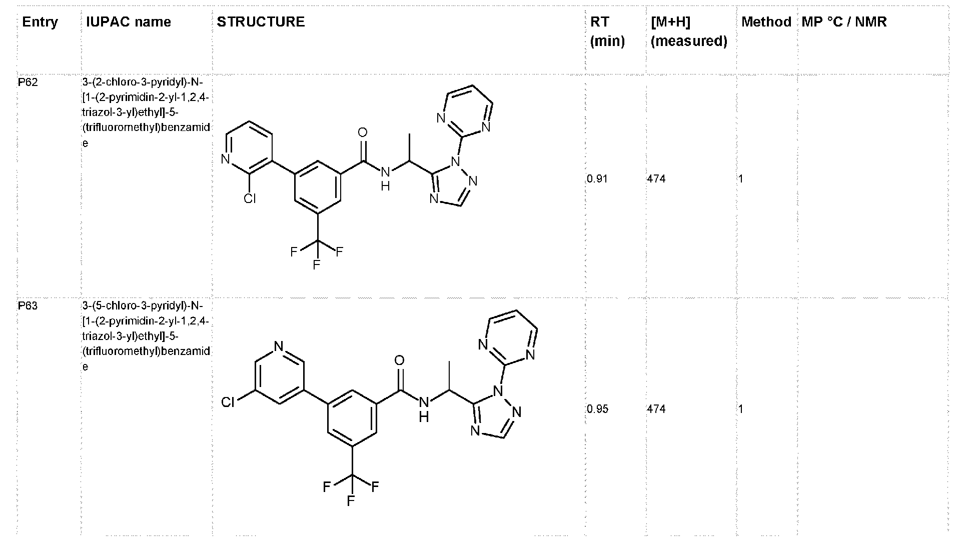

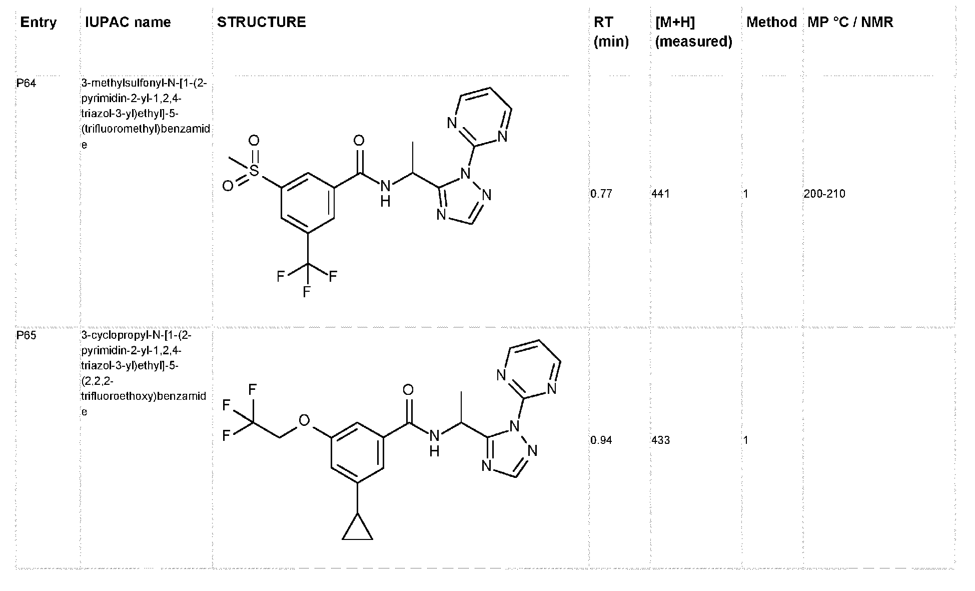

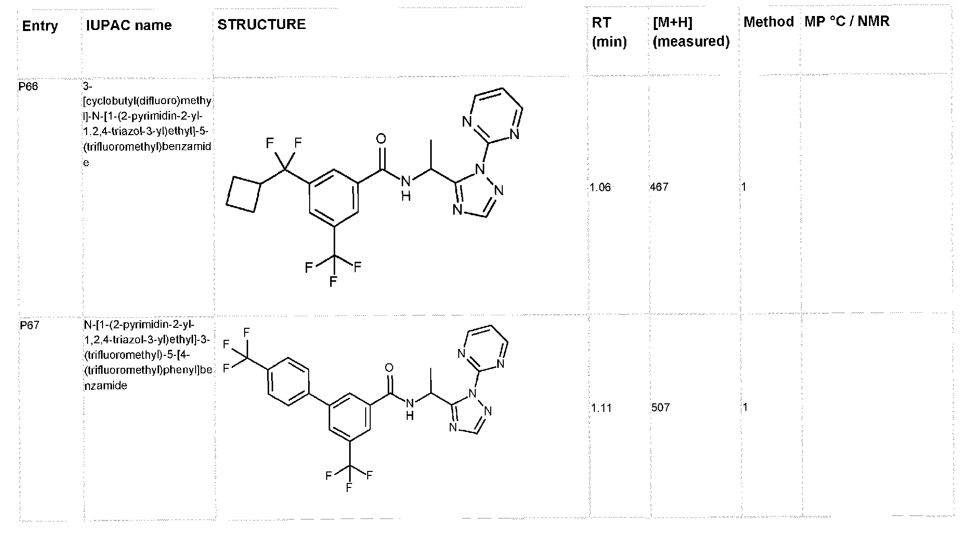

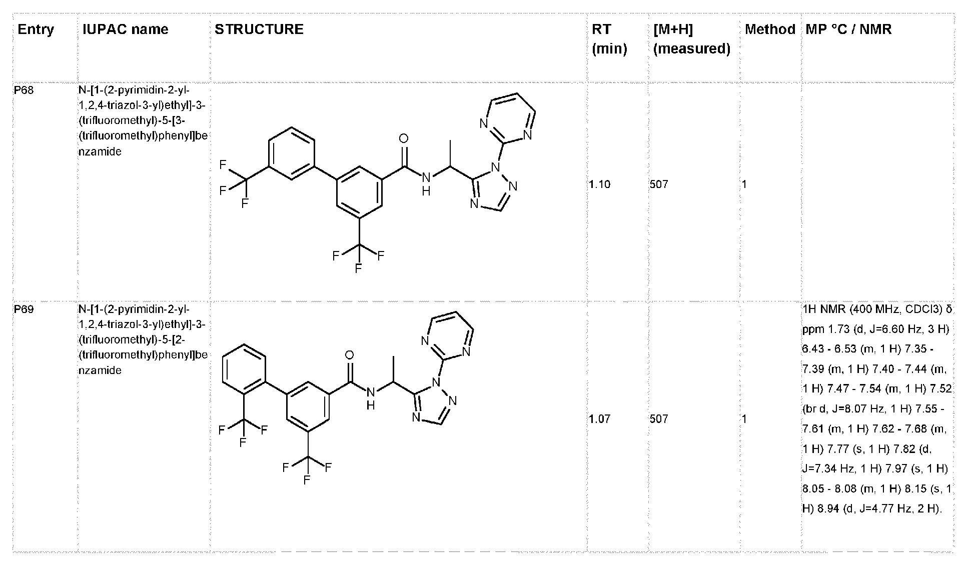

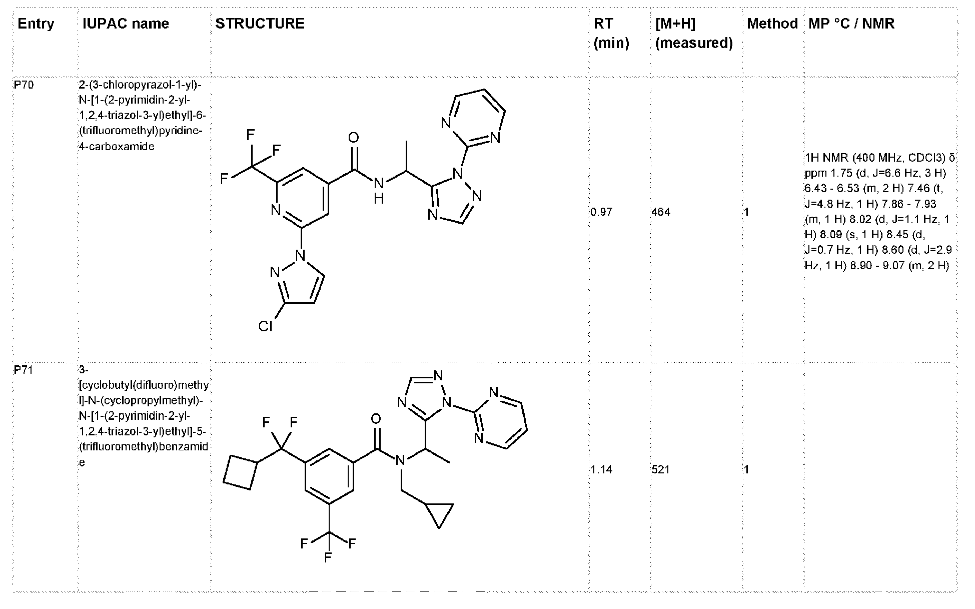

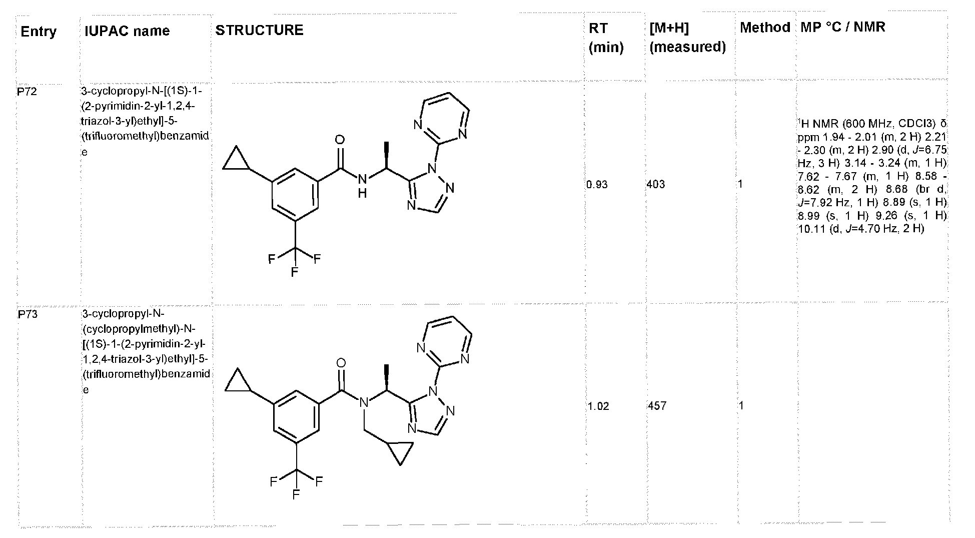

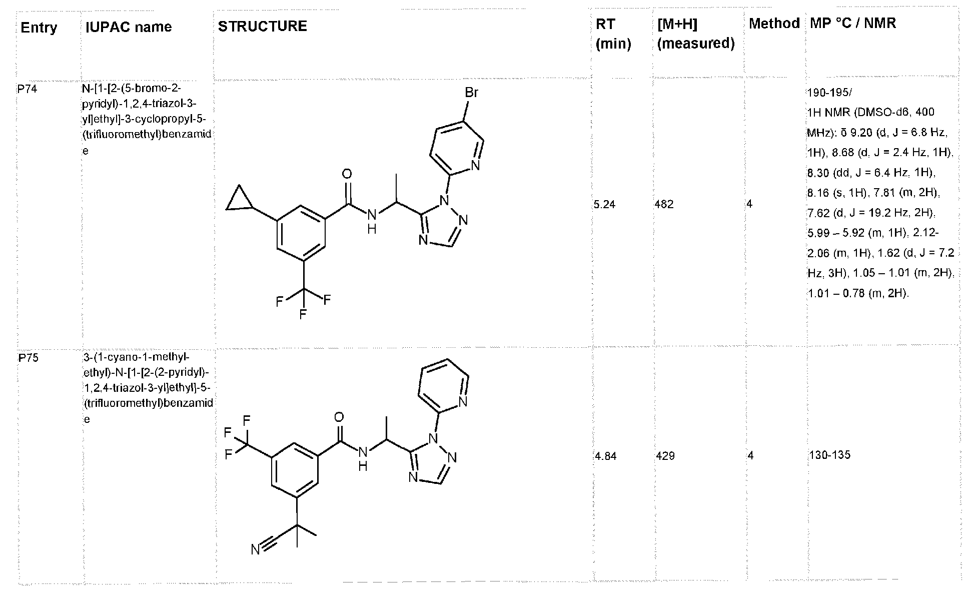

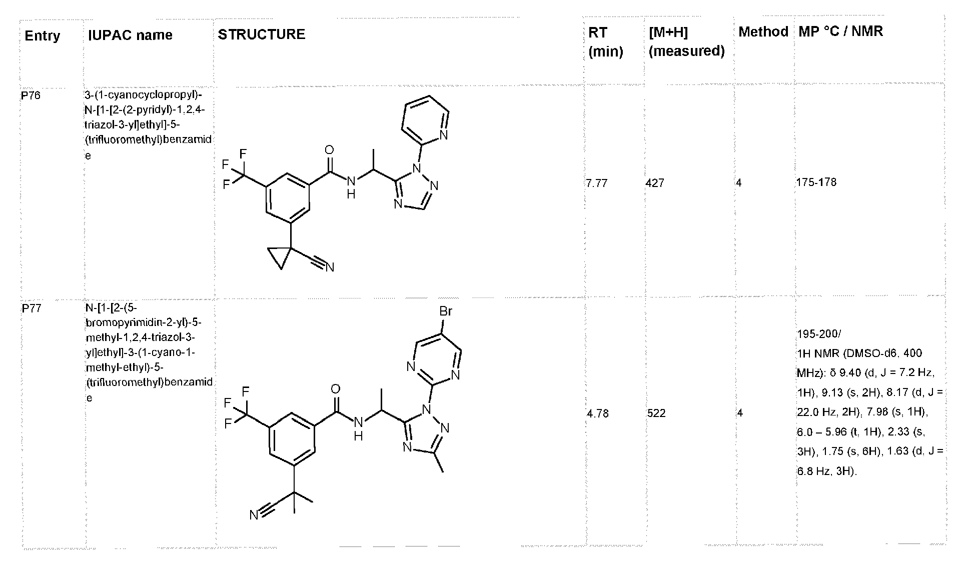

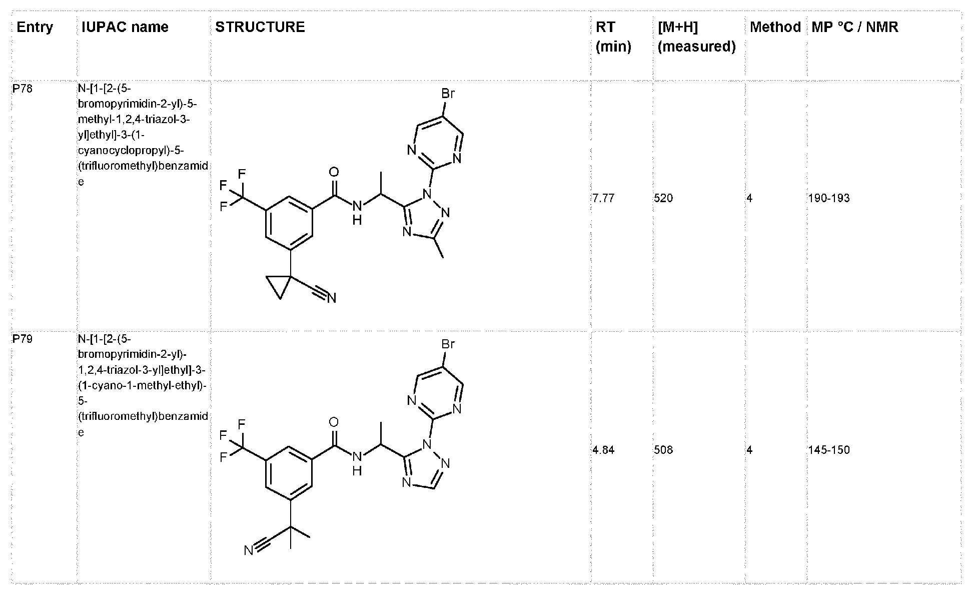

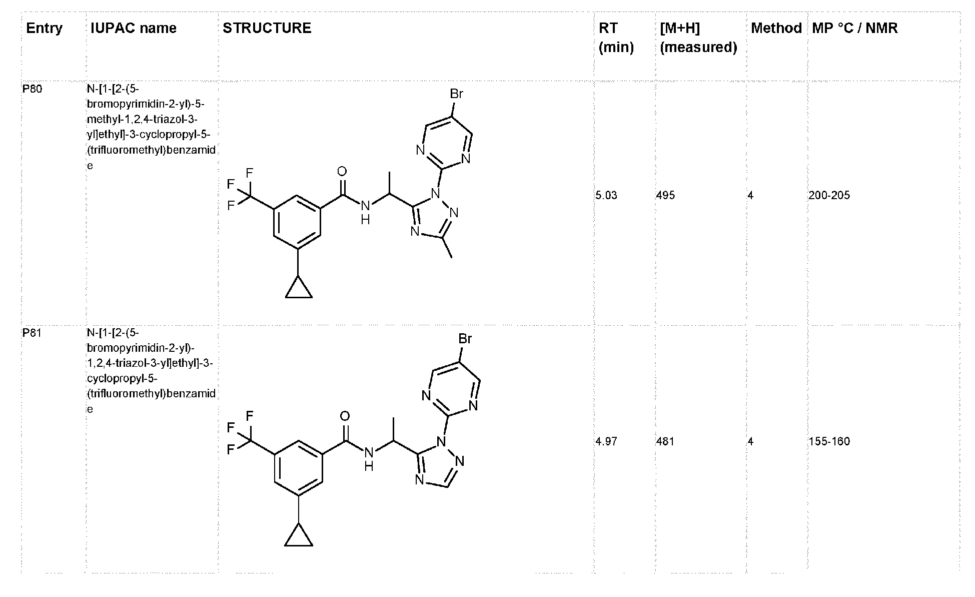

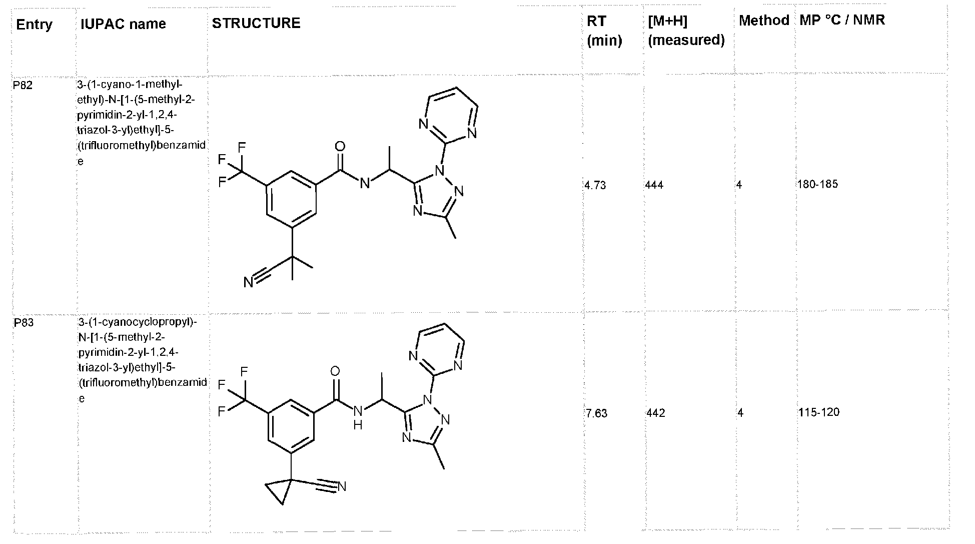

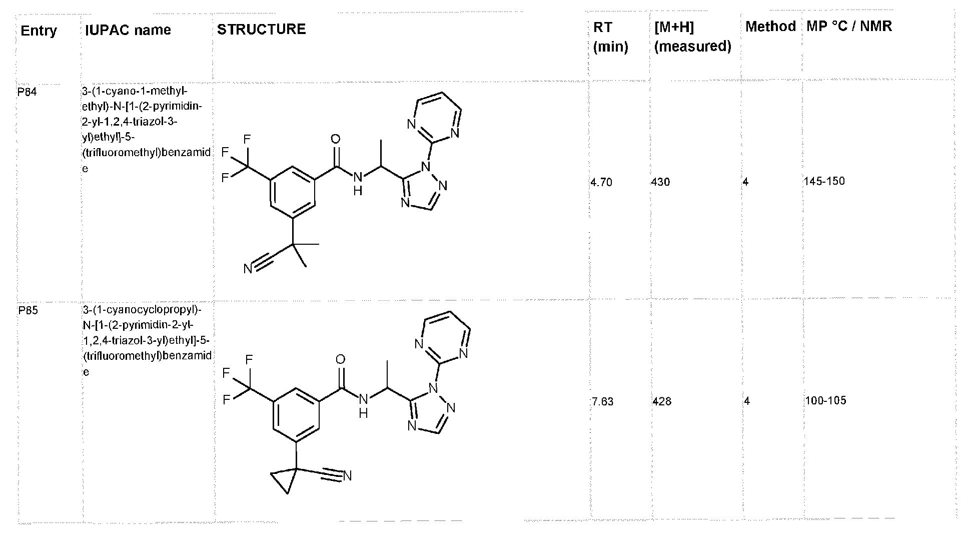

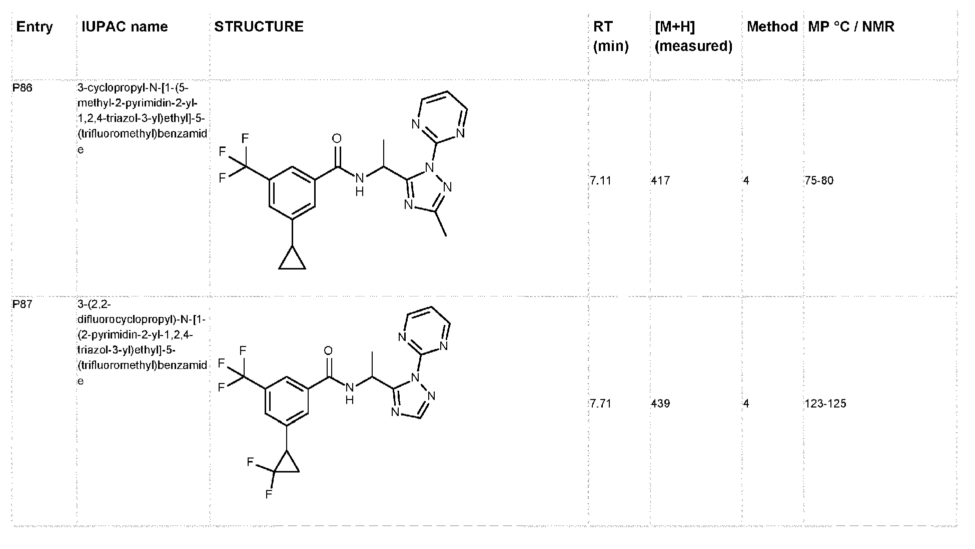

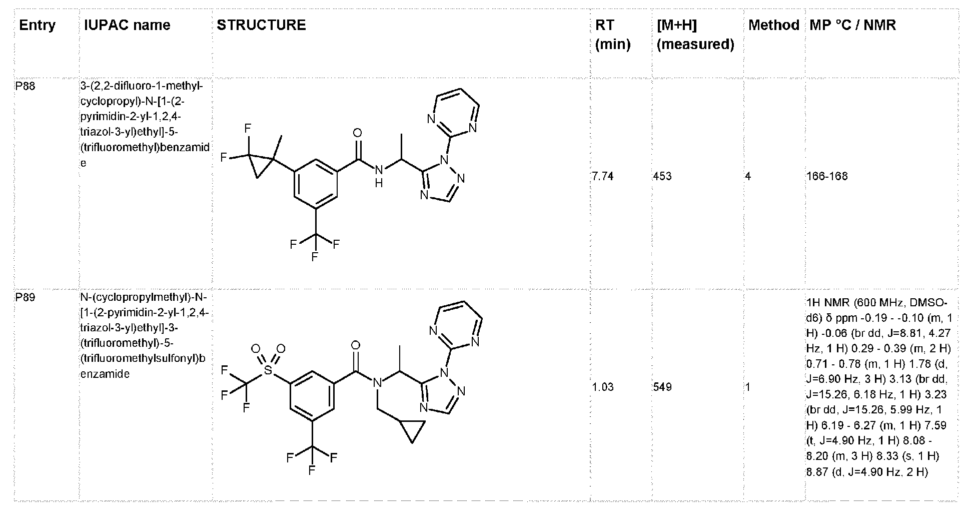

**Table I: Table of Intermediates**

| **Entry** | **IUPAC name** | **STRUCTURE** | **RT (min)** | **[M+H] (measured)** | **Method** | **NMR** |
|---|---|---|---|---|---|---|
| I1 | 2-[5-(1-bromoethyl)-1,2,4-triazol-1-yl]pyrimidine | 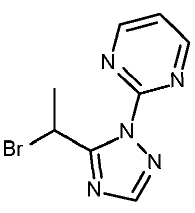 | 0.64 | 255 | 1 | ¹H-NMR (400 MHz, CDCl₃, ppm): □ = 8.90 (d, J=4.8 Hz, 2H), 8.07 (s, 1 H), 7.40 (t, J=4.8 Hz, 1H), 6.41 (q, J=6.9 Hz, 1H), 2.24 (d, J=6.9 Hz, 3H) |
| I2 | N-(cyclopropylmethyl)-1-(2-pyrimidin-2-yl-1,2,4-triazol-3-yl)ethanamine | 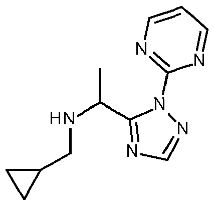 | 0.19 | 245 | 1 | ¹H-NMR (400 MHz, CDCl₃): □ = 8.88 (d, J=4.8 Hz, 2H), 8.05-8.05 (m, 1H), 8.03 (s, 1H), 7.37 (t, J=4.8 Hz, 1H), 4.96 (q, J=7.0 Hz, 1H), 2.55-2.33 (m, 2H), 2.22 (dd, J=7.5, 11.6 Hz, 1H), 1.54 (d, J=7.0 Hz, 3H), 0.96-0.81 (m, 1H), 0.43-0.34 (m, 2H), 0.07-0.09 (m, 2H) |
| I3 | 4-[2-(trifluoromethyl)cycloprop yl]benzoic acid | 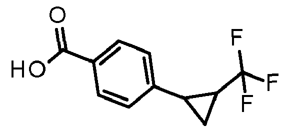 | 0.90 | 231 | 1 | |
| I4 | 3-[2-(trifluoromethyl)cycloprop yl]benzoic acid | 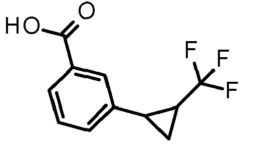 | 0.91 | 229 (M-H+) | 1 | |
| I5 | 3-cyclopropyl-5-(trifluoromethyl)benzoic acid | 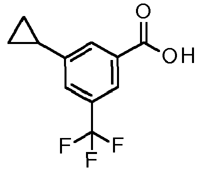 | 0.96 | 229 (M-H+) | 1 | ¹H NMR (DMSO-*d₆*, 400 MHz): δ 13.44 (s, 1 H), 7.90 (d, *J* = 21.6 Hz, 2H), 7.69 (s, 1 H), 2.20 - 2.13 (m, 1H), 1.08 - 1.03 (m, 2H), 0.83 - 0.79 (m, 2H). |
| I6 | 3-(trifluoromethyl)-5-[2-(trifluoromethyl)cycloprop yl]benzoic acid | 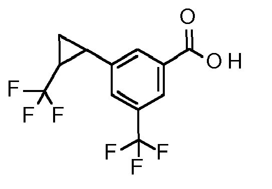 | 1.00 | 297 (M-H+) | 1 | |
| I7 | 3-(trifluoromethyl)-5-(trifluoromethylsulfinyl)benzoic acid | 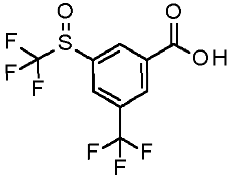 | 0.89 | 307 | 1 | ¹H NMR (400 MHz, DMSO-d6) δ ppm 8.47 (s, 1 H) 8.51 (s, 1 H) 8.64 (s, 1 H) 14.06 (br s, 1 H) |
| I8 | 6-chloro-4-(1-cyano-1-methyl- ethyl)pyridin e-2-carboxylic acid | 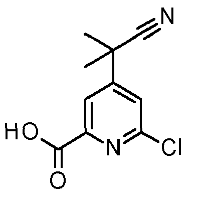 | 0.66 | 225 | 1 | ¹H NMR (400 MHz, CDCl3) δ ppm 1.78 (s, 6 H) 7.69 (s, 1 H) 8.18 (d, *J*=1.47 Hz, 1 H) |
| I9 | 4-chloro-6-(1-cyano-1-methyl-ethyl)pyridine-2-carboxylic acid | 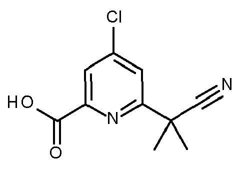 | 0.75 | 225 | 1 | ¹H NMR (400 MHz, CDCl3) δ ppm 1.82 (s, 6 H) 7.84 (d, *J*=1.83 Hz, 1 H) 8.20 (d, *J*=1.83 Hz, 1 H) |
| I10 | 4-chloro-6-(1-cyanocyclopropyl)pyridin e-2-carboxylic acid | 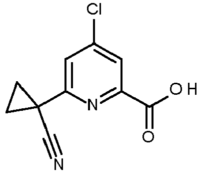 | 0.61 | 223 | 1 | ¹H NMR (400 MHz, DMSO-d6) δ ppm 7.50 (t, 1 H) 8.48 (s, 1 H) 8.59 - 8.73 (m, 2 H) 14.28 (br s, 1 H) |
| I11 | 3-(difluoromethylsulfonyl)-5-(trifluoromethyl)benzoic acid | 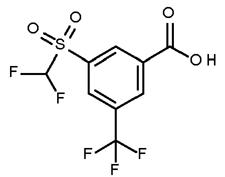 | 1.21 | 303 (M-H+) | 1 | |
| I12 | 6-(1-cyano-1-methylethyl)-4-(trifluoromethyl)pyridine-2-carboxylic acid | 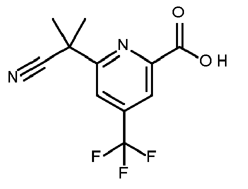 | 0.80 | 257 (M-H+) | 1 | ¹H NMR (400 MHz, CDCl3-d) δ ppm 8.46 (1 H, s), 806 (1 H, s), 1.88 (6 H, s) |
| I13 | 2-(1-cyanocyclopropyl)-6-(trifluoromethyl)pyridine-4-carboxylic acid | 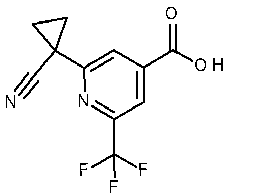 | | | | ¹H NMR (400 MHz, DMSO-d6) δ ppm: 1.76 - 1.83 (m, 2 H) 1.96 - 2.03 (m, 2 H) 8.07 (d, *J* = 1.10 Hz, 1 H) 8.17 (s, 1 H) 13.35 - 15.45 (m, 1 H). |
| I14 | 6-(1-cyanocyclopropyl)-4-(trifluoromethyl)pyridine-2-carboxylic acid | 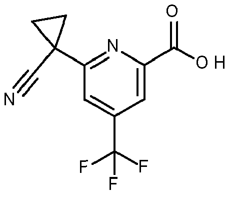 | 0.79 | 257 | 1 | 1 H NMR (400 MHz, CDCl3) δ ppm 9.74 - 10.27 (1 H, br s), 8.37 (1 H, m), 8.17 (1 H, m), 1.97 - 2.04 (2 H, m), 1.88 - 1.96 (2 H, m). |
| I15 | 3-(1-cyano-1-methylethyl)-5-(trifluoromethyl)benzoic acid | 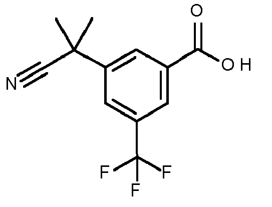 | | | | ¹H NMR (DMSO-*d*₆, 400 MHz): δ 13.6 (brs, 1H), 8.35 (s, 1H), 8.14 (d, *J* = 9.2 Hz, 2H), 1.78 (s, 6H). |
| I16 | 4-[cyclopropyl(difluoro)met hyl]benzoic acid | 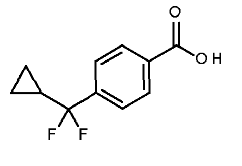 | 0.88 | 211 (M-H+) | 1 | |
| I17 | 3-(1-methylcyclopropyl)-5-(trifluoromethyl)benzoic acid | 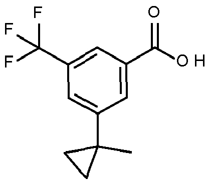 | 1.02 | 243 | 1 | 1H NMR (400 MHz, CDCl3) δ ppm 8.17 (1 H, m), 8.16 (1 H, m), 7.74 (1 H, m), 1.48 (3 H, s), 0.91 - 0.98 (2 H, m), 0.79 - 0.90 (2 H, m). |
| I18 | 3-[cyclopropyl(difluoro)met hyl]benzoic acid | 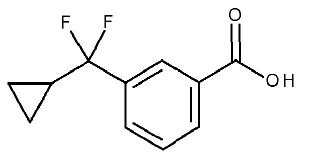 | 0.87 | 211 (M-H+) | 1 | |
| I19 | 3-[2,2-difluoro-1-(trifluoromethyl)cycloprop yl]-5-(trifluoromethyl)benzoic acid | 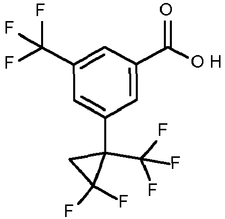 | 1.01 | 333 (M-H+) | 1 | |
| I20 | 3-[1-methyl-2-(trifluoromethyl)cycloprop yl]-5-(trifluoromethyl)benzoic acid | 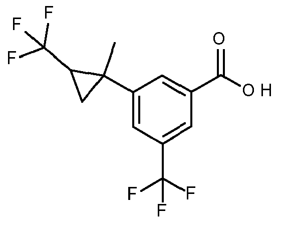 | 1.05 | 311 (M-H+) | 1 | |
| I21 | 3-(2,2-difluoro-1-methylcyclopropyl)-5-(trifluoromethyl)benzoic acid | 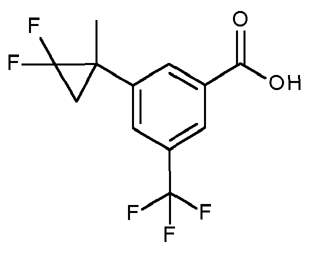 | 0.98 | 279 (M-H+) | 1 | 1H NMR (400 MHz, CDCI3) δ ppm 8.31 (1 H, s), 8.26 (1 H, s), 7.83 (1 H, s), 1.75 - 1.81 (1 H, m), 1.60 (3 H, m), 0.54 - 1.60 (1 H, m). |
| I22 | 3-(trifluoromethyl)-5-[1-(trifluoromethyl)cycloprop yl]benzoic acid | 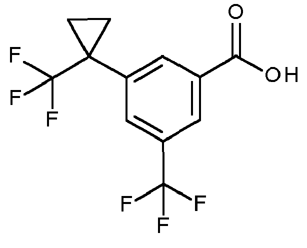 | 1.03 | 297 (M-H+) | 1 | ¹H NMR (400 MHz, CDCl3) δ ppm 1.10 - 1.18 (m, 2 H) 1.21 - 1.35 (m, 1 H) 1.47 - 1.54 (m, 2 H) 7.98 (s, 1 H) 8.34 - 8.42 (m, 2 H) |
| I23 | 3-(1-cyanocyclopropyl)-5-(trifluoromethyl)benzoic acid | 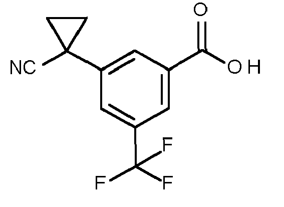 | 0.85 | 254 (M-H+) | 1 | |
| I24 | 3-(cyclopropylmethoxy)-5-(trifluoromethyl)benzoic acid | 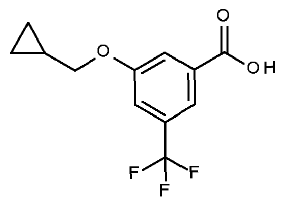 | 1.00 | 259 (M-H+) | 1 | |
| I25 | 3-(cyclobutoxy)-5-(trifluoromethyl)benzoic acid | 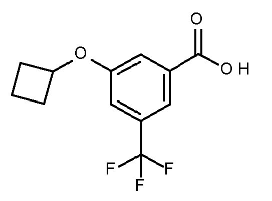 | 1.03 | 261 | 1 | |
| I26 | 3-[cyclopropyl(difluoro)met hyl]-5- (trifluoromethyl)benzoic acid | 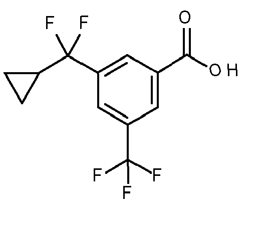 | | | | ¹H NMR (400 MHz, CDCl₃, ppm): δ = 10.70 - 12.60 (1 H, br s), 8.48 (1 H, s), 8.45 (1 H, s), 8.05 (1 H, m), 1.46 - 1.58 (1 H, m), 0.80 - 0.88 (2 H, m), 0.73 - 0.79 (2 H, m). |
| I27 | 3-(2,2-difluorocyclopropyl)-5-(trifluoromethyl)benzoic acid | 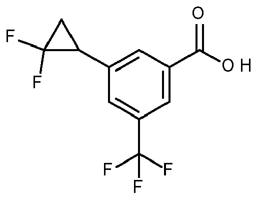 | 0.91 | 267 | 1 | |
| I28 | 3-(cyclopropoxy)-5-(trifluoromethyl)benzoic acid | 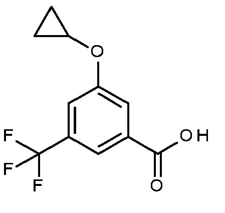 | 0.95 | 245 (M-H+) | 1 | |
| I29 | 2-cyclopropyl-6-(trifluoromethyl)pyridine-4-carboxylic acid | 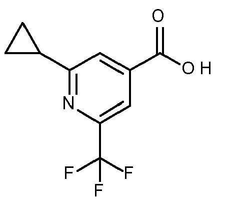 | 0.94 | 232 | 1 | ¹H NMR (400 MHz, DMSO-d6) δ ppm: 0.94 - 1.03 (m, 2 H), 1.06 - 1.15 (m, 2 H), 2.37 - 2.46 (m, 1 H), 7.88 (d, *J*=1.10 Hz, 1 H), 8.05 (d, *J*=0.73 Hz, 1 H), 13.89 - 14.33 (m, 1 H). |
| I30 | 3-(1-cyano-1-methyl-ethoxy)-5-(trifluoromethyl)benzoic acid | 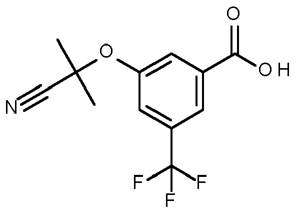 | 0.92 | 272 (M-H+) | 1 | |
| I31 | 3,5-bis(cyclopropoxy)benzoic acid | 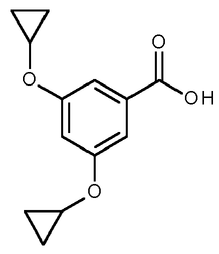 | 0.91 | 233 (M-H+) | 1 | |
| I32 | 3-cyclopropyl-5-(2,2-difluoroethoxy)benzoic acid | 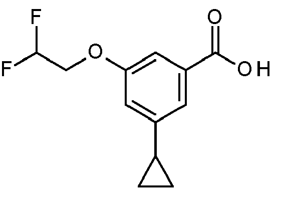 | 0.91 | 241 (M-H+) | 1 | 1H NMR (400 MHz, DMSO) δ ppm 0.70 - 0.77 (m, 2 H) 0.94 - 1.00 (m, 2 H) 1.95 - 2.06 (m, 1 H) 4.36 (td, J=14.76, 3.48 Hz, 2 H) 6.21 - 6.54 (m, 1 H) 6.91 - 6.97 (m, 1 H) 7.26 (dd, J=2.38, 1.28 Hz, 1 H) 7.31 (t, J=1.28 Hz, 1 H) 11.73 - 13.25 (m, 1 H) |
| I33 | 3-(2-chloro-3-pyridyl)-5-(trifluoromethyl)benzoic acid | 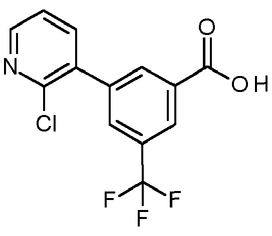 | 0.93 | 302 | 1 | 1H NMR (400 MHz, DMSO) δ ppm 7.57 - 7.61 (m, 1 H) 8.03 - 8.07 (m, 1 H) 8.16 - 8.20 (m, 1 H) 8.24 - 8.27 (m, 1 H) 8.29 - 8.32 (m, 1 H) 8.50 - 8.55 (m, 1 H) 13.64 - 13.77 (m, 1 H) |
| I34 | 3-(5-chloro-3-pyridyl)-5-(trifluoromethyl)benzoic acid | 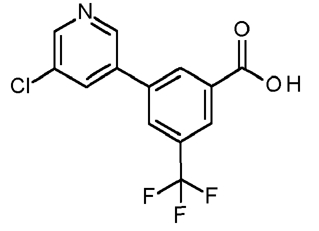 | 0.97 | 302 | 1 | 1H NMR (400 MHz,DMSO) δ ppm 8.22 - 8.26 (m, 1 H) 8.42 - 8.45 (m, 1 H) 8.48 (s, 1 H) 8.53 (s, 1 H) 8.71 - 8.74 (m, 1 H) 8.97 - 9.02 (m, 1 H) 13.63 13.78 (m, 1 H) |
| I35 | 3-cyclopropyl-5-(2,2,2-trifluoroethoxy)benzoic acid | 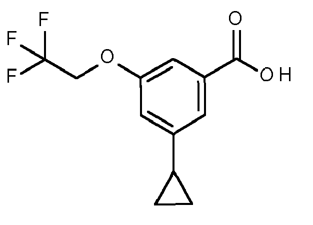 | 0.98 | 259 (M-H+) | 1 | |
| I36 | 3-[cyclobutyl(difluoro)methy l]-5-(trifluoromethyl)benzoic acid | 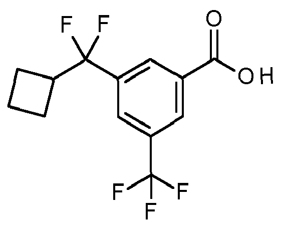 | 1.06 | 293 (M-H+) | 1 | |
| I37 | 3-(trifluoromethyl)-5-[4-(trifluoromethyl)phenyl]be nzoic acid | 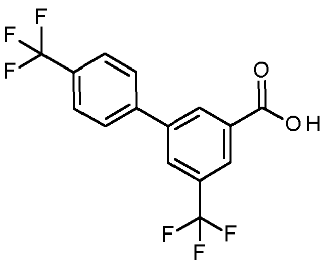 | 1.15 | 333 (M-H+) | 1 | 1H NMR (400 MHz, Solvent) δ ppm 7.87 (d, J=8.44 Hz, 2 H) 8.06 (d, J=8.44 Hz, 2 H) 8.23 (s, H) 8.34 (s, 1 H) 8.49 (s, 1 H) 13.72 (br s, 1 H) |
| I38 | 3-(trifluoromethyl)-5-[3-(trifluoromethyl)phenyl]be nzoic acid | 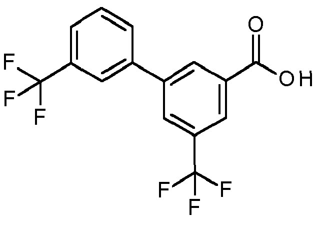 | 1.08 | 333 (M-H+) | 1 | |
| I39 | 3-(trifluoromethyl)-5-[2-(trifluoromethyl)phenyl]be nzoic acid | 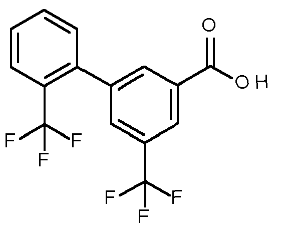 | 1.11 | 333 (M-H+) | 1 | 1H NMR (400 MHz, Solvent) δ ppm 7.53 - 7.57 (m, 1 H) 7.68 - 7.74 (m, 1 H) 7.77 - 7.84 (m, 1 H) 7.89 - 7.93 (m, 1 H) 7.95 - 7.99 (m, 1 H) 8.11 - 8.14 (m, 1 H) 8.24 - 8.27 (m, 1 H) 13.71 (br s, 1 H) |
| I40 | 2-(3-chloropyrazol-1-yl)-6-(trifluoromethyl)pyridine-4-carboxylic acid | 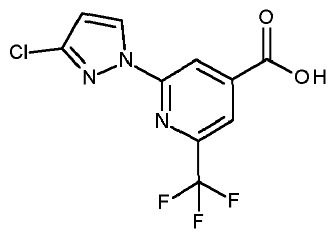 | 0.96 | 292 | 1 | ¹H NMR (400 MHz, DMSO) δ ppm 13.9 - 15.0 (m, 1 H) 8.7 (d, *J*=2.57 Hz, 1 H) 8.4 (s, 1 H) 8.1 (d, *J*=1.10 Hz, 1 H) 6.8 (d, *J*=2.57 Hz, 1 H) |
| I41 | 3-(trifluoromethyl)-5-(trifluoromethylsulfonyl)b enzoic acid | 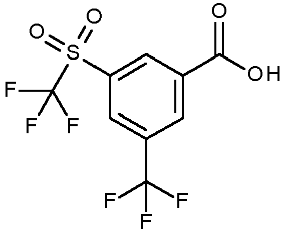 | 0.98 | 321 (M-H+) | 1 | ¹H NMR (400 MHz, DMSO-d6) δ ppm: 8.68 (s, 2 H) 8.71 - 8.76 (m, 1 H) 13.33 - 15.22 (m, 1 H). |

The activity of the compositions according to the invention can be broadened considerably, and adapted to prevailing circumstances, by adding other insecticidally, acaricidally and/or fungicidally active ingredients. The mixtures of the compounds of formula I with other insecticidally, acaricidally and/or fungicidally active ingredients may also have further surprising advantages which can also be described, in a wider sense, as synergistic activity. For example, better tolerance by plants, reduced phytotoxicity, insects can be controlled in their different development stages or better behaviour during their production, for example during grinding or mixing, during their storage or during their use.

Suitable additions to active ingredients here are, for example, representatives of the following classes of active ingredients: organophosphorus compounds, nitrophenol derivatives, thioureas, juvenile hormones, formamidines, benzophenone derivatives, ureas, pyrrole derivatives, carbamates, pyrethroids, chlorinated hydrocarbons, acylureas, pyridylmethyleneamino derivatives, macrolides, neonicotinoids and Bacillus thuringiensis preparations.

The following mixtures of the compounds of formula I with active ingredients are preferred (the abbreviation "TX" means "one compound selected from the compounds defined in the Tables A-1 to A-64 and Table P"):
an adjuvant selected from the group of substances consisting of petroleum oils (alternative name) (628) + TX,
an acaricide selected from the group of substances consisting of 1 ,1-bis(4-chlorophenyl)-2-ethoxyethanol (IUPAC name) (910) + TX, 2,4-dichlorophenyl benzenesulfonate (IUPAC/Chemical Abstracts name) (1059) + TX, 2-fluoro-*N*-methyl-*N*-1-naphthylacetamide (IUPAC name) (1295) + TX, 4-chlorophenyl phenyl sulfone (IUPAC name) (981) + TX, abamectin (1) + TX, acequinocyl (3) + TX, acetoprole [CCN] + TX, acrinathrin (9) + TX, aldicarb (16) + TX, aldoxycarb (863) + TX, alpha-cypermethrin (202) + TX, amidithion (870) + TX, amidoflumet [CCN] + TX, amidothioate (872) + TX, amiton (875) + TX, amiton hydrogen oxalate (875) + TX, amitraz (24) + TX, aramite (881) + TX, arsenous oxide (882) + TX, AVI 382 (compound code) + TX, AZ 60541 (compound code) + TX, azinphos-ethyl (44) + TX, azinphos-methyl (45) + TX, azobenzene (IUPAC name) (888) + TX, azocyclotin (46) + TX, azothoate (889) + TX, benomyl (62) + TX, benoxafos (alternative name) [CCN] + TX, benzoximate (71) + TX, benzyl benzoate (IUPAC name) [CCN] + TX, bifenazate (74) + TX, bifenthrin (76) + TX, binapacryl (907) + TX, brofenvalerate (alternative name) + TX, bromocyclen (918) + TX, bromophos (920) + TX, bromophos-ethyl (921) + TX, bromopropylate (94) + TX, buprofezin (99) + TX, butocarboxim (103) + TX, butoxycarboxim (104) + TX, butylpyridaben (alternative name) + TX, calcium polysulfide (IUPAC name) (111) + TX, camphechlor (941) + TX, carbanolate (943) + TX, carbaryl (115) + TX, carbofuran (118) + TX, carbophenothion (947) + TX, CGA 50'439 (development code) (125) + TX, chinomethionat (126) + TX, chlorbenside (959) + TX, chlordimeform (964) + TX, chlordimeform hydrochloride (964) + TX, chlorfenapyr (130) + TX, chlorfenethol (968) + TX, chlorfenson (970) + TX, chlorfensulfide (971) + TX, chlorfenvinphos (131) + TX, chlorobenzilate (975) + TX, chloromebuform (977) + TX, chloromethiuron (978) + TX, chloropropylate (983) + TX, chlorpyrifos (145) + TX, chlorpyrifos-methyl (146) + TX, chlorthiophos (994) + TX, cinerin I (696) + TX, cinerin II (696) + TX, cinerins (696) + TX, clofentezine (158) + TX, closantel (alternative name) [CCN] + TX, coumaphos (174) + TX, crotamiton (alternative name) [CCN] + TX, crotoxyphos (1010) + TX, cufraneb (1013) + TX, cyanthoate (1020) + TX, cyflumetofen (CAS Reg. No.: 400882-07-7) + TX, cyhalothrin (196) + TX, cyhexatin (199) + TX, cypermethrin (201) + TX, DCPM (1032) + TX, DDT (219) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulfon (1039) + TX, diafenthiuron (226) + TX, dialifos (1042) + TX, diazinon (227) + TX, dichlofluanid (230) + TX, dichlorvos (236) + TX, dicliphos (alternative name) + TX, dicofol (242) + TX, dicrotophos (243) + TX, dienochlor (1071) + TX, dimefox (1081) + TX, dimethoate (262) + TX, dinactin (alternative name) (653) + TX, dinex (1089) + TX, dinex-diclexine (1089) + TX, dinobuton (269) + TX, dinocap (270) + TX, dinocap-4 [CCN] + TX, dinocap-6 [CCN] + TX, dinocton (1090) + TX, dinopenton (1092) + TX, dinosulfon (1097) + TX, dinoterbon (1098) + TX, dioxathion (1102) + TX, diphenyl sulfone (IUPAC name) (1103) + TX, disulfiram (alternative name) [CCN] + TX, disulfoton (278) + TX, DNOC (282) + TX, dofenapyn (1113) + TX, doramectin (alternative name) [CCN] + TX, endosulfan (294) + TX, endothion (1121) + TX, EPN (297) + TX, eprinomectin (alternative name) [CCN] + TX, ethion (309) + TX, ethoate-methyl (1134) + TX, etoxazole (320) + TX, etrimfos (1142) + TX, fenazaflor (1147) + TX, fenazaquin (328) + TX, fenbutatin oxide (330) + TX, fenothiocarb (337) + TX, fenpropathrin (342) + TX, fenpyrad (alternative name) + TX, fenpyroximate (345) + TX, fenson (1157) + TX, fentrifanil (1161) + TX, fenvalerate (349) + TX, fipronil (354) + TX, fluacrypyrim (360) + TX, fluazuron (1166) + TX, flubenzimine (1167) + TX, flucycloxuron (366) + TX, flucythrinate (367) + TX, fluenetil (1169) + TX, flufenoxuron (370) + TX, flumethrin (372) + TX, fluorbenside (1174) + TX, fluvalinate (1184) + TX, FMC 1137 (development code) (1185) + TX, formetanate (405) + TX, formetanate hydrochloride (405) + TX, formothion (1192) + TX, formparanate (1193) + TX, gamma-HCH (430) + TX, glyodin (1205) + TX, halfenprox (424) + TX, heptenophos (432) + TX, hexadecyl cyclopropanecarboxylate (IUPAC/Chemical Abstracts name) (1216) + TX, hexythiazox (441) + TX, iodomethane (IUPAC name) (542) + TX, isocarbophos (alternative name) (473) + TX, isopropyl *O*-(methoxyaminothiophosphoryl)salicylate (IUPAC name) (473) + TX, ivermectin (alternative name) [CCN] + TX, jasmolin I (696) + TX, jasmolin II (696) + TX, jodfenphos (1248) + TX, lindane (430) + TX, lufenuron (490) + TX, malathion (492) + TX, malonoben (1254) + TX, mecarbam (502) + TX, mephosfolan (1261) + TX, mesulfen (alternative name) [CCN] + TX, methacrifos (1266) + TX, methamidophos (527) + TX, methidathion (529) + TX, methiocarb (530) + TX, methomyl (531) + TX, methyl bromide (537) + TX, metolcarb (550) + TX, mevinphos (556) + TX, mexacarbate (1290) + TX, milbemectin (557) + TX, milbemycin oxime (alternative name) [CCN] + TX, mipafox (1293) + TX, monocrotophos (561) + TX, morphothion (1300) + TX, moxidectin (alternative name) [CCN] + TX, naled (567) + TX, NC-184 (compound code) + TX, NC-512 (compound code) + TX, nifluridide (1309) + TX, nikkomycins (alternative name) [CCN] + TX, nitrilacarb (1313) + TX, nitrilacarb 1:1 zinc chloride complex (1313) + TX, NNI-0101 (compound code) + TX, NNI-0250 (compound code) + TX, omethoate (594) + TX, oxamyl (602) + TX, oxydeprofos (1324) + TX, oxydisulfoton (1325) + TX, pp'-DDT (219) + TX, parathion (615) + TX, permethrin (626) + TX, petroleum oils (alternative name) (628) + TX, phenkapton (1330) + TX, phenthoate (631) + TX, phorate (636) + TX, phosalone (637) + TX, phosfolan (1338) + TX, phosmet (638) + TX, phosphamidon (639) + TX, phoxim (642) + TX, pirimiphos-methyl (652) + TX, polychloroterpenes (traditional name) (1347) + TX, polynactins (alternative name) (653) + TX, proclonol (1350) + TX, profenofos (662) + TX, promacyl (1354) + TX, propargite (671) + TX, propetamphos (673) + TX, propoxur (678) + TX, prothidathion (1360) + TX, prothoate (1362) + TX, pyrethrin I (696) + TX, pyrethrin II (696) + TX, pyrethrins (696) + TX, pyridaben (699) + TX, pyridaphenthion (701) + TX, pyrimidifen (706) + TX, pyrimitate (1370) + TX, quinalphos (711) + TX, quintiofos (1381) + TX, R-1492 (development code) (1382) + TX, RA-17 (development code) (1383) + TX, rotenone (722) + TX, schradan (1389) + TX, sebufos (alternative name) + TX, selamectin (alternative name) [CCN] + TX, SI-0009 (compound code) + TX, sophamide (1402) + TX, spirodiclofen (738) + TX, spiromesifen (739) + TX, SSI-121 (development code) (1404) + TX, sulfiram (alternative name) [CCN] + TX, sulfluramid (750) + TX, sulfotep (753) + TX, sulfur (754) + TX, SZI-121 (development code) (757) + TX, tau-fluvalinate (398) + TX, tebufenpyrad (763) + TX, TEPP (1417) + TX, terbam (alternative name) + TX, tetrachlorvinphos (777) + TX, tetradifon (786) + TX, tetranactin (alternative name) (653) + TX, tetrasul (1425) + TX, thiafenox (alternative name) + TX, thiocarboxime (1431) + TX, thiofanox (800) + TX, thiometon (801) + TX, thioquinox (1436) + TX, thuringiensin (alternative name) [CCN] + TX, triamiphos (1441) + TX, triarathene (1443) + TX, triazophos (820) + TX, triazuron (alternative name) + TX, trichlorfon (824) + TX, trifenofos (1455) + TX, trinactin (alternative name) (653) + TX, vamidothion (847) + TX, vaniliprole [CCN] and YI-5302 (compound code) + TX,
an algicide selected from the group of substances consisting of bethoxazin [CCN] + TX, copper dioctanoate (IUPAC name) (170) + TX, copper sulfate (172) + TX, cybutryne [CCN] + TX, dichlone (1052) + TX, dichlorophen (232) + TX, endothal (295) + TX, fentin (347) + TX, hydrated lime [CCN] + TX, nabam (566) + TX, quinoclamine (714) + TX, quinonamid (1379) + TX, simazine (730) + TX, triphenyltin acetate (IUPAC name) (347) and triphenyltin hydroxide (IUPAC name) (347) + TX,
an anthelmintic selected from the group of substances consisting of abamectin (1) + TX, crufomate (1011) + TX, doramectin (alternative name) [CCN] + TX, emamectin (291) + TX, emamectin benzoate (291) + TX, eprinomectin (alternative name) [CCN] + TX, ivermectin (alternative name) [CCN] + TX, milbemycin oxime (alternative name) [CCN] + TX, moxidectin (alternative name) [CCN] + TX, piperazine [CCN] + TX, selamectin (alternative name) [CCN] + TX, spinosad (737) and thiophanate (1435) + TX,
an avicide selected from the group of substances consisting of chloralose (127) + TX, endrin (1122) + TX, fenthion (346) + TX, pyridin-4-amine (IUPAC name) (23) and strychnine (745) + TX,
a bactericide selected from the group of substances consisting of 1-hydroxy-1*H*-pyridine-2-thione (IUPAC name) (1222) + TX, 4-(quinoxalin-2-ylamino)benzenesulfonamide (IUPAC name) (748) + TX, 8-hydroxyquinoline sulfate (446) + TX, bronopol (97) + TX, copper dioctanoate (IUPAC name) (170) + TX, copper hydroxide (IUPAC name) (169) + TX, cresol [CCN] + TX, dichlorophen (232) + TX, dipyrithione (1105) + TX, dodicin (1112) + TX, fenaminosulf (1144) + TX, formaldehyde (404) + TX, hydrargaphen (alternative name) [CCN] + TX, kasugamycin (483) + TX, kasugamycin hydrochloride hydrate (483) + TX, nickel bis(dimethyldithiocarbamate) (IUPAC name) (1308) + TX, nitrapyrin (580) + TX, octhilinone (590) + TX, oxolinic acid (606) + TX, oxytetracycline (611) + TX, potassium hydroxyquinoline sulfate (446) + TX, probenazole (658) + TX, streptomycin (744) + TX, streptomycin sesquisulfate (744) + TX, tecloftalam (766) + TX, and thiomersal (alternative name) [CCN] + TX,
a biological agent selected from the group of substances consisting of *Adoxophyes orana* GV (alternative name) (12) + TX, *Agrobacterium radiobacter* (alternative name) (13) + TX, *Amblyseius* spp. (alternative name) (19) + TX, *Anagrapha falcifera* NPV (alternative name) (28) + TX, *Anagrus atomus* (alternative name) (29) + TX, *Aphelinus abdominalis* (alternative name) (33) + TX, *Aphidius colemani* (alternative name) (34) + TX, *Aphidoletes aphidimyza* (alternative name) (35) + TX, *Autographa californica* NPV (alternative name) (38) + TX, *Bacillus firmus* (alternative name) (48) + TX, *Bacillus sphaericus* Neide (scientific name) (49) + TX, *Bacillus thuringiensis* Berliner (scientific name) (51) + TX, *Bacillus thuringiensis* subsp. *aizawai* (scientific name) (51) + TX, *Bacillus thuringiensis* subsp. *israelensis* (scientific name) (51) + TX, *Bacillus thuringiensis* subsp. *japonensis* (scientific name) (51) + TX, *Bacillus thuringiensis* subsp. *kurstaki* (scientific name) (51) + TX, *Bacillus thuringiensis* subsp. *tenebrionis* (scientific name) (51) + TX, *Beauveria bassiana* (alternative name) (53) + TX, *Beauveria brongniartii* (alternative name) (54) + TX, *Chrysoperla carnea* (alternative name) (151) + TX, *Cryptolaemus montrouzieri* (alternative name) (178) + TX, *Cydia pomonella* GV (alternative name) (191) + TX, *Dacnusa sibirica* (alternative name) (212) + TX, *Diglyphus isaea* (alternative name) (254) + TX, *Encarsia formosa* (scientific name) (293) + TX, *Eretmocerus eremicus* (alternative name) (300) + TX, *Helicoverpa zea* NPV (alternative name) (431) + TX, *Heterorhabditis bacteriophora* and *H. megidis* (alternative name) (433) + TX, *Hippodamia convergens* (alternative name) (442) + TX, *Leptomastix dactylopii* (alternative name) (488) + TX, *Macrolophus caliginosus* (alternative name) (491) + TX, *Mamestra brassicae* NPV (alternative name) (494) + TX, *Metaphycus helvolus* (alternative name) (522) + TX, *Metarhizium anisopliae* var. *acridum* (scientific name) (523) + TX, *Metarhizium anisopliae* var. *anisopliae* (scientific name) (523) + TX, *Neodiprion sertifer* NPV and *N. lecontei* NPV (alternative name) (575) + TX, *Orius* spp. (alternative name) (596) + TX, *Paecilomyces fumosoroseus* (alternative name) (613) + TX, *Phytoseiulus persimilis* (alternative name) (644) + TX, *Spodoptera exigua* multicapsid nuclear polyhedrosis virus (scientific name) (741) + TX, *Steinernema bibionis* (alternative name) (742) + TX, *Steinernema carpocapsae* (alternative name) (742) + TX, *Steinernema feltiae* (alternative name) (742) + TX, *Steinernema glaseri* (alternative name) (742) + TX, *Steinernema riobrave* (alternative name) (742) + TX, *Steinernema riobravis* (alternative name) (742) + TX, *Steinernema scapterisci* (alternative name) (742) + TX, *Steinernema* spp. (alternative name) (742) + TX, *Trichogramma* spp. (alternative name) (826) + TX, *Typhlodromus occidentalis* (alternative name) (844) and *Verticillium lecanii* (alternative name) (848) + TX,
a soil sterilant selected from the group of substances consisting of iodomethane (IUPAC name) (542) and methyl bromide (537) + TX,
a chemosterilant selected from the group of substances consisting of apholate [CCN] + TX, bisazir (alternative name) [CCN] + TX, busulfan (alternative name) [CCN] + TX, diflubenzuron (250) + TX, dimatif (alternative name) [CCN] + TX, hemel [CCN] + TX, hempa [CCN] + TX, metepa [CCN] + TX, methiotepa [CCN] + TX, methyl apholate [CCN] + TX, morzid [CCN] + TX, penfluron (alternative name) [CCN] + TX, tepa [CCN] + TX, thiohempa (alternative name) [CCN] + TX, thiotepa (alternative name) [CCN] + TX, tretamine (alternative name) [CCN] and uredepa (alternative name) [CCN] + TX,
an insect pheromone selected from the group of substances consisting of (E)-dec-5-en-1-yl acetate with (E)-dec-5-en-1-ol (IUPAC name) (222) + TX, (E)-tridec-4-en-1-yl acetate (IUPAC name) (829) + TX, (E)-6-methylhept-2-en-4-ol (IUPAC name) (541) + TX, (E,Z)-tetradeca-4,10-dien-1-yl acetate (IUPAC name) (779) + TX, (Z)-dodec-7-en-1-yl acetate (IUPAC name) (285) + TX, (Z)-hexadec-11-enal (IUPAC name) (436) + TX, (*Z*)-hexadec-11-en-1-yl acetate (IUPAC name) (437) + TX, (Z)-hexadec-13-en-11-yn-1-yl acetate (IUPAC name) (438) + TX, (Z)-icos-13-en-10-one (IUPAC name) (448) + TX, (Z)-tetradec-7-en-1-al (IUPAC name) (782) + TX, (Z)-tetradec-9-en-1-ol (IUPAC name) (783) + TX, (Z)-tetradec-9-en-1-yl acetate (IUPAC name) (784) + TX, (7E,9Z)-dodeca-7,9-dien-1-yl acetate (IUPAC name) (283) + TX, (9Z, 11 E)-tetradeca-9, 11-dien-1-yl acetate (IUPAC name) (780) + TX, (9Z,12E)-tetradeca-9,12-dien-1-yl acetate (IUPAC name) (781) + TX, 14-methyloctadec-1-ene (IUPAC name) (545) + TX, 4-methylnonan-5-ol with 4-methylnonan-5-one (IUPAC name) (544) + TX, alpha-multistriatin (alternative name) [CCN] + TX, brevicomin (alternative name) [CCN] + TX, codlelure (alternative name) [CCN] + TX, codlemone (alternative name) (167) + TX, cuelure (alternative name) (179) + TX, disparlure (277) + TX, dodec-8-en-1-yl acetate (IUPAC name) (286) + TX, dodec-9-en-1-yl acetate (IUPAC name) (287) + TX, dodeca-8 + TX, 10-dien-1-yl acetate (IUPAC name) (284) + TX, dominicalure (alternative name) [CCN] + TX, ethyl 4-methyloctanoate (IUPAC name) (317) + TX, eugenol (alternative name) [CCN] + TX, frontalin (alternative name) [CCN] + TX, gossyplure (alternative name) (420) + TX, grandlure (421) + TX, grandlure I (alternative name) (421) + TX, grand lure II (alternative name) (421) + TX, grandlure III (alternative name) (421) + TX, grand lure IV (alternative name) (421) + TX, hexalure [CCN] + TX, ipsdienol (alternative name) [CCN] + TX, ipsenol (alternative name) [CCN] + TX, japonilure (alternative name) (481) + TX, lineatin (alternative name) [CCN] + TX, litlure (alternative name) [CCN] + TX, looplure (alternative name) [CCN] + TX, medlure [CCN] + TX, megatomoic acid (alternative name) [CCN] + TX, methyl eugenol (alternative name) (540) + TX, muscalure (563) + TX, octadeca-2,13-dien-1-yl acetate (IUPAC name) (588) + TX, octadeca-3,13-dien-1-yl acetate (IUPAC name) (589) + TX, orfralure (alternative name) [CCN] + TX, oryctalure (alternative name) (317) + TX, ostramone (alternative name) [CCN] + TX, siglure [CCN] + TX, sordidin (alternative name) (736) + TX, sulcatol (alternative name) [CCN] + TX, tetradec-11-en-1-yl acetate (IUPAC name) (785) + TX, trimedlure (839) + TX, trimedlure A (alternative name) (839) + TX, trimedlure B₁ (alternative name) (839) + TX, trimedlure B₂ (alternative name) (839) + TX, trimedlure C (alternative name) (839) and trunc-call (alternative name) [CCN] + TX,
an insect repellent selected from the group of substances consisting of 2-(octylthio)ethanol (IUPAC name) (591) + TX, butopyronoxyl (933) + TX, butoxy(polypropylene glycol) (936) + TX, dibutyl adipate (IUPAC name) (1046) + TX, dibutyl phthalate (1047) + TX, dibutyl succinate (IUPAC name) (1048) + TX, diethyltoluamide [CCN] + TX, dimethyl carbate [CCN] + TX, dimethyl phthalate [CCN] + TX, ethyl hexanediol (1137) + TX, hexamide [CCN] + TX, methoquin-butyl (1276) + TX, methylneodecanamide [CCN] + TX, oxamate [CCN] and picaridin [CCN] + TX,
an insecticide selected from the group of substances consisting of 1-dichloro-1-nitroethane (IUPAC/Chemical Abstracts name) (1058) + TX, 1,1-dichloro-2,2-bis(4-ethylphenyl)ethane (IUPAC name) (1056), + TX, 1,2-dichloropropane (IUPAC/Chemical Abstracts name) (1062) + TX, 1,2-dichloropropane with 1,3-dichloropropene (IUPAC name) (1063) + TX, 1-bromo-2-chloroethane (IUPAC/Chemical Abstracts name) (916) + TX, 2,2,2-trichloro-1-(3,4-dichlorophenyl)ethyl acetate (IUPAC name) (1451) + TX, 2,2-dichlorovinyl 2-ethylsulfinylethyl methyl phosphate (IUPAC name) (1066) + TX, 2-(1,3-dithiolan-2-yl)phenyl dimethylcarbamate (IUPAC/ Chemical Abstracts name) (1109) + TX, 2-(2-butoxyethoxy)ethyl thiocyanate (IUPAC/Chemical Abstracts name) (935) + TX, 2-(4,5-dimethyl-1,3-dioxolan-2-yl)phenyl methylcarbamate (IUPAC/ Chemical Abstracts name) (1084) + TX, 2-(4-chloro-3,5-xylyloxy)ethanol (IUPAC name) (986) + TX, 2-chlorovinyl diethyl phosphate (IUPAC name) (984) + TX, 2-imidazolidone (IUPAC name) (1225) + TX, 2-isovalerylindan-1,3-dione (IUPAC name) (1246) + TX, 2-methyl(prop-2-ynyl)aminophenyl methylcarbamate (IUPAC name) (1284) + TX, 2-thiocyanatoethyl laurate (IUPAC name) (1433) + TX, 3-bromo-1-chloroprop-1-ene (IUPAC name) (917) + TX, 3-methyl-1-phenylpyrazol-5-yl dimethylcarbamate (IUPAC name) (1283) + TX, 4-methyl(prop-2-ynyl)amino-3,5-xylyl methylcarbamate (IUPAC name) (1285) + TX, 5,5-dimethyl-3-oxocyclohex-1-enyl dimethylcarbamate (IUPAC name) (1085) + TX, abamectin (1) + TX, acephate (2) + TX, acetamiprid (4) + TX, acethion (alternative name) [CCN] + TX, acetoprole [CCN] + TX, acrinathrin (9) + TX, acrylonitrile (IUPAC name) (861) + TX, alanycarb (15) + TX, aldicarb (16) + TX, aldoxycarb (863) + TX, aldrin (864) + TX, allethrin (17) + TX, allosamidin (alternative name) [CCN] + TX, allyxycarb (866) + TX, alpha-cypermethrin (202) + TX, alpha-ecdysone (alternative name) [CCN] + TX, aluminium phosphide (640) + TX, amidithion (870) + TX, amidothioate (872) + TX, aminocarb (873) + TX, amiton (875) + TX, amiton hydrogen oxalate (875) + TX, amitraz (24) + TX, anabasine (877) + TX, athidathion (883) + TX, AVI 382 (compound code) + TX, AZ 60541 (compound code) + TX, azadirachtin (alternative name) (41) + TX, azamethiphos (42) + TX, azinphos-ethyl (44) + TX, azinphos-methyl (45) + TX, azothoate (889) + TX, *Bacillus thuringiensis* delta endotoxins (alternative name) (52) + TX, barium hexafluorosilicate (alternative name) [CCN] + TX, barium polysulfide (IUPAC/Chemical Abstracts name) (892) + TX, barthrin [CCN] + TX, Bayer 22/190 (development code) (893) + TX, Bayer 22408 (development code) (894) + TX, bendiocarb (58) + TX, benfuracarb (60) + TX, bensultap (66) + TX, beta-cyfluthrin (194) + TX, beta-cypermethrin (203) + TX, bifenthrin (76) + TX, bioallethrin (78) + TX, bioallethrin *S*-cyclopentenyl isomer (alternative name) (79) + TX, bioethanomethrin [CCN] + TX, biopermethrin (908) + TX, bioresmethrin (80) + TX, bis(2-chloroethyl) ether (IUPAC name) (909) + TX, bistrifluron (83) + TX, borax (86) + TX, brofenvalerate (alternative name) + TX, bromfenvinfos (914) + TX, bromocyclen (918) + TX, bromo-DDT (alternative name) [CCN] + TX, bromophos (920) + TX, bromophos-ethyl (921) + TX, bufencarb (924) + TX, buprofezin (99) + TX, butacarb (926) + TX, butathiofos (927) + TX, butocarboxim (103) + TX, butonate (932) + TX, butoxycarboxim (104) + TX, butylpyridaben (alternative name) + TX, cadusafos (109) + TX, calcium arsenate [CCN] + TX, calcium cyanide (444) + TX, calcium polysulfide (IUPAC name) (111) + TX, camphechlor (941) + TX, carbanolate (943) + TX, carbaryl (115) + TX, carbofuran (118) + TX, carbon disulfide (IUPAC/Chemical Abstracts name) (945) + TX, carbon tetrachloride (IUPAC name) (946) + TX, carbophenothion (947) + TX, carbosulfan (119) + TX, cartap (123) + TX, cartap hydrochloride (123) + TX, cevadine (alternative name) (725) + TX, chlorbicyclen (960) + TX, chlordane (128) + TX, chlordecone (963) + TX, chlordimeform (964) + TX, chlordimeform hydrochloride (964) + TX, chlorethoxyfos (129) + TX, chlorfenapyr (130) + TX, chlorfenvinphos (131) + TX, chlorfluazuron (132) + TX, chlormephos (136) + TX, chloroform [CCN] + TX, chloropicrin (141) + TX, chlorphoxim (989) + TX, chlorprazophos (990) + TX, chlorpyrifos (145) + TX, chlorpyrifos-methyl (146) + TX, chlorthiophos (994) + TX, chromafenozide (150) + TX, cinerin I (696) + TX, cinerin II (696) + TX, cinerins (696) + TX, cis-resmethrin (alternative name) + TX, cismethrin (80) + TX, clocythrin (alternative name) + TX, cloethocarb (999) + TX, closantel (alternative name) [CCN] + TX, clothianidin (165) + TX, copper acetoarsenite [CCN] + TX, copper arsenate [CCN] + TX, copper oleate [CCN] + TX, coumaphos (174) + TX, coumithoate (1006) + TX, crotamiton (alternative name) [CCN] + TX, crotoxyphos (1010) + TX, crufomate (1011) + TX, cryolite (alternative name) (177) + TX, CS 708 (development code) (1012) + TX, cyanofenphos (1019) + TX, cyanophos (184) + TX, cyanthoate (1020) + TX, cyclethrin [CCN] + TX, cycloprothrin (188) + TX, cyfluthrin (193) + TX, cyhalothrin (196) + TX, cypermethrin (201) + TX, cyphenothrin (206) + TX, cyromazine (209) + TX, cythioate (alternative name) [CCN] + TX, d-limonene (alternative name) [CCN] + TX, d-tetramethrin (alternative name) (788) + TX, DAEP (1031) + TX, dazomet (216) + TX, DDT (219) + TX, decarbofuran (1034) + TX, deltamethrin (223) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulphon (1039) + TX, diafenthiuron (226) + TX, dialifos (1042) + TX, diamidafos (1044) + TX, diazinon (227) + TX, dicapthon (1050) + TX, dichlofenthion (1051) + TX, dichlorvos (236) + TX, dicliphos (alternative name) + TX, dicresyl (alternative name) [CCN] + TX, dicrotophos (243) + TX, dicyclanil (244) + TX, dieldrin (1070) + TX, diethyl 5-methylpyrazol-3-yl phosphate (IUPAC name) (1076) + TX, diflubenzuron (250) + TX, dilor (alternative name) [CCN] + TX, dimefluthrin [CCN] + TX, dimefox (1081) + TX, dimetan (1085) + TX, dimethoate (262) + TX, dimethrin (1083) + TX, dimethylvinphos (265) + TX, dimetilan (1086) + TX, dinex (1089) + TX, dinex-diclexine (1089) + TX, dinoprop (1093) + TX, dinosam (1094) + TX, dinoseb (1095) + TX, dinotefuran (271) + TX, diofenolan (1099) + TX, dioxabenzofos (1100) + TX, dioxacarb (1101) + TX, dioxathion (1102) + TX, disulfoton (278) + TX, dithicrofos (1108) + TX, DNOC (282) + TX, doramectin (alternative name) [CCN] + TX, DSP (1115) + TX, ecdysterone (alternative name) [CCN] + TX, EI 1642 (development code) (1118) + TX, emamectin (291) + TX, emamectin benzoate (291) + TX, EMPC (1120) + TX, empenthrin (292) + TX, endosulfan (294) + TX, endothion (1121) + TX, endrin (1122) + TX, EPBP (1123) + TX, EPN (297) + TX, epofenonane (1124) + TX, eprinomectin (alternative name) [CCN] + TX, esfenvalerate (302) + TX, etaphos (alternative name) [CCN] + TX, ethiofencarb (308) + TX, ethion (309) + TX, ethiprole (310) + TX, ethoate-methyl (1134) + TX, ethoprophos (312) + TX, ethyl formate (IUPAC name) [CCN] + TX, ethyl-DDD (alternative name) (1056) + TX, ethylene dibromide (316) + TX, ethylene dichloride (chemical name) (1136) + TX, ethylene oxide [CCN] + TX, etofenprox (319) + TX, etrimfos (1142) + TX, EXD (1143) + TX, famphur (323) + TX, fenamiphos (326) + TX, fenazaflor (1147) + TX, fenchlorphos (1148) + TX, fenethacarb (1149) + TX, fenfluthrin (1150) + TX, fenitrothion (335) + TX, fenobucarb (336) + TX, fenoxacrim (1153) + TX, fenoxycarb (340) + TX, fenpirithrin (1155) + TX, fenpropathrin (342) + TX, fenpyrad (alternative name) + TX, fensulfothion (1158) + TX, fenthion (346) + TX, fenthion-ethyl [CCN] + TX, fenvalerate (349) + TX, fipronil (354) + TX, flonicamid (358) + TX, flubendiamide (CAS. Reg. No.: 272451-65-7) + TX, flucofuron (1168) + TX, flucycloxuron (366) + TX, flucythrinate (367) + TX, fluenetil (1169) + TX, flufenerim [CCN] + TX, flufenoxuron (370) + TX, flufenprox (1171) + TX, flumethrin (372) + TX, fluvalinate (1184) + TX, FMC 1137 (development code) (1185) + TX, fonofos (1191) + TX, formetanate (405) + TX, formetanate hydrochloride (405) + TX, formothion (1192) + TX, formparanate (1193) + TX, fosmethilan (1194) + TX, fospirate (1195) + TX, fosthiazate (408) + TX, fosthietan (1196) + TX, furathiocarb (412) + TX, furethrin (1200) + TX, gamma-cyhalothrin (197) + TX, gamma-HCH (430) + TX, guazatine (422) + TX, guazatine acetates (422) + TX, GY-81 (development code) (423) + TX, halfenprox (424) + TX, halofenozide (425) + TX, HCH (430) + TX, HEOD (1070) + TX, heptachlor (1211) + TX, heptenophos (432) + TX, heterophos [CCN] + TX, hexaflumuron (439) + TX, HHDN (864) + TX, hydramethylnon (443) + TX, hydrogen cyanide (444) + TX, hydroprene (445) + TX, hyquincarb (1223) + TX, imidacloprid (458) + TX, imiprothrin (460) + TX, indoxacarb (465) + TX, iodomethane (IUPAC name) (542) + TX, IPSP (1229) + TX, isazofos (1231) + TX, isobenzan (1232) + TX, isocarbophos (alternative name) (473) + TX, isodrin (1235) + TX, isofenphos (1236) + TX, isolane (1237) + TX, isoprocarb (472) + TX, isopropyl *O*-(methoxy-aminothiophosphoryl)salicylate (IUPAC name) (473) + TX, isoprothiolane (474) + TX, isothioate (1244) + TX, isoxathion (480) + TX, ivermectin (alternative name) [CCN] + TX, jasmolin I (696) + TX, jasmolin II (696) + TX, jodfenphos (1248) + TX, juvenile hormone I (alternative name) [CCN] + TX, juvenile hormone II (alternative name) [CCN] + TX, juvenile hormone III (alternative name) [CCN] + TX, kelevan (1249) + TX, kinoprene (484) + TX, lambda-cyhalothrin (198) + TX, lead arsenate [CCN] + TX, lepimectin (CCN) + TX, leptophos (1250) + TX, lindane (430) + TX, lirimfos (1251) + TX, lufenuron (490) + TX, lythidathion (1253) + TX, m-cumenyl methylcarbamate (IUPAC name) (1014) + TX, magnesium phosphide (IUPAC name) (640) + TX, malathion (492) + TX, malonoben (1254) + TX, mazidox (1255) + TX, mecarbam (502) + TX, mecarphon (1258) + TX, menazon (1260) + TX, mephosfolan (1261) + TX, mercurous chloride (513) + TX, mesulfenfos (1263) + TX, metaflumizone (CCN) + TX, metam (519) + TX, metam-potassium (alternative name) (519) + TX, metam-sodium (519) + TX, methacrifos (1266) + TX, methamidophos (527) + TX, methanesulfonyl fluoride (IUPAC/Chemical Abstracts name) (1268) + TX, methidathion (529) + TX, methiocarb (530) + TX, methocrotophos (1273) + TX, methomyl (531) + TX, methoprene (532) + TX, methoquin-butyl (1276) + TX, methothrin (alternative name) (533) + TX, methoxychlor (534) + TX, methoxyfenozide (535) + TX, methyl bromide (537) + TX, methyl isothiocyanate (543) + TX, methylchloroform (alternative name) [CCN] + TX, methylene chloride [CCN] + TX, metofluthrin [CCN] + TX, metolcarb (550) + TX, metoxadiazone (1288) + TX, mevinphos (556) + TX, mexacarbate (1290) + TX, milbemectin (557) + TX, milbemycin oxime (alternative name) [CCN] + TX, mipafox (1293) + TX, mirex (1294) + TX, monocrotophos (561) + TX, morphothion (1300) + TX, moxidectin (alternative name) [CCN] + TX, naftalofos (alternative name) [CCN] + TX, naled (567) + TX, naphthalene (lUPAC/Chemical Abstracts name) (1303) + TX, NC-170 (development code) (1306) + TX, NC-184 (compound code) + TX, nicotine (578) + TX, nicotine sulfate (578) + TX, nifluridide (1309) + TX, nitenpyram (579) + TX, nithiazine (1311) + TX, nitrilacarb (1313) + TX, nitrilacarb 1:1 zinc chloride complex (1313) + TX, NNI-0101 (compound code) + TX, NNI-0250 (compound code) + TX, nornicotine (traditional name) (1319) + TX, novaluron (585) + TX, noviflumuron (586) + TX, *O*-5-dichloro-4-iodophenyl *O*-ethyl ethylphosphonothioate (IUPAC name) (1057) + TX, O,O-diethyl *O*-4-methyl-2-oxo-2H-chromen-7-yl phosphorothioate (IUPAC name) (1074) + TX, O,O-diethyl *O*-6-methyl-2-propylpyrimidin-4-yl phosphorothioate (IUPAC name) (1075) + TX, O,O,O',O'-tetrapropyl dithiopyrophosphate (IUPAC name) (1424) + TX, oleic acid (IUPAC name) (593) + TX, omethoate (594) + TX, oxamyl (602) + TX, oxydemeton-methyl (609) + TX, oxydeprofos (1324) + TX, oxydisulfoton (1325) + TX, pp'-DDT (219) + TX, para-dichlorobenzene [CCN] + TX, parathion (615) + TX, parathion-methyl (616) + TX, penfluron (alternative name) [CCN] + TX, pentachlorophenol (623) + TX, pentachlorophenyl laurate (IUPAC name) (623) + TX, permethrin (626) + TX, petroleum oils (alternative name) (628) + TX, PH 60-38 (development code) (1328) + TX, phenkapton (1330) + TX, phenothrin (630) + TX, phenthoate (631) + TX, phorate (636) + TX, phosalone (637) + TX, phosfolan (1338) + TX, phosmet (638) + TX, phosnichlor (1339) + TX, phosphamidon (639) + TX, phosphine (IUPAC name) (640) + TX, phoxim (642) + TX, phoxim-methyl (1340) + TX, pirimetaphos (1344) + TX, pirimicarb (651) + TX, pirimiphos-ethyl (1345) + TX, pirimiphos-methyl (652) + TX, polychlorodicyclopentadiene isomers (IUPAC name) (1346) + TX, polychloroterpenes (traditional name) (1347) + TX, potassium arsenite [CCN] + TX, potassium thiocyanate [CCN] + TX, prallethrin (655) + TX, precocene I (alternative name) [CCN] + TX, precocene II (alternative name) [CCN] + TX, precocene III (alternative name) [CCN] + TX, primidophos (1349) + TX, profenofos (662) + TX, profluthrin [CCN] + TX, promacyl (1354) + TX, promecarb (1355) + TX, propaphos (1356) + TX, propetamphos (673) + TX, propoxur (678) + TX, prothidathion (1360) + TX, prothiofos (686) + TX, prothoate (1362) + TX, protrifenbute [CCN] + TX, pymetrozine (688) + TX, pyraclofos (689) + TX, pyrazophos (693) + TX, pyresmethrin (1367) + TX, pyrethrin I (696) + TX, pyrethrin II (696) + TX, pyrethrins (696) + TX, pyridaben (699) + TX, pyridalyl (700) + TX, pyridaphenthion (701) + TX, pyrimidifen (706) + TX, pyrimitate (1370) + TX, pyriproxyfen (708) + TX, quassia (alternative name) [CCN] + TX, quinalphos (711) + TX, quinalphos-methyl (1376) + TX, quinothion (1380) + TX, quintiofos (1381) + TX, R-1492 (development code) (1382) + TX, rafoxanide (alternative name) [CCN] + TX, resmethrin (719) + TX, rotenone (722) + TX, RU 15525 (development code) (723) + TX, RU 25475 (development code) (1386) + TX, ryania (alternative name) (1387) + TX, ryanodine (traditional name) (1387) + TX, sabadilla (alternative name) (725) + TX, schradan (1389) + TX, sebufos (alternative name) + TX, selamectin (alternative name) [CCN] + TX, SI-0009 (compound code) + TX, SI-0205 (compound code) + TX, SI-0404 (compound code) + TX, SI-0405 (compound code) + TX, silafluofen (728) + TX, SN 72129 (development code) (1397) + TX, sodium arsenite [CCN] + TX, sodium cyanide (444) + TX, sodium fluoride (IUPAC/Chemical Abstracts name) (1399) + TX, sodium hexafluorosilicate (1400) + TX, sodium pentachlorophenoxide (623) + TX, sodium selenate (IUPAC name) (1401) + TX, sodium thiocyanate [CCN] + TX, sophamide (1402) + TX, spinosad (737) + TX, spiromesifen (739) + TX, spirotetrmat (CCN) + TX, sulcofuron (746) + TX, sulcofuron-sodium (746) + TX, sulfluramid (750) + TX, sulfotep (753) + TX, sulfuryl fluoride (756) + TX, sulprofos (1408) + TX, tar oils (alternative name) (758) + TX, tau-fluvalinate (398) + TX, tazimcarb (1412) + TX, TDE (1414) + TX, tebufenozide (762) + TX, tebufenpyrad (763) + TX, tebupirimfos (764) + TX, teflubenzuron (768) + TX, tefluthrin (769) + TX, temephos (770) + TX, TEPP (1417) + TX, terallethrin (1418) + TX, terbam (alternative name) + TX, terbufos (773) + TX, tetrachloroethane [CCN] + TX, tetrachlorvinphos (777) + TX, tetramethrin (787) + TX, theta-cypermethrin (204) + TX, thiacloprid (791) + TX, thiafenox (alternative name) + TX, thiamethoxam (792) + TX, thicrofos (1428) + TX, thiocarboxime (1431) + TX, thiocyclam (798) + TX, thiocyclam hydrogen oxalate (798) + TX, thiodicarb (799) + TX, thiofanox (800) + TX, thiometon (801) + TX, thionazin (1434) + TX, thiosultap (803) + TX, thiosultap-sodium (803) + TX, thuringiensin (alternative name) [CCN] + TX, tolfenpyrad (809) + TX, tralomethrin (812) + TX, transfluthrin (813) + TX, transpermethrin (1440) + TX, triamiphos (1441) + TX, triazamate (818) + TX, triazophos (820) + TX, triazuron (alternative name) + TX, trichlorfon (824) + TX, trichlormetaphos-3 (alternative name) [CCN] + TX, trichloronat (1452) + TX, trifenofos (1455) + TX, triflumuron (835) + TX, trimethacarb (840) + TX, triprene (1459) + TX, vamidothion (847) + TX, vaniliprole [CCN] + TX, veratridine (alternative name) (725) + TX, veratrine (alternative name) (725) + TX, XMC (853) + TX, xylylcarb (854) + TX, YI-5302 (compound code) + TX, zeta-cypermethrin (205) + TX, zetamethrin (alternative name) + TX, zinc phosphide (640) + TX, zolaprofos (1469) and ZXI 8901 (development code) (858) + TX, cyantraniliprole [736994-63-19 + TX, chlorantraniliprole [500008-45-7] + TX, cyenopyrafen [560121-52-0] + TX, cyflumetofen [400882-07-7] + TX, pyrifluquinazon [337458-27-2] + TX, spinetoram [187166-40-1 + 187166-15-0] + TX, spirotetramat [203313-25-1] + TX, sulfoxaflor [946578-00-3] + TX, flufiprole [704886-18-0] + TX, meperfluthrin [915288-13-0] + TX, tetramethylfluthrin [84937-88-2] + TX, triflumezopyrim (disclosed in WO 2012/092115) + TX, fluxametamide (WO 2007/026965) + TX, epsilon-metofluthrin [240494-71-7] + TX, epsilon-momfluorothrin [1065124-65-3] + TX, fluazaindolizine [1254304-22-7] + TX, chloroprallethrin [399572-87-3] + TX, fluxametamide [928783-29-3] + TX, cyhalodiamide [1262605-53-7] + TX, tioxazafen [330459-31-9] + TX, broflanilide [1207727-04-5] + TX, flufiprole [704886-18-0] + TX, cyclaniliprole [1031756-98-5] + TX, tetraniliprole [1229654-66-3] + TX, guadipyr (described in WO2010/060231) + TX, cycloxaprid (described in WO 2005/077934) + TX, spiropidion + TX, Afidopyropen + TX, flupyrimin + TX, Momfluorothrin + TX, kappa-bifenthrin + TX, kappa-tefluthrin + TX, Dichloromezotiaz + TX, Tetrachloraniliprole + TX, benzpyrimoxan + TX;
a molluscicide selected from the group of substances consisting of bis(tributyltin) oxide (IUPAC name) (913) + TX, bromoacetamide [CCN] + TX, calcium arsenate [CCN] + TX, cloethocarb (999) + TX, copper acetoarsenite [CCN] + TX, copper sulfate (172) + TX, fentin (347) + TX, ferric phosphate (IUPAC name) (352) + TX, metaldehyde (518) + TX, methiocarb (530) + TX, niclosamide (576) + TX, niclosamide-olamine (576) + TX, pentachlorophenol (623) + TX, sodium pentachlorophenoxide (623) + TX, tazimcarb (1412) + TX, thiodicarb (799) + TX, tributyltin oxide (913) + TX, trifenmorph (1454) + TX, trimethacarb (840) + TX, triphenyltin acetate (IUPAC name) (347) and triphenyltin hydroxide (IUPAC name) (347) + TX, pyriprole [394730-71-3] + TX,
a nematicide selected from the group of substances consisting of AKD-3088 (compound code) + TX, 1,2-dibromo-3-chloropropane (IUPAC/Chemical Abstracts name) (1045) + TX, 1,2-dichloropropane (IUPAC/ Chemical Abstracts name) (1062) + TX, 1,2-dichloropropane with 1,3-dichloropropene (IUPAC name) (1063) + TX, 1,3-dichloropropene (233) + TX, 3,4-dichlorotetrahydrothiophene 1,1-dioxide (IUPAC/ChemicalAbstracts name) (1065) + TX, 3-(4-chlorophenyl)-5-methylrhodanine (IUPAC name) (980) + TX, 5-methyl-6-thioxo-1,3,5-thiadiazinan-3-ylacetic acid (IUPAC name) (1286) + TX, 6-isopentenylaminopurine (alternative name) (210) + TX, abamectin (1) + TX, acetoprole [CCN] + TX, alanycarb (15) + TX, aldicarb (16) + TX, aldoxycarb (863) + TX, AZ 60541 (compound code) + TX, benclothiaz [CCN] + TX, benomyl (62) + TX, butylpyridaben (alternative name) + TX, cadusafos (109) + TX, carbofuran (118) + TX, carbon disulfide (945) + TX, carbosulfan (119) + TX, chloropicrin (141) + TX, chlorpyrifos (145) + TX, cloethocarb (999) + TX, cytokinins (alternative name) (210) + TX, dazomet (216) + TX, DBCP (1045) + TX, DCIP (218) + TX, diamidafos (1044) + TX, dichlofenthion (1051) + TX, dicliphos (alternative name) + TX, dimethoate (262) + TX, doramectin (alternative name) [CCN] + TX, emamectin (291) + TX, emamectin benzoate (291) + TX, eprinomectin (alternative name) [CCN] + TX, ethoprophos (312) + TX, ethylene dibromide (316) + TX, fenamiphos (326) + TX, fenpyrad (alternative name) + TX, fensulfothion (1158) + TX, fosthiazate (408) + TX, fosthietan (1196) + TX, furfural (alternative name) [CCN] + TX, GY-81 (development code) (423) + TX, heterophos [CCN] + TX, iodomethane (IUPAC name) (542) + TX, isamidofos (1230) + TX, isazofos (1231) + TX, ivermectin (alternative name) [CCN] + TX, kinetin (alternative name) (210) + TX, mecarphon (1258) + TX, metam (519) + TX, metam-potassium (alternative name) (519) + TX, metam-sodium (519) + TX, methyl bromide (537) + TX, methyl isothiocyanate (543) + TX, milbemycin oxime (alternative name) [CCN] + TX, moxidectin (alternative name) [CCN] + TX, *Myrothecium verrucaria* composition (alternative name) (565) + TX, NC-184 (compound code) + TX, oxamyl (602) + TX, phorate (636) + TX, phosphamidon (639) + TX, phosphocarb [CCN] + TX, sebufos (alternative name) + TX, selamectin (alternative name) [CCN] + TX, spinosad (737) + TX, terbam (alternative name) + TX, terbufos (773) + TX, tetrachlorothiophene (IUPAC/ Chemical Abstracts name) (1422) + TX, thiafenox (alternative name) + TX, thionazin (1434) + TX, triazophos (820) + TX, triazuron (alternative name) + TX, xylenols [CCN] + TX, YI-5302 (compound code) and zeatin (alternative name) (210) + TX, fluensulfone [318290-98-1] + TX, fluopyram + TX,
a nitrification inhibitor selected from the group of substances consisting of potassium ethylxanthate [CCN] and nitrapyrin (580) + TX,
a plant activator selected from the group of substances consisting of acibenzolar (6) + TX, acibenzolar-S-methyl (6) + TX, probenazole (658) and *Reynoutria sachalinensis* extract (alternative name) (720) + TX,
a rodenticide selected from the group of substances consisting of 2-isovalerylindan-1,3-dione (IUPAC name) (1246) + TX, 4-(quinoxalin-2-ylamino)benzenesulfonamide (IUPAC name) (748) + TX, alpha-chlorohydrin [CCN] + TX, aluminium phosphide (640) + TX, antu (880) + TX, arsenous oxide (882) + TX, barium carbonate (891) + TX, bisthiosemi (912) + TX, brodifacoum (89) + TX, bromadiolone (91) + TX, bromethalin (92) + TX, calcium cyanide (444) + TX, chloralose (127) + TX, chlorophacinone (140) + TX, cholecalciferol (alternative name) (850) + TX, coumachlor (1004) + TX, coumafuryl (1005) + TX, coumatetralyl (175) + TX, crimidine (1009) + TX, difenacoum (246) + TX, difethialone (249) + TX, diphacinone (273) + TX, ergocalciferol (301) + TX, flocoumafen (357) + TX, fluoroacetamide (379) + TX, flupropadine (1183) + TX, flupropadine hydrochloride (1183) + TX, gamma-HCH (430) + TX, HCH (430) + TX, hydrogen cyanide (444) + TX, iodomethane (IUPAC name) (542) + TX, lindane (430) + TX, magnesium phosphide (IUPAC name) (640) + TX, methyl bromide (537) + TX, norbormide (1318) + TX, phosacetim (1336) + TX, phosphine (IUPAC name) (640) + TX, phosphorus [CCN] + TX, pindone (1341) + TX, potassium arsenite [CCN] + TX, pyrinuron (1371) + TX, scilliroside (1390) + TX, sodium arsenite [CCN] + TX, sodium cyanide (444) + TX, sodium fluoroacetate (735) + TX, strychnine (745) + TX, thallium sulfate [CCN] + TX, warfarin (851) and zinc phosphide (640) + TX,
a synergist selected from the group of substances consisting of 2-(2-butoxyethoxy)ethyl piperonylate (IUPAC name) (934) + TX, 5-(1,3-benzodioxol-5-yl)-3-hexylcyclohex-2-enone (IUPAC name) (903) + TX, farnesol with nerolidol (alternative name) (324) + TX, MB-599 (development code) (498) + TX, MGK 264 (development code) (296) + TX, piperonyl butoxide (649) + TX, piprotal (1343) + TX, propyl isomer (1358) + TX, S421 (development code) (724) + TX, sesamex (1393) + TX, sesasmolin (1394) and sulfoxide (1406) + TX,
an animal repellent selected from the group of substances consisting of anthraquinone (32) + TX, chloralose (127) + TX, copper naphthenate [CCN] + TX, copper oxychloride (171) + TX, diazinon (227) + TX, dicyclopentadiene (chemical name) (1069) + TX, guazatine (422) + TX, guazatine acetates (422) + TX, methiocarb (530) + TX, pyridin-4-amine (IUPAC name) (23) + TX, thiram (804) + TX, trimethacarb (840) + TX, zinc naphthenate [CCN] and ziram (856) + TX,
a virucide selected from the group of substances consisting of imanin (alternative name) [CCN] and ribavirin (alternative name) [CCN] + TX,
a wound protectant selected from the group of substances consisting of mercuric oxide (512) + TX, octhilinone (590) and thiophanate-methyl (802) + TX,
and biologically active compounds selected from the group consisting of azaconazole (60207-31-0] + TX, bitertanol [70585-36-3] + TX, bromuconazole [116255-48-2] + TX, cyproconazole [94361-06-5] + TX, difenoconazole [119446-68-3] + TX, diniconazole [83657-24-3] + TX, epoxiconazole [106325-08-0] + TX, fenbuconazole [114369-43-6] + TX, fluquinconazole [136426-54-5] + TX, flusilazole [85509-19-9] + TX, flutriafol [76674-21-0] + TX, hexaconazole [79983-71-4] + TX, imazalil [35554-44-0] + TX, imibenconazole [86598-92-7] + TX, ipconazole [125225-28-7] + TX, metconazole [125116-23-6] + TX, myclobutanil [88671-89-0] + TX, pefurazoate [101903-30-4] + TX, penconazole [66246-88-6] + TX, prothioconazole [178928-70-6] + TX, pyrifenox [88283-41-4] + TX, prochloraz [67747-09-5] + TX, propiconazole [60207-90-1] + TX, simeconazole [149508-90-7] + TX, tebuconazole [107534-96-3] + TX, tetraconazole [112281-77-3] + TX, triadimefon [43121-43-3] + TX, triadimenol [55219-65-3] + TX, triflumizole [99387-89-0] + TX, triticonazole [131983-72-7] + TX, ancymidol [12771-68-5] + TX, fenarimol [60168-88-9] + TX, nuarimol [63284-71-9] + TX, bupirimate [41483-43-6] + TX, dimethirimol [5221-53-4] + TX, ethirimol [23947-60-6] + TX, dodemorph [1593-77-7] + TX, fenpropidine [67306-00-7] + TX, fenpropimorph [67564-91-4] + TX, spiroxamine [118134-30-8] + TX, tridemorph [81412-43-3] + TX, cyprodinil [121552-61-2] + TX, mepanipyrim [110235-47-7] + TX, pyrimethanil [53112-28-0] + TX, fenpiclonil [74738-17-3] + TX, fludioxonil [131341-86-1] + TX, benalaxyl [71626-11-4] + TX, furalaxyl [57646-30-7] + TX, metalaxyl [57837-19-1] + TX, R-metalaxyl [70630-17-0] + TX, ofurace [58810-48-3] + TX, oxadixyl [77732-09-3] + TX, benomyl [17804-35-2] + TX, carbendazim [10605-21-7] + TX, debacarb [62732-91-6] + TX, fuberidazole [3878-19-1] + TX, thiabendazole [148-79-8] + TX, chlozolinate [84332-86-5] + TX, dichlozoline [24201-58-9] + TX, iprodione [36734-19-7] + TX, myclozoline [54864-61-8] + TX, procymidone [32809-16-8] + TX, vinclozoline [50471-44-8] + TX, boscalid [188425-85-6] + TX, carboxin [5234-68-4] + TX, fenfuram [24691-80-3] + TX, flutolanil [66332-96-5] + TX, mepronil [55814-41-0] + TX, oxycarboxin [5259-88-1] + TX, penthiopyrad [183675-82-3] + TX, thifluzamide [130000-40-7] + TX, guazatine [108173-90-6] + TX, dodine [2439-10-3] [112-65-2] (free base) + TX, iminoctadine [13516-27-3] + TX, azoxystrobin [131860-33-8] + TX, dimoxystrobin [149961-52-4] + TX, enestroburin {Proc. BCPC, Int. Congr., Glasgow, 2003, 1, 93} + TX, fluoxastrobin [361377-29-9] + TX, kresoxim-methyl [143390-89-0] + TX, metominostrobin [133408-50-1] + TX, trifloxystrobin [141517-21-7] + TX, orysastrobin [248593-16-0] + TX, picoxystrobin [117428-22-5] + TX, pyraclostrobin [175013-18-0] + TX, ferbam [14484-64-1] + TX, mancozeb [8018-01-7] + TX, maneb [12427-38-2] + TX, metiram [9006-42-2] + TX, propineb [12071-83-9] + TX, thiram [137-26-8] + TX, zineb [12122-67-7] + TX, ziram [137-30-4] + TX, captafol [2425-06-1] + TX, captan [133-06-2] + TX, dichlofluanid [1085-98-9] + TX, fluoroimide [41205-21-4] + TX, folpet [133-07-3] + TX, tolylfluanid [731-27-1] + TX, bordeaux mixture [8011-63-0] + TX, copperhydroxid [20427-59-2] + TX, copperoxychlorid [1332-40-7] + TX, coppersulfat [7758-98-7] + TX, copperoxid [1317-39-1] + TX, mancopper [53988-93-5] + TX, oxine-copper [10380-28-6] + TX, dinocap [131-72-6] + TX, nitrothal-isopropyl [10552-74-6] + TX, edifenphos [17109-49-8] + TX, iprobenphos [26087-47-8] + TX, isoprothiolane [50512-35-1] + TX, phosdiphen [36519-00-3] + TX, pyrazophos [13457-18-6] + TX, tolclofos-methyl [57018-04-9] + TX, acibenzolar-S-methyl [135158-54-2] + TX, anilazine [101-05-3] + TX, benthiavalicarb [413615-35-7] + TX, blasticidin-S [2079-00-7] + TX, chinomethionat [2439-01-2] + TX, chloroneb [2675-77-6] + TX, chlorothalonil [1897-45-6] + TX, cyflufenamid *[180409-60-3]* + TX, cymoxanil [57966-95-7] + TX, dichlone *[117-80-6]* + TX, diclocymet [139920-32-4] + TX, diclomezine [62865-36-5]+ TX, dicloran [99-30-9] + TX, diethofencarb *[87130-20-9]* + TX, dimethomorph *[110488-70-5]* + TX, SYP-LI90 (Flumorph) *[211867-47-9]* + TX, dithianon *[3347-22-6]* + TX, ethaboxam *[162650-77-3]* + TX, etridiazole *[2593-15-9]* + TX, famoxadone *[131807-57-3]* + *TX,* fenamidone *[161326-34-7]* + TX, fenoxanil *[115852-48-7]* + TX, fentin *[668-34-8]* + TX, ferimzone *[89269-64-7]* + TX, fluazinam *[79622-59-6]* + TX, fluopicolide [239110-15-7] + TX, flusulfamide [106917-52-6] + TX, fenhexamid [126833-17-8] + TX, fosetyl-aluminium [39148-24-8] + TX, hymexazol [10004-44-1] + TX, iprovalicarb [140923-17-7] + TX, IKF-916 (Cyazofamid) *[120116-88-3]* + TX, kasugamycin [6980-18-3] + TX, methasulfocarb *[66952-49-6]* + TX, metrafenone *[220899-03-6]* + TX, pencycuron [66063-*05-6]* + TX, phthalide [27355-22-2] + TX, polyoxins [11113-80-7] + TX, probenazole [27605-76-1] + TX, propamocarb [25606-41-1] + TX, proquinazid [189278-12-4] + TX, pyroquilon [57369-32-1] + TX, quinoxyfen [124495-18-7] + TX, quintozene [82-68-8] + TX, sulfur [7704-34-9] + TX, tiadinil [223580-51-6] + TX, triazoxide [72459-58-6] + TX, tricyclazole [41814-78-2] + TX, triforine [26644-46-2] + TX, validamycin [37248-47-8] + TX, zoxamide (RH7281) [156052-68-5] + TX, mandipropamid [374726-62-2] + TX, isopyrazam [881685-58-1] + TX, sedaxane [874967-67-6] + TX, 3-difluoromethyl-1-methyl-1H-pyrazole-4-carboxylic acid (9-dichloromethylene-1,2,3,4-tetrahydro-1,4-methano-naphthalen-5-yl)-amide (disclosed in WO 2007/048556) + TX, 3-difluoromethyl-1-methyl-1H-pyrazole-4-carboxylic acid (3',4',5'-trifluoro-biphenyl-2-yl)-amide (disclosed in WO 2006/087343) + TX, [(3*S*,4*R,*4a*R*,6*S*,6a*S*,12*R*,12a*S*,12b*S*)-3-[(cyclopropylcarbonyl)oxy]- 1,3,4,4a,5,6,6a,12,12a,12b-decahydro-6,12-dihydroxy-4,6a,12b-trimethyl-11-oxo-9-(3-pyridinyl)-2*H*,11*H*naphtho[2,1-*b*]pyrano[3,4-e]pyran-4-yl]methyl-cyclopropanecarboxylate [915972-17-7] + TX and 1,3,5-trimethyl-N-(2-methyl-1-oxopropyl)-N-[3-(2-methylpropyl)-4-[2,2,2-trifluoro-1-methoxy-1-(trifluoromethyl)ethyl]phenyl]-1H-pyrazole-4-carboxamide [926914-55-8] + TX, lancotrione [1486617-21-3] + TX, florpyrauxifen [943832-81-3] + TX, ipfentrifluconazole[1417782-08-1] + TX, mefentrifluconazole [1417782-03-6] + TX, quinofumelin [861647-84-9] + TX, chloroprallethrin [399572-87-3] + TX, cyhalodiamide [1262605-53-7] + TX, fluazaindolizine [1254304-22-7] + TX, fluxametamide [928783-29-3] + TX, epsilon-metofluthrin [240494-71-7] + TX, epsilon-momfluorothrin [1065124-65-3] + TX, pydiflumetofen [1228284-64-7] + TX, kappa-bifenthrin [439680-76-9] + TX, broflanilide [1207727-04-5] + TX, dicloromezotiaz [1263629-39-5] + TX, dipymetitrone [16114-35-5] + TX, pyraziflumid [942515-63-1] + TX and kappa-tefluthrin [391634-71-2] + TX; and
microbials including: *Acinetobacter Iwoffii* + TX, *Acremonium alternatum* + TX + TX, *Acremonium cephalosporium* + TX + TX, *Acremonium diospyri* + TX, *Acremonium obclavatum* + TX, *Adoxophyes orana granulovirus* (AdoxGV) (Capex^{®}) + TX, *Agrobacterium radiobacter* strain K84 (Galltrol-A^{®}) + TX, *Alternaria alternate* + TX, *Alternaria cassia* + TX, *Alternaria destruens* (Smolder^{®}) + TX, *Ampelomyces quisqualis* (AQ10^{®}) + TX, *Aspergillus flavus* AF36 (AF36^{®}) + TX, *Aspergillus flavus* NRRL 21882 (Aflaguard^{®}) + TX, *Aspergillus* spp. + TX, *Aureobasidium pullulans* + TX, *Azospirillum +* TX, (MicroAZ^{®} + TX, TAZO B^{®}) + TX, *Azotobacter* + TX, *Azotobacter chroocuccum* (Azotomeal^{®}) + TX, *Azotobacter* cysts (Bionatural Blooming Blossoms@) + TX, *Bacillus amyloliquefaciens* + TX, *Bacillus cereus* + TX, *Bacillus chitinosporus* strain CM-1 + TX, *Bacillus chitinosporus* strain AQ746 + TX, *Bacillus licheniformis* strain HB-2 (Biostart^{™} Rhizoboost^{®}) + TX, *Bacillus licheniformis* strain 3086 (EcoGuard^{®} + TX, Green Releaf^{®}) + TX, *Bacillus circulans* + TX, *Bacillus firmus* (BioSafe^{®} + TX, BioNem-WP^{®} + TX, VOTiVO^{®}) + TX, *Bacillus firmus* strain I-1582 + TX, *Bacillus macerans* + TX, *Bacillus marismortui* + TX, *Bacillus megaterium* + TX, *Bacillus mycoides* strain AQ726 + TX, *Bacillus papillae* (Milky Spore Powder^{®}) + TX, *Bacillus pumilus* spp. + TX, *Bacillus pumilus* strain GB34 (Yield Shield^{®}) + TX, *Bacillus pumilus* strain AQ717 + TX, *Bacillus pumilus* strain QST 2808 (Sonata^{®} + TX, Ballad Plus^{®}) + TX, *Bacillus spahericus* (VectoLex^{®}) + TX, *Bacillus* spp. + TX, *Bacillus* spp. strain AQ175 + TX, *Bacillus* spp. strain AQ177 + TX, *Bacillus* spp. strain AQ178 + TX, *Bacillus subtilis* strain QST 713 (CEASE^{®} + TX, Serenade^{®} + TX, Rhapsody^{®}) + TX, *Bacillus subtilis* strain QST 714 (JAZZ@) + TX, *Bacillus subtilis* strain AQ153 + TX, *Bacillus subtilis* strain AQ743 + TX, *Bacillus subtilis* strain QST3002 + TX, *Bacillus subtilis* strain QST3004 + TX, *Bacillus subtilis var. amyloliquefaciens* strain FZB24 (Taegro^{®} + TX, Rhizopro^{®}) + TX, *Bacillus thuringiensis* Cry 2Ae + TX, *Bacillus thuringiensis* Cry1Ab + TX, *Bacillus thuringiensis aizawai* GC 91 (Agree^{®}) + TX, *Bacillus thuringiensis israelensis* (BMP123^{®} + TX, Aquabac^{®} + TX, VectoBac^{®}) + TX, *Bacillus thuringiensis kurstaki* (Javelin^{®} + TX, Deliver^{®} + TX, CryMax^{®} + TX, Bonide^{®} + TX, Scutella WP^{®} + TX, Turilav WP ^{®} + TX, Astuto^{®} + TX, Dipel WP^{®} + TX, Biobit^{®} + TX, Foray^{®}) + TX, *Bacillus thuringiensis kurstaki* BMP 123 (Baritone^{®}) + TX, *Bacillus thuringiensis kurstaki* HD-1 (Bioprotec-CAF / 3P^{®}) + TX, *Bacillus thuringiensis* strain BD#32 + TX, *Bacillus thuringiensis* strain AQ52 + TX, *Bacillus thuringiensis var. aizawai* (XenTari^{®} + TX, DiPeI^{®}) + TX, bacteria spp. (GROWMEND^{®} + TX, GROWSWEET^{®} + TX, Shootup^{®}) + TX, bacteriophage of *Clavipacter michiganensis* (AgriPhage^{®}) + TX, Bakflor^{®} + TX, *Beauveria bassiana* (Beaugenic^{®} + TX, Brocaril WP^{®}) + TX, *Beauveria bassiana* GHA (Mycotrol ES^{®} + TX, Mycotrol O^{®} + TX, BotaniGuard^{®}) + TX, *Beauveria brongniartii* (Engerlingspilz^{®} + TX, Schweizer Beauveria^{®} + TX, Melocont^{®}) + TX, *Beauveria* spp. + TX, *Botrytis cineria* + TX, *Bradyrhizobium japonicum* (TerraMax^{®}) + TX, *Brevibacillus brevis* + TX, *Bacillus thuringiensis tenebrionis* (Novodor^{®}) + TX, BtBooster + TX, *Burkholderia cepacia* (Deny^{®} + TX, Intercept^{®} + TX, Blue Circle^{®}) + TX, *Burkholderia gladii* + TX, *Burkholderia gladioli* + TX, *Burkholderia* spp. + TX, Canadian thistle fungus (CBH Canadian Bioherbicide^{®}) + TX, *Candida butyri* + TX, *Candida famata +* TX, *Candida fructus* + TX, *Candida glabrata* + TX, *Candida guilliermondii* + TX, *Candida melibiosica +* TX, *Candida oleophila* strain O + TX, *Candida parapsilosis* + TX, *Candida pelliculosa* + TX, *Candida pulcherrima* + TX, *Candida reukaufii* + TX, *Candida saitoana* (Bio-Coat^{®} + TX, Biocure^{®}) + TX, *Candida sake* + TX, *Candida* spp. + TX, *Candida tenius* + TX, *Cedecea dravisae* + TX, *Cellulomonas flavigena* + TX, *Chaetomium cochliodes* (Nova-Cide^{®}) + TX, *Chaetomium globosum* (Nova-Cide^{®}) + TX, *Chromobacterium subtsugae* strain PRAA4-1T (Grandevo^{®}) + TX, *Cladosporium cladosporioides* + TX, *Cladosporium oxysporum* + TX, *Cladosporium chlorocephalum* + TX, *Cladosporium* spp. + TX, *Cladosporium tenuissimum* + TX, *Clonostachys rosea* (EndoFine^{®}) + TX, *Colletotrichum acutatum +* TX, *Coniothyrium minitans* (Cotans WG^{®}) + TX, *Coniothyrium* spp. + TX, *Cryptococcus albidus* (YIELDPLUS^{®}) + TX, *Cryptococcus humicola* + TX, *Cryptococcus infirmo-miniatus* + TX, *Cryptococcus laurentii* + TX, *Cryptophlebia leucotreta granulovirus* (Cryptex^{®}) + TX, *Cupriavidus campinensis* + TX, *Cydia pomonella granulovirus* (CYD-X^{®}) + TX, *Cydia pomonella granulovirus* (Madex^{®} + TX, Madex Plus^{®} + TX, Madex Maxi Carpovirusine^{®}) + TX, *Cylindrobasidium laeve* (Stumpout^{®}) + TX, *Cylindrocladium* + TX, *Debaryomyces hansenii* + TX, *Drechslera hawaiinensis +* TX, *Enterobacter cloacae* + TX, *Enterobacteriaceae* + TX, *Entomophtora virulenta* (Vektor^{®}) + TX, *Epicoccum nigrum* + TX, *Epicoccum purpurascens* + TX, *Epicoccum* spp. + TX, *Filobasidium floriforme* + TX, *Fusarium acuminatum* + TX, *Fusarium chlamydosporum* + TX, *Fusarium oxysporum* (Fusaclean^{®} / Biofox C^{®}) + TX, *Fusarium proliferatum* + TX, *Fusarium* spp. + TX, *Galactomyces geotrichum* + TX, *Gliocladium catenulatum* (Primastop^{®} + TX, Prestop^{®}) + TX, *Gliocladium roseum +* TX, *Gliocladium* spp. (SoilGard^{®}) + TX, *Gliocladium virens* (Soilgard^{®}) + TX, *Granulovirus* (Granupom^{®}) + TX, *Halobacillus halophilus* + TX, *Halobacillus litoralis* + TX, *Halobacillus trueperi +* TX, *Halomonas* spp. + TX, *Halomonas subglaciescola* + TX, *Halovibrio variabilis* + TX, *Hanseniaspora uvarum* + TX, *Helicoverpa armigera nucleopolyhedrovirus* (Helicovex^{®}) + TX, *Helicoverpa zea nuclear polyhedrosis virus* (Gemstar^{®}) + TX, Isoflavone - formononetin (Myconate^{®}) + TX, *Kloeckera apiculata* + TX, *Kloeckera* spp. + TX, *Lagenidium giganteum* (Laginex^{®}) + TX, *Lecanicillium longisporum* (Vertiblast^{®}) + TX, *Lecanicillium muscarium* (Vertikil^{®}) + TX, *Lymantria Dispar nucleopolyhedrosis virus* (Disparvirus^{®}) + TX, *Marinococcus halophilus* + TX, *Meira geulakonigii* + TX, *Metarhizium anisopliae* (Met52^{®}) + TX, *Metarhizium anisopliae* (Destruxin WP^{®}) + TX, *Metschnikowia fruticola* (Shemer^{®}) + TX, *Metschnikowia pulcherrima* + TX, *Microdochium dimerum* (Antibot^{®}) + TX, *Micromonospora coerulea* + TX, *Microsphaeropsis ochracea* + TX, *Muscodor albus* 620 (Muscudor^{®}) + TX, *Muscodor roseus* strain A3-5 + TX, *Mycorrhizae* spp. (AMykor^{®} + TX, Root Maximizer^{®}) + TX, *Myrothecium verrucaria* strain AARC-0255 (DiTera^{®}) + TX, BROS PLUS^{®} + TX, *Ophiostoma piliferum* strain D97 (Sylvanex^{®}) + TX, *Paecilomyces farinosus* + TX, *Paecilomyces fumosoroseus* (PFR-97^{®} + TX, PreFeRal^{®}) + TX, *Paecilomyces linacinus* (Biostat WP^{®}) + TX, *Paecilomyces lilacinus* strain 251 (MeloCon WG^{®}) + TX, *Paenibacillus polymyxa* + TX, *Pantoea agglomerans* (BlightBan C9-1^{®}) + TX, *Pantoea* spp. + TX, *Pasteuria* spp. (Econem^{®}) + TX, *Pasteuria nishizawae* + TX, *Penicillium aurantiogriseum* + TX, *Penicillium billai* (Jumpstart^{®} + TX, TagTeam^{®}) + TX, *Penicillium brevicompactum* + TX, *Penicillium frequentans* + TX, *Penicillium griseofulvum* + TX, *Penicillium purpurogenum* + TX, *Penicillium* spp. + TX, *Penicillium viridicatum* + TX, *Phlebiopsis gigantean* (Rotstop^{®}) + TX, phosphate solubilizing bacteria (Phosphomeal^{®}) + TX, *Phytophthora cryptogea +* TX, *Phytophthora palmivora* (Devine^{®}) + TX, *Pichia anomala* + TX, *Pichia guilermondii* + TX, *Pichia membranaefaciens* + TX, *Pichia onychis* + TX, *Pichia stipites* + TX, *Pseudomonas aeruginosa* + TX, *Pseudomonas aureofasciens* (Spot-Less Biofungicide^{®}) + TX, *Pseudomonas cepacia* + TX, *Pseudomonas chlororaphis* (AtEze^{®}) + TX, *Pseudomonas corrugate* + TX, *Pseudomonas fluorescens* strain A506 (BlightBan A506^{®}) + TX, *Pseudomonas putida* + TX, *Pseudomonas reactans* + TX, *Pseudomonas* spp. + TX, *Pseudomonas syringae* (Bio-Save^{®}) + TX, *Pseudomonas viridiflava* + TX, *Pseudomons fluorescens* (Zequanox^{®}) + TX, *Pseudozyma flocculosa* strain PF-A22 UL (Sporodex L^{®}) + TX, *Puccinia canaliculata* + TX, *Puccinia thlaspeos* (Wood Warrior^{®}) + TX, *Pythium paroecandrum* + TX, *Pythium oligandrum* (Polygandron^{®} + TX, Polyversum^{®}) + TX, *Pythium periplocum* + TX, *Rhanella aquatilis* + TX, *Rhanella* spp. + TX, *Rhizobia* (Dormal^{®} + TX, Vault^{®}) + TX, *Rhizoctonia* + TX, *Rhodococcus globerulus* strain AQ719 + TX, *Rhodosporidium diobovatum* + TX, *Rhodosporidium toruloides* + TX, *Rhodotorula* spp. + TX, *Rhodotorula glutinis* + TX, *Rhodotorula graminis* + TX, *Rhodotorula mucilagnosa* + TX, *Rhodotorula rubra* + TX, *Saccharomyces cerevisiae +* TX, *Salinococcus roseus* + TX, *Sclerotinia minor* + TX, *Sclerotinia minor* (SARRITOR^{®}) + TX, *Scytalidium* spp. + TX, *Scytalidium uredinicola* + TX, *Spodoptera exigua nuclear polyhedrosis virus* (Spod-X^{®} + TX, Spexit^{®}) + TX, *Serratia marcescens* + TX, *Serratia plymuthica* + TX, *Serratia* spp. + TX, *Sordaria fimicola* + TX, *Spodoptera littoralis nucleopolyhedrovirus* (Littovir^{®}) + TX, *Sporobolomyces roseus* + TX, *Stenotrophomonas maltophilia* + TX, *Streptomyces ahygroscopicus +* TX, *Streptomyces albaduncus* + TX, *Streptomyces exfoliates* + TX, *Streptomyces galbus* + TX, *Streptomyces griseoplanus* + TX, *Streptomyces griseoviridis* (Mycostop^{®}) + TX, *Streptomyces lydicus* (Actinovate^{®}) + TX, *Streptomyces lydicus* WYEC-108 (ActinoGrow^{®}) + TX, *Streptomyces violaceus +* TX, *Tilletiopsis minor* + TX, *Tilletiopsis* spp. + TX, *Trichoderma asperellum* (T34 Biocontrol^{®}) + TX, *Trichoderma gamsii* (Tenet^{®}) + TX, *Trichoderma atroviride* (Plantmate^{®}) + TX, *Trichoderma hamatum* TH 382 + TX, *Trichoderma harzianum rifai* (Mycostar^{®}) + TX, *Trichoderma harzianum* T-22 (Trianum-P^{®} + TX, PlantShield HC^{®} + TX, Rootshield^{®} + TX, Trianum-G^{®}) + TX, *Trichoderma harzianum* T-39 (Trichodex^{®}) + TX, *Trichoderma inhamatum* + TX, *Trichoderma koningii* + TX, *Trichoderma* spp. LC 52 (Sentinel^{®}) + TX, *Trichoderma lignorum* + TX, *Trichoderma longibrachiatum* + TX, *Trichoderma polysporum* (Binab T^{®}) + TX, *Trichoderma taxi* + TX, *Trichoderma virens* + TX, *Trichoderma virens* (formerly Gliocladium virens GL-21) (SoilGuard^{®}) + TX, *Trichoderma viride* + TX, *Trichoderma viride* strain ICC 080 (Remedier^{®}) + TX, *Trichosporon pullulans* + TX, *Trichosporon* spp. + TX, *Trichothecium* spp. + TX, *Trichothecium roseum* + TX, *Typhula phacorrhiza* strain 94670 + TX, *Typhula phacorrhiza* strain 94671 + TX, *Ulocladium atrum* + TX, *Ulocladium oudemansii* (Botry-Zen^{®}) + TX, *Ustilago maydis* + TX, various bacteria and supplementary micronutrients (Natural II^{®}) + TX, various fungi (Millennium Microbes^{®}) + TX, *Verticillium chlamydosporium* + TX, *Verticillium lecanii* (Mycotal^{®} + TX, Vertalec^{®}) + TX, Vip3Aa20 (VIPtera^{®}) + TX, *Virgibaclillus marismortui* + TX, *Xanthomonas campestris pv. Poae* (Camperico^{®}) + TX, *Xenorhabdus bovienii* + TX, *Xenorhabdus nematophilus;* and
Plant extracts including: pine oil (Retenol^{®}) + TX, azadirachtin (Plasma Neem Oil^{®} + TX, AzaGuard^{®} + TX, MeemAzal^{®} + TX, Molt-X^{®} + TX, Botanical IGR (Neemazad^{®} + TX, Neemix^{®}) + TX, canola oil (Lilly Miller Vegol^{®}) + TX, *Chenopodium ambrosioides near ambrosioides* (Requiem^{®}) + TX, *Chrysanthemum* extract (Crisant^{®}) + TX, extract of neem oil (Trilogy^{®}) + TX, essentials oils of *Labiatae* (Botania^{®}) + TX, extracts of clove rosemary peppermint and thyme oil (Garden insect killer^{®}) + TX, Glycinebetaine (Greenstim^{®}) + TX, garlic + TX, lemongrass oil (GreenMatch^{®}) + TX, neem oil + TX, *Nepeta cataria* (Catnip oil) + TX, *Nepeta catarina* + TX, nicotine + TX, oregano oil (MossBuster^{®}) + TX, *Pedaliaceae* oil (Nematon^{®}) + TX, pyrethrum + TX, *Quillaja saponaria* (NemaQ^{®}) + TX, *Reynoutria sachalinensis* (Regalia^{®} + TX, Sakalia^{®}) + TX, rotenone (Eco Roten^{®}) + TX, *Rutaceae* plant extract (Soleo^{®}) + TX, soybean oil (Ortho ecosense^{®}) + TX, tea tree oil (Timorex Gold^{®}) + TX, thymus oil + TX, AGNIQUE^{®} MMF + TX, BugOil^{®} + TX, mixture of rosemary sesame pepermint thyme and cinnamon extracts (EF 300^{®}) + TX, mixture of clove rosemary and peppermint extract (EF 400^{®}) + TX, mixture of clove pepermint garlic oil and mint (Soil Shot^{®}) + TX, kaolin (Screen^{®}) + TX, storage glucam of brown algae (Laminarin^{®}); and
pheromones including: blackheaded fireworm pheromone (3M Sprayable Blackheaded Fireworm Pheromone^{®}) + TX, Codling Moth Pheromone (Paramount dispenser-(CM)/ Isomate C-Plus^{®}) + TX, Grape Berry Moth Pheromone (3M MEC-GBM Sprayable Pheromone^{®}) + TX, Leafroller pheromone (3M MEC - LR Sprayable Pheromone^{®}) + TX, Muscamone (Snip7 Fly Bait^{®} + TX, Starbar Premium Fly Bait^{®}) + TX, Oriental Fruit Moth Pheromone (3M oriental fruit moth sprayable pheromone^{®}) + TX, Peachtree Borer Pheromone (Isomate-P^{®}) + TX, Tomato Pinworm Pheromone (3M Sprayable pheromone^{®}) + TX, Entostat powder (extract from palm tree) (Exosex CM^{®}) + TX, (E + TX,Z + TX,Z)-3 + TX,8 + TX,11 Tetradecatrienyl acetate + TX, (Z + TX,Z + TX,E)-7 + TX,11 + TX,13-Hexadecatrienal + TX, (E + TX,Z)-7 + TX,9-Dodecadien-1-yl acetate + TX, 2-Methyl-1-butanol + TX, Calcium acetate + TX, Scenturion^{®} + TX, Biolure^{®} + TX, Check-Mate^{®} + TX, Lavandulyl senecioate; and
Macrobials including: *Aphelinus abdominalis* + TX, *Aphidius ervi* (Aphelinus-System^{®}) + TX, *Acerophagus papaya* + TX, *Adalia bipunctata* (Adalia-System^{®}) + TX, *Adalia bipunctata* (Adaline^{®}) + TX, *Adalia bipunctata* (Aphidalia^{®}) + TX, *Ageniaspis citricola* + TX, *Ageniaspis fuscicollis* + TX, *Amblyseius andersoni* (Anderline^{®} + TX, Andersoni-System^{®}) + TX, *Amblyseius californicus* (Amblyline^{®} + TX, Spical^{®}) + TX, *Amblyseius cucumeris* (Thripex^{®} + TX, Bugline cucumeris^{®}) + TX, *Amblyseius fallacis* (Fallacis^{®}) + TX, *Amblyseius swirskii* (Bugline swirskii^{®} + TX, Swirskii-Mite^{®}) + TX, *Amblyseius womersleyi* (WomerMite^{®}) + TX, *Amitus hesperidum* + TX, *Anagrus atomus* + TX, *Anagyrus fusciventris* + TX, *Anagyrus kamali* + TX, *Anagyrus loecki* + TX, *Anagyrus pseudococci* (Citripar^{®}) + TX, *Anicetus benefices* + TX, *Anisopteromalus calandrae* + TX, *Anthocoris nemoralis* (Anthocoris-System^{®}) + TX, *Aphelinus abdominalis* (Apheline^{®} + TX, Aphiline^{®}) + TX, *Aphelinus asychis* + TX, *Aphidius colemani* (Aphipar^{®}) + TX, *Aphidius ervi* (Ervipar^{®}) + TX, *Aphidius gifuensis +* TX, *Aphidius matricariae* (Aphipar-M^{®}) + TX, *Aphidoletes aphidimyza* (Aphidend^{®}) + TX, *Aphidoletes aphidimyza* (Aphidoline^{®}) + TX, *Aphytis lingnanensis* + TX, *Aphytis melinus* + TX, *Aprostocetus hagenowii* + TX, *Atheta coriaria* (Staphyline^{®}) + TX, *Bombus* spp. + TX, *Bombus terrestris* (Natupol Beehive^{®}) + TX, *Bombus terrestris* (Beeline^{®} + TX, Tripol^{®}) + TX, *Cephalonomia stephanoderis +* TX, *Chilocorus nigritus* + TX, *Chrysoperla carnea* (Chrysoline^{®}) + TX, *Chrysoperla carnea* (Chrysopa^{®}) + TX, *Chrysoperla rufilabris* + TX, *Cirrospilus ingenuus* + TX, *Cirrospilus quadristriatus +* TX, *Citrostichus phyllocnistoides* + TX, *Closterocerus chamaeleon* + TX, *Closterocerus* spp. + TX, *Coccidoxenoides perminutus* (Pianopar^{®}) + TX, *Coccophagus cowperi* + TX, *Coccophagus lycimnia +* TX, *Cotesia flavipes* + TX, *Cotesia plutellae* + TX, *Cryptolaemus montrouzieri* (Cryptobug^{®} + TX, Cryptoline^{®}) + TX, *Cybocephalus nipponicus* + TX, *Dacnusa sibirica* + TX, *Dacnusa sibirica* (Minusa^{®}) + TX, *Diglyphus isaea* (Diminex^{®}) + TX, *Delphastus catalinae* (Delphastus^{®}) + TX, *Delphastus pusillus* + TX, *Diachasmimorpha krausii* + TX, *Diachasmimorpha longicaudata* + TX, *Diaparsis jucunda* + TX, *Diaphorencyrtus aligarhensis* + TX, *Diglyphus isaea* + TX, *Diglyphus isaea* (Miglyphus^{®} + TX, Digline^{®}) + TX, *Dacnusa sibirica* (DacDigline^{®} + TX, Minex^{®}) + TX, *Diversinervus* spp. + TX, *Encarsia citrina* + TX, *Encarsia formosa* (Encarsia max^{®} + TX, Encarline^{®} + TX, En-Strip^{®}) + TX, *Eretmocerus eremicus* (Enermix^{®}) + TX, *Encarsia guadeloupae* + TX, *Encarsia haitiensis* + TX, *Episyrphus balteatus* (Syrphidend^{®}) + TX, *Eretmoceris siphonini* + TX, *Eretmocerus californicus* + TX, *Eretmocerus eremicus* (Ercal^{®} + TX, Eretline e^{®}) + TX, *Eretmocerus eremicus* (Bemimix^{®}) + TX, *Eretmocerus hayati* + TX, *Eretmocerus mundus* (Bemipar^{®} + TX, Eretline m^{®}) + TX, *Eretmocerus siphonini* + TX, *Exochomus quadripustulatus* + TX, *Feltiella acarisuga* (Spidend^{®}) + TX, *Feltiella acarisuga* (Feltiline^{®}) + TX, *Fopius arisanus* + TX, *Fopius ceratitivorus* + TX, Formononetin (Wirless Beehome^{®}) + TX, *Franklinothrips vespiformis* (Vespop^{®}) + TX, *Galendromus occidentalis* + TX, *Goniozus legneri* + TX, *Habrobracon hebetor* + TX, *Harmonia axyridis* (HarmoBeetle^{®}) + TX, *Heterorhabditis* spp. (Lawn Patrol^{®}) + TX, *Heterorhabditis bacteriophora* (NemaShield HB^{®} + TX, Nemaseek^{®} + TX, Terranem-Nam^{®} + TX, Terranem^{®} + TX, Larvanem^{®} + TX, B-Green^{®} + TX, NemAttack ^{®} + TX, Nematop^{®}) + TX, *Heterorhabditis megidis* (Nemasys H^{®} + TX, BioNem H^{®} + TX, Exhibitline hm^{®} + TX, Larvanem-M^{®}) + TX, *Hippodamia convergens* + TX, *Hypoaspis aculeifer* (Aculeifer-System^{®} + TX, Entomite-A^{®}) + TX, *Hypoaspis miles* (Hypoline m^{®} + TX, Entomite-M^{®}) + TX, *Lbalia leucospoides* + TX, *Lecanoideus floccissimus* + TX, *Lemophagus errabundus* + TX, *Leptomastidea abnormis* + TX, *Leptomastix dactylopii* (Leptopar^{®}) + TX, *Leptomastix epona* + TX, *Lindorus lophanthae* + TX, *Lipolexis oregmae* + TX, *Lucilia caesar* (Natufly^{®}) + TX, *Lysiphlebus testaceipes* + TX, *Macrolophus caliginosus* (Mirical-N^{®} + TX, Macroline c^{®} + TX, Mirical^{®}) + TX, *Mesoseiulus longipes* + TX, *Metaphycus flavus* + TX, *Metaphycus lounsburyi* + TX, *Micromus angulatus* (Milacewing^{®}) + TX, *Microterys flavus* + TX, *Muscidifurax raptorellus* and *Spalangia cameroni* (Biopar^{®}) + TX, *Neodryinus typhlocybae* + TX, *Neoseiulus californicus* + TX, *Neoseiulus cucumeris* (THRYPEX^{®}) + TX, *Neoseiulus fallacis* + TX, *Nesideocoris tenuis* (NesidioBug^{®} + TX, Nesibug^{®}) + TX, *Ophyra aenescens* (Biofly^{®}) + TX, *Orius insidiosus* (Thripor-I^{®} + TX, Online i^{®}) + TX, *Orius laevigatus* (Thripor-L^{®} + TX, Online I^{®}) + TX, *Onus majusculus* (Oriline m^{®}) + TX, *Orius strigicollis* (Thripor-S^{®}) + TX, *Pauesia juniperorum* + TX, *Pediobius foveolatus* + TX, *Phasmarhabditis hermaphrodita* (Nemaslug^{®}) + TX, *Phymastichus coffea* + TX, *Phytoseiulus macro pilus* + TX, *Phytoseiulus persimilis* (Spidex^{®} + TX, Phytoline p^{®}) + TX, *Podisus maculiventris* (Podisus^{®}) + TX, *Pseudacteon curvatus* + TX, *Pseudacteon obtusus* + TX, *Pseudacteon tricuspis +* TX, *Pseudaphycus maculipennis* + TX, *Pseudleptomastix mexicana* + TX, *Psyllaephagus pilosus +* TX, *Psy(talia concolor* (complex) + TX, *Quadrastichus* spp. + TX, *Rhyzobius lophanthae* + TX, *Rodolia cardinalis* + TX, *Rumina decollate* + TX, *Semielacher petiolatus* + TX, *Sitobion avenae* (Ervibank^{®}) + TX, *Steinernema carpocapsae* (Nematac C^{®} + TX, Millenium^{®} + TX, BioNem C^{®} + TX, NemAttack^{®} + TX, Nemastar^{®} + TX, Capsanem^{®}) + TX, *Steinernema feltiae* (NemaShield^{®} + TX, Nemasys F^{®} + TX, BioNem F^{®} + TX, Steinernema-System^{®} + TX, NemAttack^{®} + TX, Nemaplus^{®} + TX, Exhibitline sf^{®} + TX, Scia-rid^{®} + TX, Entonem^{®}) + TX, *Steinernema kraussei* (Nemasys L^{®} + TX, BioNem L^{®} + TX, Exhibitline srb^{®}) + TX, *Steinernema riobrave* (BioVector^{®} + TX, BioVektor^{®}) + TX, *Steinernema scapterisci* (Nematac S^{®}) + TX, *Steinernema* spp. + TX, *Steinernematid* spp. (Guardian Nematodes^{®}) + TX, *Stethorus punctillum* (Stethorus^{®}) + TX, *Tamarixia radiate* + TX, *Tetrastichus setifer* + TX, *Thripobius semiluteus* + TX, *Torymus sinensis* + TX, *Trichogramma brassicae* (Tricholine b^{®}) + TX, *Trichogramma brassicae* (Tricho-Strip^{®}) + TX, *Trichogramma evanescens* + TX, *Trichogramma minutum* + TX, *Trichogramma ostriniae* + TX, *Trichogramma platneri* + TX, *Trichogramma pretiosum +* TX, *Xanthopimpla stemmator;* and
other biologicals including: abscisic acid + TX, bioSea^{®} + TX, *Chondrostereum purpureum* (Chontrol Paste^{®}) + TX, *Colletotrichum gloeosporioides* (Collego^{®}) + TX, Copper Octanoate (Cueva^{®}) + TX, Delta traps (Trapline d^{®}) + TX, Erwinia amylovora (Harpin) (ProAct^{®} + TX, Ni-HIBIT Gold CST^{®}) + TX, Ferri-phosphate (Ferramol^{®}) + TX, Funnel traps (Trapline y^{®}) + TX, Gallex^{®} + TX, Grower's Secret^{®} + TX, Homo-brassonolide + TX, Iron Phosphate (Lilly Miller Worry Free Ferramol Slug & Snail Bait^{®}) + TX, MCP hail trap (Trapline f^{®}) + TX, *Microctonus hyperodae* + TX, *Mycoleptodiscus terrestris* (Des-X^{®}) + TX, BioGain^{®} + TX, Aminomite^{®} + TX, Zenox^{®} + TX, Pheromone trap (Thripline ams^{®}) + TX, potassium bicarbonate (MilStop^{®}) + TX, potassium salts of fatty acids (Sanova^{®}) + TX, potassium silicate solution (Sil-Matrix^{®}) + TX, potassium iodide + potassiumthiocyanate (Enzicur^{®}) + TX, SuffOil-X^{®} + TX, Spider venom + TX, *Nosema locustae* (Semaspore Organic Grasshopper Control^{®}) + TX, Sticky traps (Trapline YF^{®} + TX, Rebell Amarillo@) + TX and Traps (Takitrapline y + b^{®}) + TX;
or a biologically active compound or agent selected from: Brofluthrinate + TX, Diflovidazine + TX, Flometoquin + TX, Fluhexafon + TX, Plutella xylostella Granulosis virus + TX, Cydia pomonella Granulosis virus + TX, Imicyafos + TX, Heliothis virescens Nucleopolyhedrovirus + TX, Heliothis punctigera Nucleopolyhedrovirus + TX, Helicoverpa zea Nucleopolyhedrovirus + TX, Spodoptera frugiperda Nucleopolyhedrovirus + TX, Plutella xylostella Nucleopolyhedrovirus + TX, p-cymene + TX, Pyflubumide + TX, Pyrafluprole + TX, QRD 420 + TX, QRD 452 + TX, QRD 460 + TX, Terpenoid blends + TX, Terpenoids + TX, Tetraniliprole + TX, and α-terpinene + TX;
or an active substance referenced by a code + TX, such as code AE 1887196 (BSC-BX60309) + TX, code NNI-0745 GR + TX, code IKI-3106 + TX, code JT-L001 + TX, code ZNQ-08056 + TX, code IPPA152201 + TX, code HNPC-A9908 (CAS: [660411-21-2]) + TX, code HNPC-A2005 (CAS: [860028-12-2]) + TX, code JS118 + TX, code ZJ0967 + TX, code ZJ2242 + TX, code JS7119 (CAS: [929545-74-4]) + TX, code SN-1172 + TX, code HNPC-A9835 + TX, code HNPC-A9955 + TX, code HNPC-A3061 + TX, code Chuanhua 89-1 + TX, code IPP-10 + TX, code ZJ3265 + TX, code JS9117 + TX, code ZJ3757 + TX, code ZJ4042 + TX, code ZJ4014 + TX, code ITM-121 + TX, code DPX-RAB55 (DKI-2301) + TX, code NA-89 + TX, code MIE-1209 + TX, code MCI-8007 + TX, code BCS-CL73507 + TX, code S-1871 + TX, code DPX-RDS63 + TX, Quinofumelin + TX, mefentrifluconazol + TX, fenpicoxamid + TX, fluindapyr + TX, inpyrfluxam + TX or indiflumetpyr + TX, isoflucypram + TX, pyrapropoyne + TX, florylpicoxamid + TX, metyltetraprole + TX, ipflufenoquin + TX, pyridachlometyl + TX or chlopyridiflu + TX, tetrachlorantraniliprole + TX, tetrachloraniliprole + TX, Tyclopyrazoflor + TX, flupyrimin + TX or pyrifluramide + TX, benzpyrimoxan + TX, Benzosufyl + TX or oxazosulfyl + TX, etpyrafen + TX, acynonapyr + TX or pyrinonafen + TX, oxotrione + TX, bixlozone + TX or
clofendizone + TX or dicloroxizone + TX, cyclopyranil + TX or pyrazocyclonil + TX or cyclopyrazonil + TX , alpha-bromadiolone + TX, code AKD-1193 + TX, Oxathiapiprolin + TX, Fluopyram + TX, Penflufen+ TX, Fluoxopyrosad+ TX, and Flupyradifurone + TX.

The references in brackets behind the active ingredients, e.g. [3878-19-1] refer to the Chemical Abstracts Registry number. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright © 1995-2004]; for example, the compound "acetoprole" is described under the internet address http://www.alanwood.net/pesticides/acetoprole.html.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/Chemical Abstracts name, a "chemical name", a "traditional name", a "compound name" or a "development code" is used or, if neither one of those designations nor a "common name" is used, an "alternative name" is employed. "CAS Reg. No" means the Chemical Abstracts Registry Number.

The active ingredient mixture of the compounds of formula I selected from the Tables A-1 to A-64 and Table P with active ingredients described above comprises a compound selected from the Tables A-1 to A-64 and Table P and an active ingredient as described above preferably in a mixing ratio of from 100:1 to 1:6000, especially from 50:1 to 1:50, more especially in a ratio of from 20:1 to 1:20, even more especially from 10:1 to 1:10, very especially from 5:1 and 1:5, special preference being given to a ratio of from 2:1 to 1:2, and a ratio of from 4:1 to 2:1 being likewise preferred, above all in a ratio of 1:1, or 5:1, or 5:2, or 5:3, or 5:4, or 4:1, or 4:2, or 4:3, or 3:1, or 3:2, or 2:1, or 1:5, or 2:5, or 3:5, or 4:5, or 1:4, or 2:4, or 3:4, or 1:3, or 2:3, or 1:2, or 1:600, or 1:300, or 1:150, or 1:35, or 2:35, or 4:35, or 1:75, or 2:75, or 4:75, or 1:6000, or 1:3000, or 1:1500, or 1:350, or 2:350, or 4:350, or 1:750, or 2:750, or 4:750. Those mixing ratios are by weight.

The mixtures as described above can be used in a method for controlling pests, which comprises applying a composition comprising a mixture as described above to the pests or their environment, with the exception of a method for treatment of the human or animal body by surgery or therapy and diagnostic methods practised on the human or animal body.

The mixtures comprising a compound of formula I selected from the Tables A-1 to A-64 and Table P and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compounds of formula I selected from the Tables A-1 to A-64 and Table P and the active ingredients as described above is not essential for working the present invention.

The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds I for the preparation of these compositions are also a subject of the invention.

The application methods for the compositions, that is the methods of controlling pests of the abovementioned type, such as spraying, atomizing, dusting, brushing on, dressing, scattering or pouring - which are to be selected to suit the intended aims of the prevailing circumstances - and the use of the compositions for controlling pests of the abovementioned type are other subjects of the invention. Typical rates of concentration are between 0.1 and 1000 ppm, preferably between 0.1 and 500 ppm, of active ingredient. The rate of application per hectare is generally 1 to 2000 g of active ingredient per hectare, in particular 10 to 1000 g/ha, preferably 10 to 600 g/ha.

A preferred method of application in the field of crop protection is application to the foliage of the plants (foliar application), it being possible to select frequency and rate of application to match the danger of infestation with the pest in question. Alternatively, the active ingredient can reach the plants via the root system (systemic action), by drenching the locus of the plants with a liquid composition or by incorporating the active ingredient in solid form into the locus of the plants, for example into the soil, for example in the form of granules (soil application). In the case of paddy rice crops, such granules can be metered into the flooded paddy-field.

The compounds of formula (I) of the invention and compositions thereof are also be suitable for the protection of plant propagation material, for example seeds, such as fruit, tubers or kernels, or nursery plants, against pests of the abovementioned type. The propagation material can be treated with the compound prior to planting, for example seed can be treated prior to sowing. Alternatively, the compound can be applied to seed kernels (coating), either by soaking the kernels in a liquid composition or by applying a layer of a solid composition. It is also possible to apply the compositions when the propagation material is planted to the site of application, for example into the seed furrow during drilling. These treatment methods for plant propagation material and the plant propagation material thus treated are further subjects of the invention. Typical treatment rates would depend on the plant and pest/fungi to be controlled and are generally between 1 to 200 grams per 100 kg of seeds, preferably between 5 to 150 grams per 100 kg of seeds, such as between 10 to 100 grams per 100 kg of seeds.

The term seed embraces seeds and plant propagules of all kinds including but not limited to true seeds, seed pieces, suckers, corns, bulbs, fruit, tubers, grains, rhizomes, cuttings, cut shoots and the like and means in a preferred embodiment true seeds.

The present invention also comprises seeds coated or treated with or containing a compound of formula (I). The term "coated or treated with and/or containing" generally signifies that the active ingredient is for the most part on the surface of the seed at the time of application, although a greater or lesser part of the ingredient may penetrate into the seed material, depending on the method of application. When the said seed product is (re)planted, it may absorb the active ingredient. In an embodiment, the present invention makes available a plant propagation material adhered thereto with a compound of formula (I). Further, it is hereby made available, a composition comprising a plant propagation material treated with a compound of formula (I).

Seed treatment comprises all suitable seed treatment techniques known in the art, such as seed dressing, seed coating, seed dusting, seed soaking and seed pelleting. The seed treatment application of the compound formula (I) can be carried out by any known methods, such as spraying or by dusting the seeds before sowing or during the sowing/planting of the seeds.

### Biological Examples:

The Examples which follow serve to illustrate the invention. Certain compounds of the invention can be distinguished from known compounds by virtue of greater efficacy at low application rates, which can be verified by the person skilled in the art using the experimental procedures outlined in the Examples, using lower application rates if necessary, for example 50 ppm, 12.5 ppm, 6 ppm, 3 ppm, 1.5 ppm, 0.8 ppm or 0.2 ppm.

### Example B1: Diabrotica balteata (Corn root worm)

Maize sprouts placed onto an agar layer in 24-well microtiter plates were treated with aqueous test solutions prepared from 10'000 ppm DMSO stock solutions by spraying. After drying, the plates were infested with L2 larvae (6 to 10 per well). The samples were assessed for mortality and growth inhibition in comparison to untreated samples 4 days after infestation.

The following compounds gave an effect of at least 80% in at least one of the two categories (mortality or growth inhibition) at an application rate of 200 ppm:
P16, P24, P25, P26, P29, P30, P31, P32, P33, P34, P35, P40, P41, P42, P43, P44, P45, P46, P49, P50, P51, P52, P54, P55, P56, P58, P59, P60, P64, P67, P68, P69, P72, P73, P74, P75, P76, P77, P78, P79, P80, P81, P82, P83, P84, P85, P86, P87, P88, P89

### Example B2: Euschistus heros (Neotropical Brown Stink Bug)

Soybean leaves on agar in 24-well microtiter plates were sprayed with aqueous test solutions prepared from 10'000 ppm DMSO stock solutions. After drying the leaves were infested with N2 nymphs. The samples were assessed for mortality and growth inhibition in comparison to untreated samples 5 days after infestation.

The following compounds gave an effect of at least 80% in at least one of the two categories (mortality or growth inhibition) at an application rate of 200 ppm:
P24, P25, P26, P27, P29, P30, P32, P34, P35, P41, P42, P44, P45, P46, P49, P52, P53, P54, P55, P58, P59, P61, P64, P72, P73, P77, P78, P79, P80, P81, P82, P83, P84, P85, P86, P87, P88, P89

### Example B3: Frankliniella occidentalis (Western flower thrips):Feeding/contact activity

Sunflower leaf discs were placed on agar in 24-well microtiter plates and sprayed with aqueous test solutions prepared from 10'000 DMSO stock solutions. After drying the leaf discs were infested with a Frankliniella population of mixed ages. The samples were assessed for mortality 7 days after infestation.

The following compounds resulted in at least 80% mortality at an application rate of 200 ppm: P45, P64, P72, P78, P83, P85

### Example B4: Chilo suppressalis (Striped rice stemborer)

24-well microtiter plates with artificial diet were treated with aqueous test solutions prepared from 10'000 ppm DMSO stock solutions by pipetting. After drying, the plates were infested with L2 larvae (6-8 per well). The samples were assessed for mortality, anti-feeding effect, and growth inhibition in comparison to untreated samples 6 days after infestation. Control of Chilo suppressalis by a test sample is given when at least one of the categories (i.e. mortality, anti-feedant effect, and growth inhibition) is higher than the untreated sample.

The following compounds resulted in at least 80% control at an application rate of 200 ppm:
P25, P26, P32, P42, P43, P49, P50, P58, P59, P61, P64, P66, P67, P68, P69, P72, P73, P74, P75, P76, P77, P78, P79, P80, P81, P82, P83, P84, P85, P86, P87, P88, P89

### Example B5: Plutella xylostella (Diamond back moth)

24-well microtiter plates with artificial diet were treated with aqueous test solutions prepared from 10'000 ppm DMSO stock solutions by pipetting. After drying, Plutella eggs were pipetted through a plastic stencil onto a gel blotting paper and the plate was closed with it. The samples were assessed for mortality and growth inhibition in comparison to untreated samples 8 days after infestation.

The following compounds gave an effect of at least 80% in at least one of the two categories (mortality or growth inhibition) at an application rate of 200 ppm:
P3, P5, P8, P9, P15, P23, P24, P25, P26, P27, P29, P30, P31, P32, P33, P34, P35, P36, P38, P40, P41, P42, P43, P44, P45, P46, P47, P49, P50, P51, P52, P54, P55, P56, P58, P59, P60, P61, P63, P64, P67, P68, P69, P70, P72, P73, P74, P75, P76, P77, P78, P79, P80, P81, P82, P83, P84, P85, P86, P87, P88, P89

### Example B6: Mvzus persicae (Green peach aphid):Feedinq/Contact activity

Sunflower leaf discs were placed onto agar in a 24-well microtiter plate and sprayed with aqueous test solutions prepared from 10'000 ppm DMSO stock solutions. After drying, the leaf discs were infested with an aphid population of mixed ages. The samples were assessed for mortality 6 days after infestation.

The following compounds resulted in at least 80% mortality at an application rate of 200 ppm: P24, P26, P34, P42, P46, P50, P61, P64, P72, P73, P82, P83, P85, P87, P89

### Example B7: Myzus persicae (Green peach aphid). Systemic activity

Roots of pea seedlings infested with an aphid population of mixed ages were placed directly into aqueous test solutions prepared from 10'000 DMSO stock solutions. The samples were assessed for mortality 6 days after placing seedlings into test solutions.

The following compounds resulted in at least 80% mortality at a test rate of 24 ppm: P26, P34, P35, P45, P46, P64, P82, P83, P84, P89

### Example B8: Myzus persicae (Green peach aphid). Intrinsic activity

Test compounds prepared from 10'000 ppm DMSO stock solutions were applied by pipette into 24-well microtiter plates and mixed with sucrose solution. The plates were closed with a stretched Parafilm. A plastic stencil with 24 holes was placed onto the plate and infested pea seedlings were placed directly on the Parafilm. The infested plate was closed with a gel blotting paper and another plastic stencil and then turned upside down. The samples were assessed for mortality 5 days after infestation.

The following compounds resulted in at least 80% mortality at a test rate of 12 ppm: P21, P23, P24, P25, P26, P29, P30, P34, P35, P42, P43, P45, P46, P49, P50, P52, P54, P55, P58, P59, P61, P64, P72, P73, P75, P77, P78, P82, P83, P84, P85, P86, P87, P88, P89

### Example B9: Spodoptera littoralis (Egyptian cotton leaf worm)

Cotton leaf discs were placed onto agar in 24-well microtiter plates and sprayed with aqueous test solutions prepared from 10'000 ppm DMSO stock solutions. After drying the leaf discs were infested with five L1 larvae. The samples were assessed for mortality, anti-feeding effect, and growth inhibition in comparison to untreated samples 3 days after infestation. Control of Spodoptera littoralis by a test sample is given when at least one of the three categories (i.e. mortality, anti-feedant effect, and growth inhibition) is higher than the untreated sample.

The following compounds resulted in at least 80% control at an application rate of 200 ppm: P24, P25, P26, P32, P35, P36, P40, P41, P42, P43, P44, P45, P46, P49, P50, P51, P52, P54, P56, P58, P59, P61, P64, P68, P69, P70, P72, P73, P74, P75, P76, P77, P78, P79, P80, P81, P82, P83, P84, P85, P86, P87, P88, P89

### Example B10: Spodoptera littoralis (Egyptian cotton leaf worm)

Test compounds were applied by pipette from 10'000 ppm DMSO stock solutions into 24-well plates and mixed with agar. Lettuce seeds were placed onto the agar and the multi well plate was closed by another plate which contained also agar. After 7 days the compound was absorbed by the roots and the lettuce grew into the lid plate. The lettuce leaves were then cut off into the lid plate. Spodoptera eggs were pipetted through a plastic stencil onto a humid gel blotting paper and the lid plate was closed with it. The samples were assessed for mortality, anti-feedant effect and growth inhibition in comparison to untreated samples 6 days after infestation.

The following compounds gave an effect of at least 80% in at least one of the three categories (mortality, anti-feeding, or growth inhibition) at a test rate of 12.5 ppm: P25, P26, P32, P42, P43, P45, P49, P50, P54, P55, P58, P59, P85, P87, P89

### Example B11: Tetranychus urticae (Two-spotted spider mite):Feeding/contact activity

Bean leaf discs on agar in 24-well microtiter plates were sprayed with aqueous test solutions prepared from 10'000 ppm DMSO stock solutions. After drying the leaf discs were infested with a mite population of mixed ages. The samples were assessed for mortality on mixed population (mobile stages) 8 days after infestation.

The following compounds resulted in at least 80% mortality at an application rate of 200 ppm:
P27, P34, P62, P79, P83

### Example B12: Thrips tabaci (Onion thrips) Feeding/Contact activity

Sunflower leaf discs were placed on agar in 24-well microtiter plates and sprayed with aqueous test solutions prepared from 10'000 ppm DMSO stock solutions. After drying the leaf discs were infested with a thrips population of mixed ages. The samples were assessed for mortality 6 days after infestation.

The following compounds resulted in at least 80% mortality at an application rate of 200 ppm: P25, P26, P29, P30, P32, P34, P35, P42, P46, P49, P50, P52, P89

### Example B13: Activity against Myzus persicae (Green Peach Aphid):

Test compounds prepared from 10'000 ppm DMSO stock solutions were applied by a liquid handling robot into 96-well microtiter plates and mixed with a sucrose solution. Parafilm was stretched over the 96-well microtiter plate and a plastic stencil with 96 holes was placed onto the plate. Aphids were sieved into the wells directly onto the Parafilm. The infested plates were closed with a gel blotting card and a second plastic stencil and then turned upside down. The samples were assessed for mortality 5 days after infestation.

The following compounds resulted in at least 80% mortality at an application rate of 50 ppm: P11, P15, P17, P18, P20, P21, P23, P24, P25, P26, P27, P29, P30, P31, P32, P33, P34, P35, P40, P42, P43, P44, P45, P46, P47, P49, P50, P51, P52, P54, P55, P58, P59, P60, P64, P67, P68, P69, P83, P85, P87, P88, P89

### Example B14: Activity against Plutella xylostella (Diamondback Moth):

96-well microtiter plates containing artificial diet were treated with aqueous test solutions, prepared from 10'000 ppm DMSO stock solutions, by a liquid handling robot. After drying, eggs (~30 per well) were infested onto a netted lid which was suspended above the diet. The eggs hatch and L1 larvae move down to the diet. The samples were assessed for mortality 9 days after infestation.

The following compounds gave an effect of at least 80% average mortality at an application rate of 500 ppm:
P1, P2, P3, P4, P5, P9, P15, P20, P21, P23, P24, P25, P26, P27, P28, P29, P30, P31, P32, P33, P34, P35, P36, P38, P40, P41, P42, P43, P44, P45, P46, P47, P48, P49, P50, P51, P52, P54, P55, P58, P59, P60, P70, P71, P89

## Claims

1. A compound of formula I, 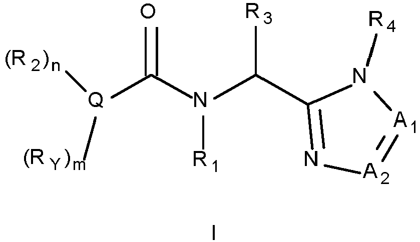
wherein A₁ is N, A₂ is CR₅ and R₅ is H, methyl, or (CH₂CH₂O)₂CH-;
R₁ is cyclopropyl-CH₂-, CH≡CCH₂-, H, or methyl;
Q is phenyl, pyridine, pyrimidine, pyrazine or pyridazine, wherein the phenyl, pyridine, pyrimidine, pyrazine or pyridazine is substituted by R₂ where n is 1 or 2, and, independent of the type of ring, is optionally substituted with R_{Y}, where m can be 0, 1 or 2;
R₂ is C₃-C₆cycloalkyl, phenyl or heteroaryl, each of which, independent of each other, is optionally substituted with one to three substituents independently selected from R_{x;} OR₆; piperidin-2-one-1-yl optionally substituted with one to two substituents independently selected from Rₓ; pyridin-2-one-1-yl optionally substituted with one to two substituents independently selected from Rₓ; azetidin-1-yl optionally substituted with one to two substituents independently selected from Rₓ; pyrrolidin-1-yl optionally substituted with one to two substituents independently selected from Rₓ; C₃-C₆cycloalkylC₁-C₄alkyl optionally substituted with one to two substituents independently selected from Rₓ; C₃-C₆cycloalkylC₁-C₃alkoxy optionally substituted with one to two substituents independently selected from Rₓ; C₁-C₅cyanoalkyl; C₁-C₅cyanoalkoxy; C₁-C₄alkylsulfonyl optionally substituted with one to two substituents independently selected from R_{x;} or C₁-C₄alkylsulfinyl optionally substituted with one to two substituents independently selected from Rₓ;
R_{Y} is selected from C₁-C₃alkyl, C₁-C₃haloalkyl, C₁-C₃haloalkylthio, C₁-C₃alkoxy, C₁-C₃haloalkoxy, halo, NO₂, SF₅, CN, C(O)NH₂, C(O)OH and C(S)NH₂;
R₃ is methyl;
R₄ is 2-pyridine or 2-pyrimidine, each optionally substituted with C₁-C₃alkoxy or halogen;
R₆ is phenyl, benzyl, heteroaryl, or C₃-C₆ cycloalkyl, each of which, independent of each other, is optionally substituted with one to three substituents independently selected from Rₓ; and
Rₓ is independently selected from halogen, C₁-C₃alkyl, C₁-C₃haloalkyl, C₁-C₃alkoxy, C₁-C₃haloalkoxy, NO₂, SF₅, CN, CONH₂, C(S)NH₂, C₁-C₄haloalkylsulfanyl, C₁-C₄haloalkylsulfinyl, C₁-C₄haloalkylsulfonyl, C₁-C₄alkylsulfanyl, C₁-C₄alkylsulfinyl and C₁-C₄alkylsulfonyl;
or agrochemically acceptable salts, stereoisomers, enantiomers, tautomers and N-oxides of the compounds of formula I.

2. The compound according to claim 1, wherein R₅ is H.

3. The compound according to claim 1 or claim 2, wherein R₄ is 2-pyrimidine.

4. The compound according to any one of claims 1 to 3, wherein Q is selected from phenyl, pyridine or pyrimidine, wherein the phenyl, pyridine or pyrimidine is substituted by R₂ where n is 1 or 2, and, independent of the type of ring, is optionally substituted with R_{Y}, where m can be 0, 1 or 2.

5. The compound according to any one of claims 1 to 4, wherein Q is selected from phenyl, wherein the phenyl, pyridine or pyrimidine is substituted by R₂ where n is 1 or 2, and, independent of the type of ring, is optionally substituted with R_{Y}, where m can be 0, 1 or 2;

6. The compound according to any one of claims 1 to 5, wherein R₂ is OR₆, wherein R₆ is selected from phenyl, benzyl, C₃-C₄ cycloalkyl, each of which, independent of each other, is optionally substituted with one substituent Rₓ.

7. The compound according to any one of claims 1 to 6, wherein Rₓ, independent of the different R₂ and R₆ groups, is independently selected from halogen, C₁-C₃alkyl, C₁-C₃haloalkyl, C₁-C₃alkoxy, C₁-C₃haloalkoxy, NO₂, SF₅, CN, CONH₂, C(S)NH₂.

8. The compound according to any one of claims 1 to 7, wherein R_{Y} is C₁-C₃alkyl, C₁-C₃haloalkyl, C₁-C₃alkoxy, C₁-C₃haloalkoxy, halo, NO₂, SF₅, and CN.

9. The compound according to any one of claims 1 to 8, wherein R₂ is selected from C₃-C₆cycloalkyl, phenyl, and pyrazolyl, and each of which, independent of each other, is optionally substituted with one to three substituents independently selected from R_{x;} OR₆; piperidin-2-one-1-yl optionally substituted with one to two substituents independently selected from Rₓ; pyridin-2-one-1-yl optionally substituted with one to two substituents independently selected from Rₓ; azetidin-1-yl optionally substituted with one to two substituents independently selected from Rₓ; C₃-C₆cycloalkylC₁-C₄alkyl optionally substituted with one to two substituents independently selected from Rₓ; C₃-C₆cycloalkylC₁-C₃alkoxy optionally substituted with one to two substituents independently selected from Rₓ; C₁-C₅cyanoalkyl; or C₁-C₄alkylsulfonyl optionally substituted with one to two substituents independently selected from Rₓ; and R_{Y} is C₁-C₃alkyl, C₁-C₃haloalkyl, C₁-C₃haloalkylthio, C₁-C₃alkoxy, C₁-C₃haloalkoxy, or halo; wherein R₆ is phenyl, benzyl, C₃-C₆ cycloalkyl, each of which, independent of each other, is optionally substituted with one to three substituents independently selected from Rₓ; and wherein Rₓ, independent of the different R₂ and R₆ groups, is independently selected from halogen, C₁-C₃alkyl, C₁-C₃haloalkyl, C₁-C₃alkoxy, C₁-C₃haloalkoxy, and CN.

10. A composition comprising a compound as defined in any one of claims 1 to 9, one or more auxiliaries and diluent, and optionally one more other active ingredient.

11. A method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound as defined as defined in any one of claims 1 to 9, or a composition as defined in claim 10, provided the method excludes a method for treatment of the human or animal body by surgery or therapy and diagnostic methods practised on the human or animal body.

12. A method for the protection of plant propagation material from the attack by insects, acarines, nematodes or molluscs, which comprises treating the propagation material or the site, where the propagation material is planted, with an effective amount of a compound as defined in any one of claims 1 to 9, or a composition as defined in claim 10.

13. A plant propagation material, such as a seed, comprising, or adhered thereto, a compound as defined in any one of claims 1 to 9, or a composition as defined in claim 10.

14. A compound as defined in any one of claims 1 to 9, or a composition as defined claim 10, for use in controlling parasites in or on an animal in need thereof.

## Patentansprüche

1. Verbindung der Formel I, 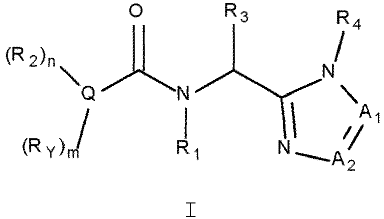
wobei A₁ für N steht, A₂ für CR₅ steht und R₅ für H, Methyl oder (CH₂CH₂O)₂CH- steht;
R₁ für Cyclopropyl-CH₂-, CH≡CCH₂-, H oder Methyl steht;
Q für Phenyl, Pyridin, Pyrimidin, Pyrazin oder Pyridazin steht, wobei das Phenyl, Pyridin, Pyrimidin, Pyrazin oder Pyridazin durch R₂ substituiert ist, wenn n für 1 oder 2 steht, und unabhängig vom Ringtyp gegebenenfalls durch R_{Y} substituiert ist, wobei m für 0, 1 oder 2 stehen kann;
R₂ für C₃-C₆-Cycloalkyl, Phenyl oder Heteroaryl, wobei jede dieser Gruppen unabhängig voneinander gegebenenfalls durch einen bis drei Substituenten, die unabhängig aus Rₓ ausgewählt sind, substituiert ist; OR₆; Piperidin-2-on-1-yl, das gegebenenfalls durch einen bis zwei Substituenten, die unabhängig aus Rₓ ausgewählt sind, substituiert ist; Pyridin-2-on-1-yl, das gegebenenfalls durch einen bis zwei Substituenten, die unabhängig aus Rₓ ausgewählt sind, substituiert ist; Azetidin-1-yl, das gegebenenfalls durch einen bis zwei Substituenten, die unabhängig aus Rₓ ausgewählt sind, substituiert ist; Pyrrolidin-1-yl, das gegebenenfalls durch einen bis zwei Substituenten, die unabhängig aus Rₓ ausgewählt sind, substituiert ist; C₃-C₆-Cycloalkyl-C₁-C₄-alkyl, das gegebenenfalls durch einen bis zwei Substituenten, die unabhängig aus Rₓ ausgewählt sind, substituiert ist; C₃-C₆-Cycloalkyl-C₁-C₃-alkoxy, das gegebenenfalls durch einen bis zwei Substituenten, die unabhängig aus Rₓ ausgewählt sind, substituiert ist; C₁-C₅-Cyanoalkyl; C₁-C₅-Cyanoalkoxy; C₁-C₄-Alkylsulfonyl, das gegebenenfalls durch einen bis zwei Substituenten, die unabhängig aus Rₓ ausgewählt sind, substituiert ist; oder C₁-C₄-Alkylsulfinyl, das gegebenenfalls durch einen bis zwei Substituenten, die unabhängig aus Rₓ ausgewählt sind, substituiert ist; steht;
R_{Y} aus C₁-C₃-Alkyl, C₁-C₃-Halogenalkyl, C₁-C₃-Halogenalkylthio, C₁-C₃-Alkoxy, C₁-C₃-Halogenalkoxy, Halogen, NO₂, SF₅, CN, C(O)NH₂, C(O)OH und C(S)NH₂ ausgewählt ist;
R₃ für Methyl steht;
R₄ für 2-Pyridin oder 2-Pyrimidin steht, wobei jede dieser Gruppen gegebenenfalls durch C₁-C₃-Alkoxy oder Halogen substituiert ist;
R₆ für Phenyl, Benzyl, Heteroaryl oder C₃-C₆-Cycloalkyl steht, wobei jede dieser Gruppen unabhängig voneinander gegebenenfalls durch einen bis drei Substituenten, die unabhängig aus Rₓ ausgewählt sind, substituiert ist; und
Rₓ unabhängig aus Halogen, C₁-C₃-Alkyl, C₁-C₃-Halogenalkyl, C₁-C₃-Alkoxy, C₁-C₃-Halogenalkoxy, NO₂, SF₅, CN, CONH₂, C(S)NH₂, C₁-C₄-Halogenalkylsulfanyl, C₁-C₄-Halogenalkylsulfinyl, C₁-C₄-Halogenalkylsulfonyl, C₁-C₄-Alkylsulfanyl, C₁-C₄-Alkylsulfinyl und C₁-C₄-Alkylsulfonyl ausgewählt ist;
oder agrochemisch unbedenkliche Salze, Stereoisomere, Enantiomere, Tautomere und N-Oxide der Verbindungen der Formel I.

2. Verbindung nach Anspruch 1, wobei R₅ für H steht.

3. Verbindung nach Anspruch 1 oder Anspruch 2, wobei R₄ für 2-Pyrimidin steht.

4. Verbindung nach einem der Ansprüche 1 bis 3, wobei Q aus Phenyl, Pyridin oder Pyrimidin ausgewählt ist, wobei das Phenyl, Pyridin oder Pyrimidin durch R₂ substituiert ist, wenn n für 1 oder 2 steht, und unabhängig vom Ringtyp gegebenenfalls durch R_{Y} substituiert ist, wobei m für 0, 1 oder 2 stehen kann.

5. Verbindung nach einem der Ansprüche 1 bis 4, wobei Q aus Phenyl ausgewählt ist, wobei das Phenyl, Pyridin oder Pyrimidin durch R₂ substituiert ist, wenn n für 1 oder 2 steht, und unabhängig vom Ringtyp gegebenenfalls durch R_{Y} substituiert ist, wobei m für 0, 1 oder 2 stehen kann.

6. Verbindung nach einem der Ansprüche 1 bis 5, wobei R₂ für OR₆ steht, wobei R₆ aus Phenyl, Benzyl und C₃-C₄-Cycloalkyl ausgewählt ist, wobei jede dieser Gruppen unabhängig voneinander gegebenenfalls durch einen Substituenten Rₓ substituiert ist.

7. Verbindung nach einem der Ansprüche 1 bis 6, wobei Rₓ unabhängig von den verschiedenen R₂- und R₆-Gruppen unabhängig aus Halogen, C₁-C₃-Alkyl, C₁-C₃-Halogenalkyl, C₁-C₃-Alkoxy, C₁-C₃-Halogenalkoxy, NO₂, SF₅, CN, CONH₂ und C(S)NH₂ ausgewählt ist.

8. Verbindung nach einem der Ansprüche 1 bis 7, wobei R_{Y} für C₁-C₃-Alkyl, C₁-C₃-Halogenalkyl, C₁-C₃-Alkoxy, C₁-C₃-Halogenalkoxy, Halogen, NO₂, SF₅ und CN steht.

9. Verbindung nach einem der Ansprüche 1 bis 8, wobei R₂ aus C₃-C₆-Cycloalkyl, Phenyl oder Pyrazolyl, wobei jede dieser Gruppen unabhängig voneinander gegebenenfalls durch einen bis drei Substituenten, die unabhängig aus Rₓ ausgewählt sind, substituiert ist; OR₆; Piperidin-2-on-1-yl, das gegebenenfalls durch einen bis zwei Substituenten, die unabhängig aus Rₓ ausgewählt sind, substituiert ist; Pyridin-2-on-1-yl, das gegebenenfalls durch einen bis zwei Substituenten, die unabhängig aus Rₓ ausgewählt sind, substituiert ist; Azetidin-1-yl, das gegebenenfalls durch einen bis zwei Substituenten, die unabhängig aus Rₓ ausgewählt sind, substituiert ist; C₃-C₆-Cycloalkyl-C₁-C₄-alkyl, das gegebenenfalls durch einen bis zwei Substituenten, die unabhängig aus Rₓ ausgewählt sind, substituiert ist; C₃-C₆-Cycloalkyl-C₁-C₃-alkoxy, das gegebenenfalls durch einen bis zwei Substituenten, die unabhängig aus Rₓ ausgewählt sind, substituiert ist; C₁-C₅-Cyanoalkyl oder C₁-C₄-Alkylsulfonyl, das gegebenenfalls durch einen bis zwei Substituenten, die unabhängig aus Rₓ ausgewählt sind, substituiert ist; ausgewählt ist und R_{Y} für C₁-C₃-Alkyl, C₁-C₃-Halogenalkyl, C₁-C₃-Halogenalkylthio, C₁-C₃-Alkoxy, C₁-C₃-Halogenalkoxy oder Halogen steht; wobei R₆ für Phenyl, Benzyl oder C₃-C₆-Cycloalkyl steht, wobei jede dieser Gruppen unabhängig voneinander gegebenenfalls durch einen bis drei Substituenten, die unabhängig aus Rₓ ausgewählt sind, substituiert ist; und wobei Rₓ unabhängig von den verschiedenen R₂- und R₆-Gruppen unabhängig aus Halogen, C₁-C₃-Alkyl, C₁-C₃-Halogenalkyl, C₁-C₃-Alkoxy, C₁-C₃-Halogenalkoxy und CN ausgewählt ist.

10. Zusammensetzung, umfassend eine Verbindung gemäß einem der Ansprüche 1 bis 9, einen oder mehrere Hilfsstoffe und Verdünnungsmittel und gegebenenfalls einen oder mehrere weitere Wirkstoffe.

11. Verfahren zur Bekämpfung von Insekten, Acarina, Nematoden oder Mollusken, bei dem man eine insektizid, akarizid, nematizid bzw. molluskizid wirksame Menge einer Verbindung gemäß einem der Ansprüche 1 bis 9 oder einer Zusammensetzung gemäß Anspruch 10 auf einen Schädling, einen Standort eines Schädlings oder eine Pflanze, die gegenüber Befall durch einen Schädling empfindlich ist, ausbringt, mit der Maßgabe, dass das Verfahren ein Verfahren zur chirurgischen oder therapeutischen Behandlung des menschlichen oder tierischen Körpers und Diagnostizierverfahren, die am menschlichen oder tierischen Körper vorgenommen werden, ausschließt.

12. Verfahren zum Schutz von Pflanzenfortpflanzungsmaterial vor Befall durch Insekten, Acarina, Nematoden oder Mollusken, bei dem man das Fortpflanzungsmaterial oder den Ort, an dem das Fortpflanzungsmaterial angepflanzt ist, mit einer wirksamen Menge einer Verbindung gemäß einem der Ansprüche 1 bis 9 oder einer Zusammensetzung gemäß Anspruch 10 behandelt.

13. Pflanzenfortpflanzungsmaterial, wie ein Samen, der eine Verbindung gemäß einem der Ansprüche 1 bis 9 oder eine Zusammensetzung gemäß Anspruch 10 umfasst oder an dem eine Verbindung gemäß einem der Ansprüche 1 bis 9 oder eine Zusammensetzung gemäß Anspruch 10 haftet.

14. Verbindung gemäß einem der Ansprüche 1 bis 9 oder Zusammensetzung gemäß Anspruch 10 zur Verwendung bei der Bekämpfung von Parasiten in oder auf einem Tier, bei dem diesbezüglicher Bedarf besteht.

## Revendications

1. Composé de formule 1, 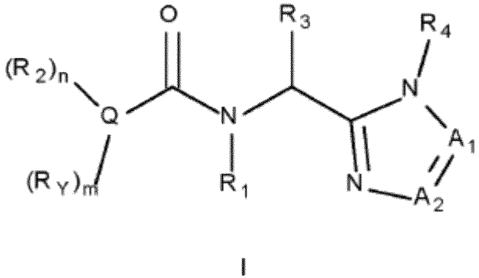
A₁ étant N, A₂ étant CR₅ et R₅ étant H, méthyle ou (CH₂CH₂O)₂CH- ;
R₁ étant cyclopropyl-CH₂-, CH≡CCH₂-, H, ou méthyle ; Q étant phényle, pyridine, pyrimidine, pyrazine ou pyridazine, le phényle, la pyridine, la pyrimidine, la pyrazine ou la pyridazine étant substitué(e) par R₂ où n est 1 ou 2, et, indépendamment du type de cycle, étant éventuellement substitu(é) par R_{Y}, où m peut être 0, 1 ou 2 ;
R₂ étant cycloalkyle en C₃ à C₆, phényle ou hétéroaryle, chacun desquels, indépendamment les uns des autres, étant éventuellement substitué par un à trois substituants indépendamment choisis parmi Rₓ; OR₆; pipéridin-2-on-1-yle éventuellement substitué par un à deux substituants indépendamment choisis parmi Rₓ ; pyridin-2-on-1-yle éventuellement substitué par un à deux substituants indépendamment choisis parmi Rₓ; azétidin-1-yle éventuellement substitué par un à deux substituants indépendamment choisis parmi Rₓ ; pyrrolidin-1-yle éventuellement substitué par un à deux substituants indépendamment choisis parmi Rₓ ; cycloalkyle en C₃ à C₆-alkyle en C₁ à C₄ éventuellement substitué par un à deux substituants indépendamment choisis parmi Rₓ; cycloalkyle en C₃ à C₆-alcoxy en C₁ à C₃ éventuellement substitué par un à deux substituants indépendamment choisis parmi Rₓ ; cyanoalkyle en C₁ à C₅; cyanoalcoxy en C₁ à C₅; alkylsulfonyle en C₁ à C₄ éventuellement substitué par un à deux substituants indépendamment choisis parmi Rₓ; alkylsulfinyle en C₁ à C₄ éventuellement substitué par un à deux substituants indépendamment choisis parmi Rₓ ;
R_{Y} étant choisi parmi alkyle en C₁ à C₃, halogénoalkyle en C₁ à C₃, halogénoalkylthio en C₁ à C₃, alcoxy en C₁ à C₃, halogénoalcoxy en C₁ à C₃, halogène, NO₂, SF₅, CN, C(O)NH₂, C(O)OH et C(S)NH₂ ; R₃ étant méthyle ;
R₄ étant 2-pyridine ou 2-pyrimidine, chacun éventuellement substitué par alcoxy en C₁ à C₃ ou halogène ;
R₆ étant phényle, benzyle, hétéroaryle, ou cycloalkyle en C₃ à C₆, chacun desquels, indépendamment les uns des autres, étant éventuellement substitué par un à trois substituants indépendamment choisis parmi Rₓ ; et
Rₓ étant indépendamment choisi parmi halogène, alkyle en C₁ à C₃, halogénoalkyle en C₁ à C₃, alcoxy en C₁ à C₃, halogénoalcoxy en C₁ à C₃, NO₂, SF₅, CN, CONH₂, C(S)NH₂, halogénoalkylsulfanyle en C₁ à C₄, halogénoalkylsulfinyle en C₁ à C₄, halogénoalkylsulfonyle en C₁ à C₄, alkylsulfanyle en C₁ à C₄, alkylsulfinyle en C₁ à C₄ et alkylsulfonyle en C₁ à C₄ ;
ou sels, stéréoisomères, énantiomères, formes tautomères et N-oxydes acceptables sur le plan agrochimique correspondant des composés de formule I.

2. Composé selon la revendication 1, R₅ étant H.

3. Composé selon la revendication 1 ou la revendication 2, R₄ étant 2-pyrimidine.

4. Composé selon l'une quelconque des revendications 1 à 3, Q étant choisi parmi phényle, pyridine, ou pyrimidine, le phényle, la pyridine, ou la pyrimidine, étant substitué(e) par R₂ où n est 1 ou 2, et, indépendamment du type de cycle, étant éventuellement substitué(e) par R_{Y}, où m peut être 0, 1 ou 2.

5. Composé selon l'une quelconque des revendications 1 à 4, Q étant choisi parmi phényle, le phényle, la pyridine, ou la pyrimidine, étant substitué(e) par R₂ où n est 1 ou 2, et, indépendamment du type de cycle, étant éventuellement substitué(e) par R_{Y}, où m peut être 0, 1 ou 2.

6. Composé selon l'une quelconque des revendications 1 à 5, R₂ étant OR₆, R₆ étant choisi parmi phényle, benzyle, cycloalkyle en C₃ à C₄, chacun desquels, indépendamment les uns des autres, étant éventuellement substitué par un substituant Rₓ.

7. Composé selon l'une quelconque des revendications 1 à 6, Rₓ, indépendamment des différents groupes R₂ et R₆, étant indépendamment choisi parmi halogène, alkyle en C₁ à C₃, halogénoalkyle en C₁ à C₃, alcoxy en C₁ à C₃, halogénoalcoxy en C₁ à C₃, NO₂, SF₅, CN, CONH₂, C(S)NH₂.

8. Composé selon l'une quelconque des revendications 1 à 7, R_{Y} étant alkyle en C₁ à C₃, halogénoalkyle en C₁ à C₃, alcoxy en C₁ à C₃, halogénoalcoxy en C₁ à C₃, halogène, NO₂, SF₅, et CN.

9. Composé selon l'une quelconque des revendications 1 à 8, R₂ étant choisi parmi cycloalkyle en C₃ à C₆, phényle et pyrazolyle, et chacun desquels, indépendamment les uns des autres, étant éventuellement substitué par un à trois substituants indépendamment choisis parmi Rₓ; OR₆; pipéridin-2-on-1-yle éventuellement substitué par un à deux substituants indépendamment choisis parmi Rₓ; pyridin-2-on-1-yle éventuellement substitué par un à deux substituants indépendamment choisis parmi Rₓ ; azétidin-1-yle éventuellement substitué par un à deux substituants indépendamment choisis parmi Rₓ ; cycloalkyle en C₃ à C₆-alkyle en C₁ à C₄ éventuellement substitué par un à deux substituants indépendamment choisis parmi Rₓ; cycloalkyle en C₃ à C₆-alcoxy en C₁ à C₃ éventuellement substitué par un à deux substituants indépendamment choisis parmi Rₓ; cyanoalkyle en C₁ à C₅; ou alkylsulfonyle en C₁ à C₄ éventuellement substitué par un à deux substituants indépendamment choisis parmi Rₓ ; et R_{Y} étant alkyle en C₁ à C₃, halogénoalkyle en C₁ à C₃, halogénoalkylthio en C₁ à C₃, alcoxy en C₁ à C₃, halogénoalcoxy en C₁ à C₃, ou halogène ; R₆ étant phényle, benzyle, cycloalkyle en C₃ à C₆, chacun desquels, indépendamment les uns des autres, étant éventuellement substitué par un à trois substituants indépendamment choisis parmi Rₓ ; et Rₓ, indépendamment des différents groupes R₂ et R₆, étant indépendamment choisi parmi halogène, alkyle en C₁ à C₃, halogénoalkyle en C₁ à C₃, alcoxy en C₁ à C₃, halogénoalcoxy en C₁ à C₃, et CN.

10. Composition comprenant un composé tel que défini dans l'une quelconque des revendication 1 à 9, un ou plusieurs auxiliaires et diluants, et éventuellement un ou plusieurs autres ingrédients actifs.

11. Procédé de lutte contre, et de régulation, des insectes, des acariens, des nématodes ou des mollusques qui comprend l'application sur un organisme nuisible, sur un site d'un organisme nuisible, ou sur un végétal susceptible d'être attaqué par un organisme nuisible, d'une quantité efficace sur le plan insecticide, sur le plan acaricide, sur le plan nématicide ou sur le plan molluscicide d'un composé tel que défini dans l'une quelconque des revendications 1 à 9, ou d'une composition telle que définie dans la revendication 10, étant entendu que le procédé exclut un procédé pour le traitement du corps humain ou animal par chirurgie ou thérapie et des procédés de diagnostic pratiqués sur le corps humain ou animal.

12. Procédé pour la protection d'un matériel de propagation végétale contre l'attaque par des insectes, des acariens, des nématodes ou des mollusques, qui comprend le traitement du matériel de propagation ou du site où le matériel de propagation est planté, par une quantité efficace d'un composé tel que défini dans l'une quelconque des revendications 1 à 9, ou d'une composition telle que définie dans la revendication 10.

13. Matériel de propagation végétale, tel qu'une graine, comprenant, ou adhérant à celui-ci/celle-ci, un composé tel que défini dans l'une quelconque des revendications 1 à 9, ou une composition telle que définie dans la revendication 10.

14. Composé tel que défini dans l'une quelconque des revendications 1 à 9, ou composition telle que définie dans la revendication 10, pour une utilisation dans la lutte contre des parasites dans ou sur un animal qui en a besoin.
